(19) Europäisches Patentamt
European Patent Office
Office européen des brevets

(11) EP 4 603 480 A1

(12) EUROPEAN PATENT APPLICATION
published in accordance with Art. 153(4) EPC

(43) Date of publication:
**20.08.2025 Bulletin 2025/34**

(21) Application number: **23876779.2**

(22) Date of filing: **12.10.2023**

(51) International Patent Classification (IPC):
***C07D 209/60*** (2006.01) ***C07D 209/08*** (2006.01)
***C07D 401/06*** (2006.01) ***C07D 401/14*** (2006.01)
***C07D 471/04*** (2006.01) ***C09K 11/06*** (2006.01)
***A61K 49/06*** (2006.01)

(52) Cooperative Patent Classification (CPC):
**A61K 49/06; C07D 209/08; C07D 209/60; C07D 401/06; C07D 401/14; C07D 471/04; C09K 11/06**

(86) International application number:
**PCT/CN2023/124246**

(87) International publication number:
**WO 2024/078579 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.10.2022 CN 202211250126**
**28.09.2023 CN 202311284553**

(71) Applicants:
- **Jiangsu Yahong Meditech Co., Ltd.**
**Taizhou, Jiangsu 225316 (CN)**
- **Asieris Pharmaceuticals (Shanghai) Co., Ltd.**
**Pudong, Shanghai 201203 (CN)**

(72) Inventors:
- **WU, Maojiang**
**Shanghai 201203 (CN)**
- **WANG, Tielin**
**Shanghai 201203 (CN)**
- **ZHENG, Peipei**
**Shanghai 201203 (CN)**

(74) Representative: **Regimbeau**
**20, rue de Chazelles**
**75847 Paris Cedex 17 (FR)**

# (54) INDOLE PHTHALOCYANINE COMPOUND, PREPARATION METHOD THEREFOR, AND USE THEREOF IN TUMOR DIAGNOSIS IMAGING

(57) The present invention relates to an indole phthalocyanine compound, a preparation method therefor, and a use thereof in tumor diagnosis imaging. Specifically, the present invention relates to a compound having a structure represented by general formula (I) or a pharmaceutically acceptable salt thereof, and a use thereof as a contrast agent. By using the contrast agent of the present invention, near-infrared real-time imaging having high contrast and a clear and distinguishable imaging result can be implemented.

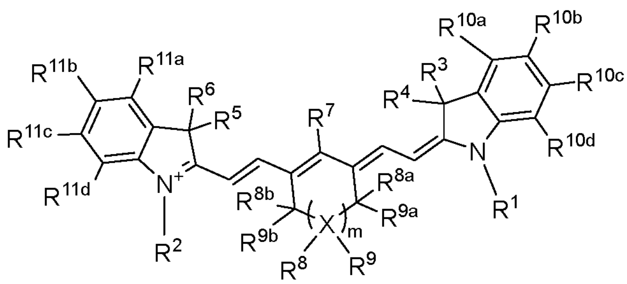

(I)

EP 4 603 480 A1

**Description**

## FIELD OF THE INVENTION

The present invention relates to indole phthalocyanine compounds, methods for preparing the same, and their uses in tumor diagnostic imaging.

## BACKGROUND OF THE INVENTION

Molecular imaging is an interdisciplinary field formed by the fusion of molecular biology and medical imaging. The molecular imaging technology has advantages such as high sensitivity and quantifiability, compared with traditional imaging examinations. The molecular imaging technology or molecular image technique mainly includes magnetic resonance (MR) molecular imaging, nuclear medicine molecular imaging, and optical molecular imaging. Among them, the Near Infrared Fluorescence (NIRF) imaging technology in optical molecular imaging has been widely used in clinical surgical procedures, and the basic principle thereof is to use near-infrared light emitted by a fluorescent tracer after excitation to identify sites such as blood vessels, lymph nodes and tumor lesions, thereby assisting clinical doctors in performing surgical procedures.

Currently, indocyanine green (ICG) has been widely used as a near-infrared fluorescent tracer in the clinical field. However, since ICG does not specifically recognize tumor tissue, the use of ICG in surgical tumor resection only enables to indirectly label tumor lesions according to vascular and tissular differences. The imaging result is poor, and specifically manifested as insufficient sensitivity, poor tumor specificity and short imaging time. There is no specific contrast diagnostic reagent for bladder cancer in clinic.

A problem to be solved is to find a contrast agent that has strong targeting to tumors, enables to clearly distinguish them from surrounding normal tissues, enables to shorten imaging time especially for bladder perfusion, has sufficient duration in tumors, and has good biocompatibility without obvious toxicity and accumulation.

## SUMMARY OF THE INVENTION

The present invention provides a novel near-infrared fluorescent labeling technology for tumors.

Therefore, the object of the present invention is to provide a compound with a structure of general formula (I) or a pharmaceutically acceptable salt thereof,

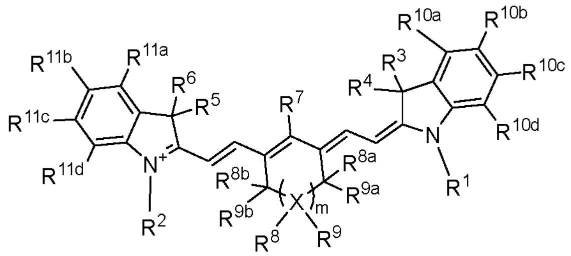

(I)

wherein:

- X is C, O, S or $N^+$;
- $R^1$ and $R^2$ are each independently selected from the group consisting of $L_1$-$L_2$-$L_3$-Q;
- $L_1$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
- $L_2$ is selected from the group consisting of bond, aryl, heteroaryl, cycloalkyl and heterocyclyl;
- $L_3$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
- Q is selected from the group consisting of $R^{12}$, $-OR^{13}$, $-C(O)R^{12}$, $-C(O)OR^{13}$, $-C(O)NHR^{13}$, $-SO_3H$ and

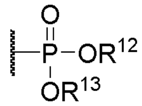

;

$R^{12}$ is selected from the group consisting of hydrogen, halogen, amino, hydroxy, thiol, alkyl, alkylamino, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^{13}$ is selected from the group consisting of hydrogen, alkyl, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^3$ and $R^4$ are each independently selected from the group consisting of hydrogen, halogen, amino, nitro, cyano, hydroxy, thiol, oxo, alkyl, alkoxy, alkenyl, alkynyl, cycloalkyl, heterocyclyl aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro,cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^5$ and $R^6$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^7$ is selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^8$ and $R^9$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^{8a}$ and $R^{9a}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^{8b}$ and $R^{9b}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^{10a}$, $R^{10b}$, $R^{10c}$ and $R^{10d}$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{10a}$, $R^{10b}$, $R^{10c}$ and $R^{10d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^{11a}$, $R^{11b}$, $R^{11c}$ and $R^{11d}$ are each independently selected from the group consisting of hydrogen, halogen, nitro, cyano, hydroxy, thiol, oxo, alkyl, $OR^a$, $NR^bR^c$, $-SO_3H$, $-S(O)_2R^a$, $-C(O)OR^a$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{11a}$, $R^{11b}$, $R^{11c}$ and $R^{11d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy,

haloalkoxy, hydroxyalkyl, alkenyl, alkynyl, cycloalkyl, heterocyclyl, aryl and heteroaryl;
$R^a$ is selected from the group consisting of hydrogen, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;
$R^b$ and $R^c$ are each independently selected from the group consisting of hydrogen, alkyl, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
m is 0 or 1;
p is an integer from 1 to 10;
q is an integer from 1 to 10;
v is an integer from 1 to 10.

In another aspect of the present invention, provided is a compound represented by general formula (IA) or a pharmaceutically acceptable salt thereof,

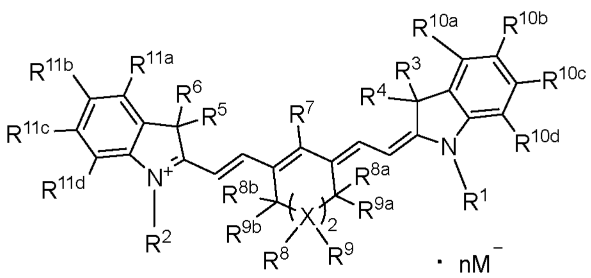

(IA)

wherein:

X is C, O, S or $N^+$;
$M^-$ is an acid ion, and preferably chloride ion, bromide ion, iodide ion, formate ion, acetate ion or trifluoroacetate ion;
n is 1 or 2, when X is one $N^+$, n is 2, and when X is C, O or S, n is 1;
$R^1$ and $R^2$ are each independently selected from the group consisting of $L_1-L_2-L_3-Q$;
$L_1$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
$L_2$ is selected from the group consisting of bond, aryl, heteroaryl, cycloalkyl and heterocyclyl;
$L_3$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
Q is selected from the group consisting of $R^{12}$, $-OR^{13}$, $-C(O)R^{12}$, $-C(O)OR^{13}$, $-C(O)NHR^{13}$, $-SO_3H$ and

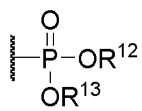

;

$R^{12}$ is selected from the group consisting of hydrogen, halogen, amino, hydroxy, thiol, alkyl, alkylamino, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{13}$ is selected from the group consisting of hydrogen, alkyl, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^3$ and $R^4$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^5$ and $R^6$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^7$ is selected from the group consisting of hydrogen, halogen, nitro, cyano, hydroxy, thiol, oxo, alkyl, $OR^a$, $SR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^8$ and $R^9$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^{8a}$ and $R^{9a}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^{8b}$ and $R^{9b}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;

$R^{10a}$, $R^{10b}$, $R^{10c}$ and $R^{10d}$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{10a}$, $R^{10b}$, $R^{10c}$ and $R^{10d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^{11a}$, $R^{11b}$, $R^{11c}$ and $R^{11d}$ are each independently selected from the group consisting of hydrogen, halogen, nitro, cyano, hydroxy, thiol, oxo, alkyl, $OR^a$, $NR^bR^c$, $-SO_3H$, $-S(O)_2R^a$, $-C(O)OR^a$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{11a}$, $R^{11b}$, $R^{11c}$ and $R^{11d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^a$ is selected from the group consisting of hydrogen, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;

$R^b$ and $R^c$ are each independently selected from the group consisting of hydrogen, alkyl, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo,

thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
m is 0 or 1;
p is an integer from 1 to 10;
q is an integer from 1 to 10;
v is an integer from 1 to 10.

In an embodiment, the compound with the structure of general formula (I) or a pharmaceutically acceptable salt thereof is a compound with a structure of general formula (II) or a pharmaceutically acceptable salt thereof,

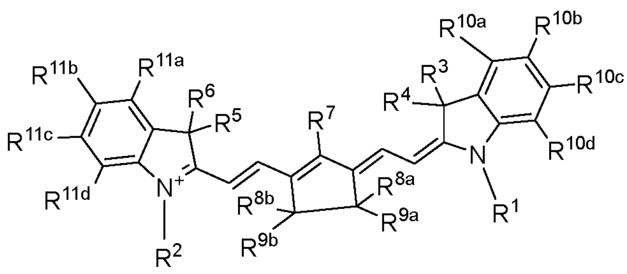

(II)

wherein, $R^1$ to $R^7$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in general formula (I).

In another embodiment, the compound with the structure of general formula (I) or a pharmaceutically acceptable salt thereof is a compound with a structure of general formula (III) or a pharmaceutically acceptable salt thereof,

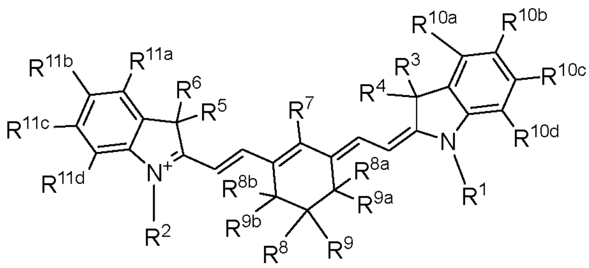

(III)

wherein , $R^1$ to $R^9$, $R^{8a}$, $R^{8b}$, $R^{9a}$, $R^{9b}$, $R^{10}$a, $R^{10b}$, $R^{10c}$, $R^{10d}$, $R^{11a}$, $R^{11b}$, $R^{11c}$ and $R^{11d}$ are as defined in general formula (I).

In another embodiment, the compound represented by general formula (IA) or a pharmaceutically acceptable salt thereof is a compound represented by general formula (IIA) or (IIIA) or a pharmaceutically acceptable salt thereof,

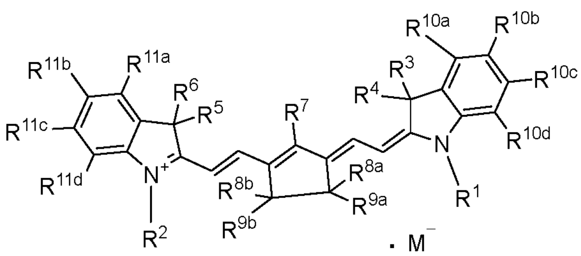

(IIA)

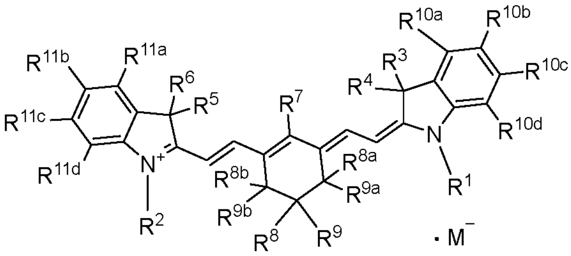

(IIIA)

wherein, $M^-$, $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in general formula (IA).

In another embodiment, the compound with the structure of general formula (I) or a pharmaceutically acceptable salt thereof is a compound with a structure of general formula (IV) or a pharmaceutically acceptable salt thereof,

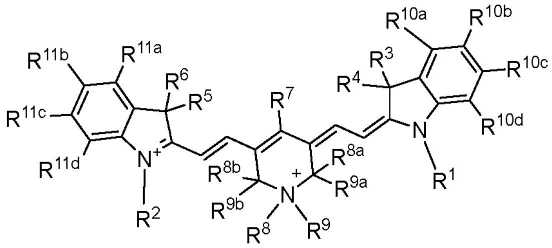

(IV)

wherein , $R^1$ to $R^9$, $R^{8a}$, $R^{8b}$, $R^{9a}$, $R^{9b}$, $R^{10a}$, $R^{10b}$, $R^{10c}$, $R^{10d}$ , $R^{11a}$, $R^{11b}$, $R^{11c}$ and $R^{11d}$ are as defined in general formula (I).

In another embodiment, the compound represented by general formula (IA) or a pharmaceutically acceptable salt thereof is a compound represented by general formula (IVA) or a pharmaceutically acceptable salt thereof,

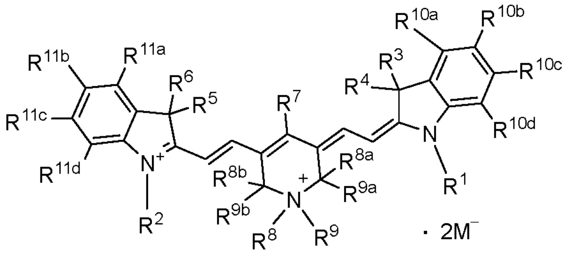

wherein, $M^-$, $R^1$ to $R^9$, $R^{8a}$, $R^{8b}$, $R^{9a}$, $R^{9b}$, $R^{10a}$, $R^{10b}$, $R^{10c}$, $R^{10d}$ , $R^{11a}$, $R^{11b}$, $R^{11c}$ and $R^{11d}$ are as defined in general formula (IA).

In another embodiment, in the compound with the structure of general formula (I), (II), (III), (IV), (IA), (IIA), (IIIA) or (IVA) or a pharmaceutically acceptable salt thereof, $R^1$ is $L_1$-$L_2$- Q;

$L_1$ is selected from the group consisting of $-(CH_2)_p-$ and $-(CH_2O)_q-(CH_2)_p-$;
$L_2$ is selected from the group consisting of bond, $C_{6-10}$ aryl and 5 to 10 membered heteroaryl; and preferably selected from the group consisting of bond and phenyl;
Q is selected from the group consisting of $R^{12}$, $-C(O)OR^{13}$, $-C(O)NHR^{13}$, $-SO_3H$and

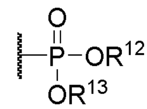

;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably selected from the group consisting of hydrogen and phenyl.

p is an integer from 1 to 10, and preferably an integer from 1 to 6;
q is an integer from 1 to 10, and preferably an integer from 1 to 6.
In another embodiment, in the compound with the structure of general formula (I), (II), (III), (IV), (IA), (IIA), (IIIA) or (IVA) or a pharmaceutically acceptable salt thereof,
$R^2$ is $L_1$- Q;
$L_1$ is $-(CH_2)_p-$;
Q is selected from the group consisting of $R^{12}$, -C(O)OR$^{13}$, -C(O)NHR$^{13}$, $-SO_3H$and

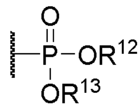
;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably selected from the group consisting of hydrogen and phenyl.
p is an integer from 1 to 10, and preferably an integer from 1 to 6.

In another embodiment, in the compound with the structure of general formula (I), (II), (III), (IV), (IA), (IIA), (IIIA) or (IVA) or a pharmaceutically acceptable salt thereof, $R^{10a}$, $R^{10b}$, $R^{10c}$ and $R^{10d}$ are each independently selected from the group consisting of hydrogen and $OR^a$, and $R^a$ is $C_{1-6}$ alkyl.

In another embodiment, in the compound with the structure of general formula (I), (II), (III), (IV), (IA), (IIA), (IIIA) or (IVA) or a pharmaceutically acceptable salt thereof, $R^{11a}$, $R^{11b}$, $R^{11c}$ and $R^{11d}$ are each independently selected from the group consisting of hydrogen, carboxy, $OR^a$, $NR^bR^c$, $-SO_3H$, $-S(O)_2R^a$ and $-C(O)OR^a$,

$R^a$ is $C_{1-6}$ alkyl;
$R^b$ and $R^c$ are each independently $C_{1-6}$ alkyl.

In another embodiment, in the compound with the structure of general formula (I), (II), (III), (IV), (IA), (IIA), (IIIA) or (IVA) or a pharmaceutically acceptable salt thereof, any two adjacent ones of $R^{10a}$, $R^{10b}$, $R^{10c}$ and $R^{10d}$, together with the atoms to which they are attached, form a $C_{6-10}$ aryl or 5 to 10 membered heteroaryl, and preferably phenyl; the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl.

In another embodiment, in the compound with the structure of general formula (I), (II), (III), (IV), (IA), (IIA), (IIIA) or (IVA) or a pharmaceutically acceptable salt thereof, any two adjacent ones of $R^{11a}$, $R^{11b}$, $R^{11c}$ and $R^{11d}$, together with the atoms to which they are attached, form a $C_{6-10}$ aryl or 5 to 10 membered heteroaryl, and preferably phenyl; the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl.

In another embodiment, the compound with the structure of general formula (I) or a pharmaceutically acceptable salt thereof is a compound with a structure of general formula (V) or a pharmaceutically acceptable salt thereof,

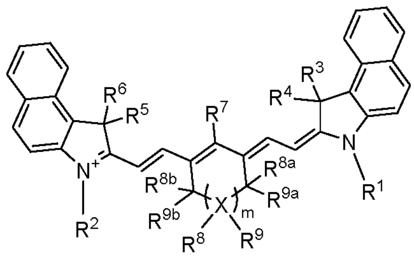

(V)

wherein, $R^1$ to $R^9$, $R^{8a}$, $R^{8b}$, $R^{9a}$, $R^{9b}$, X and m are as defined in general formula (I).

In another embodiment, in the compounds with the structure of general formula (I), (II), (III), (IV), (V), (IA), (IIA), (IIIA) or (IVA) or a pharmaceutically acceptable salt thereof,

$R^1$ is $L_1$- Q;
$L_1$ is $-(CH_2)_p-$;
Q is selected from the group consisting of $-C(O)OR^{13}$, $-C(O)NHR^{13}$, $-SO_3H$ and

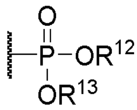
;

$R^{13}$ is selected from the group consisting of hydrogen, $C_{6-10}$ aryl and 5 to 10 membered heteroaryl; and preferably selected from the group consisting of hydrogen and phenyl;
p is an integer from 1 to 10, and preferably an integer from 1 to 6;
$R^2$ is $C_{1-6}$ alkyl.

In another embodiment, in the compound with the structure of general formula (I), (II), (III), (IV), (V), (IA), (IIA), (IIIA) or (IVA) or a pharmaceutically acceptable salt thereof,

$R^1$ is $L_1$- Q;
$L_1$ is $-(CH_2O)_q-(CH_2)_p-$;
Q is selected from the group consisting of $-C(O)OR^{13}$ and $-C(O)NHR^{13}$,
$R^{13}$ is selected from the group consisting of hydrogen, $C_{6-10}$ aryl and 5 to 10 membered heteroaryl; and preferably phenyl;
p is an integer from 1 to 6, preferably an integer from 1 to 4, and more preferably 1 or 2;
q is an integer from 1 to 6, preferably an integer from 1 to 4, and more preferably 1 or 2;
$R^2$ is $C_{1-6}$ alkyl.

In another embodiment, in the compound with the structure of general formula (I), (II), (III), (IV), (V), (IA), (IIA), (IIIA) or (IVA) or a pharmaceutically acceptable salt thereof,

$R^1$ is $L_1$-$L_2$- Q;
$L_1$ is $-(CH_2)_p-$;
$L_2$ is selected from the group consisting of $C_{6-10}$ aryl and 5 to 10 membered heteroaryl, and preferably phenyl;
Q is $-C(O)OR^{13}$,
$R^{13}$ is selected from the group consisting of hydrogen, $C_{6-10}$ aryl and 5 to 10 membered heteroaryl; and preferably phenyl;
p is an integer from 1 to 4, and preferably an integer from 1 to 2;
$R^2$ is $C_{1-6}$ alkyl.

In another embodiment, in the compound with the structure of general formula (I), (II), (III), (IV), (V), (IA), (IIA), (IIIA) or (IVA) or a pharmaceutically acceptable salt thereof,

$R^1$ is $C_{1-6}$ alkyl;
$R^2$ is $C_{1-6}$ alkyl.

In another embodiment, in the compound with the structure of general formula (I), (II), (III), (IV), (V), (IA), (IIA), (IIIA) or (IVA) or a pharmaceutically acceptable salt thereof,

$R^1$ and $R^2$ are each independently selected from $L_1$- Q;
$L_1$ is $-(CH_2)_p-$;
Q is selected from the group consisting of $R^{12}$, $-C(O)OR^{13}$, $-C(O)NHR^{13}$, $-SO_3H$ and

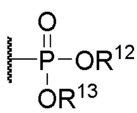
;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably

selected from the group consisting of hydrogen and phenyl;
p is an integer from 1 to 10, and preferably an integer from 1 to 6.

In another embodiment, in the compound with the structure of general formula (I), (II), (III), (IV), (V), (IA), (IIA), (IIIA) or (IVA) or a pharmaceutically acceptable salt thereof, $R^7$ is selected from the group consisting of hydrogen, halogen, hydroxy, $OR^a$, $SR^a$ and $NR^bR^c$;

$R^a$ is selected from the group consisting of $C_{6-10}$ aryl and 5 to 10 membered heteroaryl, and preferably phenyl; wherein, the aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;
$R^b$ and $R^e$ are each independently selected from the group consisting of hydrogen and $C_{1-10}$ alkyl, wherein the $C_{1-10}$ alkyl is optionally further substituted by carboxy.

In another embodiment, in the compound with the structure of general formula (I), (II), (III), (IV), (V), (IA), (IIA), (IIIA) or (IVA) or a pharmaceutically acceptable salt thereof, $R^7$ is $SR^a$; $R^a$ is $C_{1-6}$ alkyl, and the $C_{1-6}$ alkyl is optionally substituted by carboxy.
In another embodiment, in the compound with the structure of general formula (I), (II), (III), (IV), (V), (IA), (IIA), (IIIA) or (IVA) or a pharmaceutically acceptable salt thereof, $R^8$ and $R^9$ are each independently selected from the group consisting of hydrogen and $C_{1-6}$ alkyl.
In another embodiment, in the compound with the structure of general formula (I), (II), (III), (IV), (V), (IA), (IIA), (IIIA) or(IVA) or a pharmaceutically acceptable salt thereof, $R^{8a}$ and $R^{9a}$ are each independently hydrogen.
In another embodiment, in the compound with the structure of general formula (I), (II), (III), (IV), (V), (IA), (IIA), (IIIA) or (IVA) or a pharmaceutically acceptable salt thereof, $R^{8b}$ and $R^{9b}$ are each independently hydrogen.
In another embodiment, in the compound with the structure of general formula (I), (II), (III), (IV), (V), (IA), (IIA), (IIIA) or (IVA) or a pharmaceutically acceptable salt thereof, $R^3$ and $R^4$ are each independently selected from the group consisting of hydrogen and $C_{1-6}$ alkyl.
In another embodiment, in the compound with the structure of general formula (I), (II), (III), (IV), (V), (IA), (IIA), (IIIA) or (IVA) or a pharmaceutically acceptable salt thereof, $R^5$ and $R^6$ are each independently selected from the group consisting of hydrogen and $C_{1-6}$ alkyl.
In another embodiment, in the compound with the structure of general formula (I), (II), (III), (IV), (V), (IA), (IIA), (IIIA) or (IVA) or a pharmaceutically acceptable salt thereof, the pharmaceutically acceptable salt is halide salt or carboxylate salt, and preferably hydrochloride salt, formate salt, acetate salt or trifluoroacetate salt.
Typical compounds of the present invention include, but are not limited to:

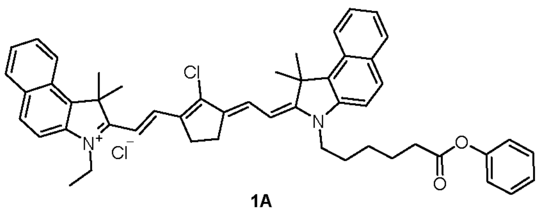
1A
,

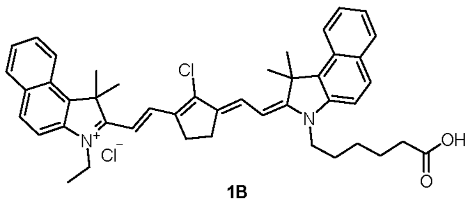
1B
,

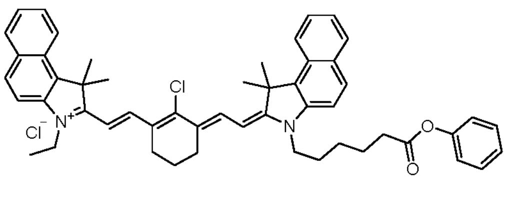
2
,

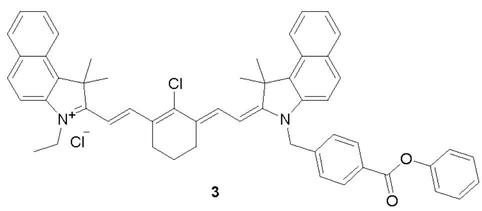
3
,

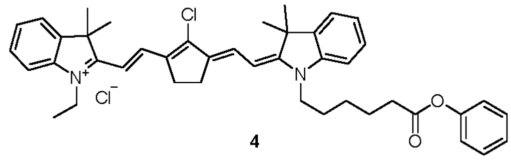

**4**,

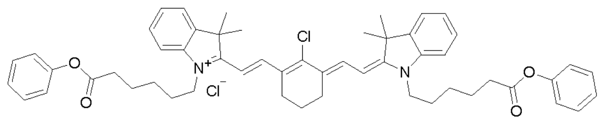

**5**,

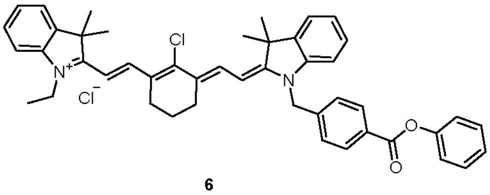

**6**,

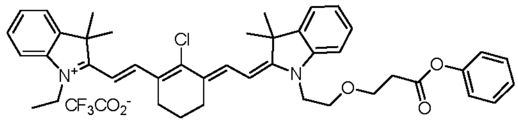

**7**,

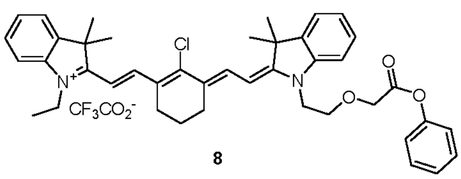

**8**,

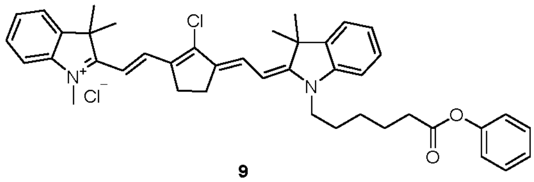

**9**,

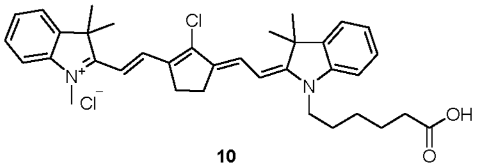

**10**,

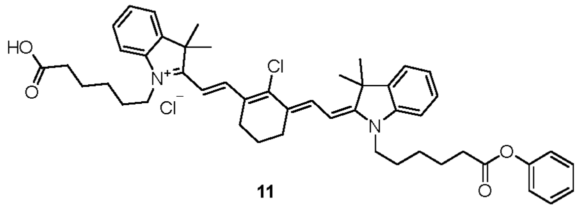

**11**,

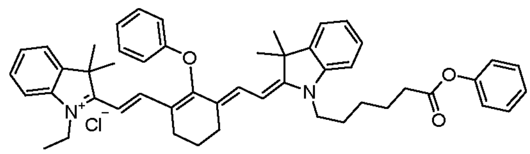

**12**,

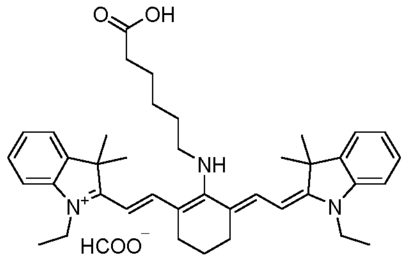

**13**,

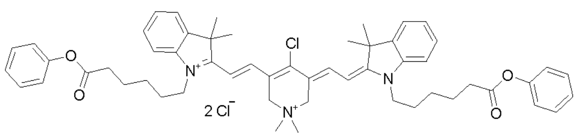

**14**,

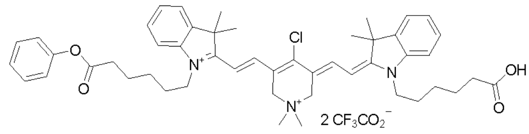

**15**,

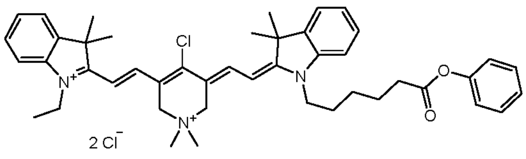

**16**,

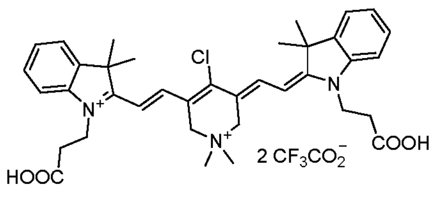

**17**,

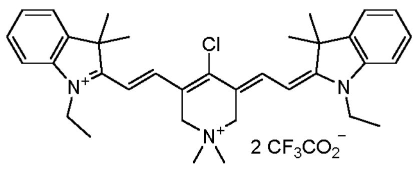

18

,

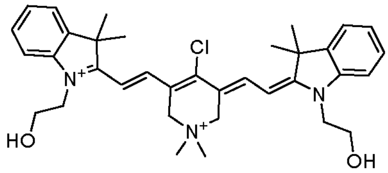

19

,

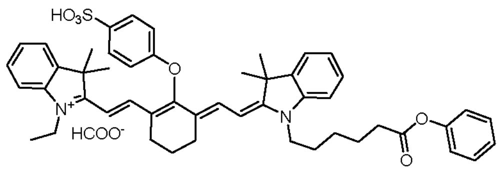

20

,

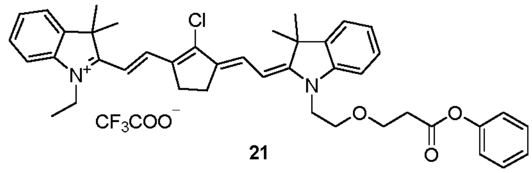

21

,

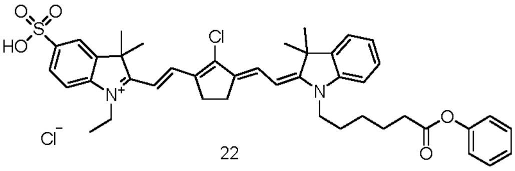

22

,

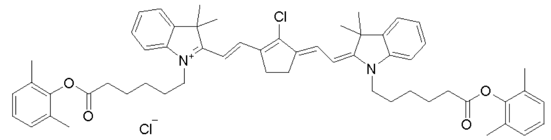

23

,

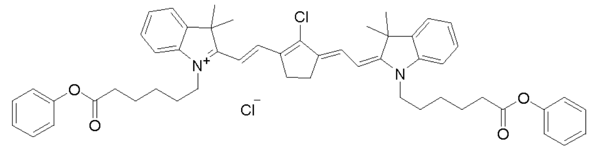

24

,

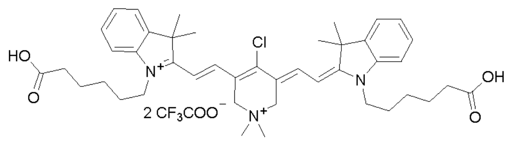

25

,

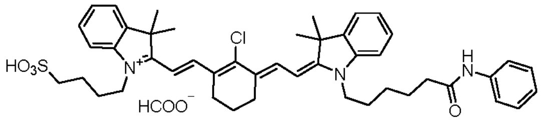

26

,

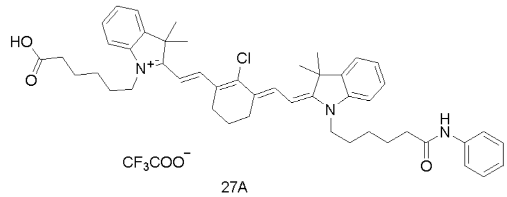

27A

,

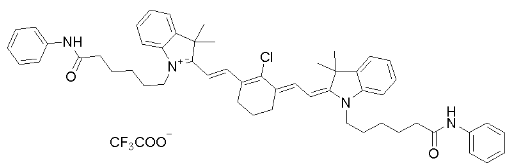

27B

,

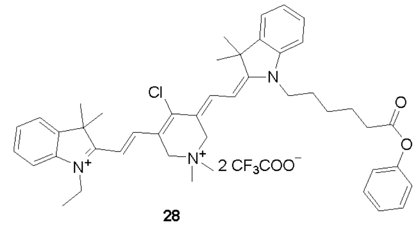

28

,

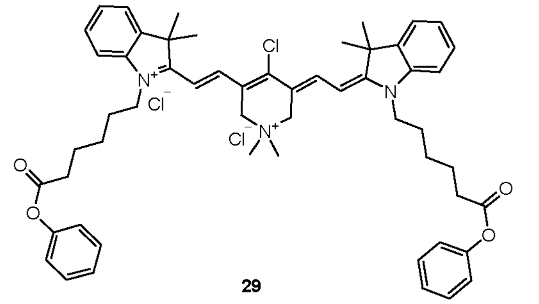

29,

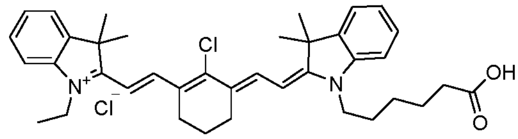

30,

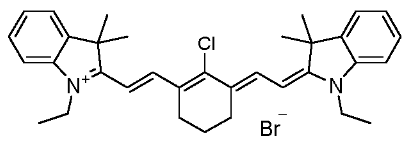

31,

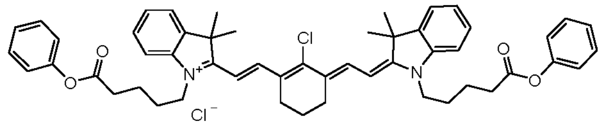

33,

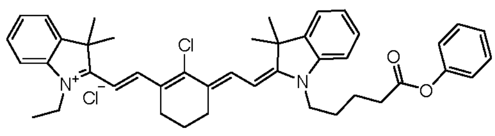

34,

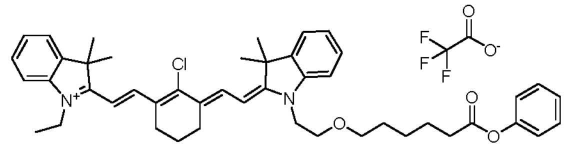

35,

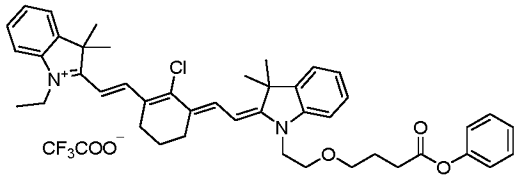

36,

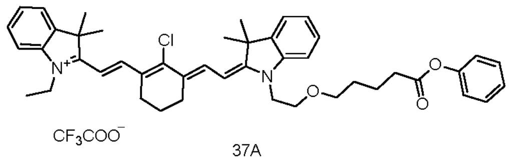

37A,

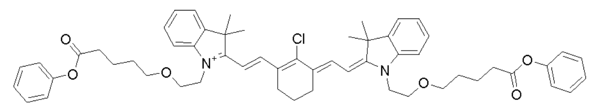

37B,

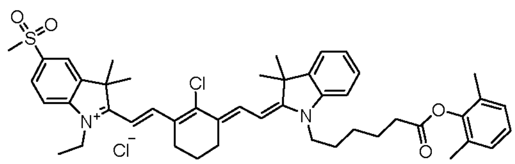

39,

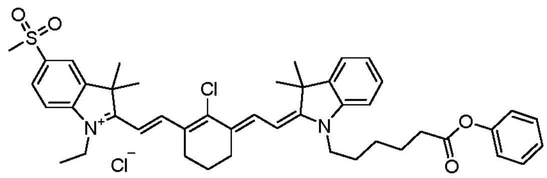

40,

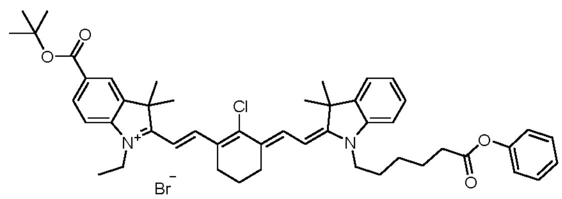

41,

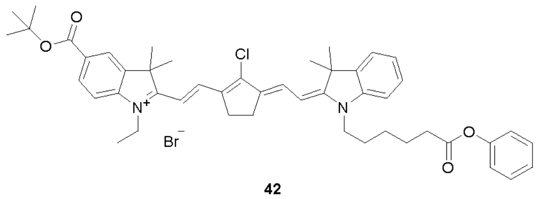
42,

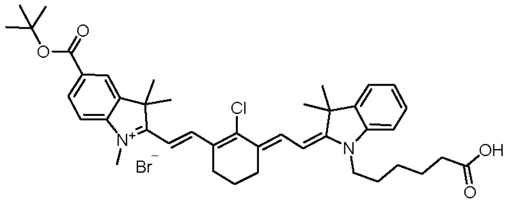
43,

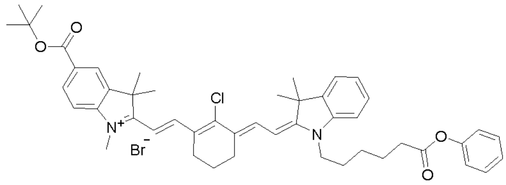
44,

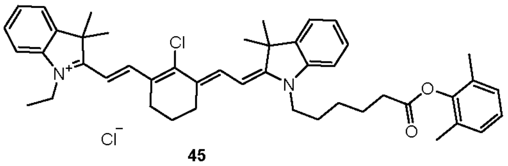
45,

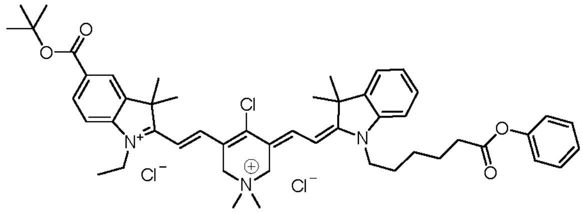
46,

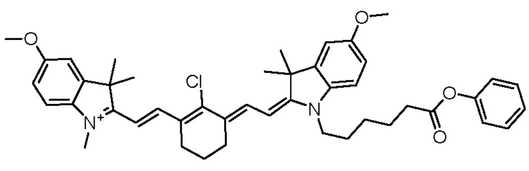
47,

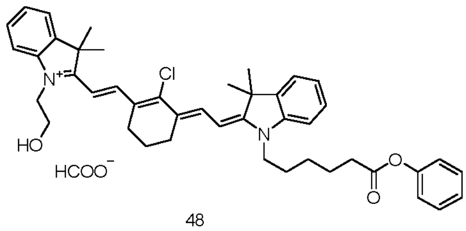
48,

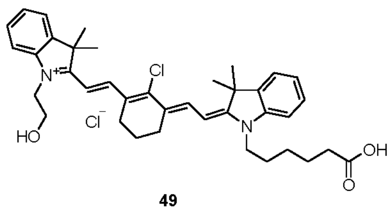
49,

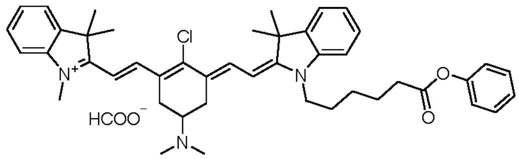
50,

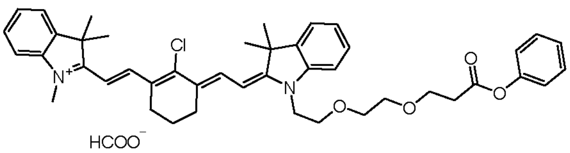
51,

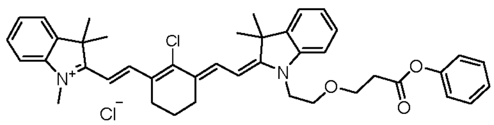
52,

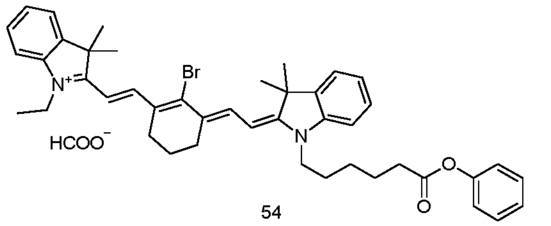
54,

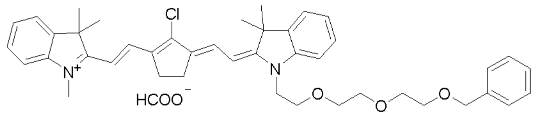

55,

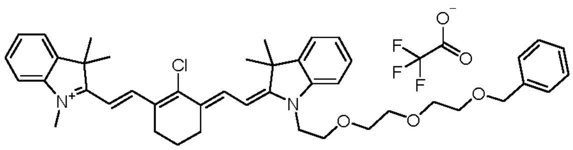

56,

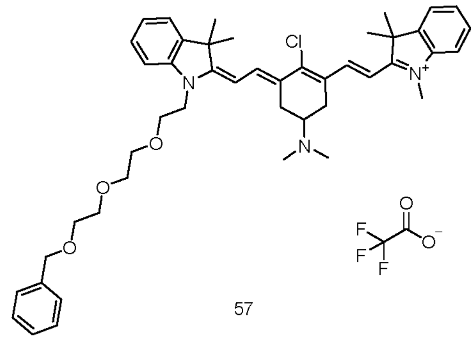

57,

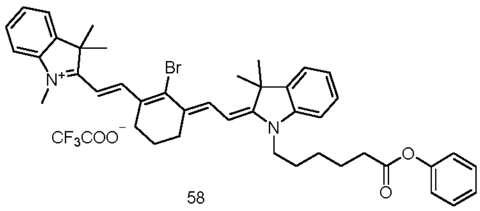

58,

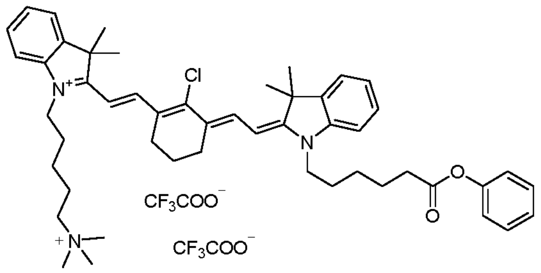

60,

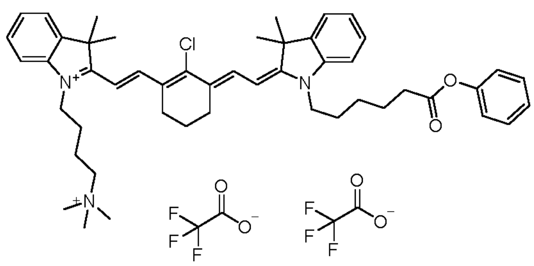

61,

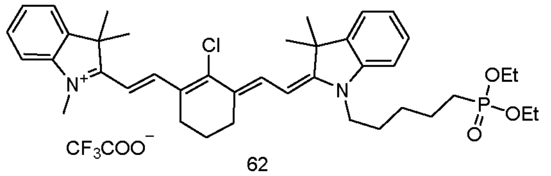

62,

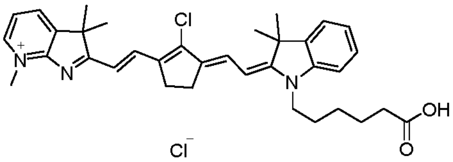

63,

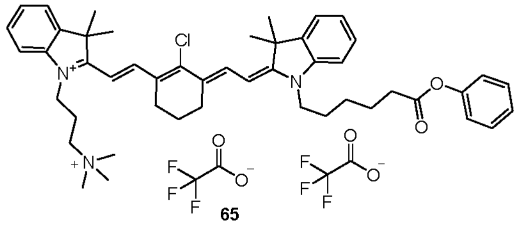

65,

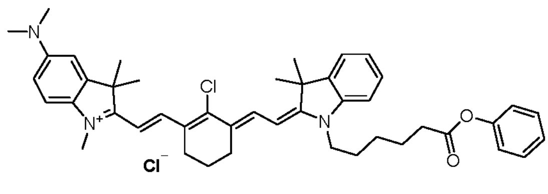

66,

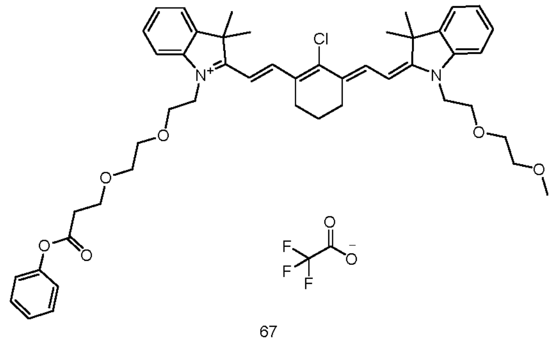

67 ,

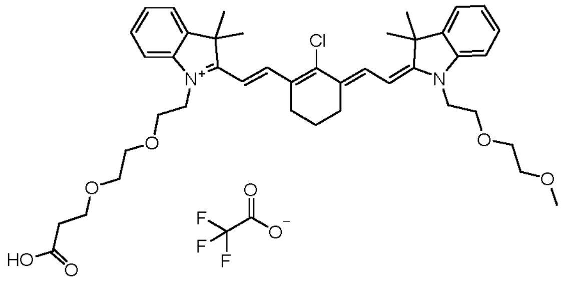

68 ,

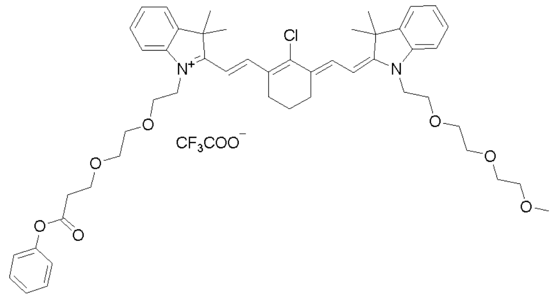

**69** ,

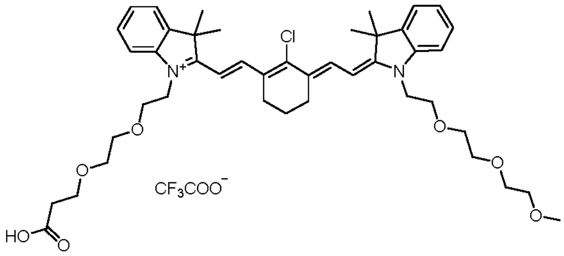

**70** ,

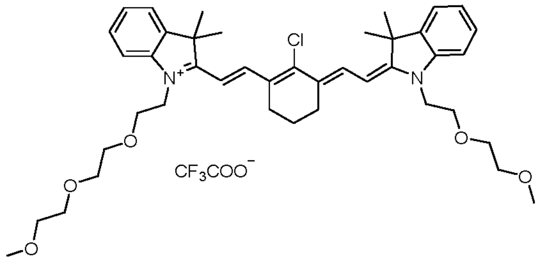

**71** ,

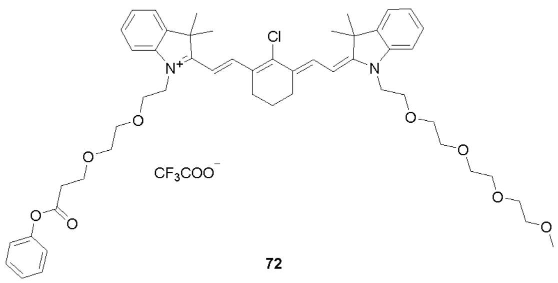

**72** ,

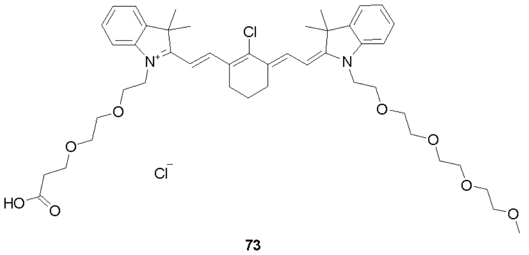

**73** ,

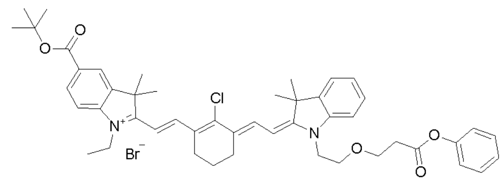

74 ,

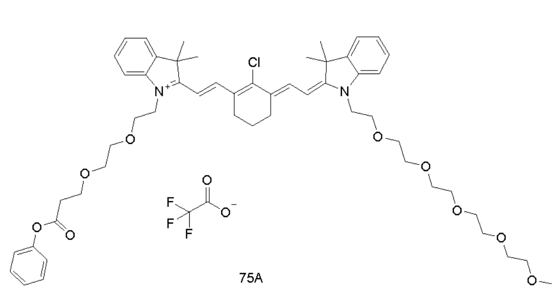

75A ,

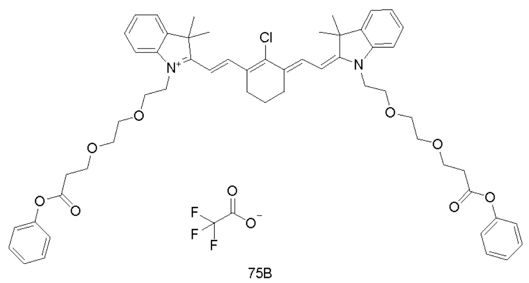

75B ,

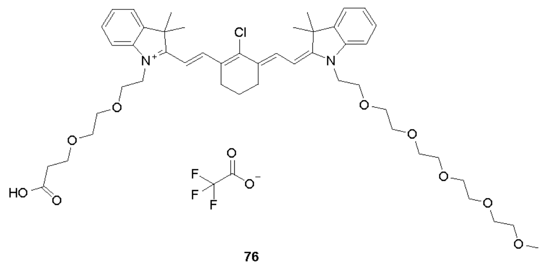

76

,

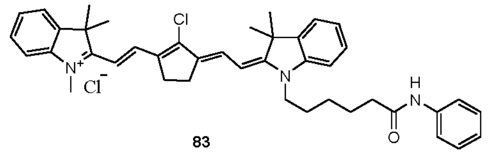

83

,

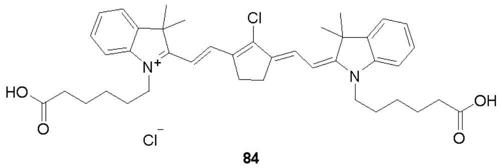

84

,

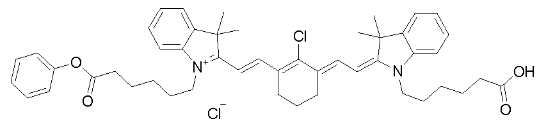

85A

,

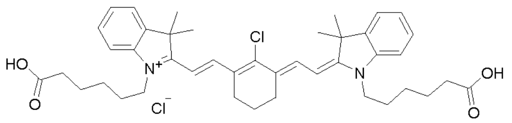

85B

,

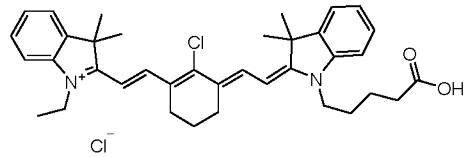

86

,

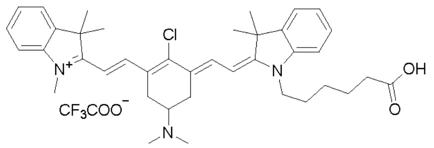

87

,

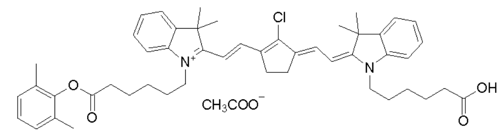

88

,

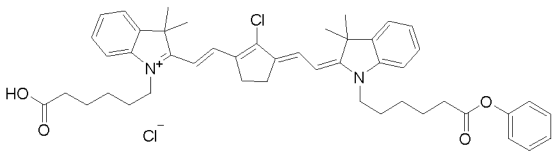

89

,

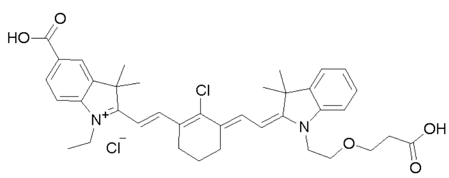

91

,

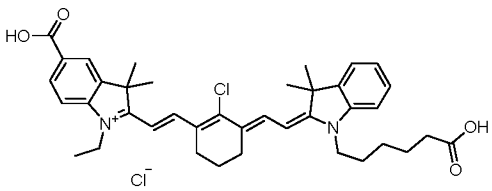

92

,

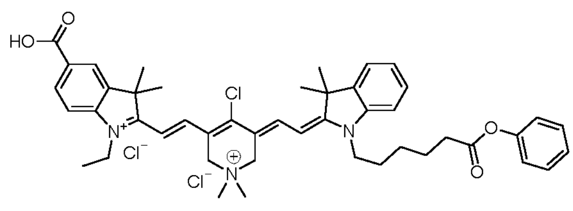

93

,

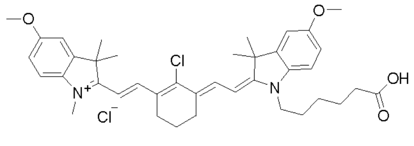

94

,

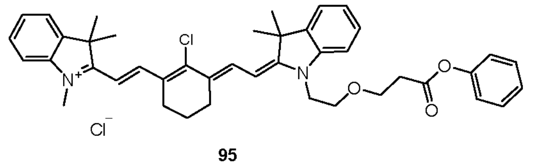

95

,

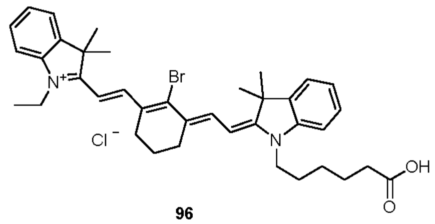

96,

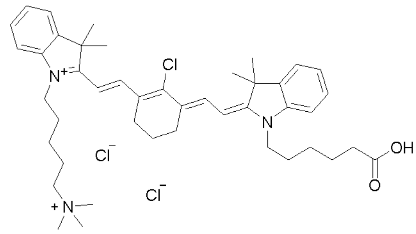

97,

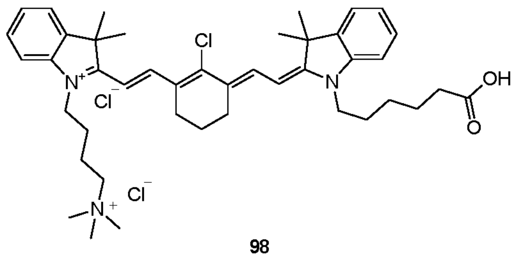

98,

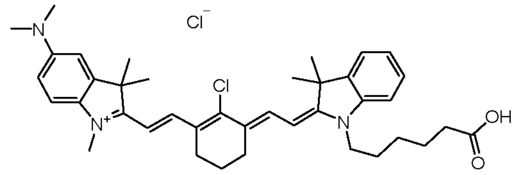

99,

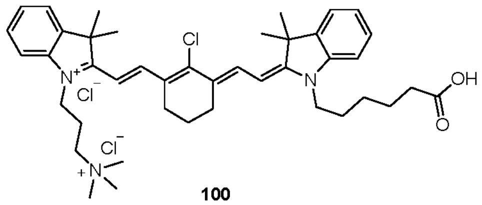

100,

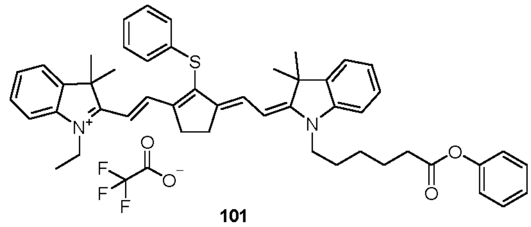

101,

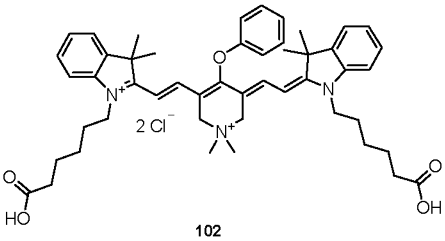

102,

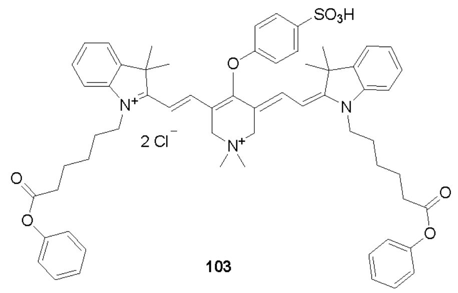

103,

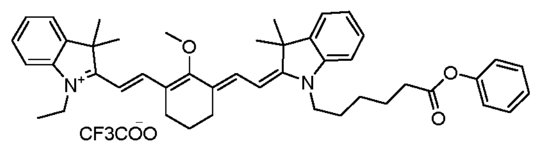

104,

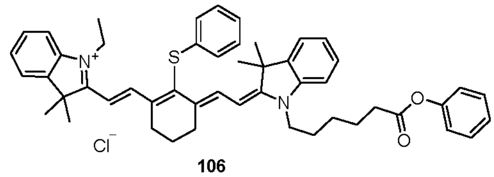

106,

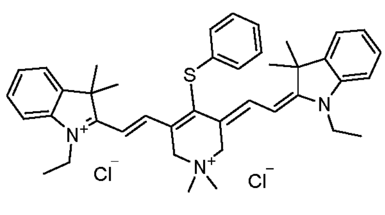

107,

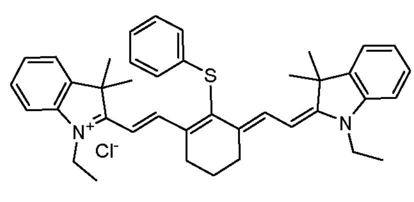

108,

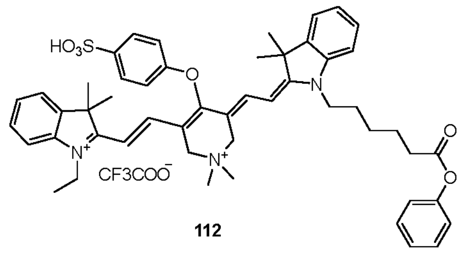

112 ,

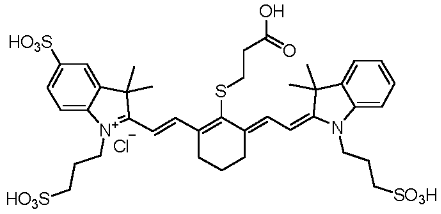

113 ,

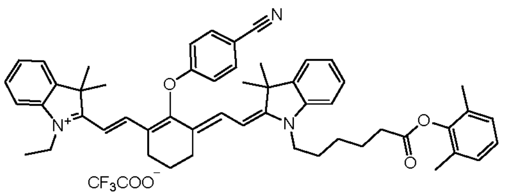

114 ,

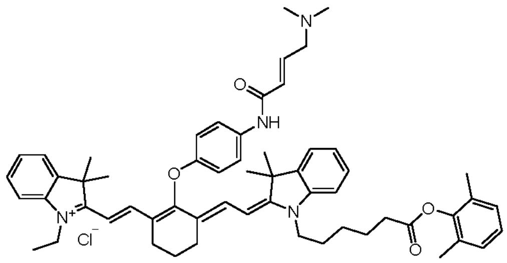

115 ,

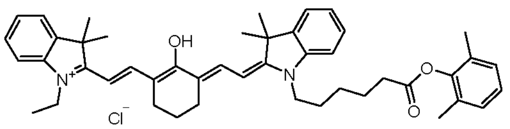

116 ,

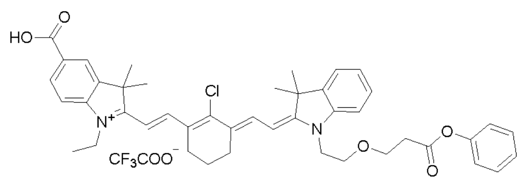

117 ,

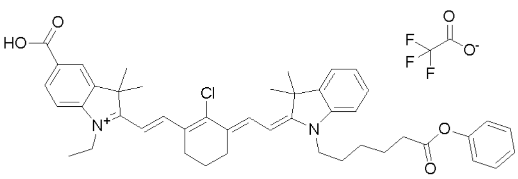

118 ,

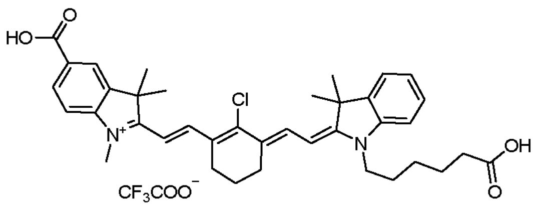

119 ,

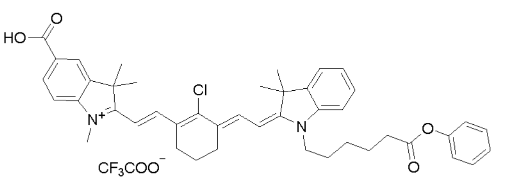

120 ,

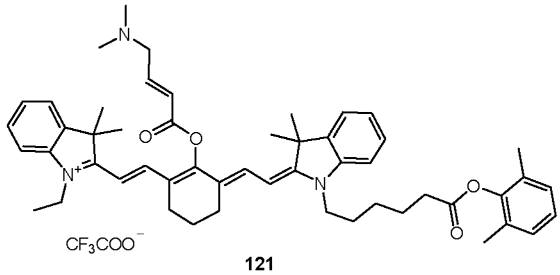

121 ,

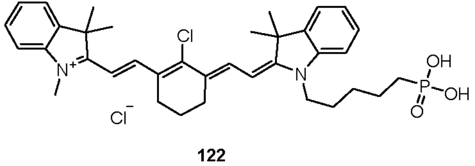

122 ,

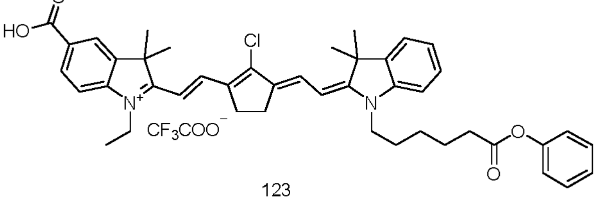

123 .

In another aspect of the present invention, provided is a compound with a structure of general formula (VI) or a pharmaceutically acceptable salt thereof,

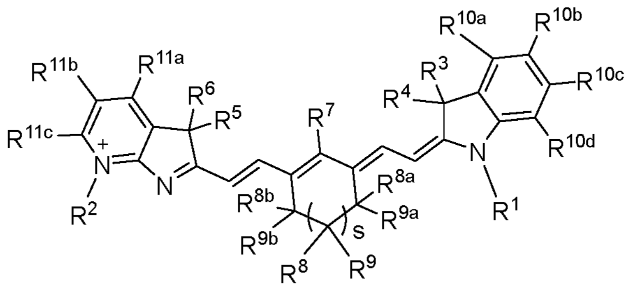

(VI)

wherein , $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$ and $R^{11c}$ are as defined in general formula (I); s is 0 or 1;

preferably,

$R^1$ is $L_1$-$L_2$- Q;

$L_1$ is $-(CH_2)_p-$;

$L_2$ is a bond;

Q is $-C(O)OR^{13}$,

$R^{13}$ is selected from the group consisting of hydrogen and phenyl;

$R^2$ is $C_{1-6}$ alkyl;

$R^3$ and $R^4$ are each independently selected from the group consisting of hydrogen and $C_{1-6}$ alkyl, and preferably $C_{1-6}$ alkyl;

$R^5$ and $R^6$ are each independently selected from the group consisting of hydrogen and $C_{1-6}$ alkyl, and preferably $C_{1-6}$ alkyl;

$R^5$ is a halogen;

$R^8$ and $R^9$ are each independently hydrogen;

$R^{8a}$ and $R^{9a}$ are each independently hydrogen;

$R^{8b}$ and $R^{9b}$ are each independently hydrogen;

$R^{10a}$, $R^{10b}$, $R^{10c}$ and $R^{10d}$ are each independently hydrogen;

$R^{11a}$, $R^{11b}$ and $R^{11c}$ are each independently hydrogen.

In a specific embodiment, the compound with the structure of general formula (VI) or a pharmaceutically acceptable salt thereof is selected from the group consisting of:

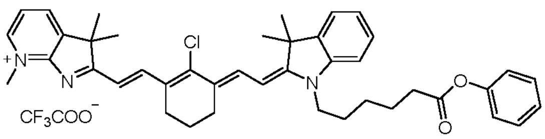

53

and

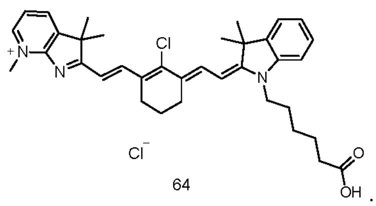

64 .

In another aspect of the present invention, provided is a compound with a structure of general formula (VII) or a pharmaceutically acceptable salt thereof,

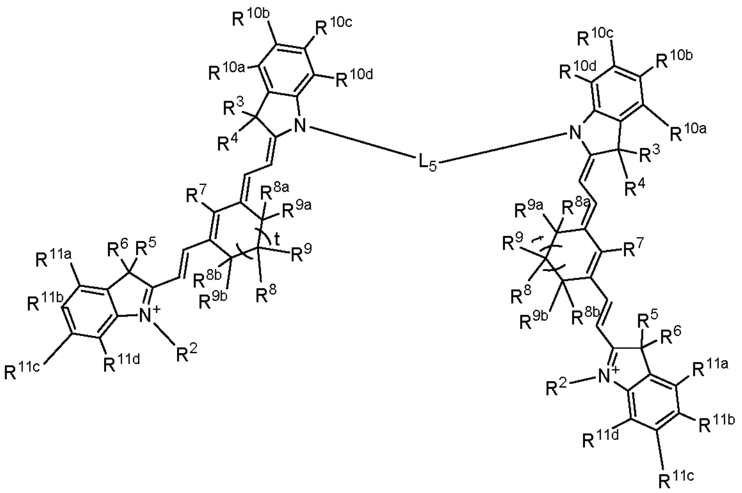

(VII)

wherein, $R^2$ to $R^9$, $R^{8a}$, $R^{8b}$, $R^{9a}$, $R^{9b}$, $R^{10a}$, $R^{10b}$, $R^{10c}$, $R^{10d}$ , $R^{11a}$, $R^{11b}$, $R^{11c}$ are as defined in general formula (I);
t is 0 or 1;
$L_5$ is $-(CH_2)_{t1}-C(O)NH-(CH_2)_{t2}-NHC(O)-(CH_2)_{t3}-$;
$t_1$, $t_2$ and $t_3$ are each independently selected from the group consisting of integers from 1 to 10; and preferably selected from the group consisting of integers from 2 to 6;
preferably,
$R^2$ is $C_{1-6}$ alkyl;
$R^3$ and $R^4$ are each independently selected from the group consisting of hydrogen and $C_{1-6}$ alkyl, and preferably $C_{1-6}$ alkyl;
$R^5$ and $R^6$ are each independently selected from the group consisting of hydrogen and $C_{1-6}$ alkyl, and preferably $C_{1-6}$ alkyl;
$R^7$ is a halogen;
$R^8$ and $R^9$ are each independently hydrogen;
$R^{8a}$ and $R^{9a}$ are each independently hydrogen;
$R^{8b}$ and $R^{9b}$ are each independently hydrogen;
$R^{10a}$, $R^{10b}$, $R^{10c}$ and $R^{10d}$ are each independently hydrogen;
$R^{11a}$, $R^{11b}$ and $R^{11c}$ are each independently hydrogen.

In a specific embodiment, the compound with the structure of general formula (VII) or a pharmaceutically acceptable salt thereof is selected from the group consisting of:

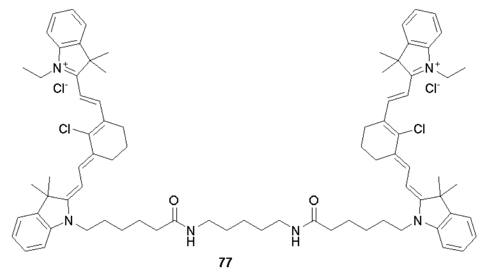

77

,

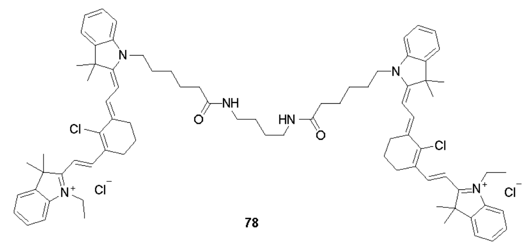

78

,

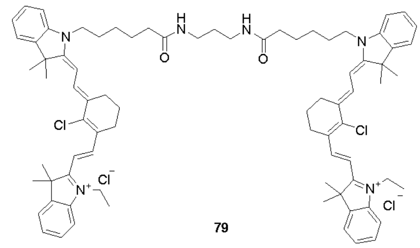

79

,

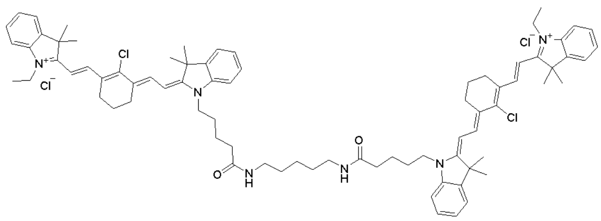

80

,

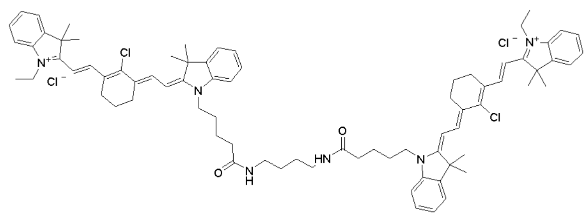
81
and
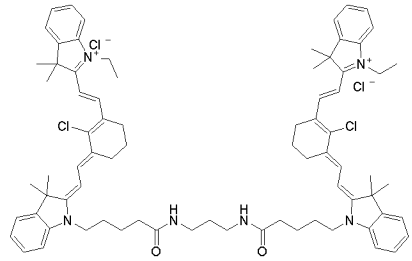
82
.

In another aspect of the present invention, provided is a compound with a structure of general formula (VIII) or a pharmaceutically acceptable salt thereof,

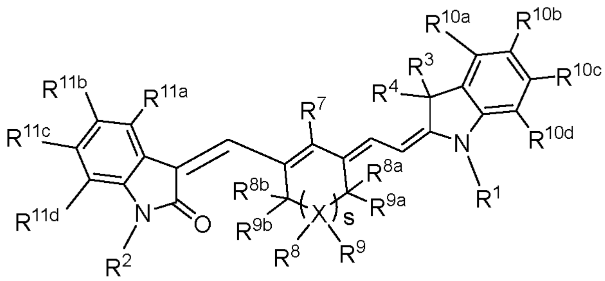
(VI)

wherein, X, $R^1$ to $R^4$, $R^7$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11}$, $R^{11c}$ and $R^{11d}$ are as defined in general formula (I); s is 0 or 1;
preferably,
X is $N^+$;
s is 1;
$R^1$ is $L_1$-$L_2$- Q;
$L_1$ is $-(CH_2)_p-$;
$L_2$ is a bond;
Q is -C(O)$OR^{13}$,
$R^{13}$ is selected from the group consisting of hydrogen and phenyl;
$R^2$ is $C_{1-6}$ alkyl;
$R^3$ and $R^4$ are each independently selected from the group consisting of hydrogen and $C_{1-6}$ alkyl, and preferably $C_{1-6}$ alkyl;
$R^7$ is a halogen;
$R^8$ and $R^9$ are each independently $C_{1-6}$ alkyl;
$R^{8a}$ and $R^{9a}$ are each independently hydrogen;
$R^{8b}$ and $R^{9b}$ are each independently hydrogen;
$R^{10a}$, $R^{10b}$, $R^{10c}$ and $R^{10d}$ are each independently hydrogen;
$R^{11a}$, $R^{11b}$ and $R^{11c}$ are each independently hydrogen.

In a specific embodiment, the compound with the structure of general formula (VIII) or a pharmaceutically acceptable salt thereof is selected from:

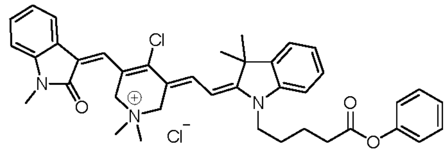
38
.

In another aspect of the present invention, provided is a contrast agent comprising the compound or a pharmaceutically acceptable salt thereof according to the present invention and an anionic surfactant.

In an embodiment, in the contrast agent, the anionic surfactant is selected from the group consisting of carboxylate salt, sulfonate salt, sulfate salt, phosphate salt and lipopeptide anionic surfactants, preferably is sulfonate salt or lipopeptide anionic surfactant, and more preferably is surfactin, sodium dodecyl sulfonate and/or sodium dodecyl benzene sulfonate.

In another embodiment, in the contrast agent, the molar ratio of the compound or a pharmaceutically acceptable salt thereof to the anionic surfactant ranges from 1:20 to 10: 1, preferably from 1: 10 to 10:1, and further preferably from 1:5 to 5:1.

In another embodiment, in the contrast agent, the compound or a pharmaceutically acceptable salt thereof has a final concentration ranging from 1 to 100 $\mu$M, and preferably from 5 to 50 $\mu$M or from 10 to 30 $\mu$M.

In another embodiment, a solution of the contrast agent has a particle size ranging from 10 to 200 nm, preferably from 30 to 150 nm, and more preferably from 50 to 100 nm.

In another embodiment, the contrast agent is in a form of solution or lyophilized powder.

The present invention also provides an assay kit comprising the compound or a pharmaceutically acceptable salt thereof according to the present invention and an anionic surfactant.

In an embodiment, in the kit, the anionic surfactant is selected from the group consisting of carboxylate salt, sulfonate salt, sulfate salt, phosphate salt and lipopeptide anionic surfactants, preferably is sulfonate salt or lipopeptide anionic surfactant, and more preferably is surfactin, sodium dodecyl sulfonate and/or sodium dodecyl benzene sulfonate.

In another embodiment, in the kit, the molar ratio of the compound or a pharmaceutically acceptable salt thereof to the anionic surfactant ranges from 1:20 to 10: 1, preferably from 1: 10 to 10:1, and further preferably from 1:5 to 5:1.

In another embodiment, in the kit, the compound or a pharmaceutically acceptable salt thereof in the kit has a final concentration ranging from 1 to 100 $\mu$M, and preferably from 5 to 50 $\mu$M or from 10 to 30 $\mu$M.

In another embodiment, in the kit,a solution of the contrast agent in the kit has a particle size ranging from 10 to 200 nm, preferably from 30 to 150 nm, and more preferably from 50 to 100 nm.

In another embodiment, in the kit, the contrast agent is in a form of solution or lyophilized powder.

The present invention also relates to a pharmaceutical composition comprising the compound or a pharmaceutically acceptable salt thereof according to the present invention, and one or more pharmaceutically acceptable carriers.

The present invention also relates to a use of the compound or a pharmaceutically acceptable salt thereof according to the present invention or a pharmaceutical composition comprising the same in the preparation of a contrast agent for tumor, wherein the tumor is preferably bladder cancer.

The present invention also relates to the compound or a pharmaceutically acceptable salt thereof according to the present invention or a pharmaceutical composition comprising the same for use as a contrast agent.

The present invention also relates to a method for tumor imaging comprising administering to a subject in need thereof the compound or a pharmaceutically acceptable salt thereof according to the present invention or a pharmaceutical composition comprising the same.

The molecules of the present invention exhibit a significant difference in uptake ability between normal cells and tumor cells in a cell model, such that tumor cells can improve their absorption exponentially, forming an obvious contrast to other cells.

The molecules of the present invention exhibit excellent tumor-specific targeting effect in an animal subcutaneous tumor model, enabling to form an obvious contrast to surrounding normal tissues, thereby facilitating tumor diagnosis.

Explanation of Terms

Unless stated to the contrary, the terms used in the specification and claims have the meanings described below.

The carbon, hydrogen, oxygen, sulfur, nitrogen or halogen involved in the groups and compounds described in the present invention all include their isotopes, i.e., the carbon, hydrogen, oxygen, sulfur, nitrogen or halogen involved in the groups and compounds described in the present invention is optionally further replaced by one or more corresponding isotopes, in which the isotope of carbon includes $^{12}C$, $^{13}C$ and $^{14}C$, the isotope of hydrogen includes protium (H), deuterium (D, also known as heavy hydrogen) and tritium (T, also known as superheavy hydrogen), the isotope of oxygen includes $^{16}O$, $^{17}O$ and $^{18}O$, the isotope of sulfur includes $^{32}S$, $^{33}S$, $^{34}S$ and $^{36}S$, the isotope of nitrogen includes $^{14}N$ and $^{15}N$, the isotope of fluorine includes $^{19}F$, the isotope of chlorine includes $^{35}C1$ and $^{37}Cl$, and the isotope of bromine includes $^{79}Br$ and $^{81}Br$.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms, an alkyl having 1 to 4 carbon atoms or an alkyl having 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl,

1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent(s) may be substituted at any available connection point. The substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group.

The term "alkylene" refers to a divalent alkyl group with the alkyl as defined above, which has 1 to 20 (for example 1, 2, 3, 4, 5, 6, 7, 8, 9, 10, 11, 12, 13, 14, 15, 16, 17, 18, 19 or 20 ) carbon atoms (i.e., $C_{1-20}$ alkylene). The alkylene is preferably an alkylene having 1 to 12 carbon atoms (i.e., $C_{1-12}$ alkylene), more preferably an alkylene having 1 to 6 carbon atoms (i.e., $C_{1-6}$ alkylene), and further preferably an alkylene having 1 to 4 carbon atoms (i.e., $C_{1-4}$ alkylene). Non-limiting examples of the alkylene include, but are not limited to, methylene ($-CH_2-$), 1,1-ethylidene ($-CH(CH_3)-$), 1,2-ethylidene ($-CH_2CH_2-$), 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$) and the like. The alkylene may be substituted or unsubstituted, and when substituted, it may be substituted at any available connection point. The substituent(s) may be one or more selected from the group consisting of alkyl, alkenyl, alkynyl, alkoxy, haloalkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkenyl" refers to a monovalent hydrocarbon group consisting of at least two carbon atoms and at least one carbon-carbon double bond, and preferably an alkenyl having 2 to 4 carbon atoms, such as ethenyl, 1-propenyl, 2-propenyl, 1-, 2- or 3-butenyl and the like. The alkenyl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynyl" refers to a monovalent hydrocarbon group consisting of at least two carbon atoms and at least one carbon-carbon triple bond, and preferably an alkynyl having 2 to 4 carbon atoms or preferably an alkynyl having 3 to 4 carbon atoms, such as ethynyl, propynyl, butynyl and the like. The alkynyl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon group, in which the cycloalkyl ring has 3 to 20 carbon atoms, preferably 3 to 12 carbon atoms, and more preferably 3 to 6 carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. The polycyclic cycloalkyl includes spiro cycloalkyl, fused cycloalkyl and bridged cycloalkyl.

The term "spiro cycloalkyl" refers to a 5 to 20 membered polycyclic group with individual rings sharing one carbon atom (called a spiro atom), which may contain one or more double bonds, but none of the rings of which has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl is divided into a mono-spiro cycloalkyl, di-spiro cycloalkyl or poly-spiro cycloalkyl, and is preferably a mono-spiro cycloalkyl and di-spiro cycloalkyl, and more preferably a 4-membered/4-membered, 4-membered/5-membered, 4-membered/6-membered, 5-membered/5-membered or 5-membered/6-membered mono-spiro cycloalkyl. Non-limiting examples of the spiro cycloalkyl include:

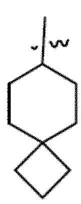 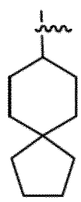 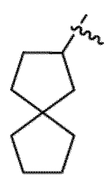 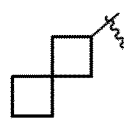 and 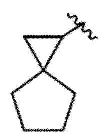 .

The term "fused cycloalkyl" refers to a 5 to 20 membered full-carbon polycyclic group, in which each ring shares a pair of adjacent carbon atoms with another ring in the system, and one or more rings may contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl may be divided into a bicyclic, tricyclic, tetracyclic or polycyclic fused cycloalkyl, and is preferably a bicyclic or tricyclic fused cycloalkyl, and more preferably a 5-membered/5-membered or 5-membered/6-membered bicyclic cycloalkyl. Non-limiting examples of the fused cycloalkyl include:

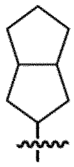 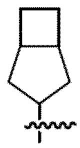 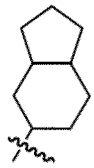 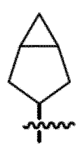 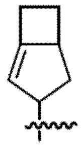 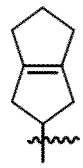 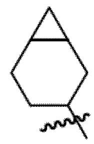 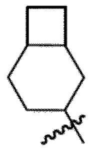 and 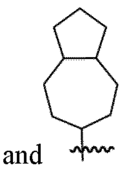 .

The term "bridged cycloalkyl" refers to a 5 to 20 membered full-carbon polycyclic group with any two of rings thereof sharing two carbon atoms that are not directly connected to each other, which may have one or more double bonds, but none of the rings of which has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl may be divided into a bicyclic, tricyclic, tetracyclic or polycyclic bridged cycloalkyl, and is preferably a bicyclic, tricyclic or tetracyclic bridged cycloalkyl, and more preferably a bicyclic or tricyclic bridged cycloalkyl. Non-limiting examples of the bridged cycloalkyl include:

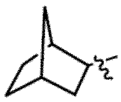 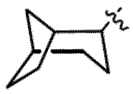 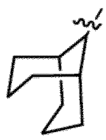 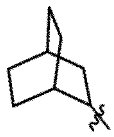 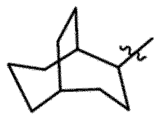

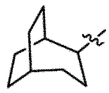 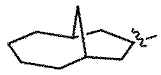 and 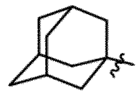 .

The cycloalkyl ring may be fused to an aryl, heteroaryl or heterocyclyl ring, in which the ring linking to the parent structure is cycloalkyl. Non-limiting examples include indanyl, tetrahydronaphthyl, benzocycloheptyl and the like. The cycloalkyl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy or carboxylic ester group.

The term "heterocyclyl" refers to a saturated or partially unsaturated monocyclic or polycyclic hydrocarbon group comprising 3 to 20 ring atoms, in which one or more ring atoms are heteroatoms selected from the group consisting of N, O and $S(O)_m$ (wherein m is an integer from 0 to 2), but excluding -O-O-, -O-S- or -S-S- in the ring, the remaining ring atoms being carbon atoms. Preferably, the heterocyclyl comprises 4 to 12 ring atoms, 1 to 4 atoms of which are heteroatoms; and more preferably 7 to 12 ring atoms, 1 to 4 atoms of which are heteroatoms. Non-limiting examples of the monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. The polycyclic heterocyclyl includes spiro heterocyclyl, fused heterocyclyl and bridged heterocyclyl.

The term "spiro heterocyclyl" refers to a 5 to 20 membered polycyclic heterocyclyl group with individual rings sharing one atom (called a spiro atom), in which one or more ring atoms are heteroatoms selected from the group consisting of N, O and $S(O)_m$ (wherein m is an integer from 0 to 2), and the remaining ring atoms are carbon atoms. The spiro heterocyclyl may contain one or more double bonds, but none of the rings thereof has a completely conjugated π-electron system. The spiro heterocyclyl is preferably a 6 to 14 membered spiro heterocyclyl, and more preferably a 7 to 12 membered spiro heterocyclyl. According to the number of the spiro atoms shared between the rings, the spiro heterocyclyl is divided into mono-spiro heterocyclyl, di-spiro heterocyclyl or poly-spiro heterocyclyl, and is preferably a mono-spiro heterocyclyl and di-spiro heterocyclyl, and more preferably a 4-membered/4-membered, 4-membered/5-membered, 4-membered/6-membered, 5-membered/5-membered or 5-membered/6-membered mono-spiro heterocyclyl. Non-limiting

examples of the spiro heterocyclyl include:

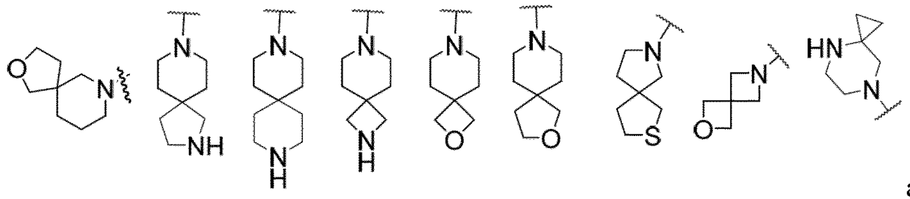

and

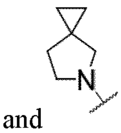

.

The term "fused heterocyclyl" refers to a 5 to 20 membered polycyclic heterocyclyl group, in which each ring shares a pair of adjacent atoms with another ring in the system, one or more rings may contain one or more double bonds, but none of the rings has a completely conjugated π-electron system, and one or more ring atoms are heteroatoms selected from the group consisting of N, O and $S(O)_m$ (wherein m is an integer from 0 to 2), with the remaining ring atoms being carbon atoms. The fused heterocyclyl is preferably a 6 to 14 membered fused heterocyclyl, and more preferably a 7 to 12 membered fused heterocyclyl. According to the number of membered rings, the fused heterocyclyl may be divided into a bicyclic, tricyclic, tetracyclic or polycyclic fused heterocyclyl, and is preferably a bicyclic or tricyclic fused heterocyclyl, and more preferably a 5-membered/5-membered or 5-membered/6-membered bicyclic fused heterocyclyl. Non-limiting examples of the fused heterocyclyl include:

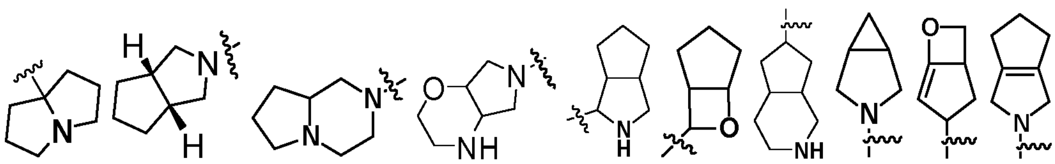

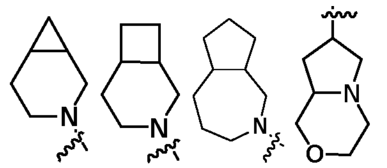

and

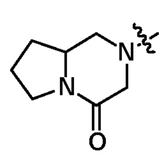

.

The term "bridged heterocyclyl" refers to a 5 to 14 membered polycyclic heterocyclyl group with any two of rings thereof sharing two atoms that are not directly connected to each other, which may have one or more double bonds, but none of the rings of which has a completely conjugated π-electron system, and in which one or more ring atoms are heteroatoms selected from the group consisting of N, O and $S(O)_m$ (wherein m is an integer from 0 to 2), with the remaining ring atoms being carbon atoms. The bridged heterocyclyl is preferably a 6 to 14 membered bridged heterocyclyl, and more preferably a 7 to 12 membered bridged heterocyclyl. According to the number of membered rings, the bridged heterocyclyl may be divided into a bicyclic, tricyclic, tetracyclic or polycyclic bridged heterocyclyl, and is preferably a bicyclic, tricyclic or tetracyclic bridged heterocyclyl, and more preferably a bicyclic or tricyclic bridged heterocyclyl. Non-limiting examples of the bridged heterocyclyl include:

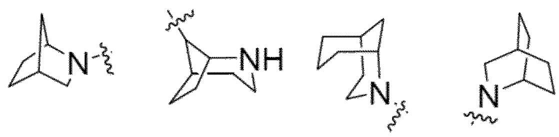

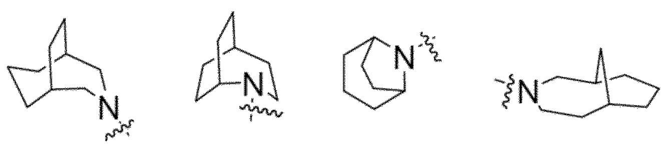

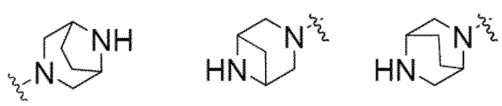

and

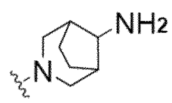

.

The heterocyclyl ring may be fused to an aryl, heteroaryl or cycloalkyl ring, in which the ring linking to the parent structure is heterocyclyl. Non-limiting examples thereof include:

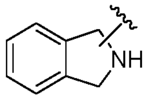 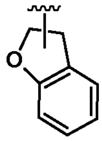 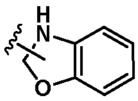 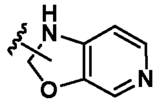 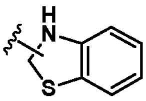

and the like.

The heterocyclyl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy or carboxylic ester group.

The term "aryl" refers to a 6 to 14 membered full-carbon monocyclic or fused polycyclic (i.e., rings sharing a pair of adjacent carbon atoms) group having a conjugated $\pi$-electron system, and preferably a 6 to 10 membered aryl, for example, phenyl and naphthyl. The aryl is more preferably phenyl. The aryl ring may be fused to a heteroaryl, heterocyclyl or cycloalkyl ring, in which the ring linking to the parent structure is an aryl ring. Non-limiting examples thereof include:

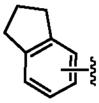 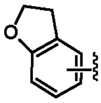 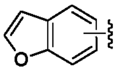 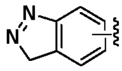 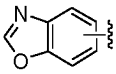 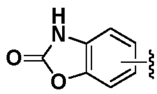 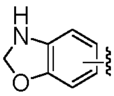 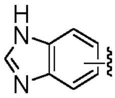 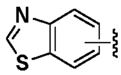

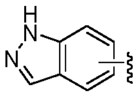 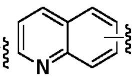 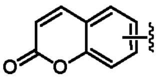 and 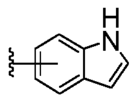 .

The aryl may be substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or carboxylic ester group.

The term "heteroaryl" refers to a heteroaromatic system having 5 to 14 ring atoms, which comprises 1 to 4 heteroatoms selected from the group consisting of O, S and N. The heteroaryl is preferably a 5 to 10 membered heteroaryl having 1 to 3 heteroatoms, and more preferably a 5 or 6 membered heteroaryl having 1 to 2 heteroatoms, for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring may be fused to a aryl, heterocyclyl or cycloalkyl ring, in which the ring linking to the parent structure is a heteroaryl ring. Non-limiting examples thereof include:

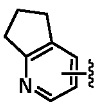 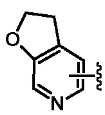 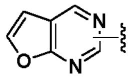 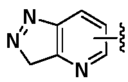 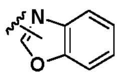 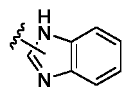 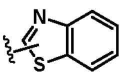 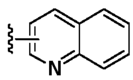

and

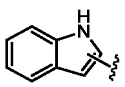 .

The heteroaryl may be optionally substituted or unsubstituted. When it is substituted, the substituent(s) may be one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or carboxylic ester group.

The term "alkoxy" refers to an -O-(alkyl) group, in which the alkyl is as described above. Non-limiting examples of the alkoxy include methoxy, ethoxy, propoxy, butoxy, cyclopropyloxy, cyclobutyloxy, cyclopentyloxy and cyclohexyloxy. The alkoxy may be optionally substituted or unsubstituted. When it is substituted, the substituent(s) may be one or more groups independently selected from the group consisting of alkyl, alkenyl, alkynyl, alkoxy, alkylthio, alkylamino, halogen,

thiol, hydroxy, nitro, cyano, cycloalkyl, heterocyclyl, aryl, heteroaryl, cycloalkoxy, heterocycloalkoxy, cycloalkylthio, heterocyclylthio, carboxy or carboxylic ester group.

The above alkenyl, alkynyl, cycloalkyl, heterocyclyl, aryl and heteroaryl comprise residues resulting from removing one hydrogen atom from the parent rings, or residues resulting from rremoving two hydrogen atoms from the same ring atom or two different ring atoms of the parent rings, i.e., "alkenylene", "alkynylene", "cycloalkylene", "heterocyclylene", "arylene" and "heteroarylene".

The term "cycloalkoxy" refers to an -O-(cycloalkyl) group, in which the cycloalkyl is as defined above.

The term "heterocycloalkoxy" refers to an -O-(heterocyclyl) group, in which the heterocyclyl is as defined above.

The term "haloalkyl" refers to an alkyl group substituted by one or more halogens, in which the alkyl is as defined above.

The term "haloalkoxy" refers to an alkoxy group substituted by one or more halogens, in which the alkoxy is as defined above.

The term "hydroxyalkyl" refers to an alkyl group substituted by a hydroxy group, in which the alkyl is as defined above.

The term "hydroxy" refers to an -OH group.

The term "halogen" refers to fluorine, chlorine, bromine or iodine.

The term "amino" refers to a $-NH_2$ group.

The term "cyano" refers to a -CN group.

The term "nitro" refers to a $-NO_2$ group.

The term "oxo" refers to an =O group.

The term "thioxo" refers to a =S group.

The term "carboxy" refers to a -C(O)OH group.

The term "thiol" refers to a -SH group.

The term "ester" refers to a -C(O)O(alkyl) or a -C(O)O(cycloalkyl) group, in which the alkyl and cycloalkyl are as defined above.

The term "acyl" refers to a compound with a -C(O)R group, wherein R is alkyl, cycloalkyl, heterocyclyl, aryl and heteroaryl.

The term "sulfonic group" refers to a $-SO_3H$ group.

The term "optional" or "optionally" means that the event or circumstance described subsequently can, but need not, occur, and such a description includes the situation in which the event or circumstance does or does not occur. For example, the expression "heterocyclyl optionally substituted by an alkyl group" means that an alkyl group can be, but need not be, present, and such a description includes the situation of the heterocyclyl being substituted by an alkyl group and the situation of the heterocyclyl being not substituted by an alkyl group.

The term "substituted" means that one or more hydrogen atoms in a group, preferably up to 5, and more preferably 1 to 3 hydrogen atoms in a group, are independently substituted by a corresponding number of substituents. It goes without saying that the substituents only exist in their possible chemical position. Those skilled in the art are able to determine whether the substitution is possible or impossible by experiments or theory without excessive efforts. For example, the combination of amino or hydroxy having free hydrogen and carbon atoms having unsaturated bond(s) (such as olefinic) may be unstable.

The term "pharmaceutical composition" represents a mixture containing one or more compounds described herein or physiologically/pharmaceutically acceptable salts or prodrugs thereof and other components such as physiologically/pharmaceutically acceptable carriers and excipients. The purpose of the pharmaceutical composition is to facilitate administration of a drug to an organism, which is conducive to absorption of an active ingredient(s) and thus exhibit biological activity.

The term "pharmaceutically acceptable salt" refers to a salt of the compound of the present invention, which is safe and effective in mammals and has desired biological activity.

The term "carrier" refers to a carrier or diluent which does not cause significant irritation to an organism and does not eliminate the biological activity and property of the compound administrated.

## DESCRIPTION OF THE DRAWINGS

Figure 1 shows the fluorescence microscopic imaging of the compounds of the invention in human bladder cancer cell line RT112.

Figure 2 shows the fluorescence microscopic imaging of the compounds of the invention in human bladder cancer cell line UM-UC-3.

Figure 3 shows the fluorescence microscopic imaging of the compounds of the invention in human bladder cancer cell

line RT112, with or without addition of BSP.

Figure 4 shows the fluorescence microscopic imaging of the compounds of the invention in human bladder cancer cell line UM-UC-3, with or without addition of BSP.

Figure 5 shows the fluorescence imaging of the compounds of the invention in the bladder cancer tumor model mice.

## DETAILED DESCRIPTION OF THE INVENTION

The compounds of the present invention and the preparation thereof are further understood through examples, which illustrate some methods of preparing or using the compounds. However, it is understood that the present invention is not limited to these examples. All currently known or further developed variations of the present invention are considered as falling within the scope of the present invention described and claimed herein.

The compounds of the present invention are prepared by using convenient starting materials and general preparation procedures. The present invention provides typical or preferential reaction conditions, such as reaction temperature, duration, solvent, pressure and molar ratio of reactants. However, unless specially specified, other reaction conditions can also be adopted. Optimal conditions may vary with the use of specific reactants or solvents, but under normal circumstances, the steps and conditions optimized for reaction can be determined.

In addition, some protecting groups may be used in the present invention to protect certain functional groups from unnecessary reactions. The protecting groups suitable for various functional groups and their protection or deprotection conditions are already well known to those skilled in the art. For example, "Protective Groups in Organic Synthesis" by T. W. Greene and G. M. Wuts (3rd edition, Wiley, New York, 1999) and the citations in the book describe the protection or deprotection of a large number of protecting groups in detail.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific methods used may be referred to the examples described in the present invention. Of course, other similar isolation and purification means can also be used. They can be characterized using conventional methods (including physical constants and spectral data).

The structures of the compounds are identified by nuclear magnetic resonance (NMR) and/or mass spectrometry (MS). NMR shift is given in units of $10^{-6}$ (ppm). NMR is determined on a Brukerdps 300 nuclear magnetic spectrometer. The solvents for determination are deuterated dimethyl sulfoxide (DMSO-$d_6$), deuterated chloroform ($CDCl_3$), and deuterated methanol ($CD_3OD$), and the internal standard is tetramethylsilane (TMS).

MS is determined on a LC (Agilent 1260 Infinity)/MS (G6125B) mass spectrometer (manufacturer: Agilent).

Preparative liquid chromatography is determined using a lc6000 high performance liquid chromatograph (manufacturer: Chuangxintongheng Science & Technology CO.,LTD.). The chromatographic column is Daisogel C18 10 $\mu$m 100A (30 mm$\times$250 mm), and the mobile phase is acetonitrile/water.

GF254 silica gel plates from Qingdao Haiyang Chemical are used for thin-layer chromatography (TLC). For the silica gel plates used, the specification is 0.20 mm to 0.25 mm for the thin-layer chromatography for monitoring of reactions, and 0.5 mm for the thin-layer chromatography for separation and purification.

Silica gels of 100 to 200 mesh, 200 to 300 mesh and 300 to 400 mesh from Qingdao Haiyang Chemical are used as a carrier for silica gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech, Nanjing PharmaBlock and the like.

Unless specifically stated in the examples, all reactions can be carried out under nitrogen atmosphere.

Argon or nitrogen atmosphere means that a reaction flask is connected to an argon or nitrogen balloon with a volume of about 1 L.

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP), pyridine and the like. Unless specifically stated in the examples, a solution refers to an aqueous solution.

The chemical reactions described in the present invention are generally carried out under normal pressure. The reaction time and conditions are, for example, between -78° C and 200° C at one atmosphere, and completed within about 1 to 24 hours. If the reaction is carried out overnight, the reaction time is generally 16 hours. Unless specifically stated in the examples, the reaction temperature is room temperature, which is 20°C to 30°C.

Reaction processes in the examples are monitored by thin-layer chromatography (TLC). The developing systems used for the reactions are: A: dichloromethane-methanol system, B: petroleum ether-ethyl acetate system,

and C: acetone, and the volume ratios of the solvents are adjusted depending on the polarity of the compounds.

The eluent systems of the column chromatography and the developing systems of the thin-layer chromatography used for purifying the compounds include: A: dichloromethane-methanol system, and B: petroleum ether-ethyl acetate system, and the volume ratios of the solvents are adjusted depending on the polarity of the compounds. A small amount of alkaline or acidic reagents such as triethylamine and trifluoroacetic acid can also be added for adjustment.

Unless otherwise defined, all professional and scientific terms used herein have the same meaning as those familiar to those skilled in the art. In addition, any methods and materials similar or equivalent to the content described herein can be applied to the methods of the present invention.

Preparation Example 1: Preparation of 2,3,3-trimethylindole-5-carboxylic acid (A8A)

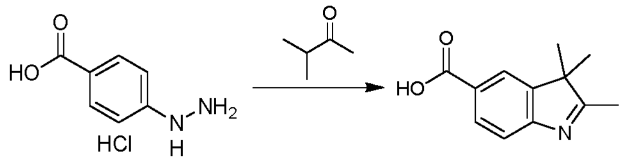

A8A

3-Methylbutan-2-one (25.1 g, 292 mmol, 31.2 mL, 1.10 eq) was added to a solution of 4-hydrazinobenzoic acid hydrochloride (50.0 g, 265 mmol, 1.00 eq) in AcOH (250 mL). The resulting mixture was kept at 120°C and stirred for 12 hours. The reaction solution was concentrated under reduced pressure, and the residues were diluted with DCM (200 mL) and adjusted to pH=7 with saturated $NaHCO_3$ solution. The organic phase was dried over anhydrous sodium sulfate, filtered, and concentrated under reduced pressure. The residues were purified by silica gel column chromatography ($SiO_2$, petroleum ether/ethyl acetate = 100/1 to 1/1) to obtain Compound A8A (25.0 g, 123 mmol, yield 46.4%) as a yellow solid.

$^1$H NMR (400 MHz, DMSO)δ 12.8 (s, 1H), 7.99 (s, 1H), 7.90-7.93 (m, 1H), 7.50 (d, *J* = 7.6 Hz, 1H), 2.25 (s, 3H), 1.27 (s, 6H).

LCMS: m/z = 204.0 $(M+H)^+$.

The following compounds were prepared according to the above preparation method using the corresponding substituted hydrazine:

| No. | Structure | Hydrazine | NMR or MS data, and appearance |
|---|---|---|---|
| A7A | 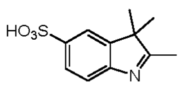 | 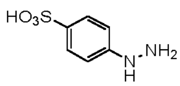 | $^1$H NMR (400MHz, DMSO) δ 7.74 (d, J = 1.2 Hz, 1H), 7.63 (dd, J1 = 8.0 Hz , J2 = 1.6 Hz, 1H), 7.43 (d, J = 8.0 Hz, 1H), 2.36 (s, 3H), 1.33 (s, 6H). Pinkish purple solid. |
| A10A | 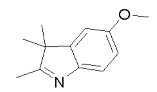 | 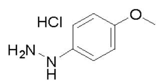 | $^1$H NMR (400MHz, $CDCl_3$) δ 7.43 (d, J = 9.2 Hz, 1H), 6.80 - 6.84 (m, 2H), 3.82 (s, 3H), 2.24 (s, 3H), 1.28 (s, 6H).<br>LCMS: m/z = 190.2 $(M+H)^+$, as a yellow oil. |
| A13B | 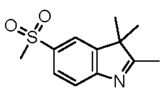 | 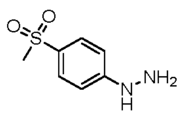 | LCMS: m/z = 238.2 $(M+H)^+$. |

Preparation Example 2: Preparation of tert-butyl 1-ethyl-2,3,3 trimethyl-indol-1-ium-5-carboxylate iodide (A8)

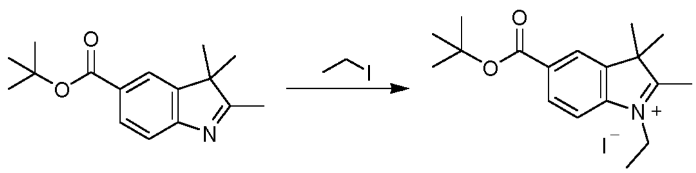

A8B A8

A mixture of Compound A8B (see Preparation Example 3 for the preparation method) (9.00 g, 34.7 mmol, 1.00 eq), iodoethane (10.8 g, 69.4 mmol, 5.55 mL, 2.00 eq) and MeCN (90.0 mL) was stirred at 100°C for 32 hours. The reaction mixture was concentrated under reduced pressure, and the residues were pulped with MTBE (30.0 mL) for 30 minutes at 25°C. Then, the resulting mixture was purified by reversed-phase chromatography (Phenomenex luna C18 250×50mm×15μm; mobile phase: [water (FA)-ACN]; B%: 50%-80%, 10 min) to obtain Compound A8 (10.0 g) as a red solid.

LCMS: m/z = 288.3 $(M+H)^+$.

The following compounds were prepared according to the above preparation method using the corresponding raw material:

| No. | Structure | Raw material | NMR or MS data, and appearance |
|---|---|---|---|
| A12 | 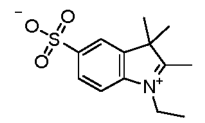 | 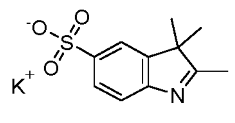 | LCMS: m/z = 268.2 $C_{13}H_{17}NO_3S$, as a yellow solid |
| A11A | 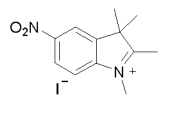 | 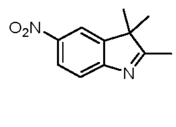 | $^1$H NMR: (400MHz, MeOD) δ 8.70 (s, 1H), 8.55 (d, J = 8. Hz, 1H), 8.11 (s, 1H), 4.14 (s, 3H), 1.70 (s, 9H).<br>LCMS: m/z = 219.2 $C_{12}H_{15}N_2O_2^+$.<br>Yellow solid. |
| A10 | 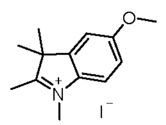 | 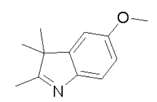 | LCMS: m/z = 204.2 $C_{13}H_{18}NO^+$. Gray solid. |
| A13 | 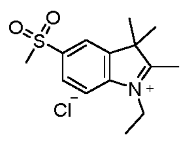 | 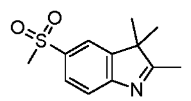 | $^1$H NMR: (400MHz, $CD_3CN$) δ 8.29 (s, 1H), 8.19 (dd, J1 = 1.6 Hz, J2 = 6.8 Hz, 1H), 8.04 (d, J = 8.8 Hz, 1H), 4.52 (dd, J1 = 7.6 Hz, J2 = 7.2 Hz, 1H), 3.17 (s, 3H), 2.84 (s, 3H), 1.62 (s, 6H), 1.52 (t, J = 7.6 Hz).<br>LCMS: m/z = 266.1 $C_{14}H_{20}NO_2S^+$. Yellow solid. |
| A9 | 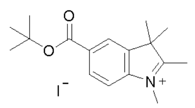 | 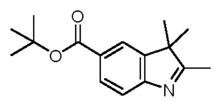 | LCMS: m/z = 274.1 $C_{17}H_{24}NO_2^+$. Red solid. |

Preparation Example 3 : Preparation of tert-butyl 2,3,3-trimethylindole-5-carboxylate (A8B)

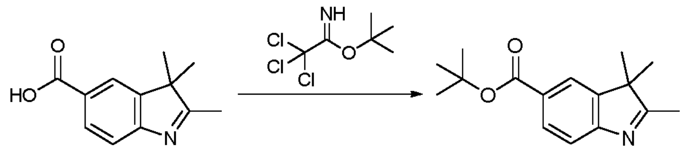

A8A A8B

$BF_3 \cdot Et_2O$ (4.75 g, 15.8 mmol, 4.12 mL, 47.0% purity, 0.400 eq) was added to a solution of 2,3,3-trimethylindole-5-carboxylic acid (8.00 g, 39.4 mmol, 1.00 eq) and tert-butyl trichloroacetimidate (17.2 g, 78.7 mmol, 14.1 mL, 2.00 eq) in DCM (80.0 mL) and THF (40.0 mL). The mixture was stirred for 12 hours at 25°C. The resulting mixture was kept around 0°C, and $NaHCO_3$ solution (50.0 mL) was added dropwise thereto. The resulting mixture was diluted with $H_2O$ (100 mL), extracted with EA (200 mL×2), washed with brine (50.0 mL×2), dried over anhydrous $Na_2SO_4$, filtered and concentrated. The residues were pulped with PE (50.0 mL) for 30 minutes at 25°C to obtain Compound A8B(9.00 g, crude) as a yellow oil.

LCMS: m/z = 260.4 $(M+H)^+$.

Preparation Example 4: Preparation of 5-amino-1,2,3,3-tetramethyl-3H-indol-1-ium iodide (A11B)

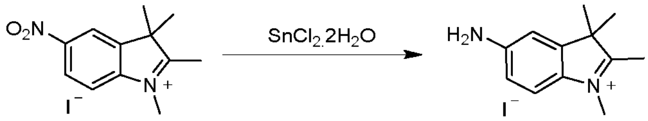


A11A A11B

$SnCl_2 \cdot 2H_2O$ (57.0 g, 253 mmol, 7.00 *eq*) was added to a solution of Compound A11A (12.5 g, 36.1 mmol, 1.00 *eq*) in HCl (60.0 mL). The mixture was stirred for 16 hours at 20°C. The resulting mixture was diluted with 300 mL of water, adjusted to pH = 10 with 5 M NaOH, and filtered. The parent solution was extracted with DCM (600 mL). The organic phase was washed with 300 mL of brine, dried over anhydrous $Na_2SO_4$ and filtered. The aqueous phase was lyophilized before addition of DCM (200 mL), stirred and filtered. The organic phases were combined, dried over anhydrous $Na_2SO_4$, filtered, and concentrated to obtain Compound **A11B** (7.05 g) as a green solid.

$^1H$ NMR (400MHz, $CDCl_3$)δ 6.57 (s, 1H), 6.52 (d, *J* = 7.2 Hz, 1H), 6.37 (s, 1H), 3.82 (s, 2H), 3.17 (s, 3H), 1.33 (s, 9H).

LCMS: m/z = 189.3, $C_{12}H_{17}N_2^+$.

Preparation Example 5 : Preparation of 5-(dimethylamino)-1,2,3,3-tetramethyl-3H-indol-1-ium iodide (A11)

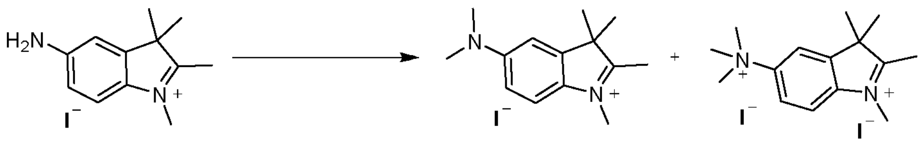

A11B A11

A mixture of Compound **A11B** (4.00 g, 12.7 mmol, 1.00 eq), MeI (8.98 g, 63.3 mmol, 3.94 mL, 5.00 eq) and ACN (20.0 mL) in a sealing tube was kept at 80°C under 15 Psi for 12 hours. The resulting mixture was concentrated under reduced pressure, and the residues were purified by reversed-phase chromatography (column: Phenomenex luna C18 (250mm×70mm, 10 μm); mobile phase: [water(HCl)-ACN]; gradient: 1%-35% B, 22 min) to obtain Compound **A11** (1.40 g, 3.97 mmol, 15.7% yield, 97.7% purity) as a yellow solid.

$^1H$ NMR (400MHz, DMSO-$_{d6}$)6 7.68 (d, J = 8.8 Hz, 1H), 7.18 (s, 1H), 6.88 (d, J = 8.4 Hz, 1H), 3.90 (s, 3H), 2.65 (s, 3H), 1.47 (s, 6H).

LCMS: m/z = 217.1, $C_{14}H_{21}N_2^+$.

In addition, trimethyl-(1,2,3,3-tetramethylindol-1-ium-5-yl)amine diiodide (720 mg, 1.38 mmol, 10.93% yield, 93.4% purity) was obtained as a yellow solid. LCMS: m/z = 231.0, $C_{15}H_{24}N_2^{2+}$.

Preparation Example 6: Preparation of (4-methylsulfonylphenyl)hydrazine (A13A)

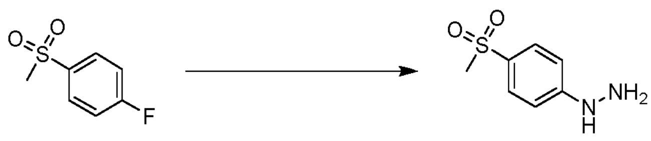

A13A

A solution of 1-fluoro-4-methylsulfonylbenzene(20.0 g, 115 mmol, 1.00 *eq*), hydrazine hydrate (13.0 g, 221 mmol, 12.6 mL, 85.0% purity, 1.92 *eq*) and EtOH (200 mL) was stirred for 16 hours at 80°C. The resulting mixture was concentrated under reduced pressure, and the residues were purified by column chromatography ($SiO_2$, PE: EA = 1: 0 to 1: 1) to obtain Compound A13A (7.80 g, 41.9 mmol, 36.5 yield ) as a yellow solid.

$^1H$ NMR (400MHz, $CDCl_3$) δ 7.74 (s, 1H), 7.71 (s, 1H), 6.90 (s, 1H), 6.87 (s, 1H), 5.74 (s, 1H), 3.70 (s, 2H), 3.01 (s, 3H).

Preparation Example 7: Preparation of (E)-2-chloro-5-(dimethylamino)-3-(hydroxymethylene)cyclohex-1-ene-1-carbaldehyde (B4)

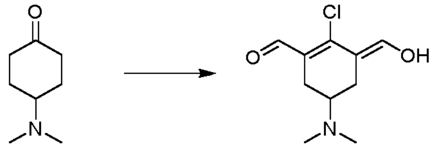

B4

$POCl_3$ (16.3 g, 106 mmol, 9.90 mL, 3.00 eq) was added to DMF (10.0 mL) at 0°C. The mixture was kept at 0 to 5°C and stirred for 30 minutes. 4-(Dimethylamino)cyclohexanone (5.00 g, 35.4 mmol, 1.00 eq) was added to the solution, heated to 80°C and stirred for 3 hours. The reaction mixture was added to HCl (1.00 M, 100 mL), stirred for 12 hours at 20°C. The mixture was filtered, and the filter cake was dried to obtain Compound B4 (3.90 g, crude) as a yellow solid.

$^1H$ NMR (400 MHz, DMSO) δ 8.89 (s, 2H), 3.40-3.44 (m, 1H), 3.02-3.07 (m, 2H), 2.76 (s, 6H), 2.36-2.40 (m, 2H).

LCMS: m/z = 216.3 $(M+H)^+$.

Preparation Example 8: Preparation of phenyl 3-[2-(2,3,3-trimethylindolin-1-ium-1-yl)ethoxy]propionate bromide (C4)

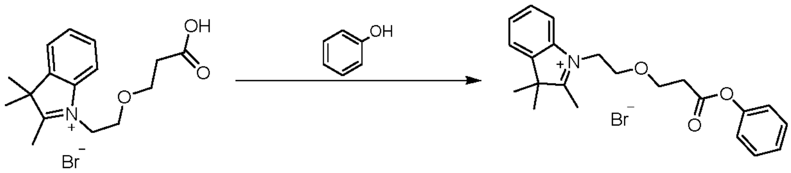

C4A C4

HBTU (7.41 g, 19.5 mmol, 1.20 eq) and DIEA (5.26 g, 40.7 mmol, 7.09 mL, 2.50 eq) was added to a solution of 3-[2-(2,3,3-trimethylindolin-1-ium-1-yl)ethoxy]propionic acid bromide (C4A) (see Preparation Example 13 for the preparation method) (5.80 g, 16.3 mmol, 1.00 eq) in DMF (50.0 mL). The mixture was stirred for 30 minutes at 25°C. Then, phenol (1.69 g, 17.9 mmol, 1.57 mL, 1.10 eq) was added, and stirred for 12 hours at 25°C. $H_2O$ (300 mL) was added to the reaction mixture, and extracted with EA (200 mL × 3). The organic phase was dried over $Na_2SO_4$ and concentrated. The residues were purified by reversed-phase HPLC (0.1% HCl condition; column: Phenomenex luna C18 250mm×50mm×15μm; mobile phase: [water(FA)-ACN]; B%: 50%-80%, 10min), and then lyophilized to obtain Compound C4 (3.10 g) as a brown oil.

LCMS: m/z = 352.2, $C_{22}H_{26}NO_3^+$.

Preparation Example 9: Preparation of 1-(6-(2,6-dimethylphenoxy)-6-oxohexyl)-2,3,3-trimethyl-3H-indol-1-ium chloride (C11)

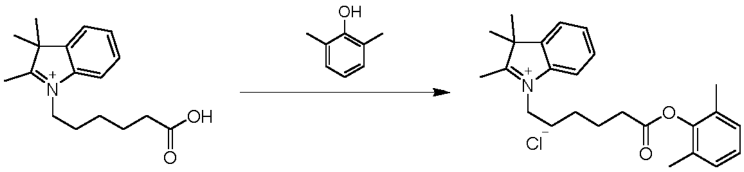

C11

Compound C11 was prepared according to the method of Preparation Example 8, except that 1-(5-carboxypentyl)-2,3,3-trimethyl-3H-indol-1-ium was used instead of C4A, and 2,6-dimethylphenol was used instead of phenol.

Preparation Example 10: Preparation of 2,3,3-trimethyl-1-(5-(trimethylammonium)pentyl)-3H-indol-1-ium bromide (C14)

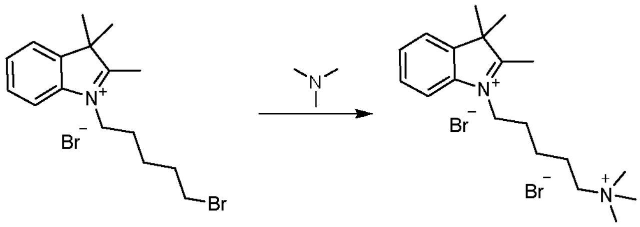

C14A C14

A mixture of 1-(5-bromopentyl)-2,3,3-trimethyl-indol-1-ium bromide (C14A) (see Preparation Example 22 for the preparation method in) (8.00 g, 20.6 mmol, 1.00 eq), N,N-dimethylcarbinamine (14.7 g, 82.2 mmol, 17.2 mL, 4.00 eq) and MeCN (80.0 mL) was stirred for 12 hours at 25°C. The resulting mixture was concentrated under reduced pressure, and purified by reversed-phase HPLC (column: Phenomenex luna, C18 150mm×40 mm×15 μm; mobile phase: [water(HCl)-ACN]; B%: 30%-60%, 10 min) to obtain Compound C14 (2.30 g, 5.13 mmol, 24.7% yield) as a pink oil.

$^{1}H$ NMR: (400 MHz, DMSO)δ 8.01-8.03 (m, 1H), 7.85-7.87 (m, 1H), 7.62-7.65 (m, 2H), 4.51 (t, J = 7.6 Hz, 2H), 3.30-3.34 (m, 2H), 3.07 (s, 9H), 2.88 (s, 3H), 1.86-1.94 (m, 2H), 1.72-1.80 (m, 2H), 1.55 (s, 6H), 1.39-1.47 (m, 2H).

LCMS: m/z = 144.3, $C_{19}H_{32}N_2^{2+}/2$.

Preparation Example 11: Preparation of 2,3,3-trimethyl-1-(4-(trimethylammonium)butyl)-3H-indol-1-ium chloride (C15)

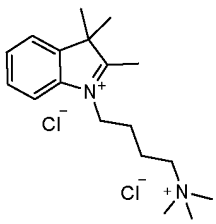

C15

Compound C15 was prepared by the same method as Preparation Example 10, except that Compound C15A was used instead of C14A.

LCMS: m/z = 273.3, $C_{18}H_{30}N_{22}^{+}$.

Preparation Example 12: Preparation of 5-methoxy-2,3,3-trimethyl-1-(6-oxo-6-phenoxyhexyl)-3H-indol-1-ium bromide (C21B)

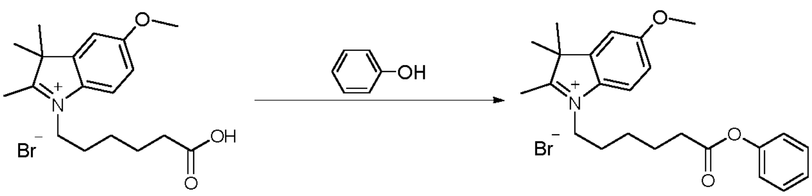

C21 C21B

Compound C21B was prepared according to the method of Preparation Example 8, except that Compound C21 was used instead of C4A.

LCMS: m/z = 380.3, $C_{24}H_{30}NO_3^{+}$.

Preparation Example 13: Preparation of Compound C4A

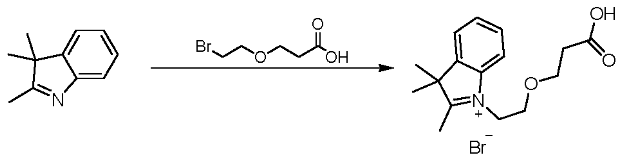

C4A

LCMS: m/z = 276.2, $C_{16}H_{22}NO_3^+$. Yellow oil.

Preparation Example 14: Preparation of Compound A7

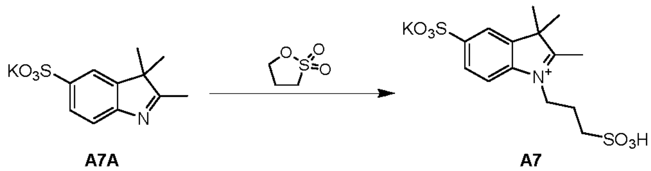

A7A A7

$^1$H NMR: (400MHz, $D_2O$)δ 8.11 (s, 1H), 8.02 (d, J = 8.4 Hz, 1H), 7.92 (d, J = 8.4 Hz, 1H), 4.65 (d, J = 16.4 Hz, 2H), 3.31 (s, 3H), 2.38-2.34 (m, 2H), 2.04 (s, 1H), 1.59 (s, 6H).

LCMS: m/z = 362. Purple solid.

Preparation Example 15: Preparation of Compound C10

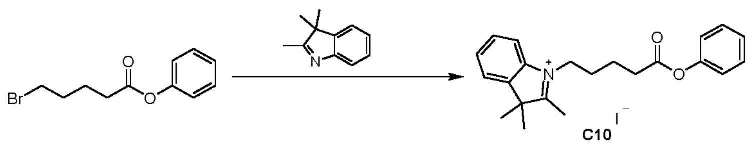

C10

LCMS: m/z = 336.2, $C_{22}H_{26}NO_2^+$. Purple solid.

Preparation Example 16: Preparation of Compound C7

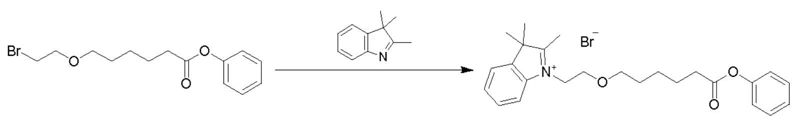

C7

LCMS: m/z = 394.3, $C_{25}H_{32}NO_3^+$. Red oil.

Preparation Example 17: Preparation of Compound C8

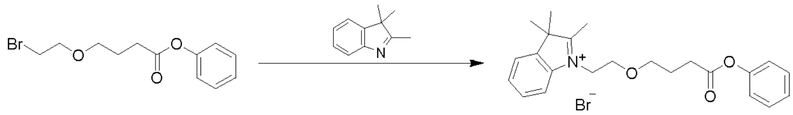

C8

LCMS: m/z = 366.2, $C_{23}H_{28}NO_3^+$. Yellow oil.

Preparation Example 18: Preparation of Compound C9

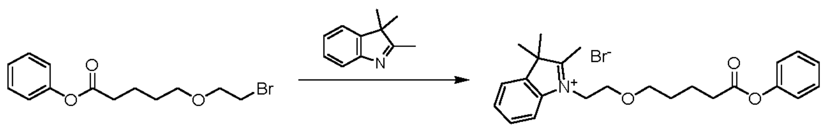

C9

LCMS: m/z = 380.2, $C_{24}H_{30}NO_3^+$. Yellow oil.

Preparation Example 19: Preparation of Compound C21

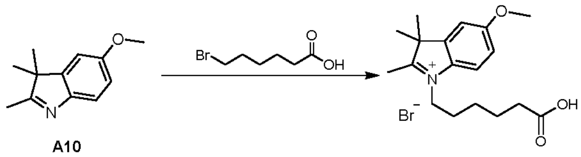

A10

C21

LCMS: m/z = 304.2, $C_{18}H_{26}NO_3^+$. Green solid.

Preparation Example 20: Preparation of Compound C13

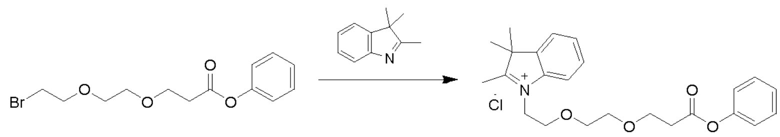

C13

LCMS: m/z = 396.3, $C_{24}H_{30}NO_4^+$. Yellow oil.

Preparation Example 21: Preparation of Compound C12

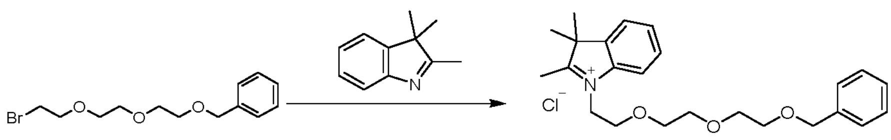

C12

$^1$H NMR (400MHz, $CDCl_3$) δ 7.66 - 7.58 (m, 1H), 7.57 - 7.56 (m, 2H), 7.55 - 7.54 (m, 1H), 7.37 - 7.32 (m, 5H), 5.83 - 5.57 (m, 4H), 4.80 (t, J = 4.80 Hz, 2H), 4.51 (s, 2H), 3.97 (d, J = 4.40 Hz, 2H), 3.56 - 3.54 (m, 2H), 3.51 - 3.50 (m, 2H), 2.74 (s, 3H), 1.47 (s, 6H).

LCMS: m/z = 382.2, $C_{24}H_{32}NO_3^+$. Brownish black solid.

Preparation Example 22: Preparation of Compound C14A

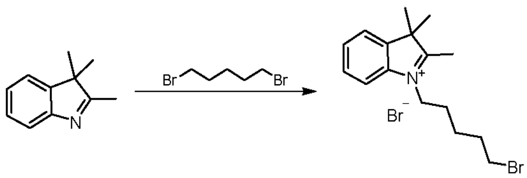

C14A

LCMS: m/z = 310.2, $C_{16}H_{23}BrN^+$. Red solid.

Preparation Example 23: Preparation of Compound C15A

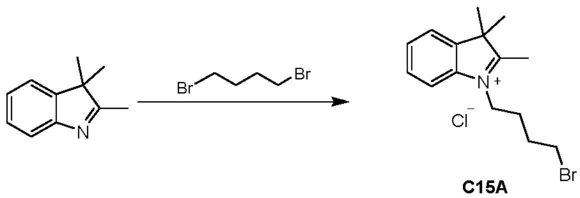

C15A

LCMS: m/z = 296.1, $C_{15}H_{21}BrN^+$. Brown oil.

Preparation Example 24: Preparation of Compound C16

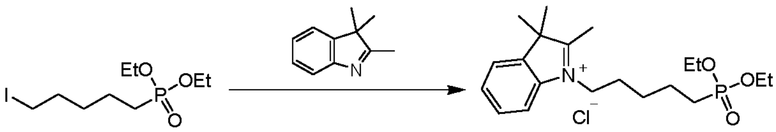

C16

$^1$H NMR (400MHz, $CDCl_3$) $\delta$ 7.55-7.68 (m, 4H), 4.85(t, J = 6.8 Hz, 2H), 4.04-4.10 (m, 4H), 3.18 (s, 3H), 1.98-2.00 (m, 3H), 1.64-1.78 (m, 11H), 1.30 (t, J = 7.2 Hz, 6H).

LCMS: m/z = 366.3, $C_{20}H_{33}NO_3P^+$. Brown oil.

Preparation Example 25: Preparation of Compound C17

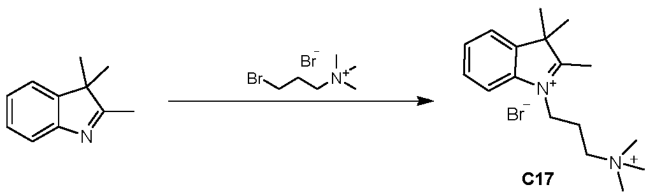

C17

LCMS: m/z = 259.3, $C_{17}H_{28}N_{22}^+$. Yellow solid.

Preparation Example 26: Preparation of Compound C18

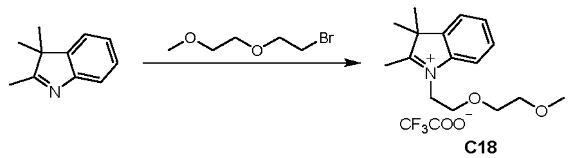

C18

LCMS: m/z = 262.2, $C_{16}H_{24}NO_2^+$. Green solid.

Preparation Example 27: Preparation of Compound C19

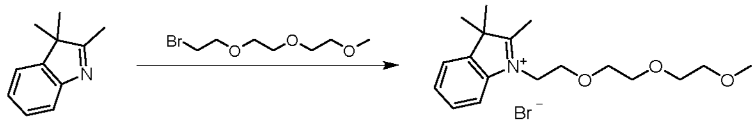

C19

$^1$H NMR (400 MHz, $CDCl_3$)δ 7.62-7.64 (m, 1H), 7.49-7.53 (m, 3H), 4.76 (s, 2H), 3.95 (t, J = 5.2 Hz, 2H), 3.55-3.57 (m, 2H), 3.48-3.52 (m, 4H), 3.42-3.44 (m, 2H), 3.32 (s, 3H), 2.00 (s, 6H), 1.55 (s, 3H).

LCMS: m/z = 306.3, $C_{18}H_{28}NO_3^+$. Red oil.

Preparation Example 28: Preparation of Compound C20

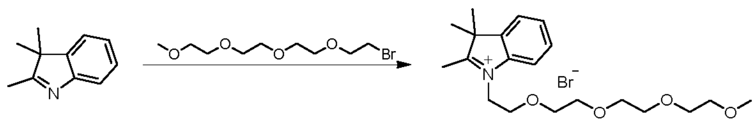

C20

LCMS: m/z = 350.3, $C_{20}H_{32}NO_4^+$. Yellow oil.

Preparation Example 29: Preparation of Compound C23

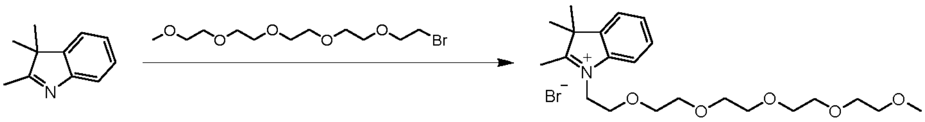

C23

LCMS: m/z = 394.2, $C_{22}H_{36}NO_5^+$. Brown oil.

Example 1: Preparation of 2-((E)-2-((E)-2-chloro-3-((E)-2-(1,1-dimethyl-3-(6-oxo-6-phenoxyhexyl)-1,3-dihydro-2 H-benzo[e]indol-2-ylidene)ethenyl)cyclopent-1-en-1-yl)ethenyl)-3-ethyl-1,1-dimethyl-1 H-benzo[e]indol-3-ium chloride (1A) and 2-((E)-2-((E)-3-((E)-2-(3-(5-carboxypentyl)-1,1-dimethyl-1,3-dihydro-2H-benzo[e]indo 1-2-ylidene)ethenyl)-2-chlorocyclopent-1-en-1-yl)ethenyl)-3-ethyl-1,1-dimethyl-1H-ben zo[e]indol-3-ium chloride (1B)

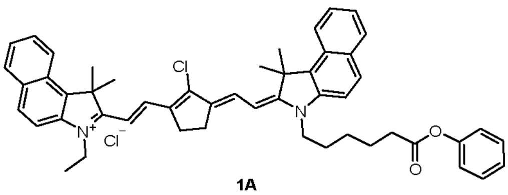

1A

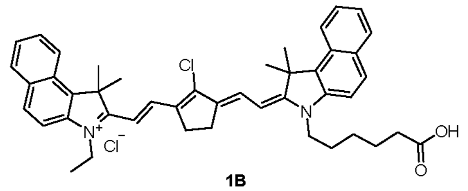

1B

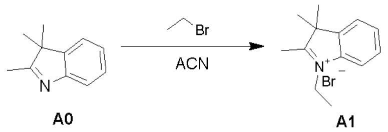


A0 A1

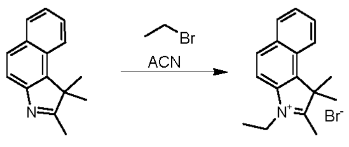


A2

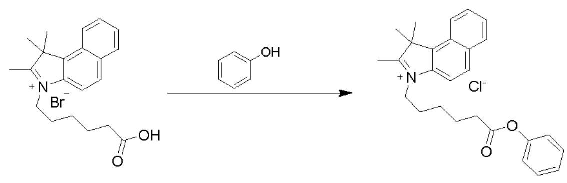

C2

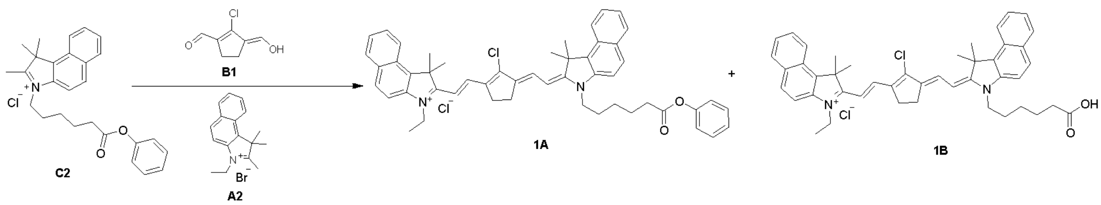

C2 B1 A2 1A + 1B

Step 1: Preparation of 1-ethyl-2,3,3-trimethyl-3H-indol-1-ium bromide (A1)

A solution of 2,3,3-trimethylindole (**A0**) (10.0 g, 62.8 mmol, 1.00 eq), bromoethane (10.3 g, 94.2 mmol, 7.03 mL, 1.50 eq) and acetonitrile (80.0 mL) was heated to 85°C and stirred for 24 hours. The reaction mixture was concentrated, and pulped with MTBE (60.0 mL) to obtain Compound **A1** (12.7 g, 47.4 mmol, yield 75.4%) as a brown solid.

$^1H$ NMR (400MHz, $CDCl_3$) $\delta$ 7.99 - 8.01 (m, 1H), 7.85 - 7.87 (m, 1H), 7.61 - 7.64 (m, 2H), 4.49 - 4.55 (m, 2H), 2.86 (s, 3H), 1.54 (s, 6H), 1.45 (t, $J$ = 7.4Hz, 3H).

LCMS: m/z = 188.1 (positive ion) .

Step 2: Preparation of 3-ethyl-1,1,2-trimethyl-1H-benzo[e]indol-3-ium bromide (**A2**)

1,1,2-Trimethylbenzo[e]indole (20.0 g, 95.5 mmol, 1.00 eq) and bromoethane (15.6 g, 143 mmol, 10.7 mL, 1.50 eq) were mixed in ACN (200 mL), evacuated and purged with $N_2$ three times. The mixture was then heated for 36 hours at 85°C. The resulting mixture was detected by LCMS, showing a target product generated with a yield of about 56%. The reaction mixture was concentrated, pulped with MTBE/DCM= 6:1 (300 mL/50.0 mL) for 12 hours at 25°C, and filtered to obtain a filter cake, i.e., Compound A2 (16.0 g, 47.9 mmol, 50.2% yield, 95.4% purity) as a product.

Step 3: Preparation of phenyl 6-(1,1,2-trimethylbenzo[e]indol-3-ium-3-yl)n-hexanoate chloride (**C2**)

6-(1,1,2-Trimethylbenzo[e]indol-3-ium-3-yl)n-hexanoic acid bromide (5.00 g, 15.4 mmol, 1.00 eq) was dissolved in a mixed solvent of DMF (15.0 mL) and DCM (20.0 mL), and EDCI (7.01 g, 18.4 mmol, 1.20 eq) and DIEA (4.98 g, 38.5 mmol, 6.71 mL, 2.50 eq) were added. The resulting mixture was stirred for 0.5 hour at 25°C, then phenol (1.60 g, 16.9 mmol, 1.49 mL, 1.10 eq) was added, and the reaction was continued for 12 hours at this temperature. The detection

showed 6-(1,1,2-trimethylbenzo[e]indol-3-ium-3-yl)n-hexanoic acid bromide was consumed completely. The reaction solution was concentrated, separated and purified by reversed-phase HPLC (0.1% HCl). Compound C2 (2.40 g, 5.99 mmol, 38.9% yield) was obtained as a brown solid after lyophilization.

LCMS: m/z = 400.2, $C_{27}H_{30}NO_2^+$.

Step 4: Preparation of Compound 1B

A solution of Compound A2(1.00 g, 3.15 mmol, 1.00 eq), Compound C2 (1.89 g, 4.72 mmol, 1.50 eq) and acetic anhydride (30.0 mL) was stirred for 1 hour at 70°C, and NaOAc (776 mg, 9.45 mmol, 3.00 eq) was added. Then (E)-2-chloro-3-(hydroxymethylene)cyclopent-1-en-1-carbaldehyde (Compound **B1**) (500 mg, 3.15 mmol, 1.00 eq) was added in portions. After stirring for 1 hour at 80°C, the solvent was removed through concentration. The residues were separated and purified by reversed-phase chromatography (0.1% HCl condition) to obtain Compound **1A** (53.7 mg, 68.4 μmol, 2.17% yield, 96.8% purity) as a brown solid, and Compound **1B** (4.90 mg, 6.80 μmol, 0.22% yield, 95.0% purity) as a white solid.

Compound 1A:

$^1H$ NMR (400MHz, $CDCl_3$) δ 8.11 - 8.15 (m, 2H), 7.93 - 7.98 (m, 5H), 7.60 - 7.62 (m, 2H), 7.44 - 7.47 (m, 4H), 7.28 - 7.32 (m, 3H), 7.19 - 7.20 (m, 1H), 6.97 - 6.99 (m, 2H), 6.25 - 6.30 (m, 2H), 4.41 - 4.49 (m, 4H), 3.11 - 3.16 (m, 4H), 2.61- 2.65 (m, 2H), 2.01 (d, J = 4.8Hz, 12H), 1.85 - 1.87 (m, 2H), 1.67 - 1.69 (m, 3H), 1.51 - 1.55 (m, 4H).

LCMS: m/z = 759.6 $(M+H)^+$.

Compound 1B:

LCMS: m/z = 683.5 $(M+H)^+$.

Example 2: Preparation of phenyl 6-[(2E)-2-[(2E)-2-[2-chloro-3-[(E)-2-(3-ethyl-1,1-dimethyl-benzo[e]indol-3-ium-2-yl)ethenyl]cyclohex-2-en-1-yl]ethylidene]-1,1-dimethyl-benzo[e]indol-3-yl]n-hexanoate (**2**)

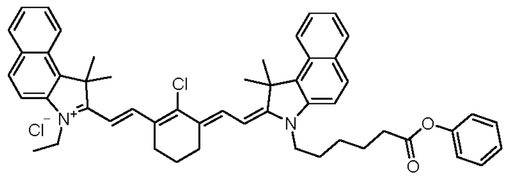

2

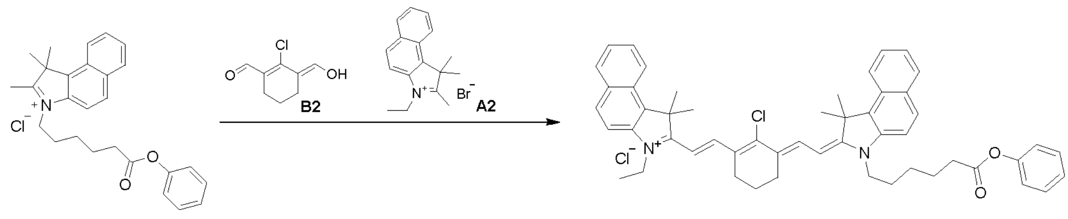


C2 2

To a solution of Compound **A2**(1.01 g, 3.16 mmol, 1.00 eq) in $Ac_2O$ (12.0 mL) was added a solution of Compound **C2** (1.90 g, 4.74 mmol, 1.50 eq) in $Ac_2O$ (12.0 mL). After keeping the temperature at 70°C for 3 hours, the reaction solution was concentrated. The residues were dissolved in EtOH (40.0 mL) and heated to 80°C. Compound **B2** (purchased from WuXi AppTec, 545 mg, 3.16 mmol, 1 eq) and NaOAc (778 mg, 9.49 mmol, 3.00 eq) were added, and stirring was continued for 2 hours at 80°C. After the reaction solution was concentrated, the residues were purified by preparative chromatography (chromatographic column: YMC Triart C18 150mm×25mm×5μm; mobile phase[water(HCl)-ACN]; B%: 76%-100%,10min), and lyophilized to obtain Compound **2** (52.47 mg, 65.9 μmol, 34.0% yield, 97.3% purity) as a green solid.

$^1H$ NMR: (400MHz, $CDCl_3$)δ 8.47 - 8.41 (m, 1H), 8.13 (d, J = 8.0 Hz, 2H), 7.98 - 7.94 (m, 4H), 7.62 (d, J = 7.2 Hz, 2H), 7.53 - 7.46 (m, 3H), 7.31 - 7.27 (m, 2H), 7.20 - 7.15 (m, 1H), 6.98 - 6.95 (m, 2H), 6.41 - 6.22 (m, 1H), 4.41 (s, 3H), 2.95 -

2.57 (m, 5H), 2.02 - 1.96 (m, 20H), 1.89 - 1.80 (m, 3H), 1.70 - 1.62 (m, 2H), 1.53 (t, J = 5.1 Hz, 3H).

Example 3: Preparation of 2-((E)-2-((E)-2-chloro-3-((E)-2-(1,1-dimethyl-3-(4-(phenoxycarbonyl)phenyl)-1,3-dihy dro-2H-benzo[e]indol-2-ylidene)ethenyl)cyclohex-1-en-1-yl)ethenyl)-3-ethyl-1,1-dimet hyl-1H-benzo[e]indol-3-ium chloride (**3**)

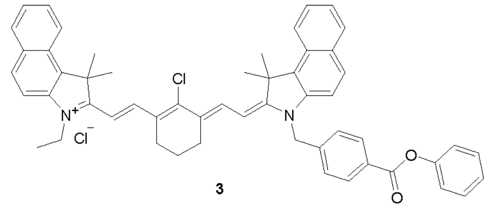

3

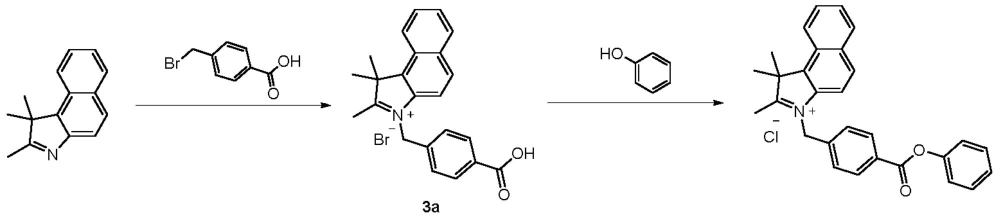

3a

C3

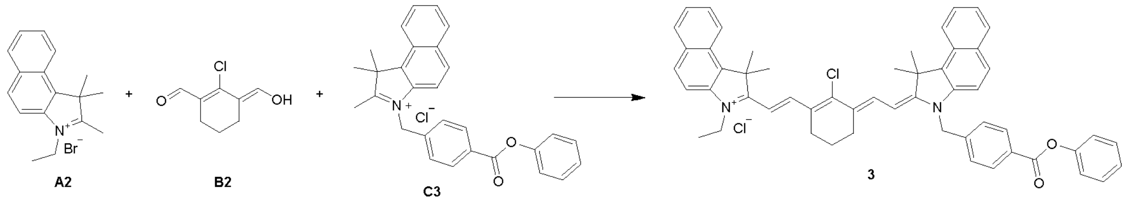

A2 B2 C3 3

Step 1: Preparation of 3-(4-carboxybromo)-1,1,2-trimethyl-1H-benzo[e]indol-3-ium bromide (**3a**)

To a solution of 1,1,2-trimethylbenzo[e]indole (3.75 g, 17.90 mmol, 1.00 eq) in ACN (50.0 mL) was added 4-(bromomethyl)benzoic acid (3.85 g, 17.9 mmol, 1.00 eq). The mixture was stirred for 12 hours at 85°C. The reaction solution was concentrated under reduced pressure, and the residues were pulped with MTBE/DCM=1:2 (210 mL/30 mL) for 30 minutes at 25°C, and filtered to obtain a filter cake, i.e., Compound 3a (6.50 g, 15.3 mmol, 85.5% yield ) as a product, which was a brown solid.

$^1$H NMR: (400MHz, $CDCl_3$)$\delta$ 8.41 (d, J = 8.4 Hz, 1H), 8.22 - 8.16 (m, 2H), 7.97 - 7.91 (m, 3H), 7.82 - 7.73 (m, 2H), 4.22 - 4.16 (m, 4H), 7.57 - 7.52 (m, 2H), 6.06 (s, 2H), 3.07 (s, 3H), 1.85 (s, 6H).

Step 2: Preparation of 1,1,2-trimethyl-3-(4-(phenoxycarbonyl)bromo)-1H-benzo[e]indol-3-ium chloride (C**3**)

Compound **3a** (4.50 g, 10.6 mmol, 1.00 eq) was dissolved in DMF (10.0 mL) and DCM (60.0 mL), and HBTU (4.83 g, 12.7 mmol, 1.20 eq) and DIEA (3.43 g, 26.5 mmol, 4.62 mL, 2.50 eq) were added. The mixed solution was stirred for 0.5 hour at 25°C, and then phenol (1.10 g, 11.6 mmol, 1.03 mL, 1.10 eq) was added. The mixture was then stirred for 12 hours at 25°C. The reaction solution was concentrated, and the residues were purified by reversed-phase chromatography (0.1%HCl condition) to obtain Compound C3 (1.7 g, 4.04 mmol, 38.12% yield ) as a brown solid.

LCMS: m/z = 420.2 $C_{29}H_{26}ClNO_2^-$.

Step 3: Preparation of Compound **3**

A mixture of Compound **A2** (353 mg, 1.11 mmol, 1.00 *eq*), 1,1,2-trimethyl-3-(4-(phenoxycarbonyl)bromo)-1H-benzo[e]indol-3-ium chloride (C3) (0.700 g, 1.66 mmol, 1.50 *eq*) and $Ac_2O$ (14.0 mL) was stirred for 1 hour at 70°C, and NaOAc (273 mg, 3.33 mmol, 3.00 *eq*) and Compound **B2** (192 mg, 1.11 mmol, 1.00 *eq*) were added. The reaction solution was heated to 80°C and stirred for 1 hour. The reaction mixture was concentrated, and the residues were purified by

preparative chromatography (HCl condition) to obtain Compound **3** (104.57 mg, 126 μmol, 11.4% yield, 95.7% purity) as a green solid.

$^1$H NMR (400MHz, $CDCl_3$)δ 8.51 - 8.55 (m, 1H), 8.29 - 8.32 (m, 1H), 8.14 -8.22 (m, 4H), 7.89 - 8.02 (m, 2H), 7.40 - 7.67 (m, 2H), 7.40 -7.58 (m, 9H), 7.31 -7.34 (m, 1H), 7.19 -7.21 (m, 2H), 6.50 -6.57 (m, 1H), 6.15 -6.18 (m, 1H), 5.65 (s, 2H), 4.58 -4.60 (m, 2H), 2.80 (s, 2H), 2.63 (s, 2H), 2.10 (s, 6H), 2.03 (s, 6H), 2.00 -2.01 (m, 2H), 1.50 -1.60 (m, 3H).

LCMS: m/z = 793.3.

Example 4: Preparation of 2-((E)-2-((E)-2-chloro-3-(2-((E)-3,3-dimethyl-1-(6-oxo-6-phenoxyhexyl)indolin-2-ylide ne) ethenyl)cyclopent-1-en-1-yl)ethenyl)-1-ethyl-3,3-dimethyl-3H-indol-1-ium chloride (**4**)

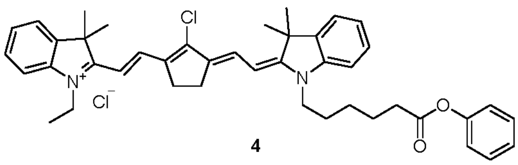

**4**

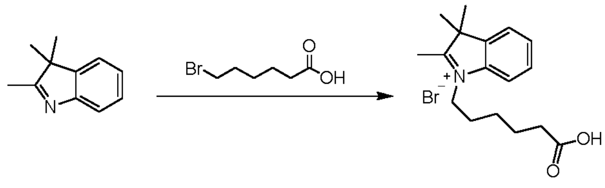

**C0**

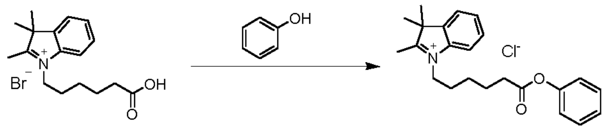

**C0** **C1**

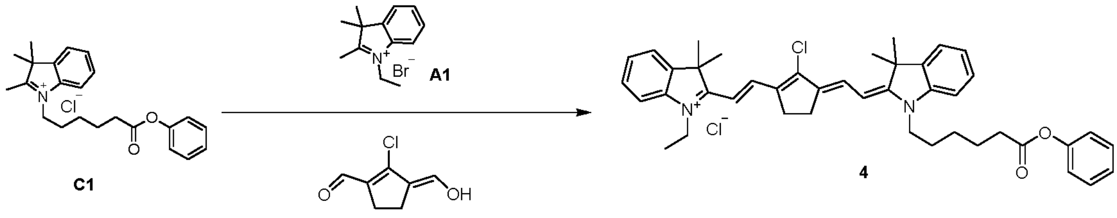

**C1** **A1** **4** **B1**

Step 1: Preparation of 1-(5-carboxypentyl)-2,3,3-trimethyl-3H-indol-1-ium bromide (**C0**)

A solution of 2,3,3-trimethylindole (5 g, 31.40 mmol, 1 eq) and 6-bromohexanoic acid (12.25 g, 62.80 mmol, 2 eq) in ACN (40 mL) was stirred for 24 hours at 85°C. The reaction solution was concentrated under reduced pressure to remove the solvent to give a crude product. Then, the crude product was pulped with DCM:MTBE=1:1 (60 mL) for 30 minutes at 0°C to obtain Compound C0 (6.9 g, 19.48 mmol, 62.02% yield) as a pink solid.

$^1$H NMR: (400MHz, $CDCl_3$)δ 7.99 -7.97 (m, 1H), 7.86 - 7.83 (m, 1H), 7.63 - 7.61 (m, 2H), 4.48 - 4.44 (m, 2H), 2.85 (s, , 3H), 2.24-2.20 (t, J = 7.2 Hz, 2H), 1.86 - 1.80 (m, 2H), 1.57 - 1.54 (m, 8H), 1.44 - 1.41 (m, 2H).

Step 2: Preparation of 2,3,3-trimethyl-1-(6-oxo-6-phenoxyhexyl)-3H-indol-1-ium chloride (**C1**)

Compound **C0** (4.00 g, 11.3 mmol, 1.00 eq) was mixed in DCM (50.0 mL), and then HBTU (5.14 g, 13.5 mmol, 1.20 eq) and DIEA (3.65 g, 28.2 mmol, 4.92 mL, 2.50 eq) were added. The resulting mixture was stirred for 0.5 hour at 25°C, then phenol (1.06 g, 11.3 mmol, 993 μL, 1.00 eq) was added, and the reaction was continued for 12 hours at 25°C. The resulting mixture was purified by reversed-phase HPLC (0.1% HCl condition), and lyophilized to obtain Compound **C1** (2.00 g, 4.53 mmol, 40.1% yield, 79.3% purity) as a product, which was a brown solid.

LCMS: m/z = 350.1, $C_{23}H_{28}NO_2^+$.

Step 3: Preparation of Compound **4**

After a solution of Compound **C1**(1.32 g, 3.78 mmol, 1.50 eq), Compound **A1**(676 mg, 2.52 mmol, 1.00 eq) and $Ac_2O$ (20.0 mL) was stirred for 1 hour at 70°C, NaOAc (621 mg, 7.56 mmol, 3.00 eq) and Compound **B1** (400 mg, 2.52 mmol, 1.00 eq) were added. The resulting mixture was heated to 80°C and stirred for 1 hour. The reaction mixture was then concentrated under reduced pressure. The residues were purified by preparative chromatography (HCl condition: column: YMC Triart C18 150 mm× 25 mm ×5 μm; mobile phase: [water(HCl)-ACN];B%: 70%-100%, 10 min), and lyophilized to obtain Compound **4** (68.07 mg, 89.0 μmol, 3.53% yield, 91% purity) as a green solid.

$^1H$ NMR (400 MHz, DMSO)δ 7.83 (t, *J* = 14 Hz, 2H), 7.34-7.43 (m, 6H), 7.22-7.23 (m, 3H), 7.11-7.16 (m, 2H), 7.02-7.06 (m, 2H), 6.05-6.11 (m, 2H), 4.16-4.21 (m, 4H), 2.63 (t, *J* = 7.2 Hz, 2H), 1.78-1.93 (m, 8H), 1.71 (d, *J* = 4.4 Hz, 12H), 1.60-1.64 (m, 2H), 1.46 (t, *J* = 7.2 Hz, 3H).

LCMS: m/z = 659.2, $C_{43}H_{48}ClN_2O_2^+$.

Example 5: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-3,3-dimethyl-1-(6-oxo-6-phenoxyhexyl)-3H-in dol-1-ium chloride (**5**)

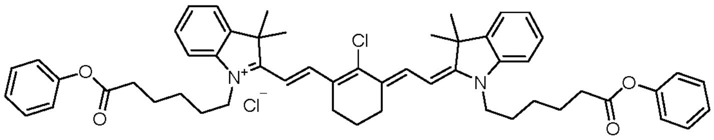

5

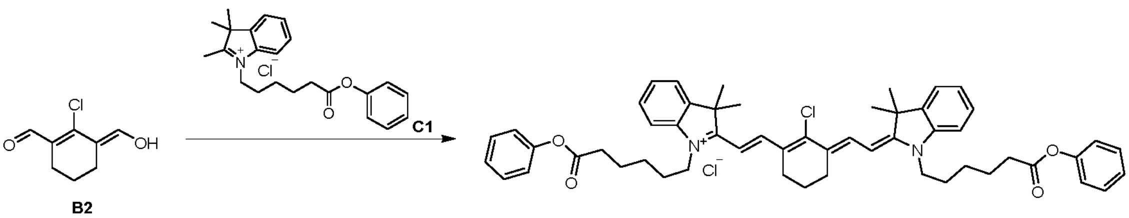

B2

C1

5

Compound **C1** (3.05 g, 8.69 mmol, 2.00 eq) was added to a solution of Compound **B2** (750 mg, 4.35 mmol, 1.00 eq), NaOAc (1.07 g, 13.0 mmol, 3.00 eq) and $Ac_2O$ (30.0 mL). The resulting mixture was stirred for 40 minutes at 70°C. The solvent was concentrated, and the residues were purified by preparative chromatography (HCl condition: column: Phenomenex luna C18 150 mm ×40 mm × 15 μm;mobile phase: [water (HCl) - ACN];B%: 70% - 100%, 10 min) to obtain Compound **5** (263 mg, 288 μmol, 6.62% yield, 95.4% purity) as a green solid.

$^1H$ NMR (400MHz, $CDCl_3$) δ 8.36 (d, *J* = 14Hz, 2H), 7.35 - 7.42 (m, 8H), 7.27 - 7.28 (m, 1H), 7.24 - 7.26 (m, 5H), 7.03 (d, *J* = 7.6Hz, 4H), 6.30 (d, *J* = 13.6Hz, 2H), 4.25 (s, 4H), 2.72 (s, 4H), 2.60 - 2.64 (m, 4H), 1.93 - 1.95 (m, 6H), 1.83 - 1.89 (m, 4H), 1.72 (s, 12H), 1.63 - 1.64 (m, 4H).

LCMS: m/z = 835.5 (M+H)+.

Example 6: Preparation of 2-((E)-2-((E)-2-chloro-3-(2-((E)-3,3-dimethyl-1-(4-(phenoxycarbonyl)bromo)indolin-2-ylidene)ethenyl)cyclohex-1-en-1-yl)ethenyl)-1-ethyl-3,3-dimethyl-3H-indol-1-ium chloride (**6**)

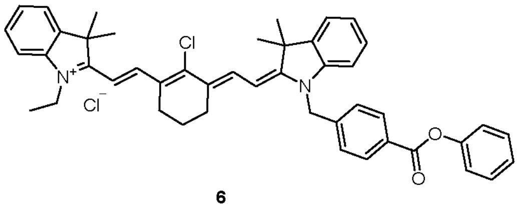

6

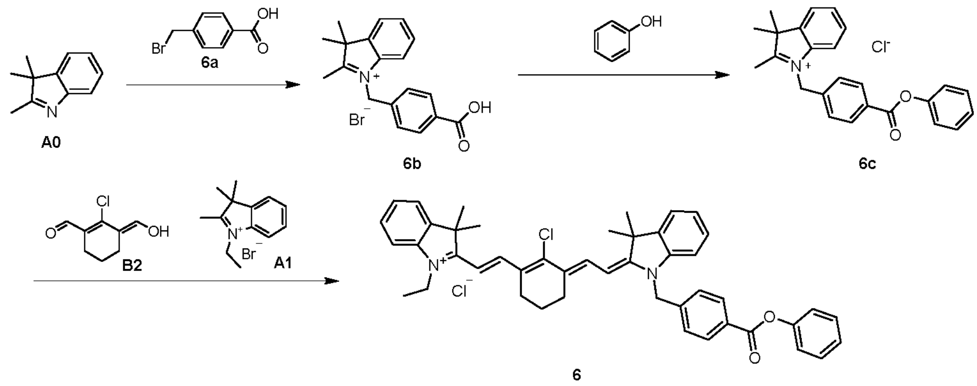

6a A0 6b 6c B2 A1 6

Step 1: Preparation of 1-(4-carboxybromo-2,3,3-trimethyl-3H-indol-1-ium bromide (**6b**)

Acetonitrile (50.0 mL) was added to compound **A0** (6.00 g, 37.6 mmol, 1.00 eq), and then 4-(bromomethyl) benzoic acid (**6a**, purchased from Shanghai Bide Pharmatech Co., Ltd.) (12.1 g, 56.5 mmol, 1.50 eq) was added. The mixture was heated for 12 hours at 85°C. The reaction solution was concentrated under reduced pressure, and then pulped with MTBE/DCM=1:2 (60.0 mL/120 mL) for 30 minutes at 25°C. The mixture was filtered to obtain Compound **6b** (11.0 g, 28.1 mmol, 74.6% yield, 95.7% purity) as a yellow solid.

$^1$H NMR (400MHz, DMSO) δ 7.97 (d, *J* = 8.0 Hz, 2H), 7.88 (d, *J* = 7.2 Hz, 1H), 7.77 (d, *J* = 7.2 Hz, 1H), 7.63 - 7.51 (m, 4H), 5.92 (s, 2H), 2.97 (s, 3H), 1.62 (s, 6H).

Step 2: Preparation of 2,3,3-trimethyl-1-(4-(phenoxycarbonyl)bromo)-3H-indol-1-ium chloride (**6c**)

Compound **6b** (6.00 g, 16.0 mmol, 1.00 eq) was dissolved in DMF (10.0 mL) and DCM (40.0 mL), and then HBTU (7.30 g, 19.2 mmol, 1.20 eq) and DIEA (5.18 g, 40.0 mmol, 6.98 mL, 2.50 eq) were added. The reaction solution was stirred for 0.5 hour at 25°C. Phenol (1.66 g, 17.6 mmol, 1.55 mL, 1.10 eq) was added to the reaction solution, and stirring was continued for 12 hours. The reaction solution was concentrated, and the residues were purified by reversed-phase preparative chromatography (0.1% HCl condition). Compound **6c** (3.6 g, 8.85 mmol, 55.22% yield, 91.1% purity) was obtained as a red solid after lyophilization.

LCMS: m/z = 370.1, $C_{25}H_{24}NO_2^+$.

Step 3: Preparation of Compound **6**

Compound **6c** (965 mg, 2.61 mmol, 1.50 *eq*) and 1-ethyl-2,3,3-methyl-3H-indol-1-ium bromide (**A1**) (466 mg, 1.74 mmol, 1.00 eq) were dissolved in $Ac_2O$ (20.0 mL), and stirred for 1 hour at 70°C. Compound **B2** (300 mg, 1.74 mmol, 1.00 eq) was added, followed by the addition of NaOAc (427 mg, 5.21 mmol, 16.3 μL, 3.00 eq). The mixture was stirred for 1 hour at 80°C. The reaction solution was concentrated, and the resulting residues were separated by reversed-phase preparative chromatography to obtain green Compound **6** (200 mg, 187 μmol, 10.7% yield, 65.0% purity). Further purification provided a green solid (83.47 mg, 113 μmol, 39.2% yield, 94.1% purity).

$^1$H NMR (400MHz, $CDCl_3$) δ 8.45 (d, *J* = 14.4 Hz, 2H), 8.26 - 8.19 (m, 3H), 7.47 - 7.38 (m, 7H), 7.35 - 7.28 (m, 3H), 7.26 - 7.22 (m, 2H), 7.20 - 7.18 (m, 2H), 7.01 (d, *J* = 8.0 Hz, 2H), 6.44 (d, *J* = 14.4 Hz, 1H), 6.03 (d, *J* = 13.2 Hz, 1H), 5.79 - 5.52 (m, 1H), 5.41 (s, 2H), 4.43 - 4.35 (m, 2H), 2.72 (s, 2H), 2.55 (s, 2H), 1.91 (s, 2H), 1.77 (s, 6H), 1.73 (s, 6H), 1.48 (t, *J* = 5.1 Hz, 3H).

Example 7: Preparation of 2-((E)-2-((E)-2-chloro-3-(2-((E)-3,3-dimethyl-1-(2-(3-oxo-3-phenoxypropoxy)ethyl)ind olin-2-ylidene)ethenyl)cyclohex-1-en-1-yl)ethenyl)-1-ethyl-3,3-dimethyl-3H-indol-1-iu m trifluoroacetate (7)

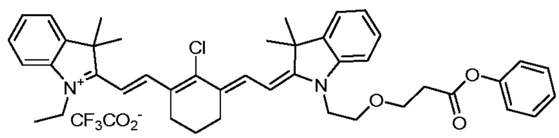

7

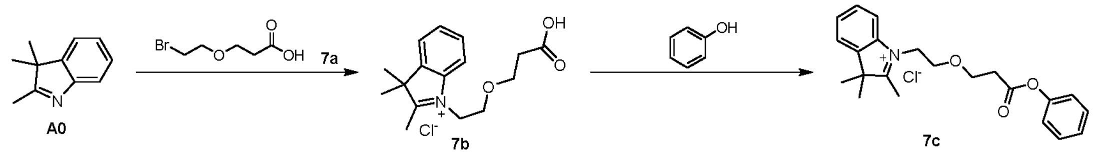


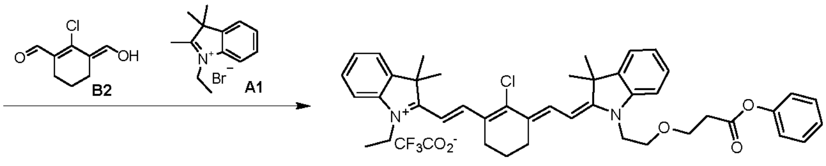


7

Step 1: Preparation of 1-(2-(2-carbonylethoxy)ethyl)-2,3,3-trimethyl-3H-indol-1-ium chloride (**7b**)

3-(2-Bromoethoxy)propionic acid (**7a**) (2.97 g, 15.1 mmol, 2.00 eq) was added to a solution of Compound **A0** (1.20 g, 7.54 mmol, 1.00 eq) and acetonitrile (15.0 mL) . The resulting mixture was stirred at 90°C for 36 hours. After the solvent was concentrated, the residues were purified by preparative chromatography (HCl condition) to obtain Compound **7b** (1.60 g, 4.49 mmol, 59.6% yield) as a colourless paste.

LCMS: m/z = 276.1 $(M+H)^+$.

Step 2: Preparation of 2,3,3-trimethyl-1-(2-(3-oxo-3-phenoxypropoxy)ethyl)-3H-indol-1-ium chloride (7c)

HBTU (2.04 g, 5.39 mmol, 1.20 eq) and DIEA (1.45 g, 11.2 mmol, 1.96 mL, 2.50 eq) were added to a solution of Compound **7b** (1.60 g, 4.49 mmol, 1.00 eq), DMF (3.00 mL) and DCM (22.0 mL), and stirred at 25°C for 30 minutes. Phenol (465 mg, 4.94 mmol, 435 μL, 1.10 eq) was added and stirred at 25°C for 12 hours. The resulting mixture was concentrated under reduced pressure, and the residues were purified by preparative chromatography (0.1% HCl condition) to obtain Compound 7c (1.40 g, 3.97 mmol, 88.5% yield) as a yellow oil.

LCMS: m/z = 352.2 $(M+H)^+$.

Step 3: Preparation of Compound 7

A solution of Compound 7c (306 mg, 869 μmol, 1.00 eq), Compound **A1** (350 mg, 1.30 mmol, 1.50 eq) and acetic anhydride (8.00 mL) was stirred at 80°C for 1.5 hours. Then NaOAc (214 mg, 2.61 mmol, 3.00 eq) and Compound **B2** (150 mg, 869 μmol, 1.00 eq) were added at 80°C, and stirring was continued for 1.5 hours. The reaction solution was concentrated, and the residues were purified by preparative chromatography (TFA condition) to obtain Compound 7 (60.0 mg, 80.3 μmol, 9.24% yield, 90.5% purity) as a brown solid.

$^1$H NMR (400MHz, $CDCl_3$) δ 8.34 (t, *J* = 14Hz, 2H), 7.36 - 7.40 (m, 4H), 7.29 - 7.35 (m, 3H), 7.26 - 7.27 (m, 2H), 7.25 - 7.26 (m, 1H), 7.13 - 7.25 (m, 1H), 6.85 (d, *J* = 7.6Hz, 2H), 6.37 (d, *J* = 14Hz, 1H), 6.13 (d, *J* = 14Hz, 1H), 4.41 (s, 2H), 4.12 - 4.17 (m, 2H), 3.97 (s, 2H), 3.82 - 3.85 (m, 2H), 2.70 - 2.74 (m, 2H), 2.65 - 2.68 (m, 4H), 1.91 - 1.93 (m, 2H), 1.74 (s, 6H), 1.72 (s, 6H), 1.44 (t, *J* = 7.2Hz, 3H).

LCMS: m/z = 675.4 $(M+H)^+$.

Example 8: Preparation of 2-((E)-2-((E)-2-chloro-3 -(2-((E)-3,3 -dimethyl- l -(2-(2-oxo-2-phenoxyethoxy)ethyl)indol in-2-ylidene)ethenyl)cyclohex-1-en-1-yl)ethenyl)-1-ethyl-3,3-dimethyl-3H-indol-1-ium trifluoroacetate (**8**)

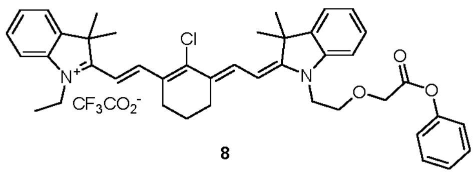

8

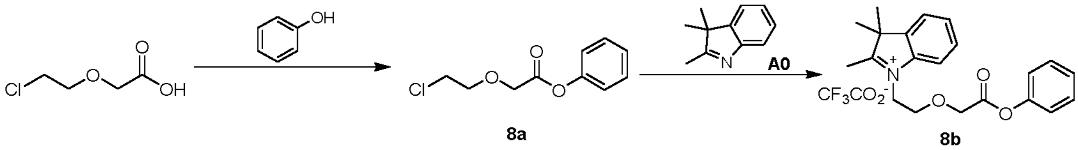


8a

8b

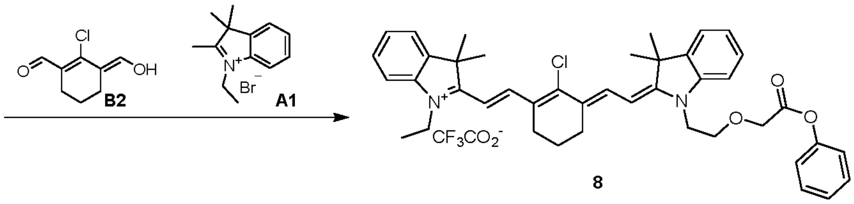


8

Step 1: Preparation of phenyl 2-(2-chloroethoxy)acetate (8a)

HBTU (16.4 g, 43.3 mmol, 1.20 eq) and DIEA (11.7 g, 90.2 mmol, 15.7 mL, 2.50 eq) were added to a solution of 2-(2-chloroethoxy)acetic acid (5.00 g, 36.1 mmol, 1.00 eq) in DCM (70.0 mL). After stirring at 25°C for 30 minutes, phenol (3.74 g, 39.7 mmol, 3.49 mL, 1.10 eq) was added and stirred for 12 hours at 25°C. The solvent was removed through concentration. Water (100 mL) was added, and the resulting mixture was extracted with ethyl acetate (100 mL×3). The organic phases were combined, dried over $Na_2SO_4$, and filtered. The filtrate was concentrated under reduced pressure. The residues were purified by silica gel column chromatography (petroleum ether/ethyl acetate = 100/1 to 1/1) and thin-layer chromatography (petroleum ether/ethyl acetate = 3/1, $R_f$ = 0.5, $KMnO_4$) to obtain Compound **8a** (6.40 g, 29.8 mmol, yield 82.6%) as a yellow solid.

$^1$H NMR (400MHz, $CDCl_3$)δ 7.39 - 7.43 (m, 2H), 7.27 - 7.29 (m, 1H), 7.13 - 7.15 (m, 2H), 4.45 (s, 2H), 3.95 (t, *J* = 5.8 Hz, 2H), 3.73 (t, *J* = 5.6 Hz, 2H).

Step 2: Preparation of 2,3,3-trimethyl-1-(2-(2-oxo-2-phenoxyethoxy)ethyl)-3H-indol-1-ium trifluoroacetate (**8b**)

A mixture of Compound **8a** (4.44 g, 20.7 mmol, 2.00 eq), Compound **A0** (1.65 g, 10.4 mmol, 1.00 eq) and NaI (7.77 g, 51.8 mmol, 5.00 eq) was heated to 180 °C, and stirred for 1 hour. After cooling, the parent solution was diluted with ACN (30.0 mL), filtered and concentrated. The concentrate was purified by preparative chromatography (TFA condition) to obtain Compound **8b** (1.20 g, 3.55 mmol) as a brown oil.

LCMS: m/z = 338.1 $(M+H)^+$.

Step 3: Preparation of Compound **8**

A solution of Compound **8b** (588 mg, 1.74 mmol, 1.50 eq) and Compound **A1** (366 mg, 1.16 mmol, 1.00 eq) in acetic anhydride (2.00 mL) was stirred at 70 °C for 1.5 hours, then NaOAc (285 mg, 3.48 mmol, 3.00 eq) and Compound **B2** (200 mg, 1.16 mmol, 1.00 eq) were added, and stirred at 80°C for another 1.5 hours. The mixture was concentrated under reduced pressure, and the residues were purified by preparative chromatography (0.1% TFA condition) to obtain Compound **8** (92.0 mg, 106 μmol, 9.17% yield, 89.8% purity, TFA) as a green solid.

$^1$H NMR (400MHz, $CDCl_3$) δ 8.30 - 8.39 (m, 2H), 7.35 - 7.42 (m, 7H), 7.24 - 7.26 (m, 3H), 7.12 (d, 8.0Hz, 1H), 7.00 - 7.02 (m, 2H), 6.44 (d, *J* = 14.4Hz, 1H), 6.13 (d, *J* = 14Hz, 1H), 4.49 - 4.51 (m, 2H), 4.35 (s, 2H), 4.10 - 4.17 (m, 4H), 2.63 - 2.69 (m, 4H), 1.92 (s, 1H), 1.90 - 1.91 (m, 2H), 1.72 (s, 12H), 1.42- 1.46 (m, 3H).

LCMS: m/z = 661.5 $(M+H)^+$.

Example 9: Preparation of 2-((E)-2-((E)-2-chloro-3-(2-((E)-3,3-dimethyl-1-(6-oxo-6-phenoxyhexyl)indolin-2-ylide ne) ethenyl)cyclopent-1-en-1-yl)ethenyl)-1,3,3-trimethyl-3H-indol-1-ium chloride (**9**)

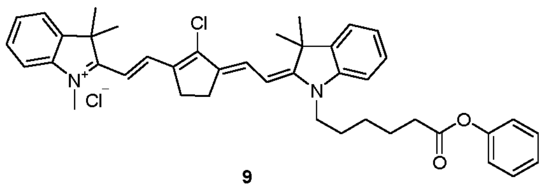

9

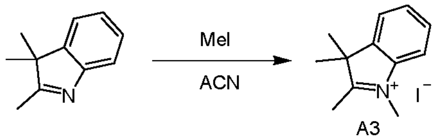


A3

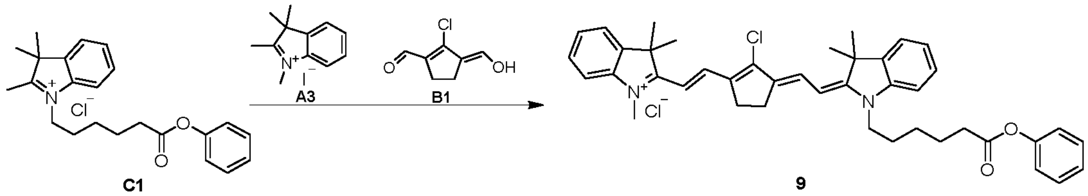


C1 9

Step 1: Preparation of 1,2,3,3-tetramethyl-3H-indol-1-ium iodide (**A3**)

A solution of 2,3,3-trimethylindole (3.00 g, 18.8 mmol, 1.00 eq) and MeI (4.01 g, 28.2 mmol, 1.76 mL, 1.50 eq) in ACN (30.0 mL) was evacuated and purged with nitrogen 3 times. The mixture was stirred at 90 °C under nitrogen atmosphere for 12 hours. The reaction solution was concentrated under reduced pressure to remove the solvent, thereby obtaining a crude produce. Then, the crude product was pulped with MTBE/DCM=5:1 (20 mL) at 25°C for 30 minutes, and filtered to obtain a filter cake, i.e., a product of Compound A3 (3.50 g, 11.4 mmol, 60.4% yield, 98% purity), which was a purple solid.

Step 2: Preparation of Compound **9**

A mixture of Compound **C1** (2.00 g, 5.71 mmol, 1.50 *eq*), Compound **A3**(1.15 g, 3.80 mmol, 1.00*eq*) and $Ac_2O$ (30.0 mL) was stirred at 70°C for 1 hour, and then NaOAc (936 mg, 11.4 mmol, 3.00 *eq*) and Compound **B1**(603 mg, 3.80 mmol, 1.00 *eq)* were added successively. The reaction mixture was stirred at 80°C for 1 hour and concentrated under reduced pressure . The residues were purified by preparative chromatography (0.1% HCl condition) to obtain Compound **9** (900 mg, 1.23 mmol, 32.21% yield, 88% purity) as a green solid.

LCMS: m/z = 645.2, $C_{42}H_{46}ClN_2O_2^+$.

Example 10: Preparation of 2-((E)-2-((E)-3-(2-((E)-1-(5-carboxypentyl)-3,3-dimethylindolin-2-ylidene)ethenyl)-2-c hlorocyclopent-1-en-1-yl)ethenyl)-1,3,3-trimethyl-3H-indol-1-ium chloride (10)

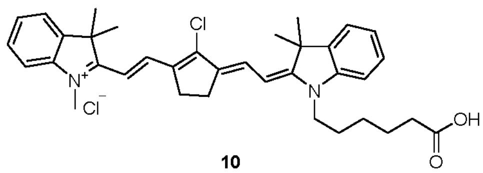

10

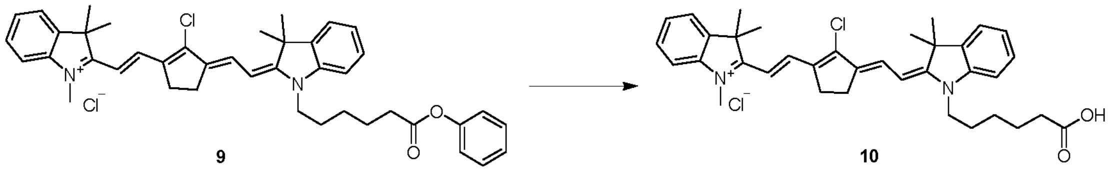

9 10

HCl/dioxane solution (1 M, 1.86 mL, 6.00eq) was added to a solution of Compound **9** (200 mg, 309 μmol, 1.00 eq) in dioxane (2.00 mL). The solution was kept at 50 °C and stirred for 2 hours. The reaction solution was concentrated, and the residues were purified by preparative chromatography (column: YMC Triart C18 150mm×25mm×5μm; mobile phase: [water(HCl)-ACN]; B%: 58%-88%, 10min) to obtain Compound **10** (41.0 mg, 71.0 μmol, 23.0% yield, 98.7% purity) as a green solid.

$^1H$ NMR (400MHz, $CDCl_3$)δ 7.85-7.81 (m, 2H), 7.41-7.36 (m, 4H), 7.25-7.22 (m, 2H), 7.15-7.13 (m, 2H), 6.08-6.03 (m, 2H), 4.08-4.06 (m, 2H), 3.67 (s, 3H), 3.03 (s, 4H), 2.54-2.49 (m, 2H), 1.87-1.76 (m, 4H), 1.71 (s, 12H), 1.60-1.54 (m, 2H).

LCMS: m/z =569.4, $C_{36}H_{42}ClN_2O_2^+$.

Example 11: Preparation of 1-(5-carboxyphenyl)-2-((E)-2-((E)-2-chloro-3-(2-((E)-3,3-dimethyl-1-(6-oxo-6-phenoxy hexyl)indolin-2-ylidene)ethenyl)cyclohex-1-en-1-yl)ethenyl)-3,3-dimethyl-3H-indol-1-i um chloride (11)

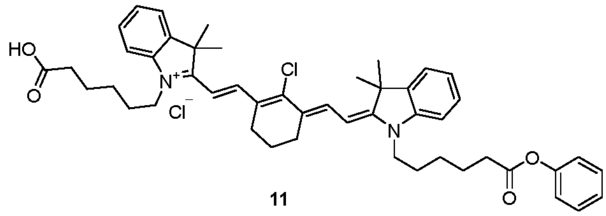

11

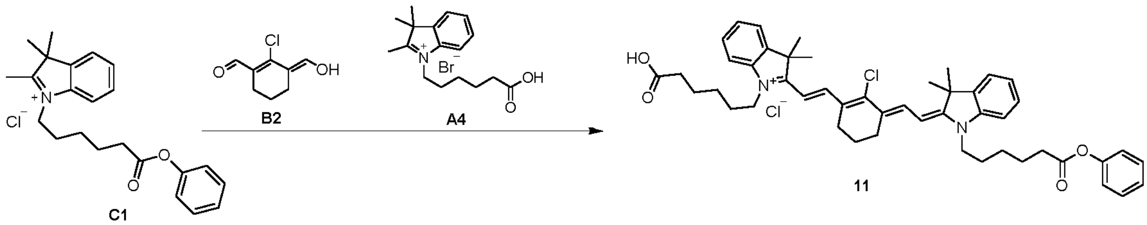

C1 B2 A4 11

NaOAc (570 mg, 6.95 mmol, 3.00 eq) and Compound **C0** (635 mg, 2.32 mmol, 1.00 eq) were added to a solution of Compound **B2** (0.400 g, 2.32 mmol, 1.00 eq) and Compound **C1** (812 mg, 2.32 mmol, 1.00 eq) in EtOH (2.00 mL). After stirring at 85°C for 2 hours, the reaction solution was concentrated, and the residues were purified by preparative chromatography (column: YMC Triart C18 150mm×25mm×5μm; mobile phase: [water(HCl) - ACN]; B%: 56%-86%, 8.5 min) to obtain Compound **11** (94.0 mg, 111 μmol, 4.81% yield, 90.2% purity) as a green solid.

$^1$H NMR (400MHz, $CDCl_3$) δ 8.40 (d, *J* = 14.0 Hz, 1H), 8.31 (d, *J* = 14.0 Hz, 1H), 7.38 - 7.43 (m, 6H), 7.35 - 7.37 (m, 1H), 7.21 - 7.23 (m, 3H), 7.02 - 7.04 (m, 3H), 6.31 (d, *J* = 14.0 Hz, 1H), 6.10 (d, *J* = 14.0 Hz, 1H), 4.06 - 4.19 (m, 4H), 2.69 - 2.75 (m, 4H), 2.61- 2.64 (m, 4H), 1.82 - 1.99 (m, 10H), 1.72 (d, *J* = 5.6 Hz, 12H), 1.59 - 1.64 (m, 4H).

LCMS: m/z = 759.4, $C_{48}H_{56}ClN_2O_4^+$.

Example 12: Preparation of 2-((E)-2-((E)-3-(2-((E)-3,3-dimethyl-1-(6-oxo-6-phenoxyhexyl)indolin-2-ylidene)ethen yl)-2-phenoxycyclohex-1-en-1-yl)ethenyl)-1-ethyl-3,3-dimethyl-3H-indol-1-ium chloride (**12**)

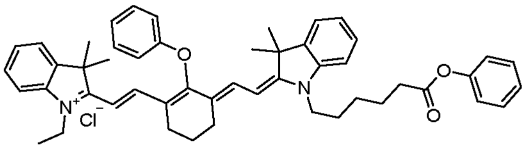

12

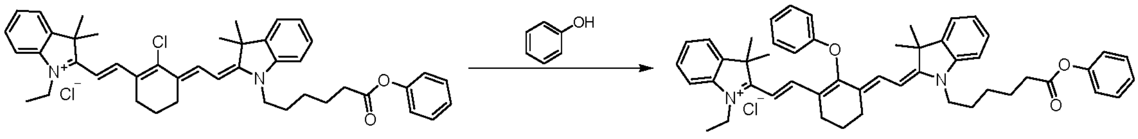

12a 12

NaH (88.9 mg, 2.22 mmol, 60.0% purity, 2.50 eq) was added to a solution of phenol (209 mg, 2.22 mmol, 196 μL, 2.50 eq) in THF(50.0 mL) at 0°C, and then heated to 25°C and stirred for 30 minutes. A solution of 2-((E)-2-((E)-2-chloro-3-(2-((E)-3,3-dimethyl-1-(6-oxo-6-phenoxyhexyl)indolin-2-ylide ne)ethenyl)cyclohex-1-en-1-yl)ethenyl)-1-ethyl-3,3-dimethyl-3H-indol-1-ium chloride (**12a**) (see Comparative Example 1 for the preparation method) (600 mg, 890 μmol, 1.00 eq) in THF (10.0 mL) was added. After stirring for 4 hours, the resulting mixture was cooled to 0°C and quenched by addition of saturated $NH_4Cl$ solution (4.00 mL). The mixture was concentrated under reduced pressure, and the residues were purified by preparative chromatography (HCl condition: column: Phenomenex luna C18 150 mm × 40 mm × 15μm; mobile phase: [water (HCl) - ACN]; B%: 60% - 90%, 10 min) to obtain Compound **12**(84.7 mg, 110 μmol,

12.4% yield, purity 95.3%) as a green solid.

$^1H$ NMR: (400MHz, $CDCl_3$) $\delta$ 7.91 (t, J = 13.8Hz, 2H), 7.34 - 7.37 (m, 6H), 7.23 - 7.27 (m, 5H), 7.02 - 7.08 (m, 7H), 6.08 (t, J = 14.4Hz, 2H), 4.06 - 4.16 (m, 4H), 2.72 (s, 4H), 2.59 - 2.71 (m, 2H), 2.02- 2.03 (m, 2H), 1.82 - 1.86 (m, 4H), 1.58 - 1.60 (m, 2H), 1.38 - 1.43 (m, 3H), 1.33 (d, J = 2.0Hz, 12H).

LCMS: m/z = 731.6 $(M+H)^+$.

Example 13: Preparation of 2-((E)-2-((E)-2-((5-carboxypentyl)amino)-3-(2-((E)-1-ethyl-3,3-dimethylindolin-2-ylide ne) ethenyl)cyclohex-1-en-1-yl)ethenyl)-1-ethyl-3,3-dimethyl-3H-indol-1-ium formate **(13)**

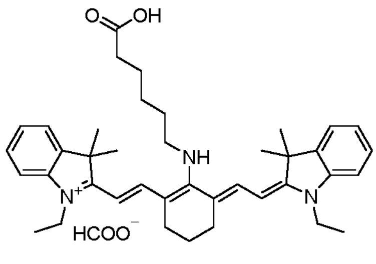

**13**

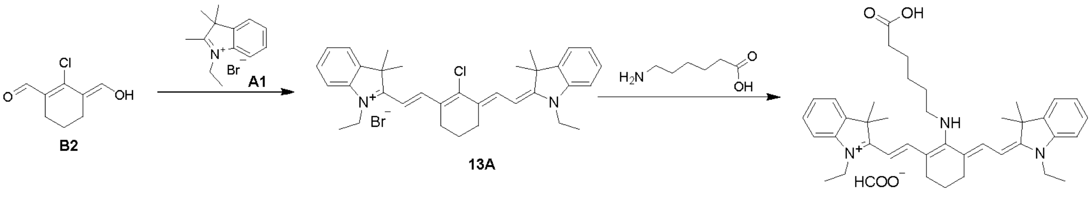

B2

A1

13A

**13**

Step 1: Preparation of 2-((E)-2-((E)-2-chloro-3-(2-((E)-1-ethyl-3,3-dimethyl-indolin-2-ylidene)ethenyl)cycloh ex-1-en-1-yl]ethenyl]-1-ethyl-3,3-dimethyl-indol-1-ium bromide **(13A)**

NaOAc (1.43 g, 17.4 mmol, 3.00 *eq)* and Compound **B2** (1.00 g, 5.79 mmol, 1.00 *eq)* were added to a mixture of Compound **A1** (3.88 g, 14.5 mmol, 2.50 *eq)* and $Ac_2O$ (50.0 mL) at 70°C. The mixture was heated to 80°C and stirred for 1 hour. The reaction solution was concentrated, and then purified by preparative chromatography (neutral condition) to obtain Compound 13A (1.30g) as a green solid after lyophilization.

LCMS: m/z = 511.2.

Step 2: Preparation of Compound **13**

Triethylamine (324 mg, 3.20 mmol, 446 $\mu$L, 16.4 *eq)* was added to a solution of Compound **13A** (0.100 g, 195 $\mu$mol, 1.00 *eq),* 6-aminohexanoic acid (169 mg, 1.29 mmol, 166 $\mu$L, 6.60 *eq)* and MeOH (5.50 mL), and the reaction mixture was stirred at 70°C for 4 hours. After concentrated under reduced pressure, the residues were purified by preparative chromatography (chromatographic column: Phenomenex luna C18 150mm$\times$25mm$\times$10$\mu$m; mobile phase: [water(FA)-ACN]; B%: 38%-68%, 10min) to obtain Compound **13** (56.0 mg, 83.9 $\mu$mol, 42.9% yield, purity 90.9%) as a green solid.

$^1H$ NMR (400MHz, $CDCl_3$) $\delta$ 8.70 (s, 1H), 7.64 - 7.67 (m, 2H), 7.28 (s, 1H), 7.23 - 7.26 (m, 3H), 7.02 - 7.06 (m, 2H), 6.81 (d, *J* = 8.4 Hz, 2H), 5.52 - 5.55 (m, 2H), 3.79 - 3.83 (m, 6H), 2.49 - 2.53 (m, 6H), 1.95 - 1.98 (m, 2H), 1.81 - 1.84 (m, 2H), 1.75 - 1.79 (m, 2H), 1.66 (s, 12H), 1.45 - 1.49 (m, 2H), 1.31 - 1.37 (m, 6H).

LCMS: m/z = 606.5.

Example 14: Preparation of 2-((E)-2-((E)-4-chloro-5-(2-((E)-3,3-dimethyl-1-(6-oxo-6-phenoxyhexyl)indolin-2-ylide ne) ethenyl)-1,1-dimethyl-1,2,5,6-tetrahydropyridin-1-ium-3-yl)ethenyl)-3,3-dimethyl-1-(6-oxo-6-phenoxyhexyl)-3H-indol-1-ium dichloride **(14)**

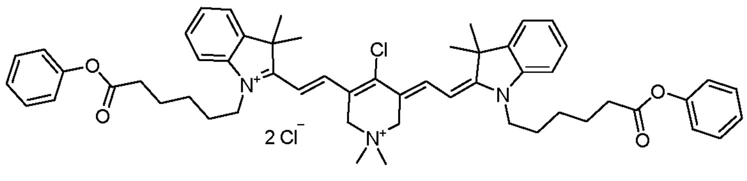

**14**

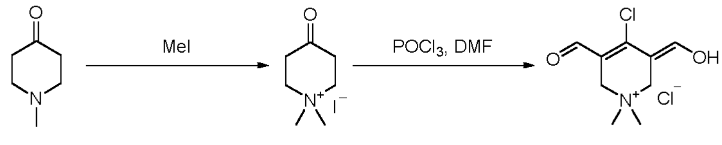


**14a** **B3**

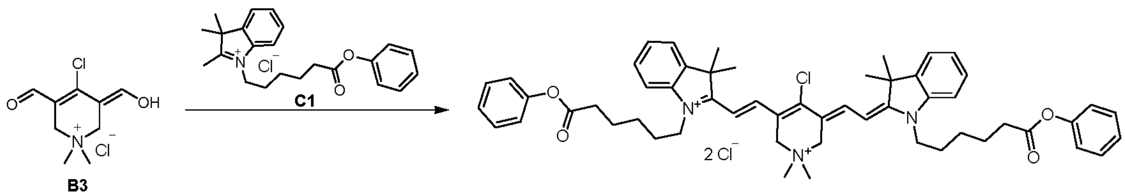


**B3** **14**

Step 1: Preparation of 1,1-dimethyl-4-oxopiperidin-1-ium iodide **(14a)**

MeI (41.02 g, 288.98 mmol, 17.99 mL, 2.18 eq) was added to a solution of 1-methyl-4-piperidone (15 g, 132.56 mmol, 15.42 mL, 1 eq) in acetone (210 mL) at 0°C, and the mixture was stirred at 25°C for 4 hours. The reaction solution was filtered, and the filter cake was washed with iced acetone (20.0 mL). The filtrate was then concentrated under reduced pressure, and the residue was a product (33 g, 129.36 mmol, 97.59% yield ) as a yellow solid.

$^1$H NMR (400MHz, $CDCl_3$)δ 3.77 -3.74 (t, *J* = 6.8 Hz, 4H), 3.28 (s, 6H), 2.73 - 2.69 (m, 4H).

Step 2: Preparation of (E)-4-chloro-5-formyl-3-(hydroxymethylene)-1,1-dimethyl-1,2,3,6-tetrahydropyridin-1-ium chloride (**B3**)

A mixture of DMF (210 mL) and $POCl_3$ (209.17 g, 1.36 mol, 126.77 mL, 3 eq) was stirred for 0.5 hour, and then the product (116 g, 454.73 mmol, 1 eq) obtained in the previous step was added to the mixture. The mixture was then stirred for at 80°C 3 hours. TLC (ethyl acetate:methanol = 5:1) showed the product obtained in the previous step was consumed completely. The reaction mixture was quenched with 20% HCl (700 mL) at 35 to 40°C, then the mixture was cooled to -20°C quickly, and the suspension was filtered. The filtrate was left at 25°C for 12 hours, then cooled to -20°C and left for 4 hours. The suspended matter was filtered. The filter cake was concentrated under reduced pressure to obtain a residue. The residues were diluted with toluene (30.0 mL), and concentrated under reduced pressure to obtain a second residue, i.e., Compound **B3** (24.2 g, 110.50 mmol, 26.26% yield ) as a yellow solid.

$^1$H NMR (400MHz, $CDCl_3$) δ 9.00 (s, 2H), 4.27 (s, 4H), 3.18 (s, 6H).

Step 3: Preparation of Compound **14**

After a mixture of Compound **C1** (865 mg, 2.47 mmol, 2.00 eq) and $Ac_2O$ (10.0 mL) was stirred at 70°C for 30 minutes, NaOAc (304 mg, 3.70 mmol, 3.00 eq) and Compound **B3** (250 mg, 1.23 mmol, 1.00 eq) were added. The mixture was kept at 80°C and stirred for 1 hours. The reaction mixture was concentrated under reduced pressure, and the residues were purified by preparative chromatography (0.1% HCl condition) to obtain Compound **14** (182.57 mg, 190 μmol, 15.4% yield, 90.1% purity) as a green solid.

$^1$H NMR (400 MHz, $CDCl_3$) δ 8.28 (d, J = 7.4 Hz, 2H), 7.34-7.44 (m, 10H), 7.19-7.25 (m, 4H), 7.04 (d, J = 8.0 Hz, 4H), 6.22-6.26 (m, 2H), 4.39 (s, 4H), 4.24-4.33 (m, 4H), 3.08 (s, 6H), 2.60 (t, J = 4.0 Hz, 4H), 2.02 (s, 2H), 1.88-1.95 (m, 4H), 1.82-1.86 (m, 4H), 1.73 (s, 12H), 1.66 (s, 2H).

LCMS: m/z = 433.4, $C_{55}H_{64}ClN_3O_4^{2+}$.

Example 15: Preparation of 2-((E)-2-((E)-5-(2-((E)-1-(5-carboxypentyl)-3,3-dimethylindolin-2-ylidene)ethenyl)-4-c hloro-1,1-dimethyl-1,2,5,6-tetrahydropyridin-1-ium-3-yl)ethenyl)-3,3-dimethyl-1-(6-oxo -6-phenoxyhexyl)-3H-indol-1-ium ditrifluoroacetate **(15)**

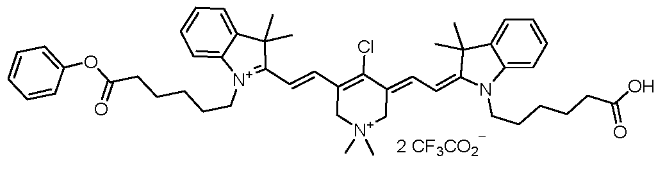

**15**

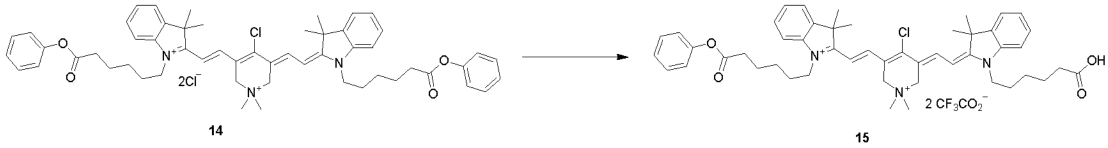

14

15

A solution of hydrogen chloride in dioxane (4.00 M, 86.6 μL, 3.00 eq) was added to a solution of Compound **14** (100 mg, 115 μmol, 1.00 eq) in MeCN (2.00 mL), and stirred at 25°C for 12 hours, followed by the addition of a solution of hydrogen chloride in dioxane (4.00 M, 289 μL, 10.0 eq) again. After stirring at 25°C for another 12 hours, the solvent was concentrated, and the residues were purified by preparative chromatography (TFA condition; column: Phenomenex Synergi Polar-RP 100mm×25 mm×4 μm; mobile phase: [water(TFA)-ACN]; B%: 38%-68%, 9 min) to obtain Compound **15** (43.44 mg, 43.0 μmol, 37.3% yield, 89.5% purity, TFA) after lyophilization as a blue paste.

$^1$H NMR (400 MHz, $CDCl_3$) δ 8.30 (d, *J* = 14.8 Hz, 2H), 7.34-7.42 (m, 8H), 7.18-7.23 (m, 3H), 7.00-7.23 (m, 2H), 6.39-6.45 (m, 2H), 4.76 (d, *J* = 14.8 Hz, 4H), 4.24-4.25 (m, 4H), 3.34 (s, 6H), 2.53-2.63 (m, 2H), 2.32-2.42 (m, 2H), 1.82-1.87 (m, 7H), 1.72 (s, 12H), 1.54-1.59 (m, 5H).

LCMS: m/z = 395.0 $C_{49}H_{60}ClN_3O_4^{2+}$.

Example 16: 2-((E)-2-((E)-4-chloro-5-(2-((E)-3,3-dimethyl-1-(6-oxo-6-phenoxyhexyl)indolin-2-ylide ne)ethenyl)-1,1-dimethyl-1,2, 5, 6-tetrahydropyridin-1-ium-3-yl)ethenyl)-1-ethyl-3,3-dim ethyl-3H-indol-1-ium dichloride **(16)**

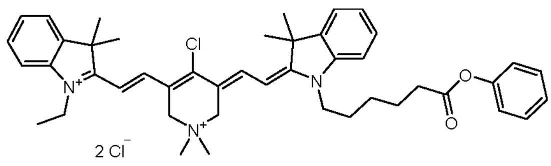

**16**

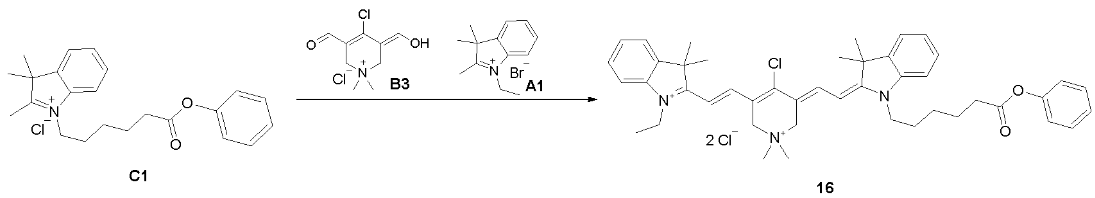


C1

16

A mixture of Compound **C1** (2.00 g, 5.71 mmol, 1.50 eq), Compound **A1** (716 mg, 3.81 mmol, 1.00 eq, the amount used being only based on positive ions) and $Ac_2O$ (30.0 mL) was heated to 80°C and stirred for 1 hours. Sodium acetate (936 mg, 11.4 mmol, 3.00 eq) and Compound **B3** (771 mg, 3.81 mmol, 1.00 eq) were added successively, and stirred at 80°C for 1 hour. The reaction solution was concentrated under reduced pressure, and the residues were purified by preparative chromatography (column: Phenomenex luna C18 150mm×40mm×15μm; mobile phase: [water (HCl)-ACN]; B%: 30%-60%, 10min) to obtain Compound **16**(121 mg, 166 μmol, 4.38% yield, 97.1% purity) as a green solid.

$^1$H NMR: (400MHz, $CDCl_3$) δ 8.28 - 8.33 (m, 2H), 7.34 - 7.45 (m, 9H), 7.24 - 7.26 (m, 2H), 7.04 - 7.06 (m, 2H), 6.20 - 6.28 (m, 2H), 4.43 (d, J = 10.8 Hz, 4H), 4.27 - 4.32 (m, 4H), 3.20 (s, 6H), 2.61 (t, J = 14.0 Hz, 2H), 1.83 - 1.93 (m, 4H), 1.74 (d,

J = 2.4 Hz, 12H), 1.59 - 1.64 (m, 2H), 1.47 - 1.50 (s, 3H).

LCMS: m/z = 352.0, $C_{45}H_{54}ClN_3O_2^{2+}$.

Example 17: Preparation of 1-(2-carboxyethyl)-2-((E)-2-((E)-5-(2-((E)-1-(2-carboxyethyl)-3,3-dimethylindolin-2-yl idene)ethenyl)-4-chloro-1,1-dimethyl-1,2,5,6-tetrahydropyridin-1-ium-3-yl)ethenyl)-3,3-dimethyl-3H-indol-1-ium ditrifluoroacetate **(17)**

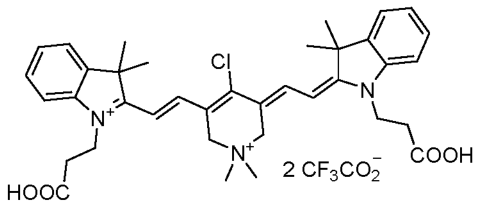

**17**

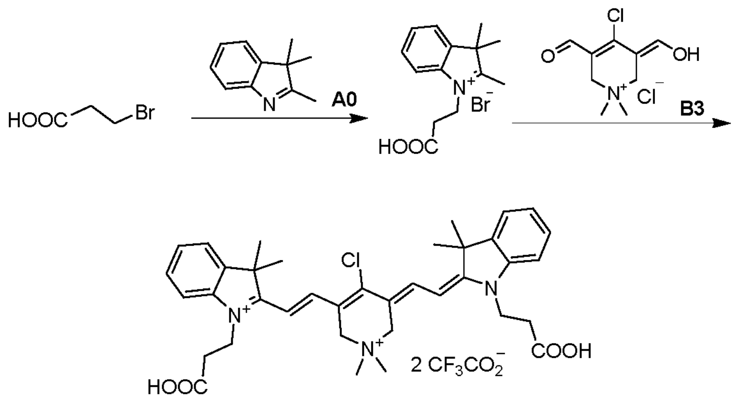


**17**

Step 1: Preparation of 1-(2-carboxyethyl)-2,3,3-trimethyl-3H-indol-1-ium bromide

2,3,3-Trimethylindole (**A0**) (3.00 g, 18.8 mmol, 1.00 *eq)* was added to a solution of 3-bromopropionic acid (5.76 g, 37.7 mmol, 3.89 mL, 2.00 *eq)* in ACN (30 mL), and stirred at 80°C for 24 hours. The reaction mixture was concentrated under reduced pressure. The residues were pulped with DCM:MTBE=5: 1 (10.0 mL) to obtain the title compound (1.50 g, 5.94 mmol, 31.5% yield, 92.0% purity) as a pink solid.

LCMS: m/z =232.2, $C_{14}H_{18}NO_2^+$.

Step 2: Preparation of Compound **17**

NaOAc (353 mg, 4.30 mmol, 1.00 *eq)* was added to a solution of the compound (1.00 g, 4.30 mmol, 1.00 *eq)* obtained in Step 1 in EtOH (50.0 mL), and the reaction mixture was stirred at 25°C for 0.5 hour. Compound **B3** (436 mg, 2.15 mmol, 0.500 *eq)* was added, and subjected to the reaction at 80°C for 5 hours. The resluting mixture was purified by preparative chromatography (column: Phenomenex Synergi Polar-RP 100mm×25mm×4μm; mobile phase: [water(TFA)-ACN]; B%: 30%-60%, 9min) to obtain Compound 17(174 mg, 247 μmol, 5.73% yield, 89.4% purity) as a green solid.

$^1$H NMR (400MHz, DMSO-d6) δ 8.30-8.26 (m, 2H), 7.44-7.37 (m, 4H), 7.32-7.28 (m, 4H), 6.56-6.52 (m, 2H), 4.78 (s, 4H), 4.54 (s, 4H), 3.43 (s, 6H), 2.91 (s, 4H), 1.71 (s, 12H).

LCMS: m/z = 314.8, $C_{37}H_{44}ClN_3O_{42}^+$.

Example 18: Preparation of 2-((E)-2-((E)-4-chloro-5-(2-((E)-1-ethyl-3,3-dimethylindolin-2-ylidene)ethenyl)-1,1-di methyl-1,2,5,6-tetrahydropyridin-1-ium-3-yl)ethenyl)-1-ethyl-3,3-dimethyl-3H-indol-1-i um ditrifluoroacetate (18)

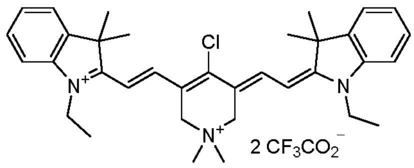

18

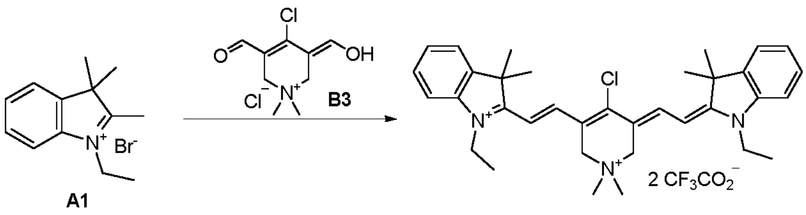


A solution of Compound **A1** (1.86 g, 9.87 mmol, 2.00 eq) in $Ac_2O$ (15.0 mL) was stirred for 0.5 h at 70°C. NaOAc (1.21 g, 14.8 mmol, 3.00 eq) and Compound **B3** (1.00 g, 4.93 mmol, 1.00 eq) were added, and the reaction mixture was stirred at 80°C for 1 hour. The reaction mixture was concentrated under reduced pressure. The residues were pulped with water for 15 minutes, and filtered. The resulting crude was purified by preparative chromatography (column: YMC Triart C18 150mm×25 mm×5 μm; mobile phase: [water(TFA)-ACN]; B%: 41%-61%, 10 min) to obtain Compound **18** (150 mg, 272 μmol, 5.51% yield, 98.2% purity) as a green solid.

$^1$H NMR (400 MHz, $CDCl_3$) δ 8.32 (d, J = 14.8 Hz, 2H), 7.41-7.46 (m, 4H), 7.36-7.34 (m, 2H), 7.22 (d, J = 8.0 Hz, 2H), 6.68 (d, J =14.8 Hz, 2H), 4.98 (s, 4H), 4.38-4.43 (m, 4H), 3.53 (s, 6H), 1.76 (s, 12H), 1.46 (t, J = 7.2 Hz, 6H).

LCMS: m/z =271.0, $C_{35}H_{44}ClN_3^{2+}$.

Example 19: Preparation of 2-((E)-2-((E)-4-chloro-5-(2-((E)-1-(2-hydroxyethyl)-3,3-dimethylindolin-2-ylidene)ethe nyl)-1,1-dimethyl-1,2,5,6-tetrahydropyridin-1-ium-3-yl)ethenyl)-1-(2-hydroxyethyl)-3,3 -dimethyl-3H-indol-1-ium ditrifluoroacetate **(19)**

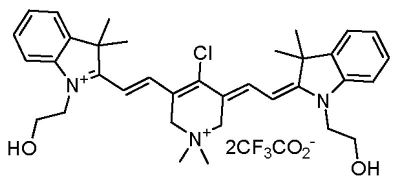

19

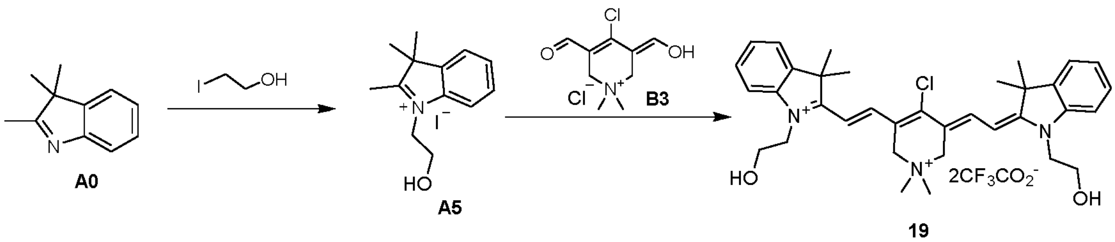


Step 1: Preparation of 1-(2-hydroxyethyl)-2,3,3-trimethyl-3H-indol-1-ium iodide **(AS)**

A mixture of Compound **A0**(5.00 g, 31.4 mmol, 1.00 *eq),* 2-iodoethanol (5.40 g, 31.4 mmol, 2.45 mL, 1.00 *eq)* and EtOH (50.0 mL) was kept at 80°C and stirred for 12 hours. Then, the reaction solution was concentrated under reduced pressure, and the residues were pulped with petroleum ether (100 mL) to obtain Compound **A5** (2.80 g, 11.2 mmol, 35.8% yield, 82.0% purity) as a pink solid.

$^1$H NMR (400MHz, $CDCl_3$) δ 7.81-7.79 (m, 1H), 7.60-7.57 (m, 3H), 4.90-4.88 (m, 2H ), 4.21 (s, 2H), 3.12 (s, 3H), 1.66 (s, 6H).

LCMS: m/z =204.2 $C_{13}H_{18}NO^+$.

Step 2: Preparation of Compound **19**

Compound **A5** (1.00 g, 4.90 mmol, 1.00 *eq)* was dissolved in EtOH (50 mL), and NaOAc (402 mg, 4.90 mmol, 1.00 *eq)* was added and stirred at 25°C for 0.5 hour. Then, Compound **B3** (496 mg, 2.45 mmol, 0.500 *eq)* was added, and kept at 80°C and stirred for 5 hours. The resulting mixture was concentrated under reduced pressure, and the residues were purified by preparative chromatography (column: Phenomenex Synergi Polar-RP 100mm×25mm×4μm; mobile phase: [water (TFA)-ACN]; B%: 28%-58%, 9min) to obtain Compound **19** (90.1 mg, 145.38 μmol, 3.34% yield, 92.65% purity) as a brown solid.

$^1H$ NMR (400MHz, DMSO-d6) δ 8.24-8.22 (m, 2H), 7.70-7.68 (m, 2H), 7.55-7.53 (m, 2H), 7.49-7.45 (m, 2H), 7.38-7.34 (m, 2H), 6.41-6.37 (m, 2H), 4.65 (s, 4H), 4.38-4.34 (m, 4H), 3.86-3.83 (m, 6H), 3.27 (s, 6H), 1.70 (s, 12H).

LCMS: m/z = 286.9, $C_{35}H_{44}ClN_3O_2^{2+}$.

Example 20: Preparation of 2-((E)-2-((E)-3-(2-((E)-3,3-dimethyl-1-(6-oxo-6-phenoxyhexyl)indol-2-methylene)ethe nyl)-2-(4-sulfophenoxy)cyclohex-1-en-1-yl)ethenyl)-1-ethyl-3,3-dimethyl-3H-indol-1-i um formate **(20)**

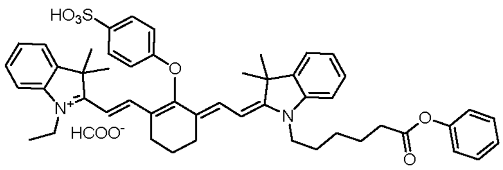

**20**

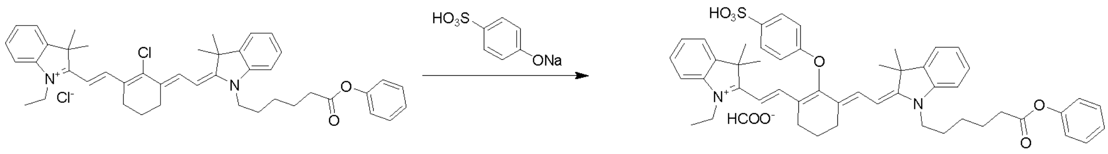

**12a** **20**

$K_2CO_3$ (30.7 mg, 222 μmol, 1.00 eq) was added to a mixture of (4-sulfophenoxy)sodium (52.3 mg, 266 μmol, 1.20 eq), Compound **12a**(150 mg, 222 μmol, 1.00 eq) and DMSO (3.00 mL), and stirred at 40°C for 12 hours. The reaction mixture was then filtered. The filtrate was purified by preparative chromatography (chromatographic column: UniSil 3-100 C18 Ultra (150mm×25mm×3μm); mobile phase [water(FA)-ACN]; B%: 67%-97%, 10min) to obtain Compound 20(85.43 mg, 100 μmol, 45.2% yield, 95.5% purity) after lyophilization as a green solid.

$^1H$ NMR (400MHz, $CDCl_3$) δ 8.03 (d, J = 8.8 Hz, 2H), 7.92 (dd, J = 14.0, 9.6 Hz, 2H), 7.39 - 7.29 (m, 5H), 7.26 - 7.15 (m, 4H), 7.07 - 7.02 (m, 4H), 6.98 - 6.96 (m, 2H), 5.92 (dd, J = 14.0, 11.6 Hz, 2H), 4.05 - 3.95 (m, 4H), 2.65- 2.59 (m, 6H), 1.02 - 1.99 (m, 2H), 1.91 - 1.82 (m, 4H), 1.60 - 1.55 (m, 2H), 1.39 (t, J = 5.4 Hz, 3H), 1.33 (s, 12H).

LCMS: m/z = 811.37, $C_{50}H_{55}N_2O_6S^+$.

Example 21: Preparation of 2-((E)-2-((E)-2-chloro-3-(2-((E)-3,3-dimethyl-1-(2-(3-oxo-3-phenoxypropoxy)ethyl)ind ol-2-methylene)ethylidene)cyclopent-1-en-1-yl)ethenyl)-1-ethyl-3,3-dimethyl-3H-indol-1-ium trifluoroacetate (21)

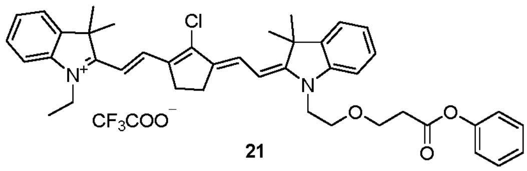

**21**

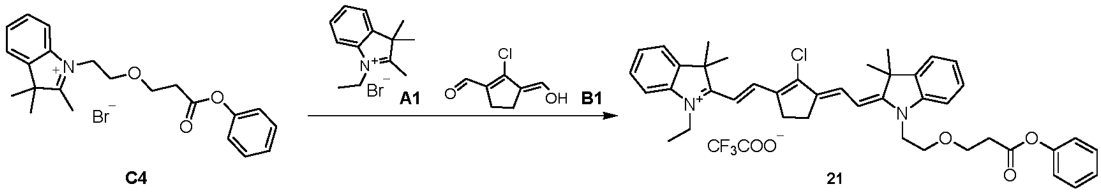


C4 21

NaOAc (776 mg, 9.46 mmol, 3.00 eq) was added to a solution of Compound **B1** (500 mg, 3.15 mmol, 1.00 eq), Compound **A1** (994 mg, 2.29 mmol, 1.00 eq) and Compound **C4** (1.36 g, 3.15 mmol, 1.00 eq) in acetic anhydride (20.0 mL). The mixture was stirred at 80°C for 3 hours. The reaction mixture was concentrated under reduced pressure, and the residues were purified by reversed-phase HPLC (Phenomenex luna C18 150×40mm× 15μm, mobile phase: [water (TFA)-ACN]; B%: 25%-55%, 10min, 0.1% HCl condition) to obtain a crude product (400 mg). The crude product was further purified by reversed-phase HPLC (TFA condition, chromatographic column: Phenomenex luna C18 150mm ×40 mm ×15 μm; mobile phase: [water (TFA)-ACN]; gradient: 60%-90% B 10 min) to obtain Compound 21 (66.41 mg, 80.4 μmol, 2.55% yield, 93.8% purity) after lyophilization as a green solid.

$^1$H NMR (400 MHz, $CDCl_3$) δ 7.88 (t, *J* = 11.2 Hz, 2H), 7.40-7.47 (m, 4H), 7.31-7.36 (m, 5H), 7.17-7.24 (m, 2H), 6.90 (d, *J* = 8.0 Hz, 2H), 5.96-6.21 (m, 2H), 4.38 (s, 2H), 4.13-4.21 (m, 2H), 4.02 (t, *J* = 4.8 Hz, 2H), 3.88 (t, *J* = 6.0 Hz, 2H), 2.97 (br, 2H), 2.78 (t, *J* = 5.6 Hz, 2H), 1.80 (s, 2H), 1.75 (d, *J* = 14 Hz, 12H), 1.49 (t, *J* = 7.2 Hz, 2H).

LCMS: m/z = 661.3, $C_{42}H_{46}ClN_2O_3^+$.

Example 22: Preparation of 2-((E)-2-((E)-2-chloro-3-(2-((E)-3,3-dimethyl-1-(6-oxo-6-phenoxyhexyl)indolin-2-ylide ne) ethenyl)cyclopent-1-en-1-yl)ethenyl)-1-ethyl-3,3-dimethyl-5-sulfonyl-3H-indol-1-iu m chloride (22)

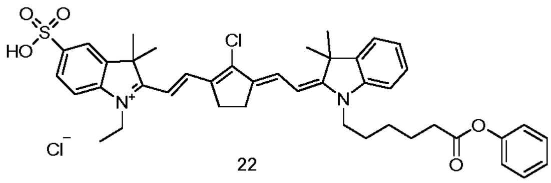

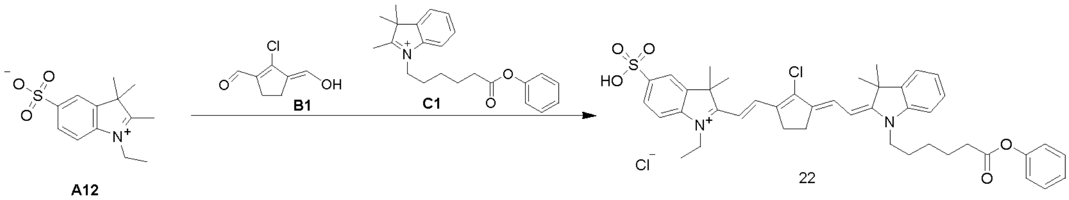


A12

Compound 22 was prepared as a green solid according to the preparation method of Example 21 with Compounds A12, B1 and C1 as raw materials.

$^1$H NMR (400MHz, DMSO-d6) δ 7.70 - 7.79 (m, 3H), 7.62 - 7.64 (m, 2H), 7.36 - 7.46 (m, 5H), 7.24 - 7.34 (m, 2H), 7.02 (d, J = 7.6 Hz, 2H), 6.10 - 6.20 (m, 2H), 4.19 - 4.23 (m, 4H), 2.91 - 2.94 (m, 4H),2.56 - 2.58 (m, 2H), 1.78 - 1.80 (m, 2H), 1.69 - 1.73 (m, 2H), 1.66 (d, J = 1.6 Hz, 12H), 1.48 - 1.50 (m, 2H), 1.29 (d, J = 7.2 Hz, 3H)

LCMS: m/z == 739.4, $C_{43}H_{48}ClN_2O_5S^+$.

Example 23: Preparation of 2-((E)-2-((E)-2-chloro-3-(2-((E)-1-(6-(2,6-dimethylphenoxy)-6-oxohexyl)-3,3-dimethyli ndolin-2-ylidene)ethenyl)cyclopent-1-en-1-yl)ethenyl)-1-(6-(2,6-dimethylphenoxy)-6-o xohexyl)-3,3-dimethyl-3H-indol-1-ium chloride (23)

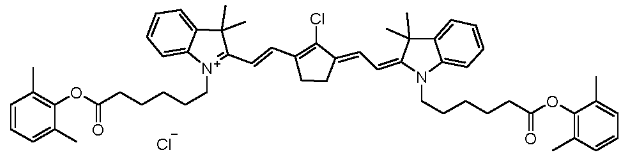

23

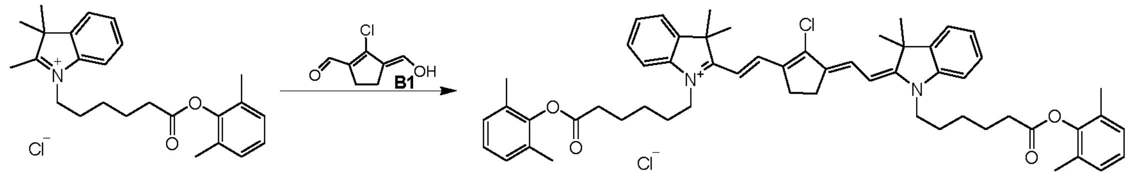


C11 23

Compound 23 was prepared as a green solid according to the preparation method of Example 21 with Compounds B1 and C11 as raw materials.

LCMS: m/z = 877.5, $C_{57}H_{66}ClN_2O_4^+$.

Example 24: Preparation of 2-((E)-2-((E)-2-chloro-3-(2-((E)-3,3-dimethyl-1-(6-oxo-6-phenoxyhexyl)indolin-2-ylide ne) ethenyl)cyclopent-1-en-1-yl)ethenyl)-3,3-dimethyl-1-(6-oxo-6-phenoxyhexyl)-3H-in dol-1-ium chloride (24)

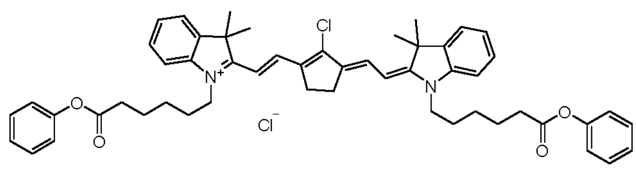

24

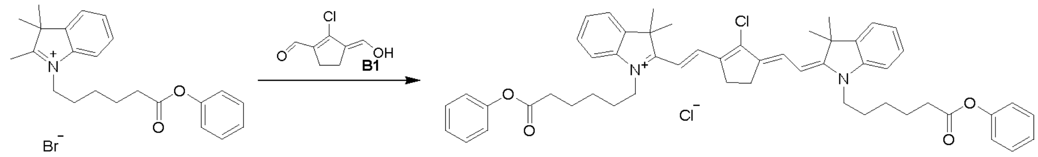


C1B 24

was used, and Compound 24 was prepared as a blue paste according to the preparation method of Example 21 with Compounds B1 and C1B (prepared according to the preparation method of Compound C1) as raw materials.

LCMS: m/z = 821.3, $C_{53}H_{58}ClN_2O_4^+$.

Example 25: Preparation of Compound 25

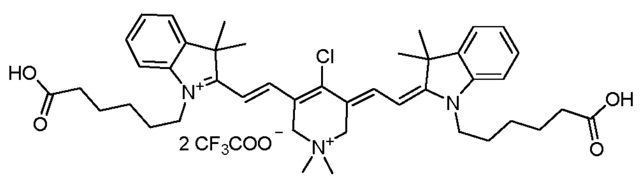

25

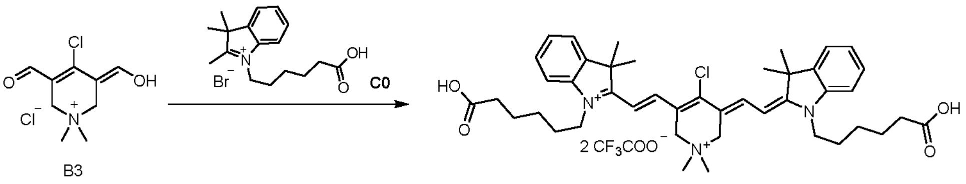


25

was used, and Compound 25 was prepared as a purple oil according to the preparation method of Example 21 with Compounds B3 and C0 as raw materials.

$^{1}H$ NMR (400 MHz, $CDCl_3$) δ 8.30 (d, *J* = 14.4 Hz, 2H), 7.40-7.45 (m, 4H), 7.30-7.34 (m, 2H), 7.18-7.20 (m, 2H), 6.38-6.42 (m, 2H), 4.83 (s, 4H), 4.24 (s, 4H), 4.48 (s, 6H), 2.33-2.37 (m, 4H), 1.78-1.87 (m, 5H), 1.73 (s, 12H), 1.44-1.54 (m, 7H).

LCMS: m/z = 357.2, $C_{43}H_{56}ClN_3O_4^{2+}$.

Example 26: Preparation of 2-((E)-2-((E)-2-chloro-3-(2-((E)-3,3-dimethyl-1-(6-oxo-6-(phenylamino)hexyl)indolin-2-ylidene)ethenyl)cyclohex-1-en-1-yl)ethenyl)-3,3-dimethyl-1-(4-sulfonylbutyl)-3H-ind ol-1-ium formate (26)

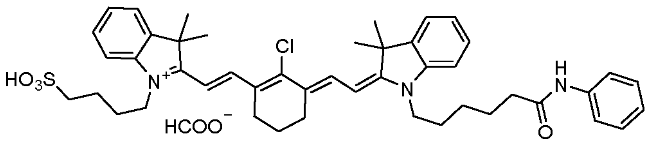

26

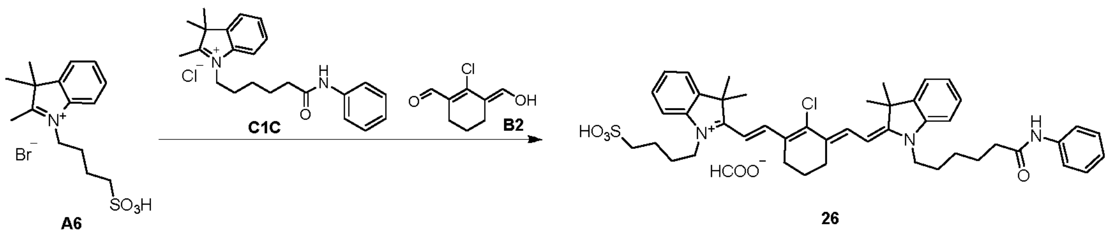


A6 26

was used, and Compound 26 was prepared as a green solid according to the preparation method of Example 21 with Compounds A6, B2 and C1C as raw materials (Compounds A6 and C1C being purchased from Bide Pharm, batch number 221007).

$^{1}H$ NMR: (400MHz, $CDCl_3$)δ 9.62 (s, 1H), 8.32 (dd, *J* = 14.0, 2.0 Hz, 2H), 7.77 (d, *J* = 7.6 Hz, 2H), 7.40 - 7.33 (m, 4H), 7.25 - 7.20 (m, 4H), 7.15 - 7.11 (m, 2H), 7.02 - 6.99 (m, 1H), 6.20 (dd, *J* = 13.6, 4.8 Hz, 2H), 4.13 - 4.04 (m, 4H), 3.04 - 3.01 (m, 2H), 2.67 - 2.60 (m, 6H), 2.11 - 2.01 (m, 4H), 1.95 - 1.91 (m, 4H), 1.85 - 1.83 (m, 1H), 1.69 (d, *J*= 2.4 Hz, 12H), 1.64 - 1.60 (m, 3H).

LCMS: m/z = 780.3, $C_{46}H_{55}ClN_3O_4S^+$.

Example 27A and 27B: Preparation of 1-(5-carboxypentyl)-2-((E)-2-((E)-2-chloro-3-(2-((E)-3,3-dimethyl-1-(6-oxo-6-(phenyla mino)hexyl)indolin-2-ylidene)ethenyl)cyclohex-1-en-1-yl)ethenyl)-3,3-dimethyl-3H-ind ol-1-ium 2,2,2-trifluoroacetate (27A) and 2-((E)-2-((E)-2-chloro-3-(2-((E)-3,3-dimethyl-1-(6-oxo-6-(phenylamino)hexyl)indolin-2-ylidene) ethenyl)cyclohex-1-en-1-yl)ethenyl)-3,3-dimethyl-1-(6-oxo-6-(phenylamino) hexyl)-3H-indol-1-ium 2,2,2-trifluoroacetate (27B)

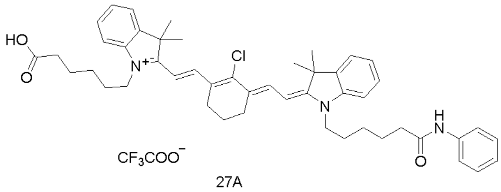

27A

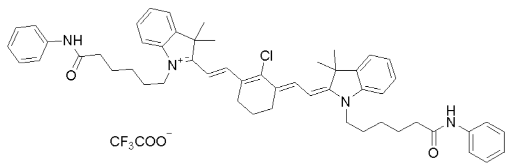

27B

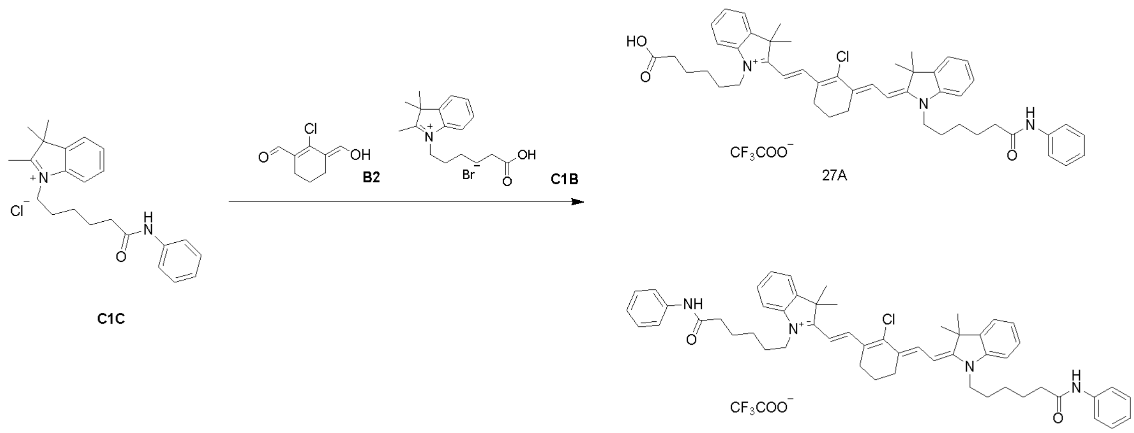


27B

Compound 27A and Compound 27B were both prepared as a green solid according to the preparation method of Example 21 with Compounds C1B, B2 and C1C as raw materials.

**Compound 27A:**

$^1$H NMR (400MHz, $CDCl_3$) $\delta$ 9.11 (s, 1H), 8.34 (t, *J* = 14.4 Hz, 2H), 7.67 (d, *J* = 8.0 Hz, 2H), 7.36 - 7.40 (m, 4H), 7.28 - 7.29 (s, 1H), 7.21 - 7.25 (m, 3H), 7.15 (d, *J* = 8.0 Hz, 1H), 7.07 (d, *J* = 8.0 Hz, 1H), 6.99 (d, *J* = 7.6 Hz, 1H), 6.03 - 6.02 (m, 2H), 3..98 - 4.09 (m, 4H), 2.63 - 2.64 (m, 4H), 2.51 (t, *J* = 6.8 Hz, 2H), 2.42 (d, *J* = 6.8 Hz, 2H), 1.76 - 1.92 (m, 9H), 1.70 (d, *J* = 12.0 Hz, 12H), 1.53 - 1.55 (m, 5H).

LCMS: m/z = 758.8, $C_{48}H_{57}ClN_30_3^+$.

**Compound 27B:**

$^1$H NMR (400MHz, $CDCl_3$) $\delta$ 9.19 (brs, 1H), 8.31 - 8.34 (m, 2H), 7.65 (d, *J* = 6.8 Hz, 2H), 7.36 - 7.40 (m, 4H), 7.22 - 7.26 (m, 3H), 7.14 (d, *J* = 8.4 Hz, 1H), 7.07 (d, *J* = 7.6 Hz, 1H), 6.99 (d, *J* = 6.4 Hz, 1H), 6.08 - 6.21 (m, 2H), 3.99 - 4.08 (m, 4H), 2.63 (s, 4H), 2.49 (t, *J* = 6.8 Hz, 4H), 1.79 - 1.88 (m, 10H), 1.71 (d, *J* = 14.4 Hz, 12H), 1.53 - 1.55 (m, 4H).

LCMS: m/z = 758.4, $C_{48}H_{57}ClN_3O_3^+$.

Example 28: Preparation of Compound 28

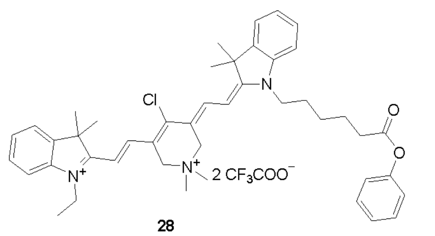

28

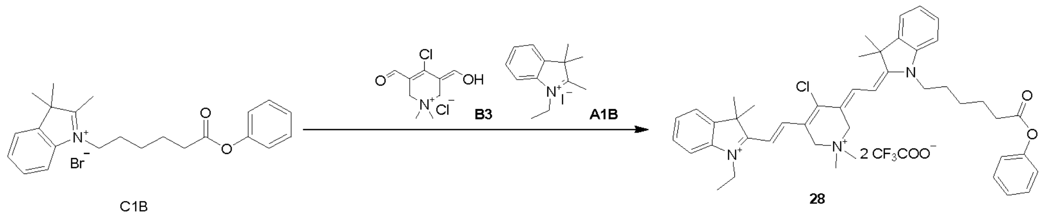

C1B B3 A1B 28

Compound 28 was prepared as a blue solid according to the preparation method of Example 21 with Compounds C1B, B3 and A1B (prepared according to the preparation method of Compound A1) as raw materials.

$^1$H NMR (400MHz, $CDCl_3$)$\delta$ 8.30 - 8.28 (m, 2H), 7.47 - 7.30 (m, 8H), 7.29 - 7.28 (m, 2H), 7.25 - 7.18 (m, 3H), 7.04 (d, $J$ = 7.6 Hz, 2H), 5.28 -5.20 (m, 2H), 4.69 - 4.59 (m, 2H), 3.62 - 3.54 (m, 4H), 2.62 - 2.59 (m, 1H), 1.98 - 1.81 (m, 5H), 1.72 (s, 20H), 1.53 -1.48 (m, 3H).

LCMS: m/z = 352.0, $C_{45}H_{54}ClN_3O_2^{2+}$.

Example 29: Preparation of 2-((E)-2-((E)-4-chloro-5-(2-((E)-3,3-dimethyl-1-(6-oxo-6-phenoxyhexyl)indolin-2-ylide ne) ethenyl)-1,1-dimethyl-1,2,5,6-tetrahydropyridin-1-ium-3-yl)ethenyl)-3,3-dimethyl-1-(6-oxo-6-phenoxyhexyl)-3H-indol-1-ium chloride (29)

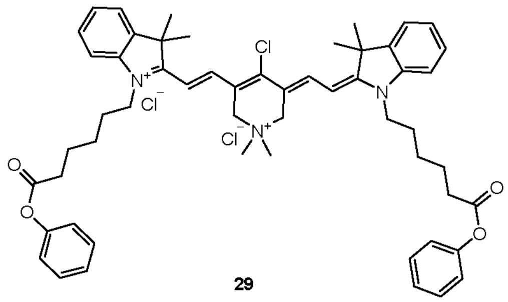

29

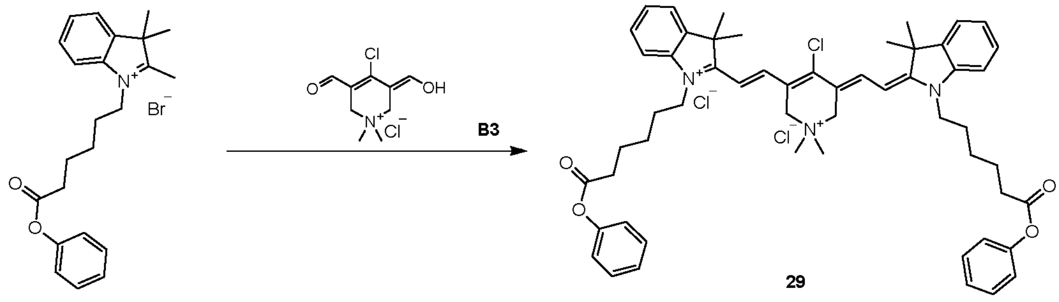

B3 29 C1B

Compound 29 was prepared as a green paste according to the preparation method of Example 21 with Compounds C1B and B3 as raw materials.

LCMS: m/z = 433.1, $C_{55}H_{64}ClN_3O_4^{2+}$.

Example 30: Preparation of 2-((E)-2-((E)-3-(2-((E)-1-(5-carboxypentyl)-3,3-dimethylindolin-2-ylidene)ethenyl)-2-c hlorocyclohex-1-en-1-yl)ethenyl)-1-ethyl-3,3-dimethyl-3H-indol-1-ium chloride (30)

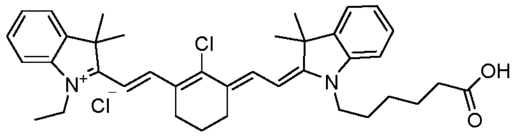

30

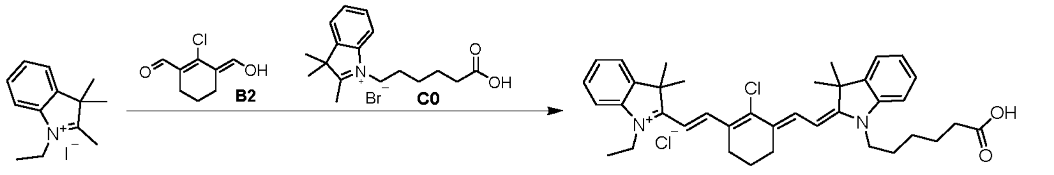


A1B

30

Compound 30 was prepared as a green solid according to the preparation method of Example 21 with Compounds A1B, B2 and C0 as raw materials.

LCMS: m/z =597.5, $C_{38}H_{46}ClN_2O_2^+$.

Example 33: Preparation of 2-((E)-2-((E)-2-chloro-3-(2-((E)-3,3-dimethyl-1-(5-oxo-5-phenoxypentyl)indolin-2-ylid ene) ethenyl)cyclohex-1-en-1-yl)ethenyl)-3,3-dimethyl-1-(5-oxo-5-phenoxypentyl)-3H-i ndol-1-ium chloride (33)

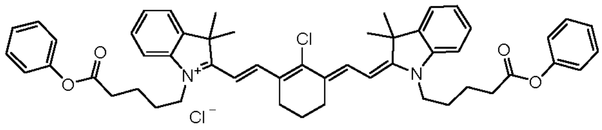

33

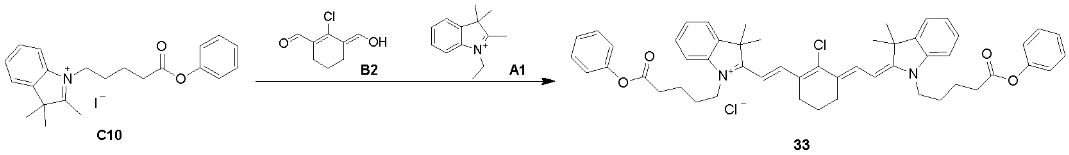


C10

33

and Compound 33 was prepared as a green solid according to the preparation method of Example 21 with Compounds B2, A1 and C10 as raw materials.

$^1$H NMR (400MHz, $CDCl_3$) δ 8.34 (d, *J* = 14.0 Hz, 2H), 7.34 - 7.38 (m, 8H), 7.20 - 7.26 (m, 6H), 7.03 (d, *J* = 7.6 Hz, 4H), 6.32 (d, *J* = 14.0 Hz, 2H), 4.31 - 4.35 (m, 4H), 2.70 - 2.75 (m, 8H), 1.90 - 2.03 (m, 10H), 1.73 (s, 12H).

LCMS: m/z = 807.4, $C_{52}H_{56}ClN_2O4^+$.

Example 34: Preparation of 2-((E)-2-((E)-2-chloro-3-(2-((E)-3,3-dimethyl-1-(5-oxo-5-phenoxypentyl)indolin-2-ylid ene) ethenyl)cyclohex-1-en-1-yl)ethenyl)-1-ethyl-3,3-dimethyl-3H-indol-1-ium chloride (34)

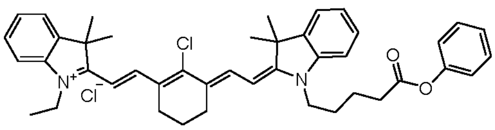

34

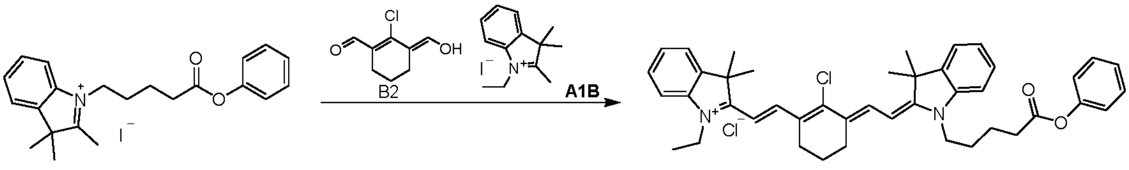


C10

34

Compound 34 was prepared as a green solid according to the preparation method of Example 21 with Compounds B2, A1B and C10 as raw materials.

$^1$H NMR (400MHz, $CDCl_3$) δ 8.32 - 8.37 (m, 2H), 7.34 - 7.42 (m, 6H), 7.20 - 7.25 (m, 5H), 7.02 - 7.04 (m, 2H), 6.25

-6.35 (m, 2H), 4.26 - 4.35 (m, 4H), 2.70 - 2.78 (m, 6H), 1.94 - 2.02 (m, 6H), 1.73 (s, 12H), 1.47 (t, *J* = 7.2 Hz, 3H).

LCMS: m/z = 659.3, $C_{43}H_{48}ClN_2O_2^+$.

Example 35: Preparation of 2-((E)-2-((E)-2-chloro-3-(2-((E)-3,3-dimethyl-1-(2-((6-oxo-6-phenoxyhexyl)oxy)ethyl)i ndolin-2-ylidene)ethenyl)cyclohex-1-en-1-yl)ethenyl)-1-ethyl-3,3-dimethyl-3H-indol-1-ium 2,2,2-trifluoroacetate (35)

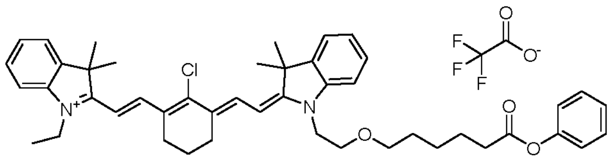

35

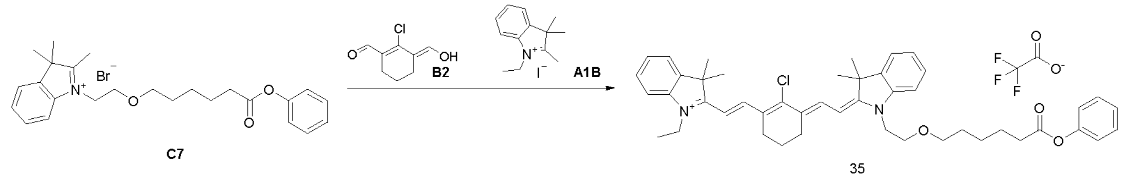


C7

35

Compound 35 was prepared as a blue solid according to the preparation method of Example 21 with Compounds B2, A1B and C7 as raw materials.

$^1$H NMR: (400 MHz, $CDCl_3$)δ 8.37 (t, *J* = 31.6 Hz, 2H), 7.37-7.40 (m, 6H), 7.32-7.34 (m, 4H), 7.24-7.25 (m, 1H), 7.02-7.04 (m, 2H), 6.33 (d, *J* = 14.4 Hz, 1H), 6.10 (d, *J* = 14 Hz, 1H), 4.31-4.34 (m, 2H), 4.09-4.14 (m, 2H), 3.83-3.86 (m, 2H), 3.42 (t, *J* = 6.4 Hz, 2H), 2.67 (s, 4H), 2.44 (t, *J* = 7.6 Hz, 2H), 1.95-1.97 (m, 2H), 1.71 (d, *J* = 10 Hz, 12H), 1.64-1.66 (m, 2H), 1.62-1.64 (m, 2H), 1.42-1.44 (m, 3H), 1.31-1.40 (m, 2H).

LCMS: m/z = 717.4, $C_{46}H_{54}ClN_2O_3^+$.

Example 36: Preparation of 2-((E)-2-((E)-2-chloro-3-(2-((E)-3,3-dimethyl-1-(2-(4-oxo-4-phenoxybutoxy)ethyl)indo lin-2-ylidene)ethenyl)cyclohex-1-en-1-yl)ethenyl)-1-ethyl-3,3-dimethyl-3H-indol-1-ium 2,2,2-trifluoroacetate (36)

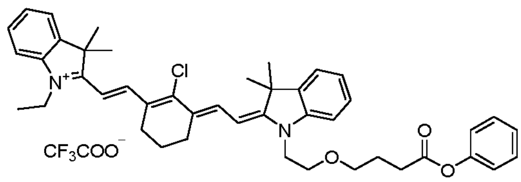

**36**

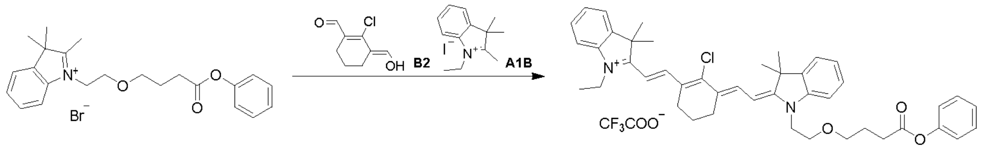


C8

36

Compound 36 was prepared as a blue solid according to the preparation method of Example 21 with Compounds B2, A1B and C8 as raw materials.

$^1$H NMR (400 MHz, $CDCl_3$) δ 8.28-8.39 (m, 2H), 7.36-7.43 (m, 5H), 7.30-7.34 (m, 2H), 7.14-7.26 (m, 4H), 6.97-7.01 (m, 2H), 6.49-6.55 (m, 1H), 6.14-6.17 (m, 1H), 4.58 (s, 2H), 4.21 (s, 2H), 3.93-3.99 (m, 2H), 3.53 (t, *J* = 6.0 Hz, 2H), 2.62-2.81 (m, 4H), 2.47 (t, *J* = 7.6 Hz, 2H), 1.86-1.96 (m, 4H), 1.72 (d, *J* = 8.4 Hz, 12H), 1.44 (t, *J* = 7.2 Hz, 3H).

$^{19}$F NMR (400 MHz, $CDCl_3$) δ 75.946.

LCMS: m/z = 689.4, $C_{44}H_{50}ClN_2O_3^+$.

Example 37: 2-((E)-2-((E)-2-chloro-3-(2-((E)-3,3-dimethyl-1-(2-((5-oxo-5-phenoxypentyl)oxy)ethyl) indolin-2-ylidene) ethenyl)cyclohex-1-en-1-yl)ethenyl)-1-ethyl-3,3-dimethyl-3H-indol-1 -ium 2,2,2-trifluoroacetate (37A) YH-0656 and 2-((E)-2-((E)-2-chloro-3-(2-((E)-3,3-dimethyl-1-(2-((5-oxo-5-phenoxypentyl)oxy)ethyl) indolin-2-ylidene)ethenyl)cyclohex-1-en-1-yl)ethenyl)-3,3-dimethyl-1-(2-((5-oxo-5-phe noxypentyl)oxy)ethyl)-3H-indol-1-ium (37B)

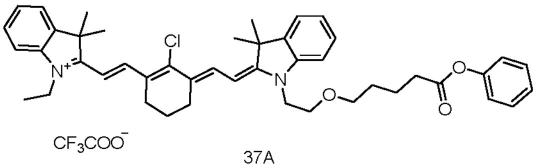

37A

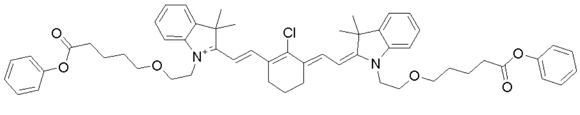

37B

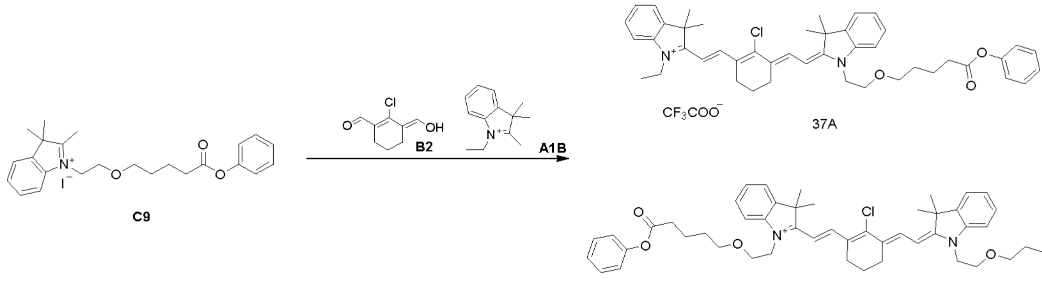


C9

37A

37B

Compound 37A and Compound 37B were both prepared as a green solid according to the preparation method of Example 21 with Compounds B2, A1B and C9 as raw materials.

Compound **37A:**

LCMS: m/z = 703.4, $C_{45}H_{52}ClN_2O_3{}^+$.

Compound **37B:**

$^1$H NMR (400MHz, $CDCl_3$)$\delta$ 8.27 (d, *J* = 14.4 Hz, 2H), 7.28 - 7.31 (m, 7H), 7.16 - 7.20 (m, 7H), 6.95 (d, *J* = 7.6 Hz, 4H), 6.24 (d, *J* = 14.0 Hz, 2H), 4.27 (t, *J* = 4.4 Hz, 4H), 3.79 (t, *J* = 4.8 Hz, 4H), 3.40 (t, *J* = 6.0 Hz, 4H), 2.59 (t, *J* = 5.2 Hz, 4H), 2.39 (t, *J* = 7.2 Hz, 4H), 1.85 - 1.86 (m, 2H), 1.65 (s, 12H), 1.58 - 1.62 (m, 4H),.1.53 - 1.54 (m, 4H).

LCMS: m/z = 859.6, $C_{56}H_{64}ClN_2O_6{}^+$.

Example 38: Preparation of (E)-4-chloro-3-(2-((E)-3,3-dimethyl-1-(5-oxo-5-phenoxypentyl)indolin-2-ylidene)ethen yl)-1,1-dimethyl-5-(((Z)-1-methyl-2-oxoindolin-2-ylidene)methyl)-1,2,3,6-tetrahydropy ridin-1-ium chloride (38)

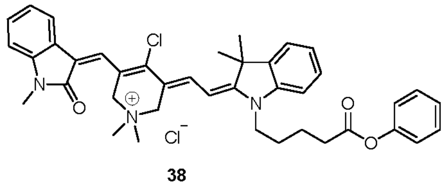

38

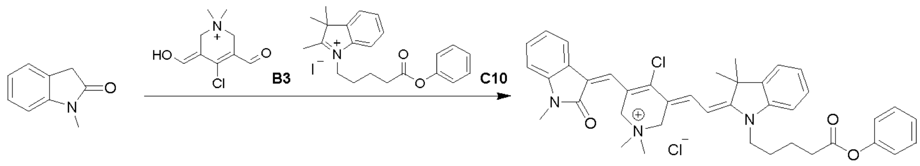


A14

38

Compound 38 was prepared as a blue solid according to the preparation method of Example 21 with Compounds B3, A14 (purchased from Bide Pharm, batch number 220918) and C10 as raw materials.

$^1$H NMR (400 MHz, $CDCl_3$) δ 8.68 (s, 2H), 7.93 (d, *J* = 13.6 Hz, 1H), 7.74 (s, 1H), 7.49 (d, *J* = 7.6 Hz, 1H), 7.36 (t, *J* = 7.6 Hz, 3H), 7.22-7.25 (m, 2H), 7.03-7.06 (m, 3H), 6.86-6.88 (m, 1H), 6.81-6.83 (m, 1H), 5.96-6.00 (m, 1H), 4.95 (s, 2H), 4.85 (s, 2H), 3.99-4.06 (m, 2H), 3.33 (s, 6H), 3.26 (s, 3H), 2.67-2.75 (m, 2H), 1.92 (s, 4H), 1.67 (s, 6H).

LCMS: m/z=648.4, $C_{40}H_{43}ClN_3O_3^+$.

Example 39: Preparation of 2-((E)-2-((E)-2-chloro-3-(2-((E)-1-(6-(2,6-dimethylphenoxy)-6-oxohexyl)-3,3-dimethyli ndolin-2-ylidene)ethenyl)cyclohex-1-en-1-yl)ethenyl)-1-ethyl-3,3-dimethyl-5-(methylsu lfonyl)-3H-indol-1-ium chloride (39)

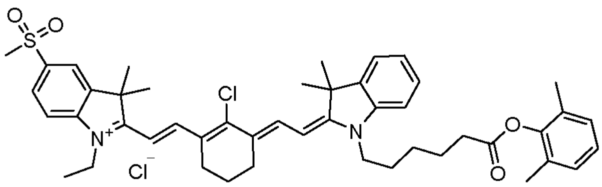

39

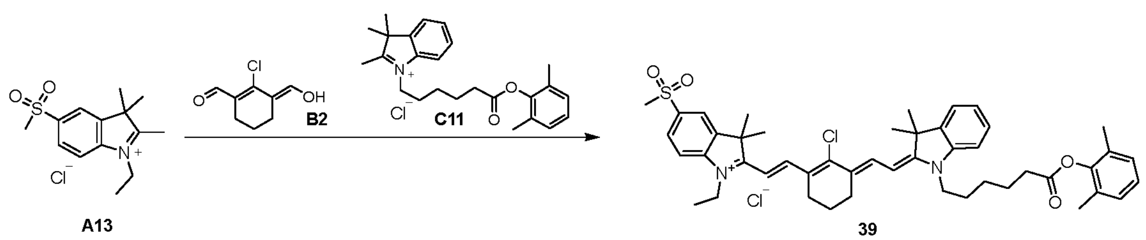


Compound 39 was prepared as a blue solid according to the preparation method of Example 21 with Compounds B2, A13 and C11 as raw materials.

$^1$H NMR (400MHz, MeOD) δ 8.66 (d, *J* = 14.8 Hz, 1H), 8.18 (d, *J* = 13.2 Hz, 1H), 7.93 - 7.90 (m, 2H), 7.68 - 7.47 (m, 4H), 7.26 (d, *J* = 8.4 Hz, 1H), 7.05 - 7.01 (m, 3H), 6.70 (d, *J* = 14.8 Hz, 1H), 6.08 (d, *J* = 13.2 Hz, 1H), 4.46 (t, *J* = 6.8 Hz, 2H), 4.07 (dd, $J_1$ = 7.2 Hz, $J_2$ = 7.2 Hz, 2H), 3.14 (s, 3H), 2.73 - 2.67 (m, 6H), 2.05 (s, 6H), 2.03 - 1.99 (m, 2H), 1.91 - 1.81 (m, 4H), 1.78 (s, 6H), 1.74 (s, 6H), 1.65 - 1.59 (m, 2H), 1.35 (t, *J*= 7.2 Hz, 3H).

LCMS: m/z = 779.5, $C_{47}H_{56}ClN_2O_4S^+$.

Example 40: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-1-ethyl-3,3-dimethyl-5-(methylsulfonyl)-3H-in dol-1-ium chloride (40)

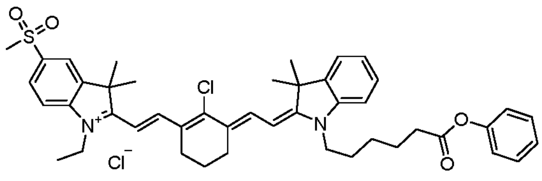

40

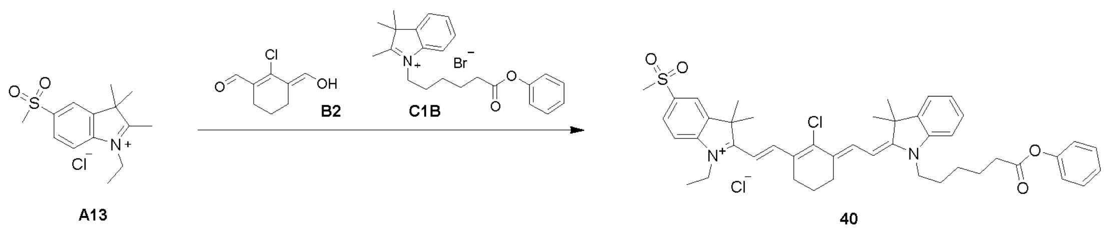

Compound 40 was prepared as a blue solid according to the preparation method of Example 21 with Compounds B2, A13 and C1B as raw materials.

$^1$H NMR (400MHz, DMSO-d6) δ 8.45 (d, *J* = 14.8 Hz, 1H), 8.05 - 8.01 (m, 2H), 7.87 (dd, $J_1$ = 1.6 Hz, $J_2$ = 6.4 Hz, 1H), 7.76 (dd, $J_1$ = 7.6 Hz, $J_2$ = 10.0 Hz, 2H), 7.55 (t, *J* = 7.2 Hz, 1H), 7.48 (t, *J* = 7.6 Hz, 1H), 7.41 - 7.37 (m, 3H), 7.27 - 7.25 (m, 1H), 7.03 (d, *J* = 8.4 Hz, 2H), 6.73 (d, *J* = 14.8 Hz, 1H), 6.10 (d, J = 13.6 Hz, 1H), 4.48 (t *J* = 2.8 Hz, 2H), 4.10 - 4.08 (m, 2H), 3.21 (s, 3H), 2.73 - 2.68 (m, 4H), 2.59 (t, *J* = 7.2 Hz, 2H), 1.88 - 1.82 (m, 4H), 1.75 - 1.68 (m, 14H), 1.52 - 1.50 (m, 2H), 1.24 (t, *J* = 7.2 Hz, 3H).

LCMS: m/z = 751.4, $C_{45}H_{52}ClN_2O_4S^+$.

Example 41: Preparation of 5-(tert-butoxycarbonyl)-2-((E)-2-((E)-2-chloro-3-(2-((E)-3,3-dimethyl-1-(6-oxo-6-phen oxyhexyl)indolin-2-ylidene)ethenyl)cyclohex-1-en-1-yl)ethenyl)-1-ethyl-3,3-dimethyl-3 H-indol-1-ium bromide (41)

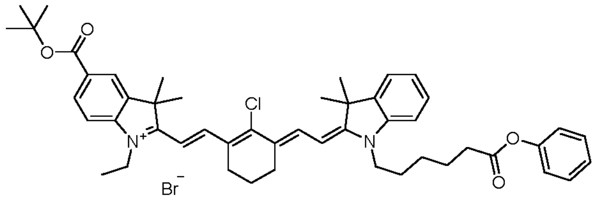

41

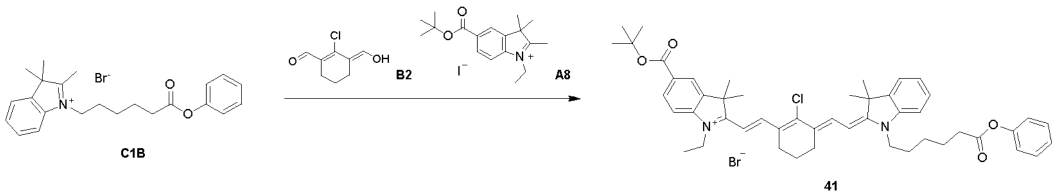

Compound 41 was prepared as a green oil according to the preparation method of Example 21 with Compounds B2, A8 and C1B as raw materials.

LCMS: m/z = 773.5, $C_{49}H_{58}ClN_2O_4^+$.

Example 42: Preparation of 5-(tert-butoxycarbonyl)-2-((E)-2-((E)-2-chloro-3-(2-((E)-3,3-dimethyl-1-(6-oxo-6-phen oxyhexyl)indolin-2-ylidene)ethenyl)cyclopent-1-en-1-yl)ethenyl)-1-ethyl-3,3-dimethyl-3H-indol-1-ium bromide (42)

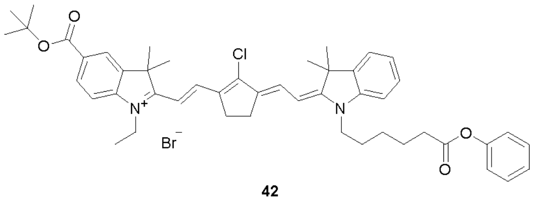

42

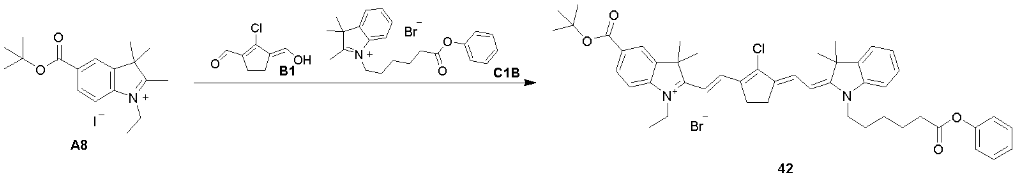

, and Compound 42 was prepared as a green oil according to the preparation method of Example 21 with Compounds B1, A8 and C1B as raw materials.

LCMS: m/z = 759.3, $C_{48}H_{56}ClN_2O_4^+$.

Example 43: Preparation of 5-(tert-butoxycarbonyl)-2-((E)-2-((E)-3-(2-((E)-1-(5-carboxypentyl)-3,3-dimethylindoli n-2-ylidene)ethenyl)-2-chlorocyclohex-1-en-1-yl)ethenyl)-1,3,3-trimethyl-3H-indol-1-iu m bromide (43)

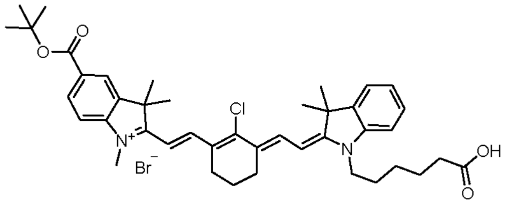

43

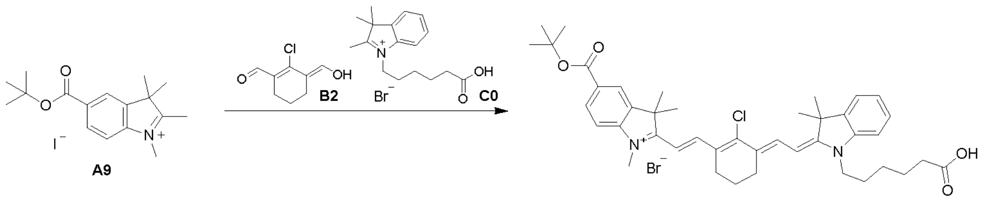


43

, and Compound 43 was prepared as a green paste according to the preparation method of Example 21 withCompounds B1, A9 and C0 as raw materials.

LCMS: m/z = 683.4, $C_{42}H_{52}ClN_2O_4^+$.

Example 44: Preparation of 5-(tert-butoxycarbonyl)-2-((E)-2-((E)-2-chloro-3-(2-((E)-3,3-dimethyl-1-(6-oxo-6-phen oxyhexyl)indolin-2-ylidene)ethenyl)cyclohex-1-en-1-yl)ethenyl)-1,3,3-trimethyl-3H-ind ol-1-ium bromide (44)

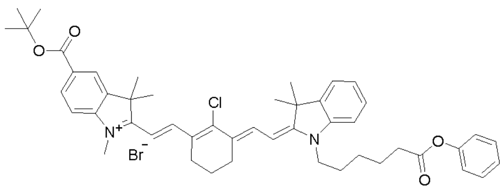

44

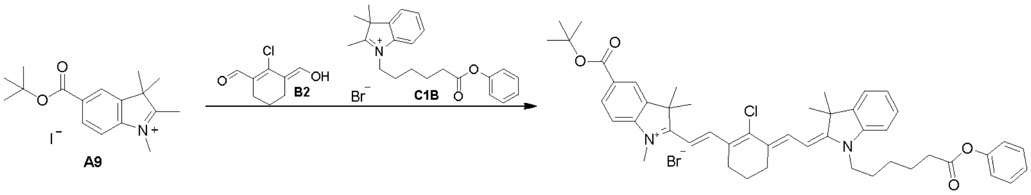


44

Compound 44 was prepared as a green paste according to the preparation method of Example 21 with Compounds B2, A9 and C1B as raw materials.

LCMS: m/z = 759.6, $C_{48}H_{56}ClN_2O_4^+$.

Example 45: Preparation of 2-((E)-2-((E)-2-chloro-3-(2-((E)-1-(6-(2,6-dimethylphenoxy)-6-oxohexyl)-3,3-dimethyli ndolin-2-ylidene)ethenyl)cyclohex-1-en-1-yl)ethenyl)-1-ethyl-3,3-dimethyl-3H-indol-1-ium chloride(45)

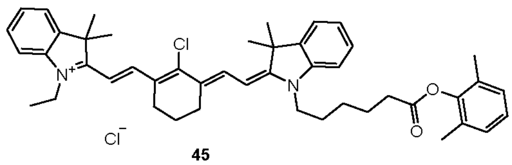

45

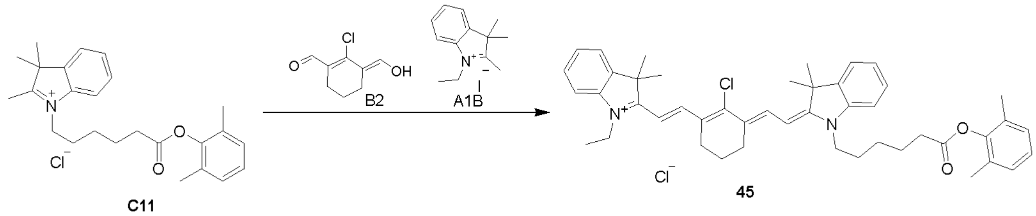


C11

45

Compound 45 was prepared as a black-brown solid according to the preparation method of Example 21 with Compounds B2, A1B and C11 as raw materials.

LCMS: m/z = 701.4, $C_{46}H_{54}ClN_2O_2^+$.

Example 46: Preparation of 5-(tert-butoxycarbonyl)-2-((E)-2-((E)-4-chloro-5-(2-((E)-3,3-dimethyl-1-(6-oxo-6-phen oxyhexyl)indolin-2-ylidene)ethenyl)-1,1-dimethyl-1,2,5,6-tetrahydropyridin-1-ium-3-yl) ethenyl)-1-ethyl-3,3-dimethyl-3H-indol-1-ium chloride (46)

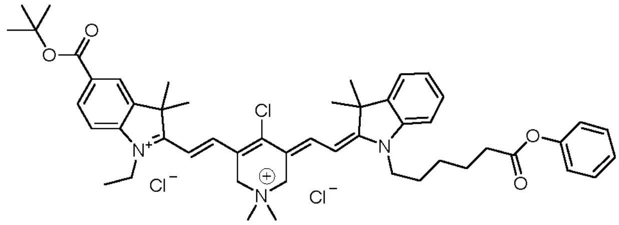

46

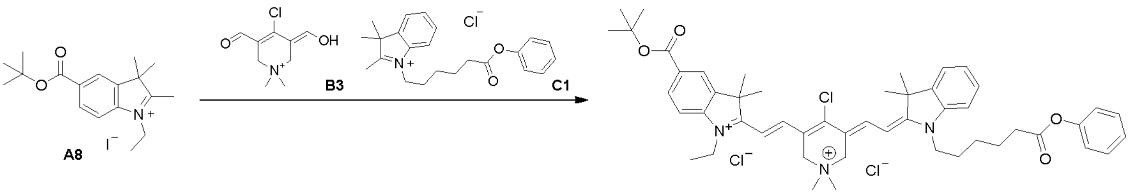


A8

46

A crude of Compound 46 was prepared as a purple oil according to the preparation method of Example 21 with Compounds B3, A8 and C1 as raw materials.

LCMS: m/z = 402.0, $C_{50}H_{62}ClN_3O_4^{2+}/2$.

Example 47: Preparation of 2-((E)-2-((E)-2-chloro-3-(2-((E)-5-methoxy-3,3-dimethyl-1-(6-oxo-6-phenoxyhexyl)ind olin-2-ylidene)ethenyl)cyclohex-1-en-1-yl)ethenyl)-5-methoxy-1,3,3-trimethyl-3H-indo 1-1-ium (47)

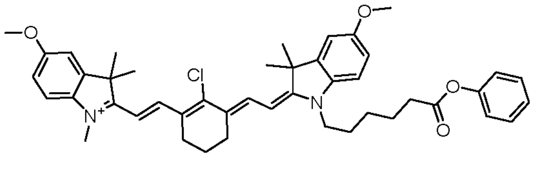

47

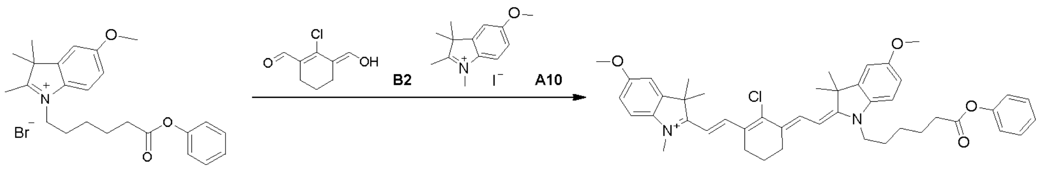


C21B

47

Compound 47 was prepared as a blue solid according to the preparation method of Example 21 with Compounds B2, A10 and C21B as raw materials.

$^{1}$H NMR (400MHz, DMSO) δ 8.15-8.21 (m, 2H), 7.56-7.41 (m, 4H), 7.29-7.31 (m, 2H), 7.25 (t, $J$ = 7.2 Hz, 1H), 6.96-7.03 (m, 4H), 6.20-6.26 (m, 2H), 4.18-4.22 (m, 2H), 3.81 (d, $J$ = 2.8 Hz, 6H), 3.67 (s, 3H), 2.66-2.67 (m, 4H), 2.58 (t, $J$ = 7.2 Hz, 2H), 1.77-1.82 (m, 4H), 1.68-1.74 (m, 2H), 1.66 (d, $J$ = 3.6 Hz, 12H), 1.46-1.50 (m, 2H).

LCMS: m/z = 719.3, $C_{45}H_{52}ClN_2O_4^+$.

Example 48: 2-((E)-2-((E)-2-chloro-3-(2-((E)-3,3-dimethyl-1-(6-oxo-6-phenoxyhexyl)indolin-2-ylide ne)ethenyl)cyclohex-1-en-1-yl)ethenyl)-1-(2-hydroxyethyl)-3,3-dimethyl-3H-indol-1-iu m formate (48)

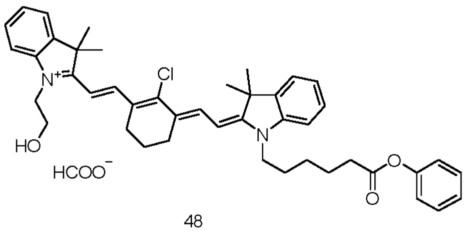

48

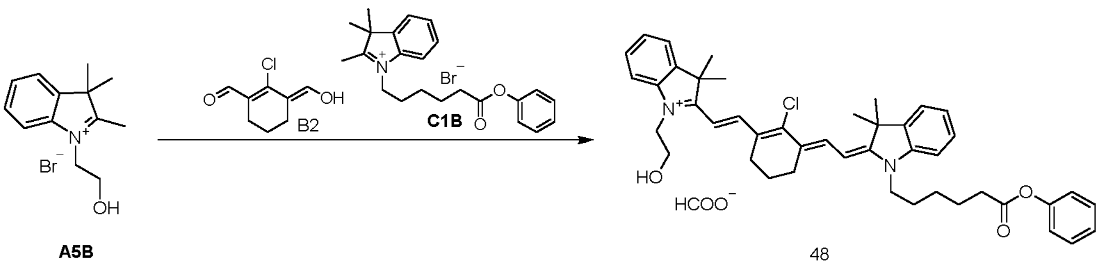


A5B

48

Compound 48 was prepared as a blue solid according to the preparation method of Example 21 withCompounds B2, A5B (prepared according to the preparation method of Compound A5) and C1B as raw materials.

$^{1}$H NMR (400 MHz, MeOD)δ 8.44 (dd, $J_1$ = 14.4 Hz, $J_2$ = 26.4 Hz, 2H), 7.53 (t, $J$ = 7.6 Hz, 2H), 7.41-7.45 (m, 2H), 7.26-7.38 (m, 6H), 7.19-7.23 (m, 1H), 6.98-7.00 (m, 2H), 6.45 (d, $J$ = 14.4 Hz, 1H), 6.27 (d, $J$ = 14.4 Hz, 1H), 4.32 (t, $J$ = 5.2 Hz, 2H), 4.20 (t, $J$= 7.2 Hz, 2H), 3.97 (t, $J$ = 5.2 Hz, 2H), 2.67-2.72 (m, 4H), 2.61 (t, $J$ = 6.4 Hz, 2H), 1.88-1.93 (m, 4H), 1.81-1.85 (m, 2H), 1.77 (s, 6H), 1.72 (s, 6H), 1.55-1.61 (m, 2H).

LCMS: m/z = 689.4, $C_{44}H_{50}ClN_2O_3^+$.

Example 49: Preparation of 2-((E)-2-((E)-3-(2-((E)-1-(5-carboxypentyl)-3,3-dimethylindolin-2-ylidene)ethenyl)-2-c hlorocyclohex-1-en-1-yl)ethenyl)-1-(2-hydroxyethyl)-3,3-dimethyl-3H-indol-1-ium chloride (49)

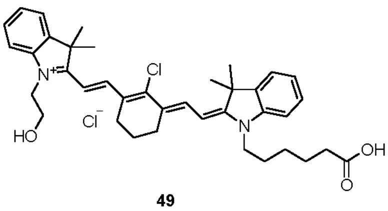

49

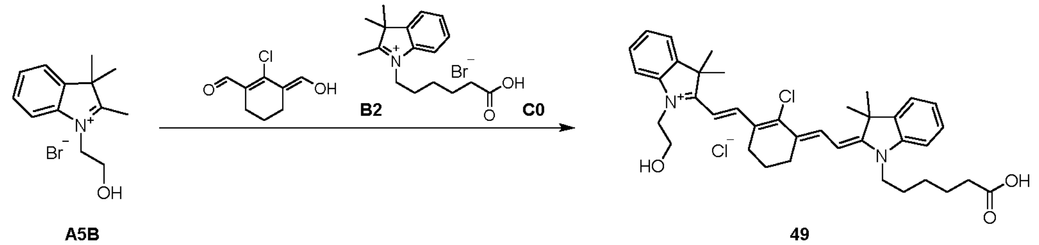

A5B

49

Compound 49 was prepared as a blue solid according to the preparation method of Example 21 with Compounds B2, A5B and C0 as raw materials.

$^{1}$H NMR (400 MHz, MeOD)δ 8.45 (br, 2H), 7.53 (d, $J$ = 8.0 Hz, 2H), 7.36-7.45 (m, 3H), 7.24-7.32 (m, 3H), 6.45 (br, 1H), 6.27 (br, 1H), 4.40 (s, 2H), 4.17 (s, 2H), 3.98 (t, $J$ = 5.2 Hz, 2H), 2.74 (br, 4H), 2.32 (t, $J$ = 7.6 Hz, 2H), 1.93-2.01 (m, 2H), 1.83-1.90 (m, 2H), 1.77 (s, 6H), 1.73 (s, 6H), 1.67-1.71 (m, 2H), 1.47-1.55 (m, 2H).

LCMS: m/z = 613.3, $C_{38}H_{46}ClN_2O_3^+$.

Example 50: Preparation of 2-((E)-2-((E)-2-chloro-3-(2-((E)-3,3-dimethyl-1-(6-oxo-6-phenoxyhexyl)indolin-2-ylide ne) ethenyl)-5-(dimethylamino)cyclohex-1-en-1-yl)ethenyl)-1,3,3-trimethyl-3H-indol-1-i um formate (50)

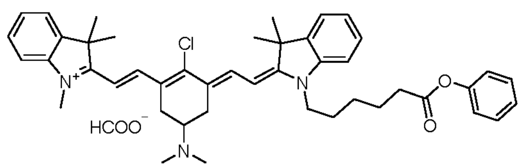

50

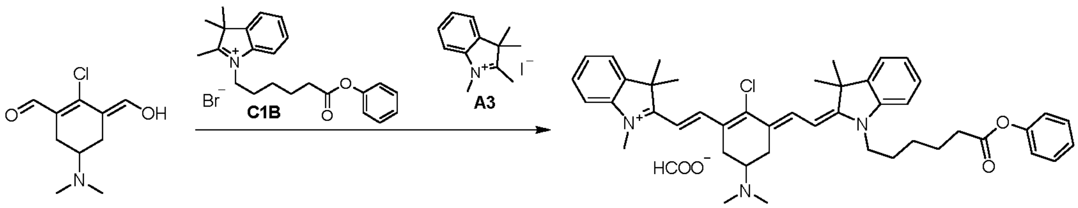

B4

50

and Compound 50 was prepared as a green solid according to the preparation method of Example 21 with Compounds B4, A3 and C1B as raw materials.

$^{1}$H NMR (400 MHz, MeOD)δ 8.46 (t, $J$ = 14.0 Hz, 2H), 7.55-7.57 (m, 2H), 7.44-7.49 (m, 2H), 7.31-7.42 (m, 6H), 7.20-7.24 (m, 1H), 6.99-7.03 (m, 2H), 6.37-6.43 (m, 2H), 4.32-4.34 (m, 2H), 3.81 (s, 3H), 3.60-3.72 (m, 1H), 3.00 (s, 6H), 2.82-2.91 (m, 2H), 2.64 (t, $J$ = 7.2 Hz, 2H), 1.81-2.00 (m, 6H), 1.75 (dd, $J_1$ = 0.8 Hz, $J_2$ = 6.4 Hz, 12H), 1.61-1.66 (m, 2H).

LCMS: m/z = 702.4, $C_{45}H_{53}ClN_3O_2^+$.

Example 51: Preparation of 2-((E)-2-((E)-2-chloro-3-(2-((E)-3,3-dimethyl-1-(2-(2-(3-oxo-3-phenoxypropoxy)ethox y) ethyl)indolin-2-ylidene)ethenyl)cyclohex-1-en-1-yl)ethenyl)-1,3,3-trimethyl-3H-indol -1-ium formate (51)

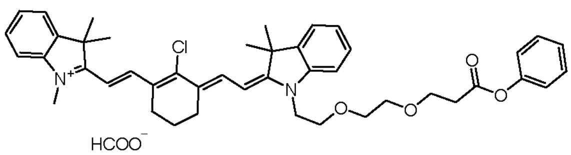

51

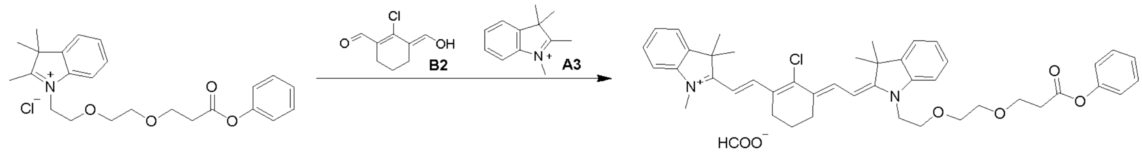


C13 51

Compound 51 was prepared as a green solid according to the preparation method of Example 21 with Compounds B2, A3 and C13 as raw materials.

$^1$H NMR (400MHz, $CDCl_3$) $\delta$ 8.26-8.31 (m, 2H), 7.29-7.31 (m, 5H), 7.15-7.22 (m, 6H), 7.00 (d, $J$ = 8.0 Hz, 2H), 6.27 (d, $J$ = 14.0 Hz, 1H), 6.10 (d, $J$ = 14.0 Hz, 1H), 4.31 (m, 2H), 3.87 (s, 2H), 3.67 (t, $J$ = 6.0 Hz, 2H), 3.62 (s, 3H), 3.52-3.56 (m, 4H), 2.62-2.68 (m, 6H), 1.89-1.90 (m, 2H), 1.65(d, $J$ = 6.8 Hz, 12H).

LCMS: m/z = 705.3, $C_{44}H_{50}ClN_2O_4^+$.

Example 52: Preparation of 2-((E)-2-((E)-2-chloro-3-(2-((E)-3,3-dimethyl-1-(2-(3-oxo-3-phenoxypropoxy)ethyl)ind olin-2-ylidene)ethenyl)cyclohex-1-en-1-yl)ethenyl)-1,3,3-trimethyl-3H-indol-1-ium chloride (52)

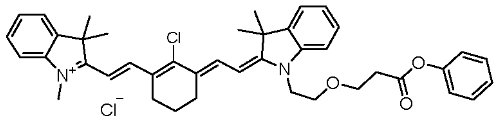

52

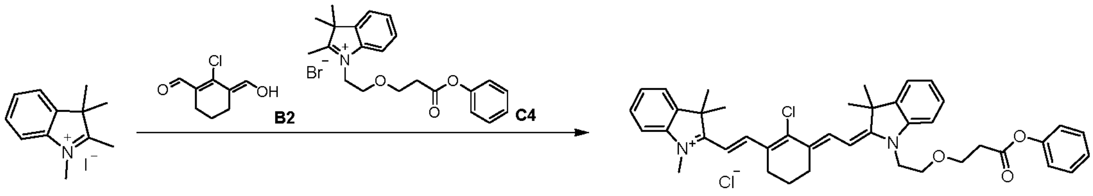


A3 52

Compound 52 was prepared as a green solid according to the preparation method of Example 21 with Compounds B2, A3 and C4 as raw materials.

$^1$H NMR (400MHz, MeOD); $\delta$ 7.52 - 7.42 (m, 8H), 7.29 - 7.26 (m, 4H), 7.18 - 7.14 (m, 2H), 6.85 - 6.83 (m, 3H), 3.98 - 3.90 (m, 2H), 3.80 (t, $J$ = 5.60 Hz, 4H), 2.74 (t, $J$= 5.60 Hz, 4H), 1.73 (d, $J$ = 6.00 Hz, 10H), 1.69 (s, 9H).

LCMS: m/z = 661.4, $C_{42}H_{46}ClN_2O_3^+$.

Example 53: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-3,3,7-trimethyl-3H-pyrrolo[2,3-b]pyridin-7-ium 2,2,2-trifluoroacetate (53)

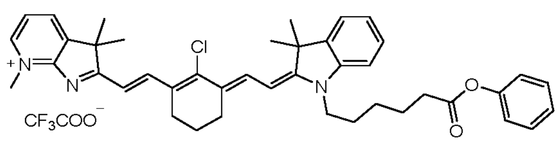

53

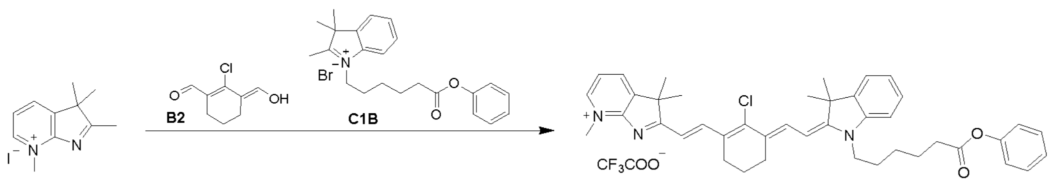


A3 53

Compound 53 was prepared as a green solid according to the preparation method of Example 21 with Compounds B2, A3 and C1B as raw materials.

$^{1}$H NMR (400MHz, DMSO-$d_6$) δ 8.42-8.47 (m, 2H), 8.30 (d, *J* = 7.2 Hz, 1H), 7.94 (d, *J* = 13.2 Hz, 1H), 7.49-7.51 (m, 2H), 7.32-7.41 (m, 3H), 7.25 (d, *J* = 7.2 Hz, 2H), 7.14 (d, *J* = 7.2 Hz, 1H), 7.03 (d, *J* = 7.6 Hz, 2H), 6.57 (d, *J* = 14.4 Hz, 1H), 6.05 (d, *J* = 14.4 Hz, 1H), 4.23 (s, 3H), 4.07 (s, 2H), 2.66 (s, 4H), 2.58 (d, *J* = 7.2 Hz, 2H), 1.67-1.78 (m, 6H), 1.62 (s, 6H), 1.49 (s, 8H).

LCMS: m/z = 660.3, $C_{42}H_{47}ClN_3O_2^{+}$.

Example 54: Preparation of 2-((E)-2-((E)-2-bromo-3-(2-((E)-3,3-dimethyl-1-(6-oxo-6-phenoxyhexyl)indolin-2-ylide ne) ethenyl)cyclohex-1-en-1-yl)ethenyl)-1-ethyl-3,3-dimethyl-3H-indol-1-ium formate (54)

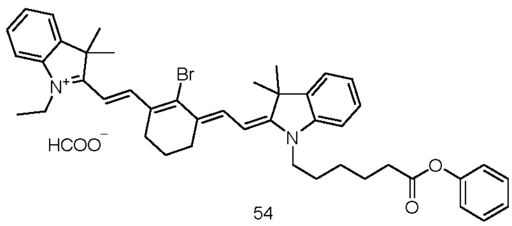

54

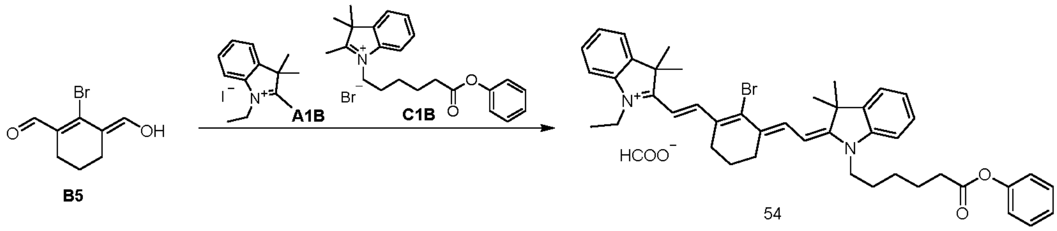


B5

54

Compound 54 was prepared as a green solid according to the preparation method of Example 21 with Compounds B5, A1B and C1B as raw materials.

$^{1}$H NMR (400 MHz, MeOD) δ 8.46 (t, *J* = 13.2 Hz, 2H), 7.53-7.56 (m, 2H), 7.42-7.47 (m, 2H), 7.30-7.37 (m, 6H), 7.19-7.23 (m, 1H), 6.98-7.01 (m, 2H), 6.31 (d, *J* = 13.6 Hz, 2H), 4.15-4.30 (m, 4H), 2.72 (s, 4H), 2.62 (t, *J* = 7.2 Hz, 2H), 1.90-1.97 (m, 4H), 1.81-1.84 (m, 2H), 1.75 (d, *J*= 4.4 Hz, 12H), 1.55-1.61 (m, 2H), 1.42 (t, *J*= 6.8 Hz, 3H).

LCMS: m/z = 719.4, $C_{44}H_{50}BrN_2O_2^{+}$.

Example 55: Preparation of 2-((E)-2-((E)-3-(2-((E)-1-(2-(2-(2-(benzyloxy)ethoxy)ethoxy)ethyl)-3,3-dimethylindolin -2-ylidene)ethenyl)-2-chlorocyclopent-1-en-1-yl)ethenyl)-1,3,3-trimethyl-3H-indol-1-iu m formate (55)

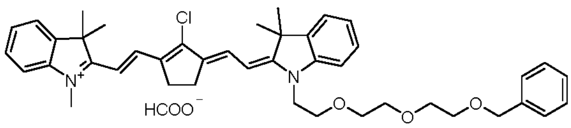

**55**

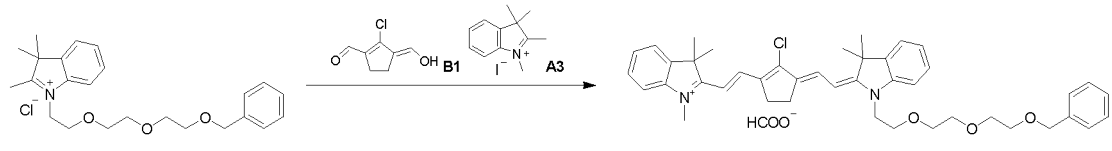


C12

55

Compound 55 was prepared as a green solid according to the preparation method of Example 21 with Compounds B1, A3 and C12 as raw materials.

$^1$H NMR (400MHz, MeOD) δ 7.95 - 7.92 (m, 2H), 7.51 - 7.49 (m, 2H), 7.43 - 7.39 (m, 2H), 7.35 - 7.34 (m, 2H), 7.32 - 7.29 (m, 6H), 7.28 - 7.25 (m, 1H), 6.28 (d, *J* = 14.0 Hz, 1H), 6.04 (d, *J* = 14.0 Hz, 1H), 4.58 (s, 1H), 4.47 (d, *J* = 5.60 Hz, 2H), 4.36 (t, *J* = 4.80 Hz, 2H), 3.92 (t, *J* = 4.80 Hz, 2H), 3.64 (s, 3H), 3.61 - 3.55 (m, 2H), 3.53 - 3.51 (m, 5H), 2.95 (d, *J* = 2.80 Hz, 4H), 1.71 (d, *J* = 2.00 Hz, 12H).

LCMS: m/z = 677.2, $C_{43}H_{50}ClN_2O_3^+$.

Example 56: Preparation of 2-((E)-2-((E)-3-(2-((E)-1-(2-(2-(2-(benzyloxy)ethoxy)ethoxy)ethyl)-3,3-dimethylindolin -2-ylidene)ethenyl)-2-chlorocyclohex-1-en-1-yl)ethenyl)-1,3,3-trimethyl-3H-indol-1-iu m 2,2,2-trifluoroacetate (56)

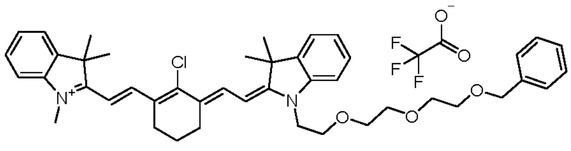

56

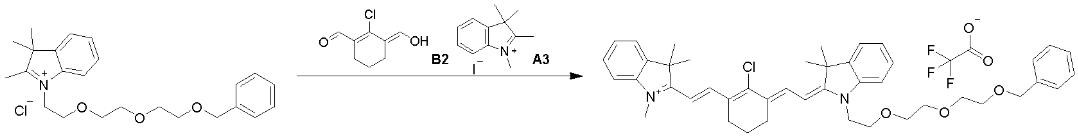


C12

56

Compound 56 was prepared as a green solid according to the preparation method of Example 21 with Compounds B2, A3 and C12 as raw materials.

$^1$H NMR (400MHz, $CDCl_3$) δ 8.32 (d, *J* = 13.2 Hz, 2H), 7.37 - 7.23 (m, 13H), 6.27 - 6.11 (m, 2H), 4.48 (s, 2H), 4.27 (s, 2H), 3.87 (t, *J* = 4.80 Hz, 2H), 3.71 - 3.62 (m, 4H), 3.49 - 3.48 (m, 8H), 2.62 (d, *J* = 1.60 Hz, 2H), 1.90 - 1.80 (m, 3H), 1.68 (d, *J* = 2.80 Hz, 12H).

LCMS: m/z = 691.6, $C_{44}H_{52}ClN_2O_3^+$.

Example 57: Preparation of 2-((E)-2-((E)-3-(2-((E)-1-(2-(2-(2-(benzyloxy)ethoxy)ethoxy)ethyl)-3,3-dimethylindolin -2-ylidene)ethenyl)-2-chloro-5-(dimethylamino)cyclohex-1-en-1-yl)ethenyl)-1,3,3-trime thyl-3H-indol-1-ium 2,2,2-trifluoroacetate (57)

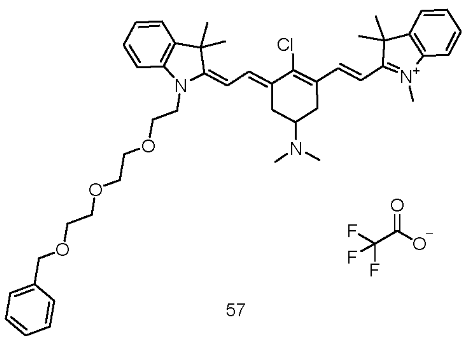

57

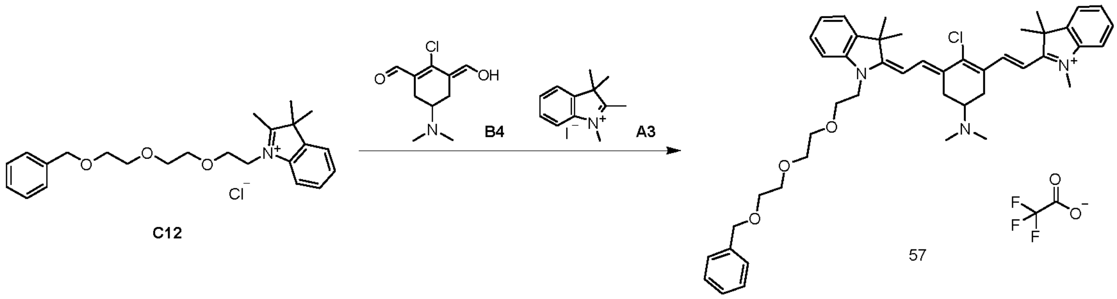


C12 57

Compound 57 was prepared as a blue paste according to the preparation method of Example 21 with Compounds B4, A3 and C12 as raw materials.

$^1$H NMR: (400MHz, MeOD)δ 8.70 - 8.42 (m, 2H), 7.50 - 7.52 (m, 2H), 7.30 - 7.32 (m, 4H), 7.30 - 7.28 (m, 7H), 6.50 (d, *J* = 14.4 Hz, 1H), 6.34 (d, *J* = 14.4 Hz, 1H), 4.48 (t, *J* = 14.8 Hz, 2H), 4.44 (s, 2H), 3.9 - 3.92 (t, *J* = 5.20 Hz, 2H), 3.75 (s, 3H), 2.70 - 3.67 (m, 1H), 3.60 - 3.58 (m, 8H), 3.3 - 3.33 (m, 2H), 3.05 (s, 6H), 2.88 - 2.79 (dd, $J_1$ = 12.0 Hz, $J_2$ = 26.4 Hz, 2H), 1.73 (d, *J* = 2.00 Hz, 2H).

LCMS: m/z = 734.4, $C_{46}H_{57}ClN_3O_3^+$.

Example 58: Preparation of 2-((E)-2-((E)-2-bromo-3-(2-((E)-3,3-dimethyl-1-(6-oxo-6-phenoxyhexyl)indolin-2-ylide ne) ethenyl)cyclohex-1-en-1-yl)ethenyl)-1,3,3-trimethyl-3H-indol-1-ium 2,2,2-trifluoroacetate (58)

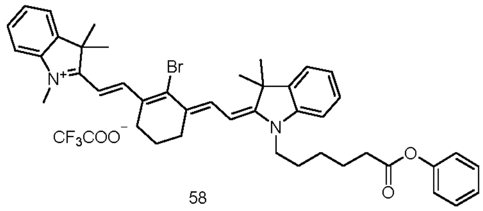

58

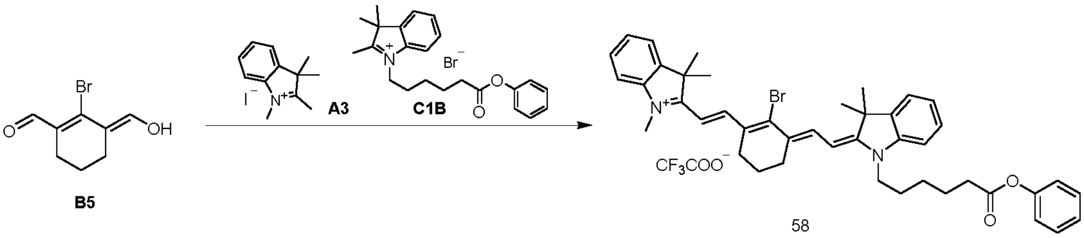


B5 58

Compound 58 was prepared as a green solid according to the preparation method of Example 21 with Compounds B5, A3 and C1B as raw materials.

$^1$H NMR (400 MHz, $CDCl_3$)δ 8.34-8.41 (m, 2H), 7.37-7.42 (m, 4H), 7.27-7.30 (m, 2H), 7.14-7.19 (m, 2H), 6.18 (dd, $J_1$ = 14.4 Hz, $J_2$ = 33.2 Hz, 2H), 4.11 (t, *J* = 7.2 Hz, 2H), 3.65 (s, 3H), 2.71 (t, *J* = 5.6 Hz, 4H), 2.46 (t, *J* = 6.8 Hz, 2H), 1.96-1.99 (m, 2H), 1.85-1.89 (m, 2H), 1.76-1.81 (m, 2H), 1.74 (s, 12H), 1.53-1.57 (m, 2H).

LCMS: m/z = 629.3, $C_{37}H_{44}BrN_2O_2^+$.

Example 60: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-3,3-dimethyl-1-(5-(trimethylammonium)pentyl) -3H-indol-1-ium 2,2,2-trifluoroacetate (60)

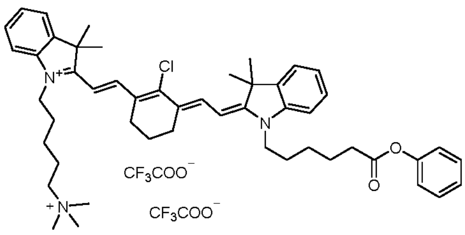

60

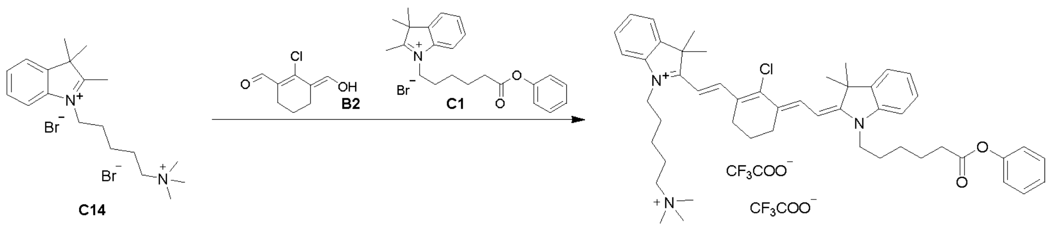


60

, and Compound 60 was prepared as a green solid according to the preparation method of Example 21 withCompounds B2, C1 and C14 as raw materials.

$^1H$ NMR (400 MHz, DMSO)δ 8.27 (t, $J$ = 14.8 Hz, 2H), 7.65 (dd, $J_1$ = 7.6 Hz, $J_2$ = 11.2 Hz, 2H), 7.37-7.52 (m, 6H), 7.23-7.34 (m, 3H), 7.02-7.04 (m, 2H), 6.35 (dd, $J_1$ = 14.0 Hz, $J_2$ = 33.2 Hz, 2H), 4.22-4.30 (m, 4H), 3.22-3.26 (m, 4H), 3.01 (s, 9H), 2.67-2.72 (m, 4H), 2.58 (t, $J$ = 7.2 Hz, 2H), 1.71-1.82 (m, 8H),1.67 (s, 12H), 1.47-1.57 (m, 2H), 1.36-1.40 (m, 2H).

LCMS: m/z = 387.6, $C_{50}H_{64}ClN_3O_2^{2+}$/2.

Example 61: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-3,3-dimethyl-1-(4-(trimethylammonium)butyl)-3H-indol-1-ium 2,2,2-trifluoroacetate (61)

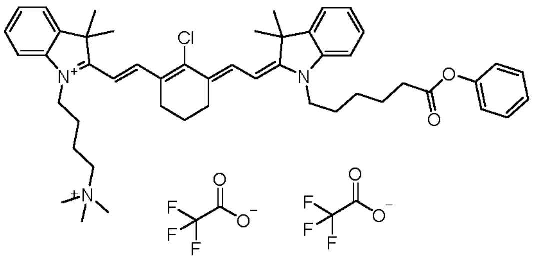

61

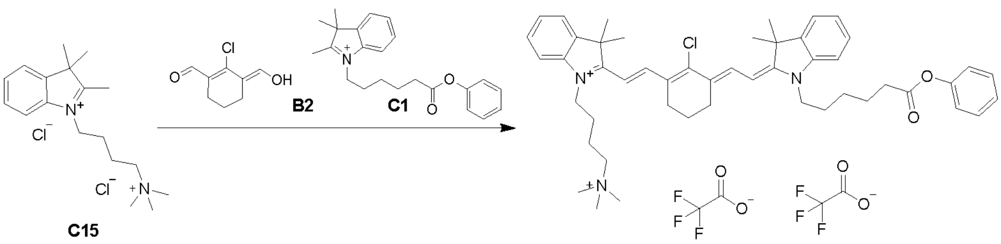


61

Compound 61 was prepared as a green solid according to the preparation method of Example 21 with Compounds B2, C1 and C15 as raw materials.

$^1H$ NMR: (400MHz, $CDCl_3$)δ 8.43 (d, $J$ = 14.4 Hz, 1H), 8.27 (t, $J$ = 13.2 Hz, 1H), 7.40-7.43 (m, 2H), 7.33-7.37 (m, 5H), 7.19-7.20 (m, 3H), 7.00-7.05 (m, 3H), 6.32 (t, $J$= 13.6 Hz, 1H), 6.05 (t, $J$ = 13.6 Hz, 1H), 4.00-4.16 (m, 4H), 3.39-3.43 (m, 2H), 3.13 (s, 9H), 2.75 (s, 2H), 2.57-2.64 (m, 4H), 2.05-2.10 (m, 2H), 1.92-1.94 (m, 3H), 1.81-1.88 (m, 5H), 1.70 (s,

12H), 1.58-1.69 (m, 2H).

LCMS: m/z = 380.2, $C_{49}H_{62}ClN_3O_{22}^{+}/2$.

Example 62: Preparation of 2-((E)-2-((E)-2-chloro-3-(2-((E)-1-(5-(diethoxyphosphonyl)pentyl)-3,3-dimethylindolin -2-ylidene)ethenyl)cyclohex-1-en-1-yl)ethenyl)-1,3,3-trimethyl-3H-indol-1-ium 2,2,2-trifluoroacetate (62)

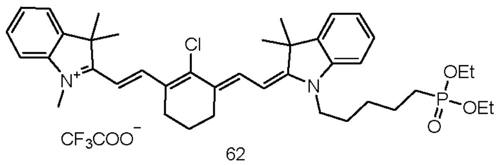

62

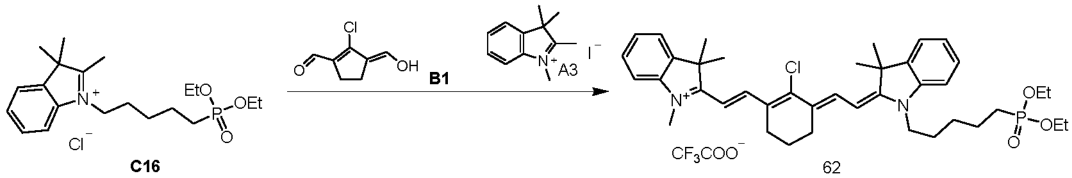


C16

62

Compound 62 was prepared as a green solid according to the preparation method of Example 21 with Compounds B1, A3 and C 16 as raw materials.

LCMS: m/z = 675.4, $C_{40}H_{53}ClN_2O_3P^{+}$.

Example 63: Preparation of 2-((E)-2-((E)-3-(2-((E)-1-(5-carboxypentyl)-3,3-dimethylindolin-2-ylidene)ethenyl)-2-c hlorocyclopent-1-en-1-yl)ethenyl)-3,3,7-trimethyl-3H-pyrrolo[2,3-b]pyridin-7-ium chloride (63)

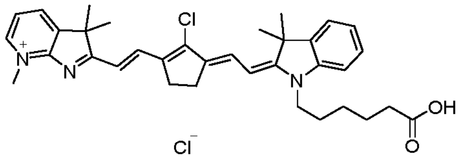

63

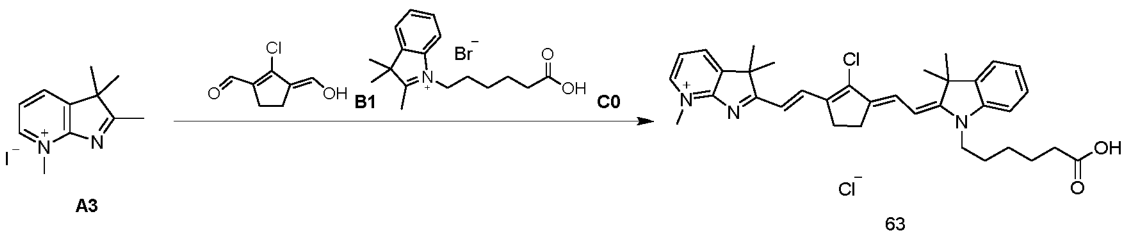


A3

63

Compound 63 was prepared as a blue paste according to the preparation method of Example 21 with Compounds B1, A3 and C0 as raw materials.

$^1$H NMR (400MHz, MeOD) δ 8.29 (d, J = 15.2 Hz, 1H), 8.01 (s, 2H), 7.83 - 7.78 (m, 2H), 7.67 - 7.56 (m, 3H), 7.17 (s, 1H), 6.92 (d, J = 15.6 Hz, 1H), 5.83 (d, J = 11.6 Hz, 1H), 4.63 (s, 2H), 4.16 (s, 3H), 3.04 (d, J = 20.0 Hz, 4H), 2.33 (t, J = 11.2 Hz, 2H), 2.04 - 2.00 (m, 2H), 1.82 (s, 6H), 1.73 - 1.67 (m, 3H), 1.62 - 1.55 (m, 7H).

LCMS: m/z = 570.4, $C_{35}H_{41}ClN_3O_2^{+}$.

Example 64: Preparation of 2-((E)-2-((E)-3-(2-((E)-1-(5-carboxypentyl)-3,3-dimethylindolin-2-ylidene)ethenyl)-2-c hlorocyclohex-1-en-1-yl)ethenyl)-3,3,7-trimethyl-3H-pyrrolo[2,3-b]pyridin-7-ium chloride (64)

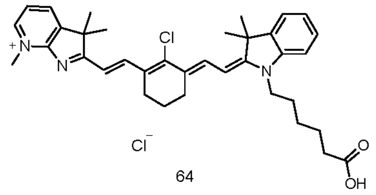

64

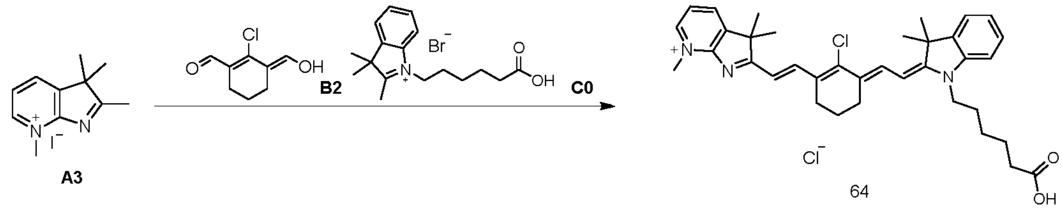


Compound 64 was prepared as a blue solid according to the preparation method of Example 21 with Compounds B2, A3 and C0 as raw materials.

$^1$H NMR (400MHz, MeOD)8.75 (d, J = 15.6 Hz, 1H), 8.01 (d, J = 7.20 Hz, 2H), 7.81 - 7.78 (m, 3H), 7.65 - 7.63 (m, 2H), 7.18 (t, J = 6.80 Hz, 1H), 7.03 (d, J = 15.6 Hz, 1H), 6.04 (t, J = 5.60 Hz, 1H), 4.62 (t, J = 7.20 Hz, 2H), 4.15 (s, 3H), 2.83 - 2.80 (m, 4H), 2.33 (t, J = 7.20 Hz, 2H), 1.99 - 1.98 (m, 4H), 1.83 (s, 6H), 1.73 - 1.67 (m, 3H), 1.58 - 1.56 (m, 7H).

LCMS: m/z = 584.4, $C_{36}H_{43}ClN_3O_2^+$.

Example 65: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-3,3-dimethyl-1-(3-(trimethylammonium)propyl )-3H-indol-1-ium 2,2,2-trifluoroacetate (65)

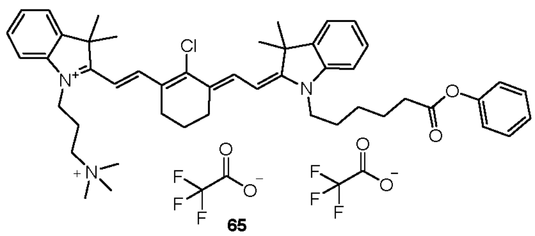

65

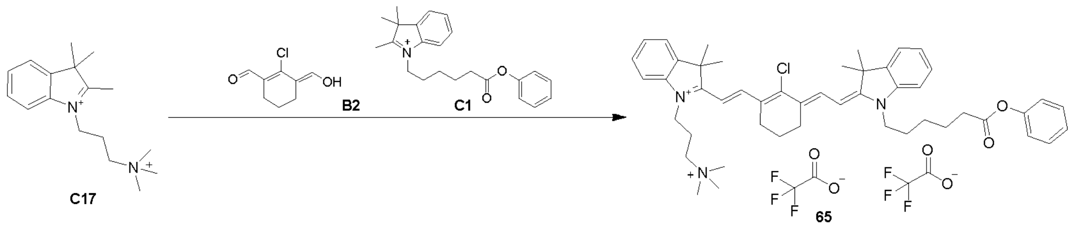


Compound 65 was prepared as a blue paste according to the preparation method of Example 21 with Compounds B2, C1 and C17 as raw materials.

$^1$H NMR: (400MHz, $CDCl_3$)$\delta$ 8.46 (d, *J* = 14.4 Hz, 1H), 8.29 (d, *J* = 13.6 Hz, 1H), 7.61 (d, *J* = 7.6 Hz, 1H), 7.46 (t, *J* = 7.6 Hz, 1H), 7.34-7.38 (m, 5H), 7.29 (s, 1H), 7.19-7.21 (m, 2H), 7.00-7.03 (m, 3H), 6.54 (d, *J* = 14.0 Hz, 1H), 6.01 (d, *J* = 14.0 Hz, 1H), 4.36 (s, 2H), 4.00-4.02 (m, 4H), 3.24 (s, 9H), 2.77 (s, 2H), 2.59-2.62 (m, 4H), 2.38 (s, 2H), 1.82-1.91 (m, 6H), 1.72 (d, *J* = 10.0 Hz, 12H), 1.56-1.58 (m, 2H).

LCMS: m/z = 373.1, $C_{48}H_{60}ClN_3O_{22}^{+}/2$.

Example 66: Preparation of 2-((E)-2-((E)-2-chloro-3-(2-((E)-3,3-dimethyl-1-(6-oxo-6-phenoxyhexyl)indolin-2-ylide ne) ethenyl)cyclohex-1-en-1-yl)ethenyl)-5-(dimethylamino)-1,3,3-trimethyl-3H-indol-1-i um chloride (66)

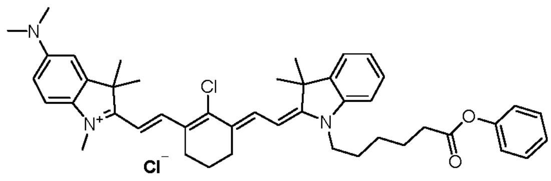

66

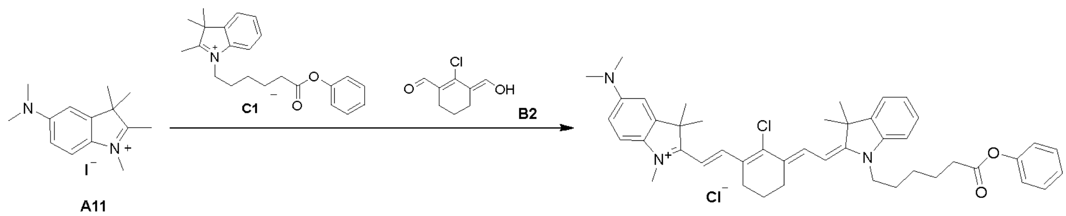


A11

66

Compound 66 was prepared as a blue solid according to the preparation method of Example 21 with Compounds B2, A11 and C1 as raw materials.

$^1$H NMR (400MHz, DMSO-$_{d6}$)δ 8.26 (d, *J* = 14.8 Hz, 1H), 7.92 (d, *J* = 13.6 Hz, 1H), 7.51-7.46 (m, 2H), 7.41-7.37 (m, 2H), 7.32-7.23 (m, 2H), 7.16 (d, *J* = 8.0 Hz, 1H), 7.09-7.04 (m, 2H), 7.03 (d, *J* = 7.6 Hz, 2H), 6.82 (d, *J* = 8.0 Hz, 1H), 6.58 (d, *J* = 15.2 Hz, 1H), 5.92 (d, *J* = 13.2 Hz, 1H), 4.00 (s, 2H), 3.85 (s, 3H), 3.03 (s, 6H), 2.66 (s, 4H), 2.58 (t, *J* = 7.2 Hz, 2H), 1.84-1.73 (m, 6H), 1.68 (s, 6H), 1.61 (s, 6H), 1.51-1.45 (m, 2H).

LCMS: m/z = 702.4, $C_{45}H_{53}ClN_3O_2^+$.

Example 67: Preparation of 2-((E)-2-((E)-2-chloro-3-(2-((E)-1-(2-(2-methoxyethoxy)ethyl)-3,3-dimethylindolin-2-y lidene)ethenyl)cyclohex-1-en-1-yl)ethenyl)-3,3-dimethyl-1-(2-(2-(3-oxo-3-phenoxyprop oxy)ethoxy)ethyl)-3H-indol-1-ium 2,2,2-trifluoroacetate (67)

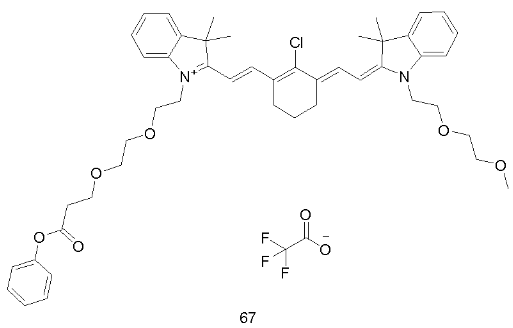

67

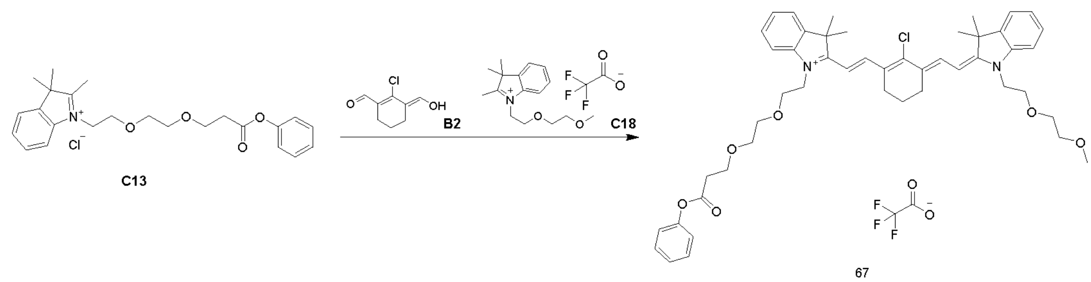


C13

67

Compound 67 was prepared as a green paste according to the preparation method of Example 21 with Compounds B2, C13 and C18 as raw materials.

$^1$H NMR (400MHz, $CDCl_3$)δ 8.35 (d, *J* = 14.4 Hz, 2H), 7.36-7.39 (m, 6H), 7.23-7.25 (m, 5H), 7.06 (d, *J* = 8.4 Hz, 2H), 6.30 (d, *J* = 14.0 Hz, 2H), 4.33-4.35 (m, 4H), 3.89-3.93 (m, 4H), 3.76 (t, *J* = 6.4 Hz, 2H), 3.58-3.63 (m, 6H), 3.43-3.46 (m, 2H), 3.28 (s, 3H), 2.67-2.75 (s, 6H), 1.95-1.96 (m, 2H), 1.73 (s, 12H).

LCMS: m/z = 793.5, $C_{48}H_{58}ClN_2O_6^+$.

Example 68: Preparation of 1-(2-(2-(2-carboxyethoxy)ethoxy)ethyl)-2-((E)-2-((E)-2-chloro-3-(2-((E)-1-(2-(2-metho xyethoxy)ethyl)-3,3-dimethylindolin-2-ylidene)ethenyl)cyclohex-1-en-1-yl)ethenyl)-3,3 -dimethyl-3H-indol-1-ium 2,2,2-trifluoroacetate (68)

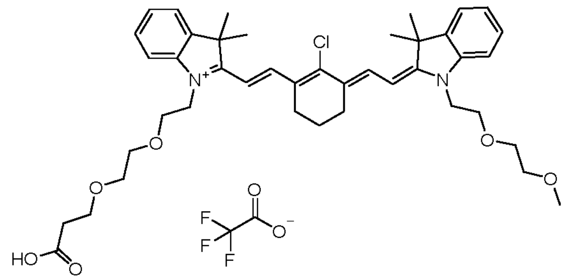

68

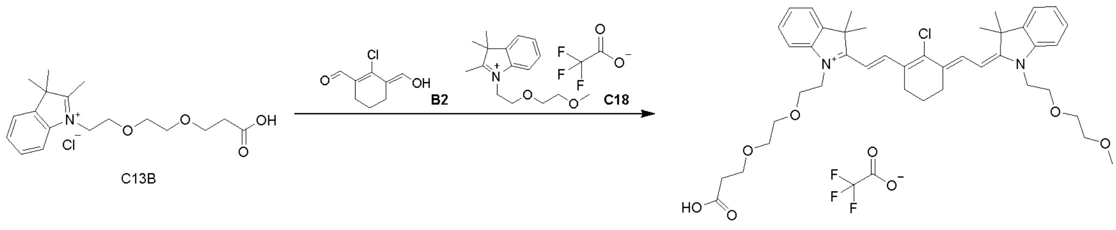


C13B

68

Compound 68 was prepared as a green paste according to the preparation method of Example 21 with Compounds B2, C13B (purchased from Bide Pharm, batch number 220503) and C18 as raw materials.

$^1$H NMR (400MHz, $CDCl_3$)δ 8.43 (d, $J$ = 14.4 Hz, 1H), 8.25 (d, $J$ = 13.6 Hz, 1H),7.30-7.43 (m, 6H), 7.19 (d, $J$ = 7.2 Hz, 1H), 7.11 (d, $J$ = 8.0 Hz, 1H), 6.57 (d, $J$ = 14.4 Hz, 1H), 6.14 (d, $J$ = 14.0 Hz, 1H), 4.46 (t, $J$ = 4.8 Hz, 2H), 4.22 (t, $J$ = 5.2 Hz, 2H), 4.00 (t, $J$ = 5.2 Hz, 2H), 3.88 (t, $J$ = 5.2 Hz, 2H), 3.74 (t, $J$ = 6.0 Hz, 2H), 3.57-3.63 (m, 4H), 3.48-3.49 (m, 4H), 3.31 (s, 3H), 2.73 (t, $J$ = 4.0 Hz, 2H), 2.60-2.66 (m, 4H), 1.95-1.97 (m, 2H), 1.73 (d, $J$ = 9.2 Hz, 12H).

LCMS: m/z = 717.4 $C_{42}H_{54}ClN_2O_6^+$.

Example 69: Preparation of 2-((E)-2-((E)-2-chloro-3-(2-((E)-1-(2-(2-(2-methoxyethoxy)ethoxy)ethyl)-3,3-dimethyli ndolin-2-ylidene)ethenyl)cyclohex-1-en-1-yl)ethenyl)-3,3-dimethyl-1-(2-(2-(3-oxo-3-ph enoxypropoxy)ethoxy)ethyl)-3H-indol-1-ium 2,2,2-trifluoroacetate (69)

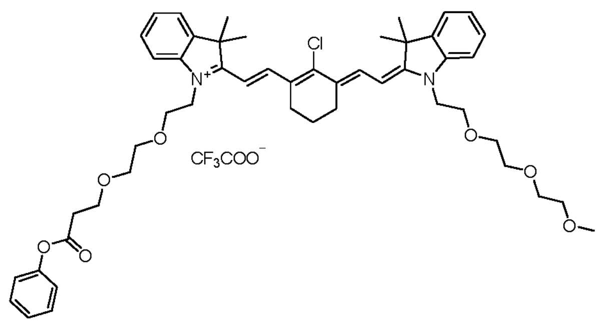

69

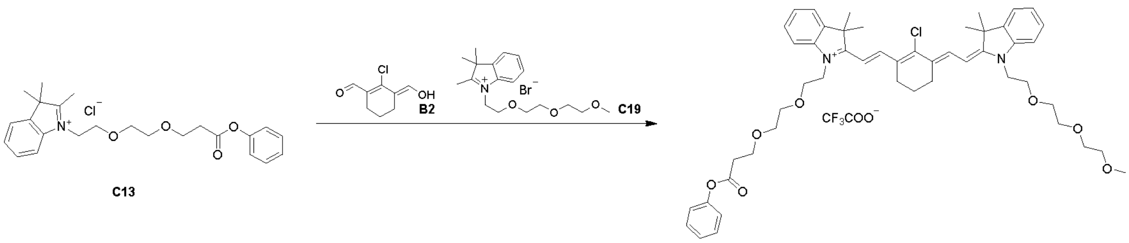


69

Compound 69 was prepared as a green paste according to the preparation method of Example 21 with Compounds B2, C13 and C19 as raw materials.

$^{1}$H NMR (400 MHz, $CDCl_3$) δ 8.35 (d, *J* = 14.0 Hz, 2H), 7.34-7.40 (m, 6H), 7.20-7.26 (m, 5H), 7.05-7.07 (m, 2H), 6.29 (d, *J* = 14.0 Hz, 2H), 4.29-4.35 (m, 4H), 3.87-3.95 (m, 4H), 3.75 (t, *J* = 6.0 Hz, 2H), 3.60-3.64 (m, 4H), 3.55-3.59 (m, 4H), 3.51-3.53 (m, 2H), 3.44-3.46 (m, 2H), 3.33 (s, 3H), 2.73 (t, *J* = 6.4 Hz, 2H), 2.62-2.69 (m, 4H), 1.88-1.99 (m, 2H), 1.73 (s, 12H).

LCMS: m/z = 837.4, $C_{50}H_{62}ClN_2O_7^+$.

Example 70: Preparation of 1-(2-(2-(2-carboxyethoxy)ethoxy)ethyl)-2-((E)-2-((E)-2-chloro-3-(2-((E)-1-(2-(2-(2-met hoxyethoxy)ethoxy)ethyl)-3,3-dimethylindolin-2-ylidene)ethenyl)cyclohex-1-en-1-yl)et henyl)-3,3-dimethyl-3H-indol-1-ium 2,2,2-trifluoroacetate (70)

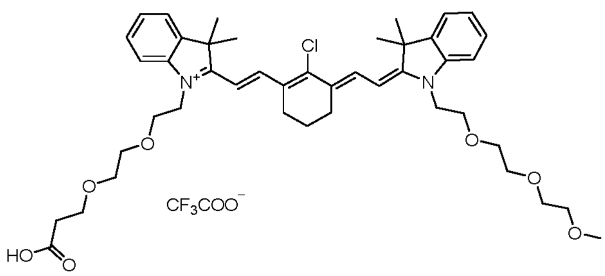

70

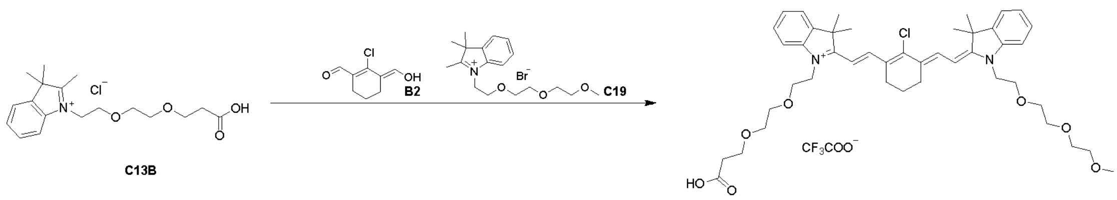


70

, and Compound 70 was prepared as a blue paste according to the preparation method of Example 21 with Compounds B2, C13B and C19 as raw materials.

$^{1}$H NMR (400 MHz, $CDCl_3$) δ 8.41 (d, *J* = 14.0 Hz, 1H), 8.28 (d, *J* = 14.0 Hz, 1H), 7.33-7.43 (m, 4H), 7.28-7.31 (m, 2H), 7.20 (d, *J* = 7.2 Hz, 1H), 7.13-7.15 (m, 1H), 6.47 (d, *J* = 14.4 Hz, 1H), 6.17 (d, *J* = 14.0 Hz, 1H), 4.36-4.42 (m, 2H), 4.24 (t, *J* = 5.2 Hz, 2H), 3.96 (t, *J* = 4.8 Hz, 2H), 3.88 (t, *J* = 4.4 Hz, 2H), 3.70 (t, *J* = 6.0 Hz, 2H), 3.61-2.63 (m, 2H), 3.52-3.58 (m, 8H), 3.45-3.47 (m, 2H), 3.34 (s, 3H), 3.64-3.71 (m, 4H), 2.59 (t, *J= 6.0* Hz, 2H), 1.90-1.98 (m, 2H), 1.72 (d, *J* = 6.8 Hz, 12H).

LCMS: m/z = 761.5, $C_{44}H_{58}ClN_2O_7^+$.

Example 71: Preparation of 2-((E)-2-((E)-2-chloro-3-(2-((E)-1-(2-(2-methoxyethoxy)ethyl)-3,3-dimethylindolin-2-y lidene)ethenyl)cyclohex-1-en-1-yl)ethenyl)-1-(2-(2-(2-methoxyethoxy)ethoxy)ethyl)-3, 3-dimethyl-3H-indol-1-ium 2,2,2-trifluoroacetate (71)

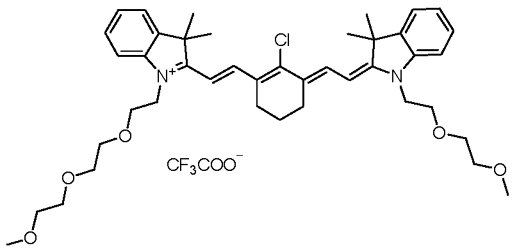

71

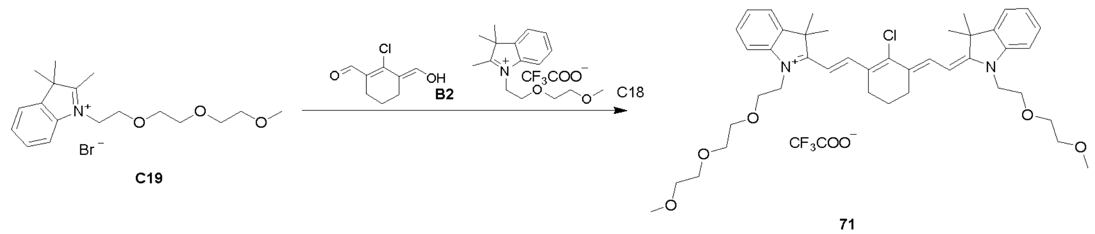


71

Compound 71 was prepared as a green paste according to the preparation method of Example 21 with Compounds B2, C18 and C19 as raw materials.

$^{1}$H NMR (400 MHz, $CDCl_3$)δ 8.33 (dd, $J_1$ = 5.6 Hz, $J_2$ = 14.0 Hz, 2H), 7.35-7.71 (m, 4H), 7.23-7.25 (m, 4H), 6.36 (t, $J_1$ = 13.6 Hz, 2H), 4.36-4.42 (m, 4H), 3.91-3.95 (m, 4H), 3.60-3.65 (m, 4H), 3.56-3.58 (m, 2H), 3.53-3.55 (m, 2H), 3.45-3.47 (m, 4H), 3.35 (s, 3H), 3.28 (s, 3H), 2.64-2.72 (m, 4H), 1.93-1.96 (m, 2H), 1.73 (s, 12H).

LCMS: m/z = 703.3, $C_{42}H_{56}ClN_2O_5^+$.

Example 72: Preparation of 2-((E)-2-((E)-2-chloro-3-(2-((E)-3,3-dimethyl-1-(2,5,8,11-tetraoxatridecan-13-yl)indoli n-2-ylidene)ethenyl)cyclohex-1-en-1-yl)ethenyl)-3,3-dimethyl-1-(2-(2-(3-oxo-3-phenox ypropoxy)ethoxy)ethyl)-3H-indol-1-ium 2,2,2-trifluoroacetate (72)

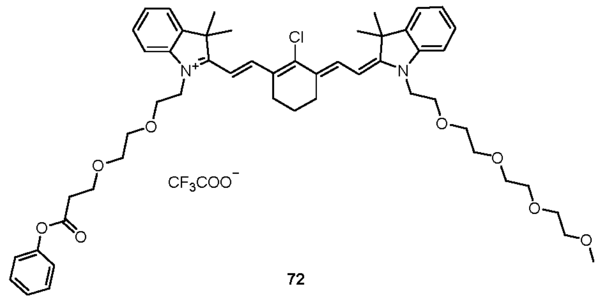

72

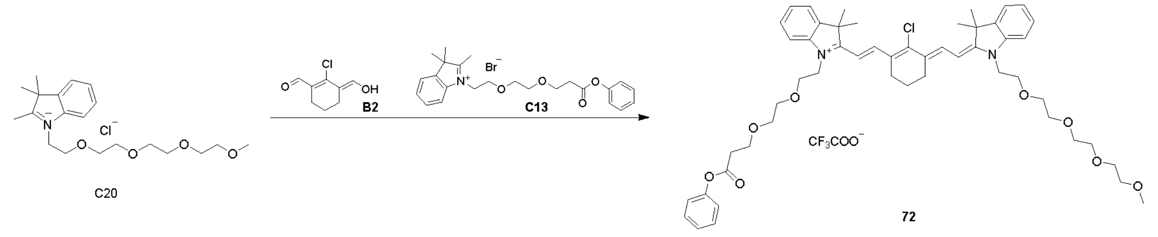


72

Compound 72 was prepared as a green paste according to the preparation method of Example 21 with Compounds B2, C13 and C20 as raw materials.

$^{1}$H NMR (400 MHz, $CDCl_3$)δ 8.35 (d, $J$ = 14.0 Hz, 2H), 7.33-7.38 (m, 6H), 7.20-7.26 (m, 5H), 7.05-7.07 (m, 2H), 6.27 (d, $J$ = 14.0 Hz, 2H), 4.25-4.34 (m, 4H), 3.88-3.96 (m, 4H), 3.74 (t, $J$ = 6.4 Hz, 2H), 2.58-2.63 (m, 8H), 2.52-2.57 (m, 8H), 3.35 (s, 3H), 2.72 (t, $J$ = 6.0 Hz, 2H), 2.63-2.69 (m, 4H), 1.91-1.99 (m, 2H), 1.72 (s, 12H).

LCMS: m/z = 881.6, $C_{52}H_{66}ClN_2O_8^+$.

Example 73: Preparation of 1-(2-(2-(2-carboxyethoxy)ethoxy)ethyl)-2-((E)-2-((E)-2-chloro-3-(2-((E)-3,3-dimethyl-1-(2,5,8,11-tetraoxatridecan-13-yl)indolin-2-ylidene)ethenyl)cyclohex-1-en-1-yl)ethenyl )-3,3-dimethyl-3H-indol-1-ium chloride (73)

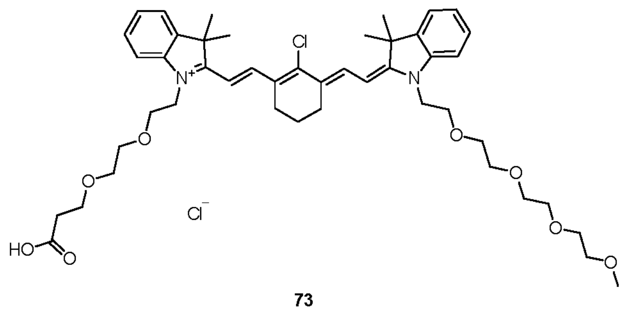

73

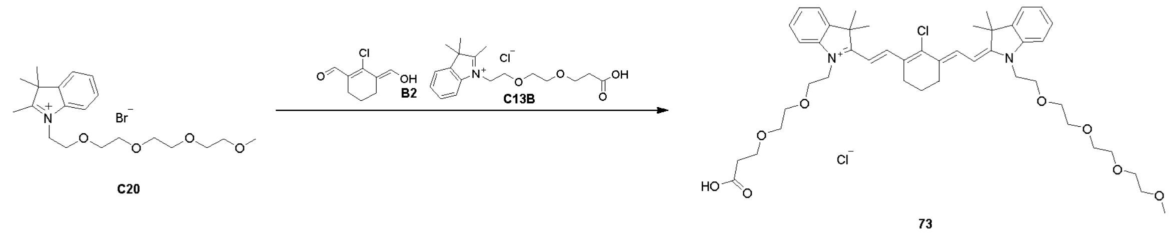


Compound 73 was prepared as a green paste according to the preparation method of Example 21 with Compounds B2, C13B and C20 as raw materials.

$^1$H NMR (400 MHz, $CDCl_3$) δ 8.42 (d, *J* = 14.0 Hz, 1H), 8.23 (d, *J* = 13.6 Hz, 1H), 7.29-7.45 (m, 6H), 7.13-7.19 (m, 2H), 6.61 (d, *J* = 14.4 Hz, 1H), 6.17 (d, *J* = 13.6 Hz, 1H), 4.53-4.60 (m, 2H), 4.23-4.30 (m, 2H), 4.00-4.07 (m, 2H), 3.86-3.96 (m, 2H), 3.73-3.76 (m, 2H), 3.57-3.63 (m, 14H), 3.51-3.54 (m, 2H), 3.36 (s, 3H), 2.76-2.83 (m, 2H), 2.64-2.72 (m, 4H), 1.93-2.02 (m, 2H), 1.72 (d, *J* = 10.0 Hz, 12H).

LCMS: m/z = 805.5, $C_{46}H_{62}ClN_2O_8^+$.

Example 74: Preparation of 5-(tert-butoxycarbonyl)-2-((E)-2-((E)-2-chloro-3-(2-((E)-3,3-dimethyl-1-(2-(3-oxo-3-ph enoxypropoxy)ethyl)indolin-2-ylidene)ethenyl)cyclohex-1-en-1-yl)ethenyl)-1-ethyl-3,3-dimethyl-3H-indol-1-ium bromide (74)

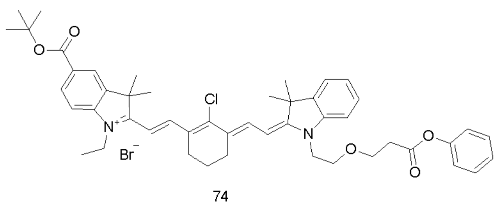

74

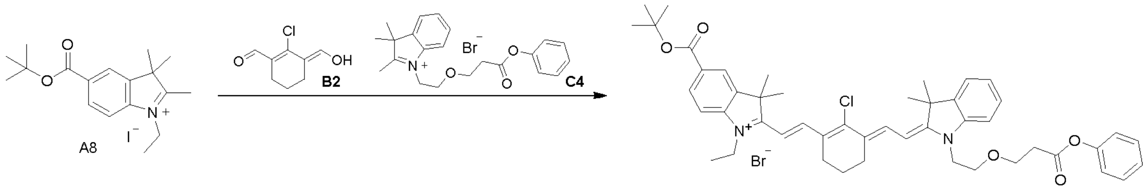


74

Compound 74 was prepared as a green oil according to the preparation method of Example 21 with Compounds B2, A8 and C4 as raw materials.

LCMS: m/z = 775.3, $C_{48}H_{56}ClN_2O_5^+$.

Example 75A and 75B: Preparation of 2-((E)-2-((E)-3-(2-((E)-1-(2,5,8,11,14-pentaoxahexadecan-16-yl)-3,3-dimethylindolin-2 -ylidene)ethenyl)-2-chlorocyclohex-1-en-1-yl)ethenyl)-3,3-dimethyl-1-(2-(2-(3-oxo-3-p henoxypropoxy) ethoxy)ethyl)-3H-indol-1-ium 2,2,2-trifluoroacetate (75A) and 2-((E)-2-((E)-2-chloro-3-(2-((E)-3,3-dimethyl-1-(2-(2-(3-oxo-3-phenoxypropoxy)ethox y)ethyl)indolin-2-ylidene)ethenyl)cyclohex-1-en-1-yl)ethenyl)-3,3-dimethyl-1-(2-(2-(3-oxo-3-phenoxypropoxy)ethoxy)ethyl)-3H-indol-1-ium 2,2,2-trifluoroacetate (75B)

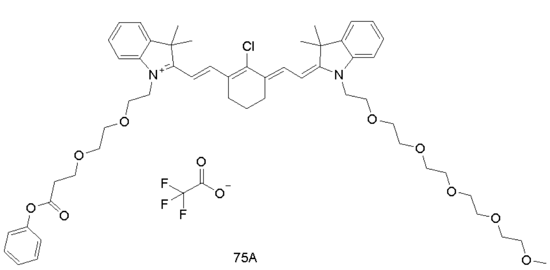

75A

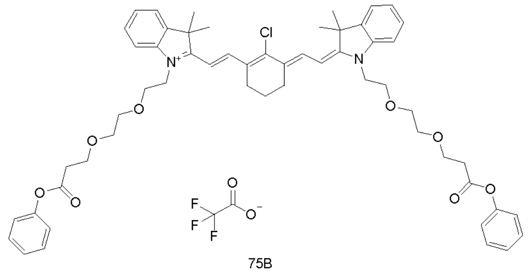

75B

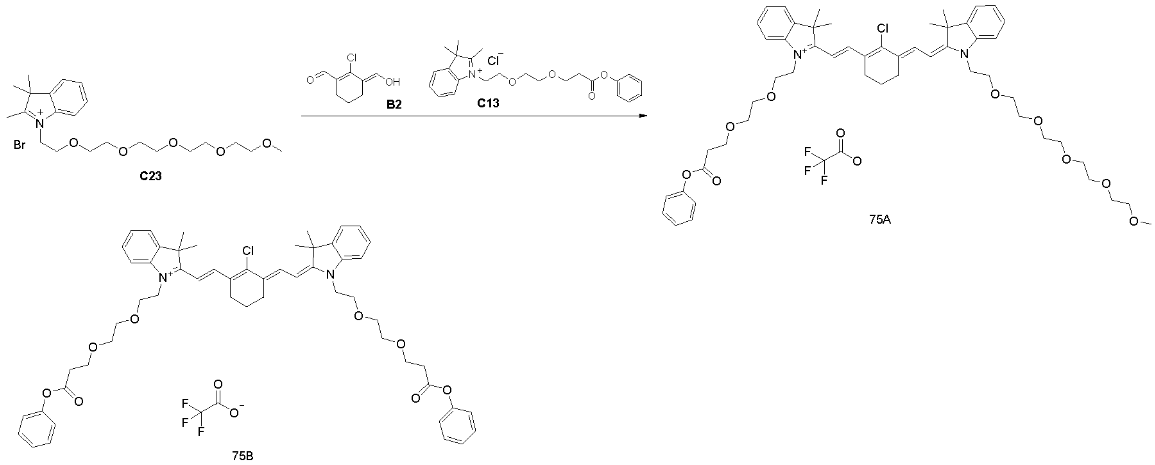


75A

75B

Compound 75A and Compound 75B were both prepared as a blue paste according to the preparation method of Example 21 with Compounds B2, C13 and C23 as raw materials.

**Compound 75A:**

$^1$H NMR (400MHz, DMSO-$d_6$);δ 8.25 (d, *J* = 14.0 Hz, 2H), 7.62 (d, *J* = 7.2 Hz, 2H), 7.36-7.44 (m, 6H), 7.23-7.28 (m, 3H), 7.05 (d, *J* = 7.6 Hz, 2H), 6.42 (d, *J* = 14.4 Hz, 2H), 4.42 (d, *J* = 4.8 Hz, 4H), 3.81-3.82 (m, 4H), 3.62 (d, *J* = 6.0 Hz, 2H), 3.50-3.55 (m,4H), 3.37-3.47 (m, 16H), 3.21 (s, 3H), 2.68 (t, *J* = 2.8 Hz, 6H), 1.83-1.86 (m, 2H) 1.66 (t, *J* = 2.4 Hz, 12H).

LCMS: m/z = 925.6, $C_{54}H_{70}ClN_2O_9^+$.

**Compound 75B:**

$^1$H NMR (400MHz, $CDCl_3$)δ 8.34 (d, *J* = 13.6 Hz, 2H), 7.34-7.38 (m, 8H), 7.22-7.26 (m, 6H), 7.06 (d, *J* = 7.6 Hz, 4H), 6.29 (d, *J* = 14.0 Hz, 2H), 4.32 (s, 4H), 3.92 (s, 4H), 3.75 (t, *J* = 6.4 Hz, 4H), 3.58-3.63 (m, 8H), 2.73 (t, *J* = 6.0 Hz, 4H), 2.66 (s, 4H), 1.94 (s, 2H), 1.72 (s, 12H)。

LCMS: m/z = 928.0, $C_{56}H_{64}ClN_2O_8^+$.

Example 76: Preparation of 2-((E)-2-((E)-3-(2-((E)-1-(2,5,8,11,14-pentaoxahexadecan-16-yl)-3,3-dimethylindolin-2 -ylidene)ethenyl)-2-chlorocyclohex-1-en-1-yl)ethenyl)-1-(2-(2-(2-carboxyethoxy)ethox y)ethyl)-3,3-dimethyl-3H-indol-1-ium 2,2,2-trifluoroacetate (76)

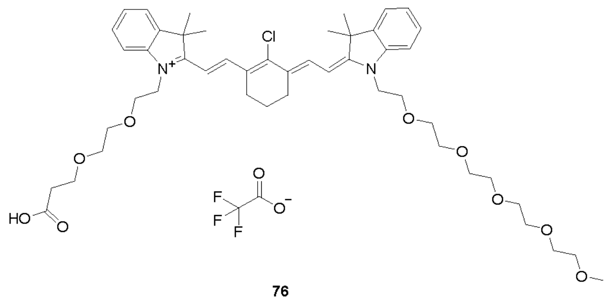

76

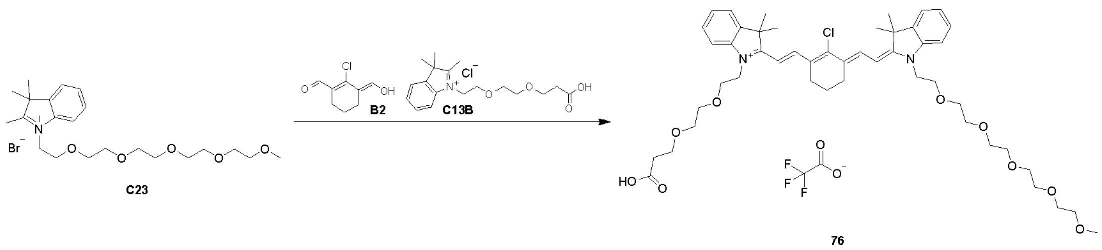

B2 C13B

C23

76

Compound 76 was prepared as a green paste according to the preparation method of Example 21 with Compounds B2, C13B and C23 as raw materials.

$^{1}H$ NMR (400MHz, $CDCl_3$);δ 8.35 (dd, $J_1$ = 14.0 Hz, $J_2$ = 29.2 Hz, 2H), 7.37-7.42 (m, 4H), 7.33-7.35 (m, 2H), 7.15-7.21 (m, 2H), 6.42 (d, *J* = 14.4 Hz, 1H), 6.18 (d, *J* = 14.0 Hz, 1H), 4.25-4.37 (m, 4H), 3.87-3.96 (m, 4H), 3.69 (t, *J* = 6.0 Hz, 2H), 3.60-3.64 (m, 9H), 3.52-3.56 (m, 11H), 3.36 (s, 3H), 2.65-2.69 (m, 4H), 2.56 (t, *J* = 6.4 Hz, 2H), 1.94 (t, *J* = 5.6 Hz, 2H), 1.72 (d, *J* = 5.6 Hz, 12H).

LCMS: m/z = 849.5, $C_{48}H_{66}ClN_2O_9^+$.

Example 77: Preparation of 2,2'-((1E,1'E)-((3E,3'E)-(((2E,2'E)-((pentane-1,5-diylbis(azanediyl))bis(6-oxohexane-6, 1-diyl))bis(3,3-dimethylindolin-1-yl-2-ylidene))bis(ethane-2,1-diylidene))bis(2-chloroc yclohex-1-en-1-yl-3-ylidene))bis(ethene -2,1-diyl))bis(1-ethyl-3,3-dimethyl-3H-indol-1-ium) chloride (77)

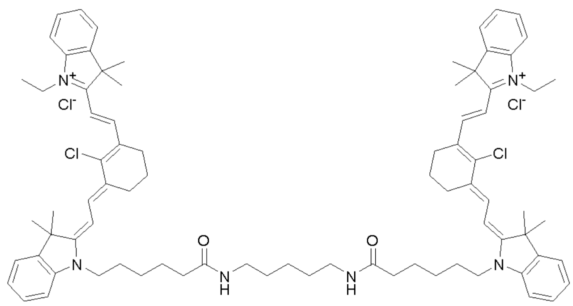

77

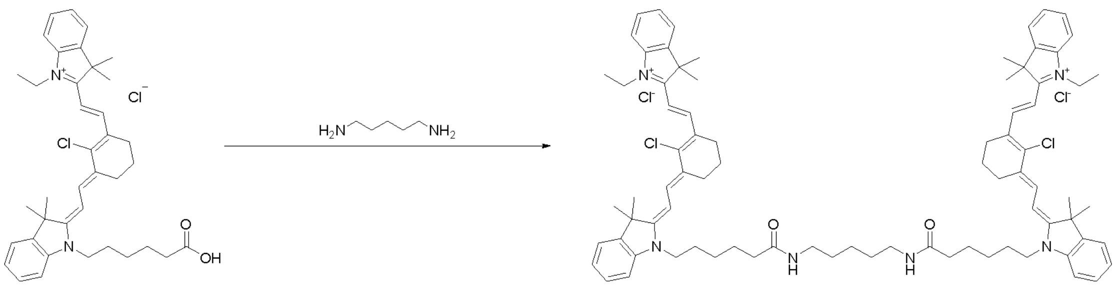

30 77

A mixture of Compound 30 (206 mg, 324 μmol, 2.00 eq), pentane-1,5-diamine (16.6 mg, 162 μmol, 19.0 μL, 1.00 eq), DIEA (62.9 mg, 486 μmol, 84.7 μL, 3.00 eq), 2-chloro-1-methyl-pyridin-1-ium iodide(62.1 mg, 243 μmol, 1.50 eq) and

DMF (3.00 mL) was stirred at 25°C under nitrogen atmosphere for 12 hours. 2-Chloro-1-methyl-pyridin-1-ium iodide (20.7 mg, 81.0 μmol, 0.500 eq) was added again, and stirring was continued for 6 hours. The resulting mixture was filtered to obtain a crude product (400 mg). The crude product was purified by reversed-phase HPLC (HCl condition; column: Phenomenex luna C18 150mm × 40 mm × 15 μm; mobile phase: [water (HCl) - ACN]; gradient: 60% - 90% B, 10 min) to obtain Compound 77 (25.27 mg, 18.4 μmol, 6.13% yield, 97.0% purity) as a blue solid.

$^1$H NMR: (400 MHz, MeOD)δ 8.44 (t, *J* = 16.0 Hz, 4H), 7.51-7.55 (m, 4H), 7.42-7.45 (m, 4H), 7.26-7.37 (m, 8H), 6.24-6.33 (m, 4H), 4.21-4.26 (m, 4H), 4.15-4.19 (m, 4H), 3.11 (t, *J* = 6.8 Hz, 4H), 2.73-2.77 (m, 8H), 2.19 (t, *J* = 7.2 Hz, 4H), 1.93-1.99 (m, 4H), 1.82-1.89 (m, 4H), 1.73 (d, *J* = 2.4 Hz, 24H), 1.65-1.70 (m, 4H), 1.40-1.49 (m, 14H), 1.27-1.33 (m, 2H).

LCMS: m/z = 631.3, $C_{81}H_{102}Cl_2N_6O_2^{2+}/2$.

According to the preparation method of Example 77 above, the following compounds were prepared using corresponding acids and diamines:

| Example No. | Compound structure and reaction formula | NMR or MS, and appearance |
|---|---|---|
| 78 | 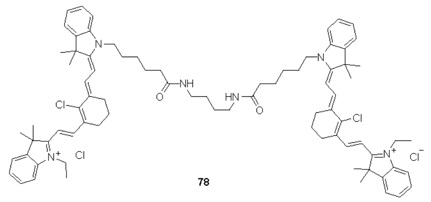 78 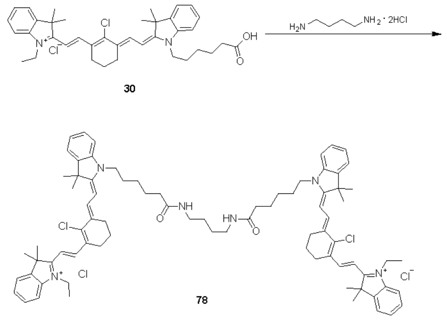 30 78 | $^1$H NMR (400MHz, $CDCl_3$) δ 8.56 - 8.55 (m, 1H), 8.38 (t, *J* = 12.8 Hz, 4H), 7.44 - 7.39 (m, 9H), 7.28 (s, 1H), 7.24 (t, *J* = 8.8 Hz, 3H), 7.18 (d, *J* = 7.6 Hz, 2H), 6.22 - 6.13 (m, 4H), 4.21-4.19 (m, 4H), 4.15 - 4.13 (m, 4H), 3.93 - 3.52 (m, 4H), 3.32 (s, 4H), 2.73 (s, 8H), 2.53 (t, *J* = 6.8 Hz 4H), 1.99 (s, 4H), 1.89 - 1.82 (m, 10H), 1.73 (s, 24H), 1.57 - 1.56 (m, 4H), 1.48 - 1.47 (m, 6H).<br>LCMS: m/z = 624.9 $C_{80}H_{100}Cl_2N_6O_2^{2+}/2$.<br>Green solid. |
| 79 | 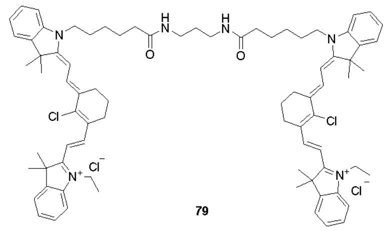 79 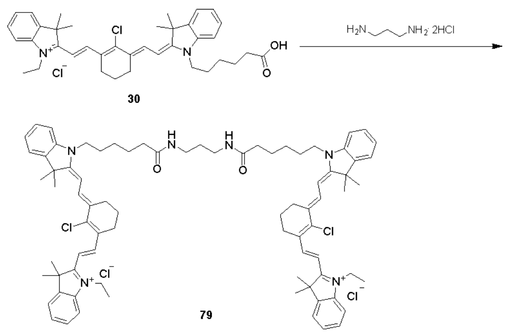 30 79 | $^1$H NMR (400MHz, $CDCl_3$)δ 8.39 - 8.34 (m, 4H), 8.11 (s, 1H), 7.44 - 7.38 (m, 9H), 7.25 - 7.8 (m, 6H), 6.18 (t, *J* = 16.0 Hz 4H), 4.26 - 4.21 (m, 4H), 4.17 - 4.10 (m, 4H), 3.30 - 3.29 (m, 4H), 2.73 (s, 8H), 2.40 (t, *J* = 6.80 Hz, 4H), 2.01 - 1.99 (m, 4H), 1.88 - 1.78 (m, 10H), 1.73 (s, 24H), 1.56 - 1.53 (m, 4H), 1.48 - 1.47 (m, 6H).<br>LCMS: m/z = 617.5 $C_{79}H_{98}Cl_2N_6O_2^{2+}/2$.<br>Green solid. |

(continued)

| Example No. | Compound structure and reaction formula | NMR or MS, and appearance |
|---|---|---|
| 80 | 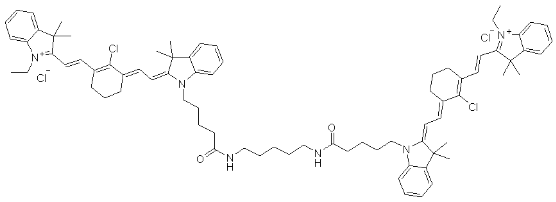 80 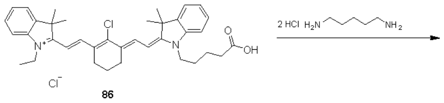 86 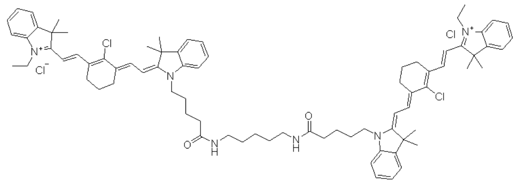 80 | $^1$H NMR: (400MHz, $CDCl_3$)δ 8.30 - 8.41 (m, 4H), 8.09 - 8.11 (m, 2H), 7.37 - 7.41 (m, 10H), 7.28 - 7.29 (m, 1H), 7.23 -7.26 (m, 3H), 7.14 - 7.16 (m, 2H), 6.28 - 6.31 (m, 2H), 6.10 (t, *J* = 14.0 Hz, 2H), 4.10 - 4.19 (m, 8H), 3.33 (s, 4H), 2.79 (s, 4H), 2.70 (t, *J* = 5.2 Hz, 8H), 1.94 - 2.02 (m, 12H), 1.72 (s, 24H), 1.62 - 1.65 (m, 6H), 1.45 (t, *J* = 7.2 Hz, 6H).<br>LCMS: m/z = 617.1 $C_{79}H_{98}Cl_2N_6O_2^{2+}/2$.<br>Green solid. |
| 81 | 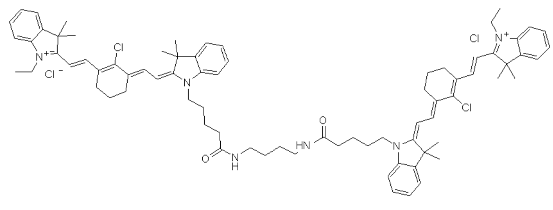 81 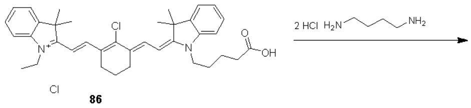 86 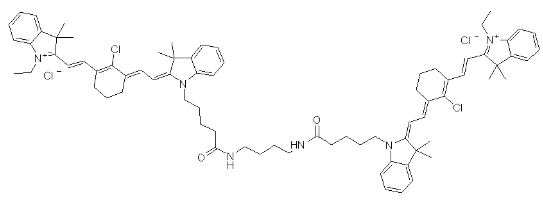 81 | $^1$H NMR (400MHz, $CDCl_3$) δ 8.31 - 8.40 (m, 4H), 7.71 - 7.72 (m, 2H), 7.38 - 7.43 (m, 9H), 7.37 (d, *J* = 3.6 Hz, 2H), 7.23 - 7.25 (m, 3H), 7.17 - 7.19 (m, 2H), 6.25 (t, *J* = 14.4 Hz, 2H), 6.11 (d, *J* = 14.0 Hz, 2H), 4.15 - 4.21 (m, 8H), 3.34 - 3.39 (m, 4H), 2.70 - 2.77 (m, 8H), 2.56 (s, 4H), 2.13 - 2.27 (m, 4H), 1.90 - 2.00 (m, 12H), 1.71 (s, 24H), 1.45 (d, *J* = 7.2 Hz, 6H).<br>LCMS: m/z = 609.6 $C_{78}H_{96}Cl_2N_6O_2^{2+}/2$.<br>Green solid |

(continued)

| Example No. | Compound structure and reaction formula | NMR or MS, and appearance |
|---|---|---|
| 82 | 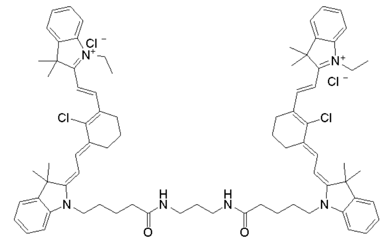<br>82<br>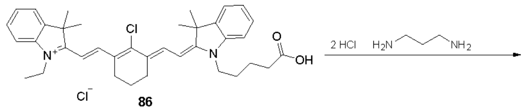<br>86<br>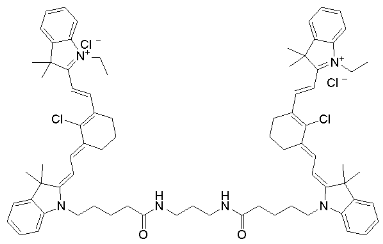<br>82 | $^1$H NMR (400 MHz, $CDCl_3$)δ 8.33-8.41 (m, 4H), 8.03 (s, 2H), 7.37-7.42 (m, 8H), 7.28-7.30 (m, 2H), 7.26-7.27 (m, 2H), 7.24-7.26 (m, 2H), 7.16-7.24 (m, 2H), 6.26 (d, *J* = 14.4 Hz, 2H), 6.13 (d, *J* = 14 Hz, 2H), 4.19-4.21 (m, 8H), 3.31-3.33 (m, 4H), 2.77-2.97 (m, 4H), 2.70-2.73 (m, 4H), 2.53 (s, 4H), 2.00-2.03 (m, 4H), 1.93 (s, 8H), 1.79-1.92 (m, 2H), 1.72 (s, 24H), 1.46 (t, *J* = 7.2 Hz, 6H).<br>LCMS: m/z = 603.3, $C_{77}H_{94}Cl_2N_6O_2{}^{2+}/2$.<br>Blue solid. |

Example 83: Preparation of 2-((E)-2-((E)-2-chloro-3-(2-((E)-3,3-dimethyl-1-(6-oxo-6-(anilino)hexyl)indol-2-methyl ene) ethylidene)cyclopent-1-en-1yl)ethenyl)-1,3,3-trimethyl-3H-indol-1-ium chloride (83)

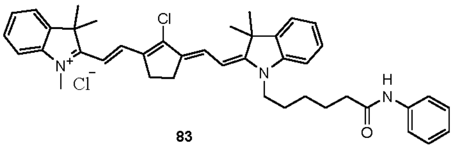

83

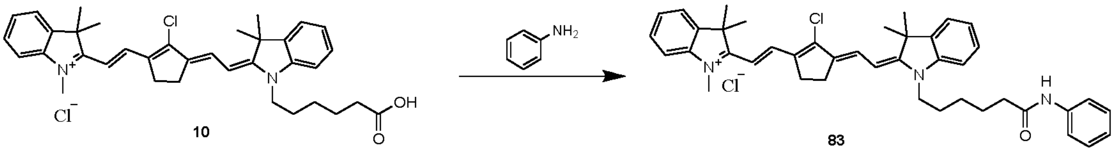

10 83

Aniline (16.3 mg, 175 μmol, 16.0 μL, 1.00 eq) and DIEA (113 mg, 877 umol, 153 uL, 5.00 eq) were added to a mixture of Compound 10 (100 mg, 175 μmol, 1.00 eq) and $T_3P$ (3.36 g, 5.28 mmol, 3.14 mL, 50% purity, 30.1 eq). The mixture was stirred at 50°C for 12 hours. The resulting mixture was concentrated under reduced pressure, and the residues were purified by preparative HPLC (HCl condition; column: YMC Triart C18 150mm×25 mm×5 μm; mobile phase: [water (HCl)-ACN]; B%: 56%-86%, 8.5 min) to obtain Compound 83 (21.57 mg, 30.3 μmol, 9.76% yield, 90.5% purity) after lyophilization as a green solid.

$^1$H NMR (400 MHz, $CDCl_3$) δ 10.8 (s, 1H), 7.96-7.98 (m, 2H), 7.86 (d, *J* = 14 Hz, 1H), 7.67 (d, *J* = 13.6 Hz, 1H), 7.37-7.44 (m, 3H), 7.30-7.34 (m, 2H), 7.17-7.24 (m, 4H), 6.95-7.03 (m, 2H), 6.39 (d, *J* = 14.4 Hz, 1H), 5.68 (d, *J* = 14.4 Hz, 1H), 4.31-4.34 (m, 2H), 3.52 (s, 3H), 3.04-3.12 (m, 2H), 2.69-2.77 (m, 4H), 1.61-1.71 (m, 18H).

LCMS: m/z = 644.3, $C_{42}H_{47}ClN_3O^+$.

Example 84: Preparation of 1-(5-carboxypentyl)-2-((E)-2-((E)-3-(2-((E)-1-(5-carboxypentyl)-3,3-dimethylindolin-2-ylidene)ethenyl)-2-chlorocyclopent-1-en-1-yl)ethenyl)-3,3-dimethyl-3H-indol-1-ium chloride (84)

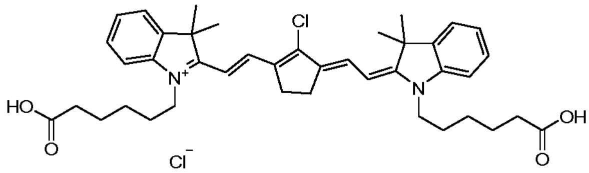

84

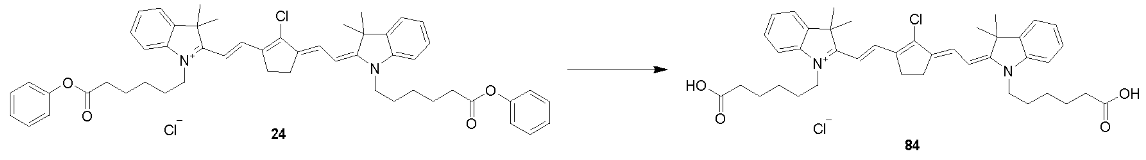

24 84

HCl/dioxane (4.00 M, 5.10 mL, 50.0 eq) solution was added to a solution of Compound 24 (350 mg, 407 μmol, 1.00 eq) in ACN (1.00 mL) and $H_2O$(73.5 mg, 4.08 mmol, 73.5 μL, 10.0 eq), and stirred at 15°C for 2 hours. The resulting mixture was concentrated under reduced pressure, and then purified by preparative chromatography (column: Phenomenex luna C18 150mm×40mm×15μm;mobile phase: [water(HCl)-ACN]; gradient: 45%-75% B, 10 min) to obtain Compound 84 (55.0 mg, 76.5 μmol, 18.7% yield, 98.2% purity) as a green solid.

$^1$H NMR (400MHz, DMSO-d6) δ 12.04 (brs, 2H), 7.76 (d, J = 13.6 Hz, 2H), 7.61 (d, J = 7.2 Hz, 2H), 7.42 - 7.44 (m, 4H), 7.25 - 7.29 (m, 2H), 6.15 (d, J = 14.0 Hz, 2H), 4.19 (t, J = 6.8 Hz, 4H), 2.94 (s, 4H), 2.21 (t, J = 7.2 Hz, 4H), 1.72 - 1.76 (m, 4H), 1.66 (s, 12H), 1.55 - 1.58 (m, 4H), 1.40 - 1.42 (m, 4H).

LCMS: m/z = 699.4, $C_{41}H_{50}ClN_2O_4^+$.

According to the preparation method of Example 84 above, the following compounds were prepared using corresponding raw materials:

| Example No. | Compound structure and reaction formula | NMR or MA, and appearance |
|---|---|---|
| 85A and 85B | 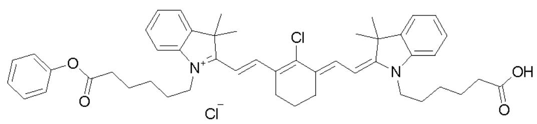<br>85A<br>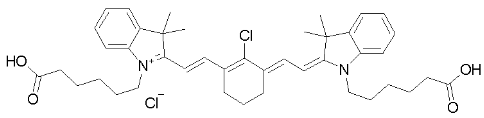<br>85B<br>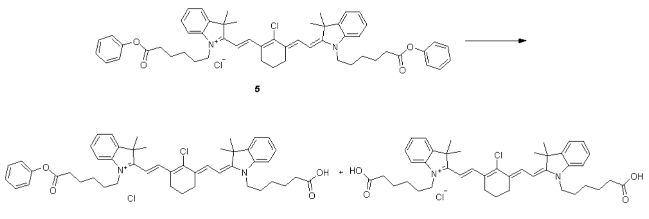<br>5 85A 85B | **85A:** $^1$H NMR (400MHz, $CDCl_3$)δ 8.42-8.3 (m, 2H), 7.44-7.34 (m, 6H), 7.30-7.28 (m, 1H), 7.25-7.19 (m, 3H), 7.11-7.09 (m, 1H), 7.03-7.01 (m, 2H), 6.27 -6.24 (m, 1H), 6.11-6.08 (m, 1H), 4.15-4.06 (m, 4H), 2.72-2.60 (m, 6H), 2.54-2.51 (m, 2H), 1.99-1.52 (m, 27H);<br>LCMS: m/z =759.5(M)$^+$.<br>85B: LCMS: m/z = 683.4(M)$^+$.<br>Both were green solid. |

(continued)

| Example No. | Compound structure and reaction formula | NMR or MA, and appearance |
|---|---|---|
| 86 | 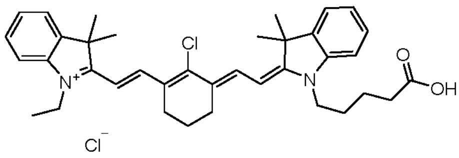 86 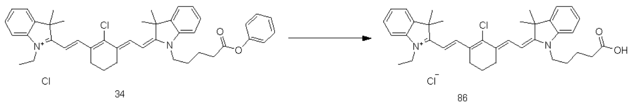 34 86 | LCMS: m/z = 583.5 $C_{37}H_{44}ClN_2O_2^+$. Green solid. |
| 87 | 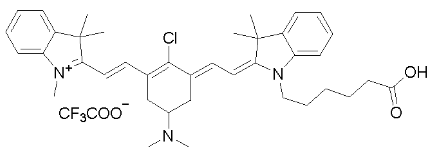 **87** 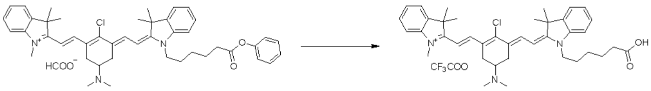 50 87 | $^1$H NMR (400 MHz, MeOD)δ 8.49 (dd, $J_1$ = 8.0 *Hz, $J_2$* = 14.0 Hz, 2H), 7.54-7.57 (m, 2H), 7.31-7.50 (m, 6H), 6.32-6.41 (m, 2H), 4.27 (t, *J* = 7.2 Hz, 2H), 3.79-3.85 (m, 1H), 3.77 (s, 3H), 3.33-3.39 (m, 2H), 3.10 (s, 6H), 2.88-2.94 (m, 2H), 2.33 (t, *J* = 7.2 Hz, 2H), 1.86-1.94 (m, 2H), 1.75 (t, *J* = 2.0 Hz, 12H), 1.67-1.71 (m, 2H), 1.50-1.56 (m, 2H);<br>LCMS: m/z = 626.5 $C_{39}H_{49}ClN_3O_2^+$. Green solid. |

Example 88: Preparation of 2-((E)-2-((E)-3-(2-((E)-1-(5-carboxypentyl)-3,3-dimethylindolin-2-ylidene)ethenyl)-2-c hlorocyclopent-1-en-1-yl)ethenyl)-1-(6-(2,6-dimethylphenoxy)-6-oxohexyl)-3,3-dimeth yl-3H-indol-1-ium acetate (88)

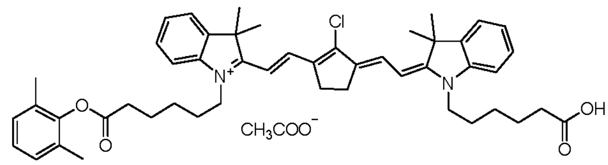

**88**

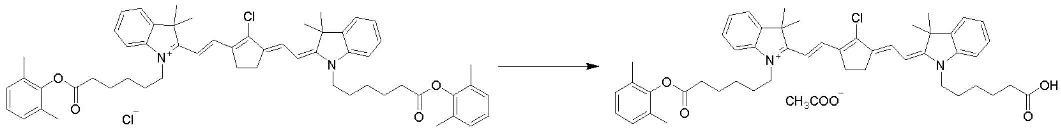

23 88

HCl/dioxane solution (4.00 M, 2.05 mL, 50.0 eq) and $H_2O$ (29.6 mg, 1.64 mmol, 29.5 μL, 10.0 eq) were added to a solution of Compound 23 (150 mg, 164 μmol, 1.00 eq) in MeCN (3 mL), and stirred at 40°C for 12 hours. The resulting mixture was filtered, and the filtrate was concentrated and purified by preparative chromatography HPLC (column: Phenomenex luna C18 150mm×25mm×10μm; mobile phase: [water(TFA)-ACN]; gradient: 55%-85% B, 10 min) to obtain Compound 88 (24.0 mg, 24.5 μmol, 14.9% yield, 90.6% purity) as a green solid.

$^1$H NMR (400MHz, $CDCl_3$) δ 7.81 - 7.87 (m, 2H), 7.36 - 7.40 (m, 4H), 7.23 - 7.25 (m, 2H), 7.07 - 7.11 (m, 2H), 7.03 (s, 3H), 6.00 - 6.05 (m, 2H), 4.05 - 4.08 (m, 4H), 2.95 (s, 2H), 2.65 -2 .67 (m, 2H), 2.44 (s, 2H), 2.10 (s, 6H), 1.85 - 1.90 (m, 8H), 1.71 - 1.76 (m, 12H), 1.54 - 1.61 (m, 6H).

LCMS: m/z = 773.4, $C_{49}H_{58}ClN_2O_4^+$.

According to the preparation method of Example 88 above, the following compounds were prepared using corresponding raw materials:

| Example No. | Compound structure and reaction formula | NMR or MS, and appearance |
|---|---|---|
| 89 | 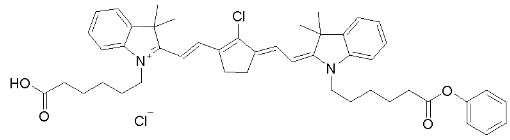<br>89<br>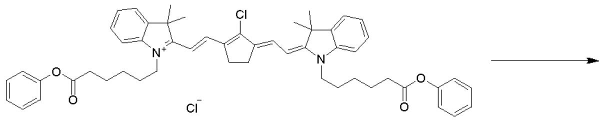<br>24<br>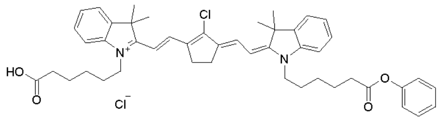<br>89 | $^1$H NMR (400MHz, $CDCl_3$) δ 7.83 - 7.87 (m, 2H), 7.33 - 7.38 (m, 6H), 7.19 - 7.22 (m, 3H), 7.13 (s, 2H), 7.02 (d, *J* = 8.0 Hz, 2H), 5.95 - 6.08 (m, 2H), 3.93 - 4.20 (m, 4H), 2.62 (s, 2H), 2.47 (s, 2H), 1.77 - 1.86 (m, 9H), 1.70 (s, 12H), 1.56 - 1.66 (m, 7H).<br>LCMS: m/z = 745.4 $C_{47}H_{54}ClN_2O_4^+$.<br>Green solid. |
| 91 | 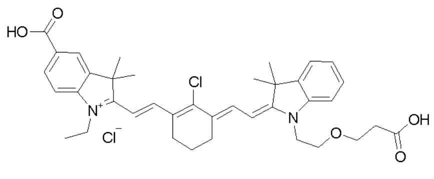<br>91<br>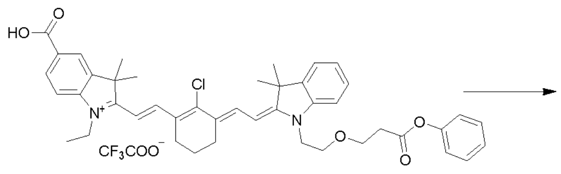<br>117<br>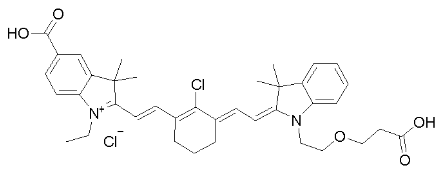<br>91 | $^1$H NMR (400 MHz, MeOD)δ 8.58 (d, *J* = 14.8 Hz, 1H), 8.27 (d, *J* = 13.6 Hz, 1H), 8.02-8.05 (m, 2H), 7.60 (d, *J* = 7.2 Hz, 1H), 7.48-7.50 (m, 2H), 7.38-7.42 (m, 1H), 7.18 (d, *J* = 8.4 Hz, 1H), 6.68 (d, *J* = 14.8 Hz, 1H), 6.14 (d, *J* = 13.6 Hz, 1H), 4.52 (t, *J* = 4.8 Hz, 2H), 4.11 (q, *J* = 7.2 Hz, 2H), 3.91 (t, *J* = 4.8 Hz, 2H), 3.67 (t, *J* = 6.0 Hz, 2H), 2.74 (t, *J* = 6.0 Hz, 4H), 2.39 (t, *J* = 6.0 Hz, 2H). 1.94-1.98 (m, 2H), 1.78 (s, 6H), 1.72 (s, 6H), 1.37 (t, *J* = 6.8 Hz, 3H).<br>LCMS: m/z = 643.2 $C_{38}H_{44}ClN_2O_5^+$.<br>Green solid. |
| 92 | 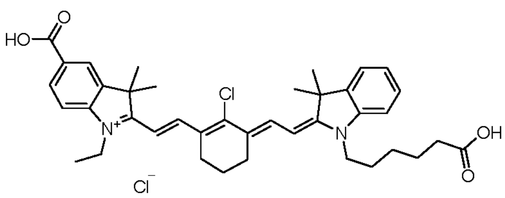<br>92 | $^1$H NMR (400 MHz, MeOD)δ 8.63 (d, *J* = 14.8 Hz, 1H), 8.28 (d, *J* = 13.6 Hz, 1H), 8.04-8.08 (m, 2H), 7.64 (d, *J* = 7.6 Hz, 1H), 7.53-7.54 (m, 2H), 7.43-7.46 (m, 1H), 7.22 (d, *J* = 8.0 Hz, 1H), 6.58 (d, *J* = 14.8 Hz, 1H), 6.14 (d, *J* = 13.6 Hz, |

(continued)

| Example No. | Compound structure and reaction formula | NMR or MS, and appearance |
|---|---|---|
| | 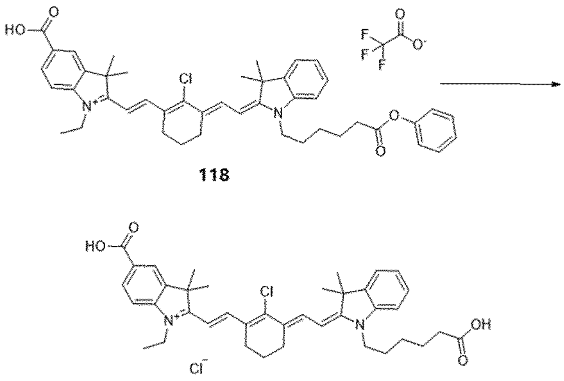 118 92 | 1H), 4.36 (t, *J* = 7.6 Hz, 2H), 4.08-4.14 (m, 2H), 2.76-2.77 (m, 4H), 2.33 (t, *J* = 7.2 Hz, 2H), 1.97-2.01 (m, 2H), 1.91-1.94 (m, 2H), 1.76 (d, *J* = 18.8 Hz, 12H), 1.67-1.71 (m, 2H), 1.50-1.56 (m, 2H), 1.38 (t, *J* = 7.2 Hz, 3H).<br>LCMS: m/z = 641.4 $C_{39}H_{46}ClN_2O_4^+$.<br>Green solid. |
| 93 | | $^1$H NMR (400 MHz, MeOD)δ 8.45 (d, *J* = 15.2 Hz, 1H), 8.39 (d, *J* = 14.8 Hz, 1H), 8.13-8.15 (m, 2H), 7.63-7.70 (m, 2H), 7.49-7.59 (m, 2H), 7.37-7.42 (m, 3H), 7.24 (t, *J* = 7.2 Hz, 1H), 7.02 (d, *J* = 7.6 Hz, 2H), 6.51 (d, *J* = 14.8 Hz, 1H), 6.19 (d, *J* = 14.4 Hz, 1H), 4.60-4.66 (m, 4H), 4.45-4.80 (m, 2H), 4.23-4.28 (m, 2H), 3.25 (s, 6H), 2.63-2.67 (m, 2H), 1.97-2.04 (m, 2H), 1.83-1.90 (m, 2H), 1.78 (d, *J* = 8.8 Hz, 12H), 1.61-1.67 (m, 2H), 1.43 (t, *J* = 7.2 Hz, 3H).<br>LCMS: m/z = 374.0 $C_{46}H_{54}ClN_3O_4^{2+}/2$.<br>Blue solid. |
| 94 | | $^1$H NMR (400 MHz, DMSO)δ 8.15-8.21 (m, 2H), 7.29-7.39 (m, 4H), 6.96-7.01 (m, 2H), 6.22 (t, *J* = 14.0 Hz, 2H), 4.16-4.17 (m, 2H), 3.81-3.82 (m, 6H), 3.67 (s, 3H), 2.67-2.68 (m, 4H), 2.20 (t, *J* = 7.2 Hz, 2H), 1.85-1.86 (m, 2H), 1.70-1.75 (m, 2H), 1.66 (d, *J* = 2.8 Hz, 12H), 1.52-1.59 (m, 2H), 1.36-1.41 (m, 2H).<br>LCMS: m/z = 643.4 $C_{39}H_{48}ClN_2O_4^+$.<br>Green solid. |

(continued)

| Example No. | Compound structure and reaction formula | NMR or MS, and appearance |
|---|---|---|
| 95 | 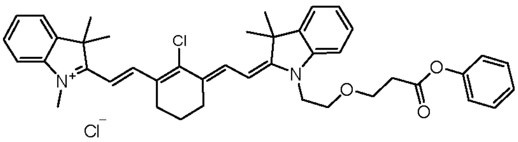<br>95<br>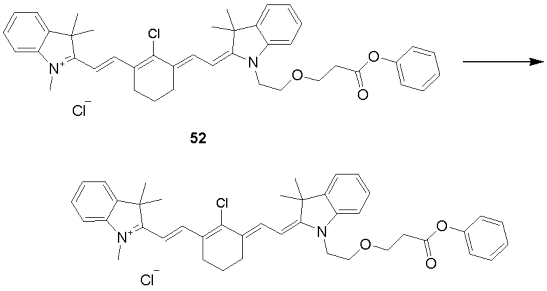<br>52<br>95 | $^1$H NMR (400MHz, $CDCl_3$);δ 8.27-8.35 (m, 1H), 7.31-7.37 (m, 7H), 7.14-7.21 (m, 5H), 4.37 (s, 2H), 3.90 (s, 3H), 3.55-3.72 (m, 4H), 2.63 (t, *J* = 5.6 Hz, 3H), 1.94 (s, 2H), 1.68 (d, *J* = 7.6 Hz, 15H);<br>LCMS: m/z = 585.3 $C_{42}H_{46}ClN_2O_3^+$.<br>Green solid. |
| 96 | 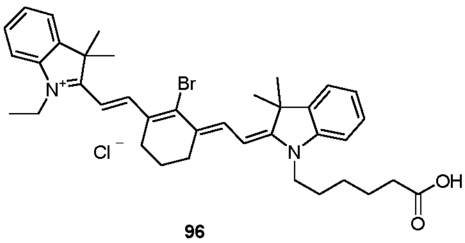<br>96<br>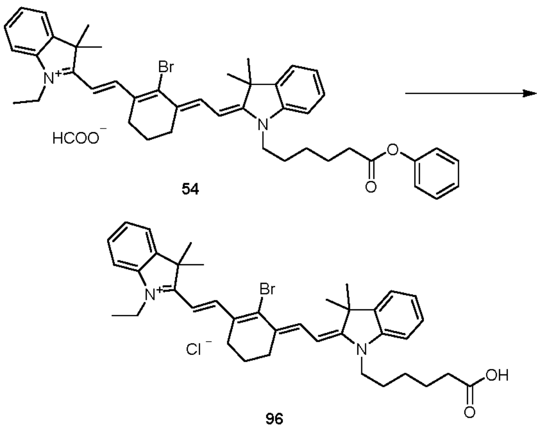<br>54<br>96 | $^1$H NMR (400 MHz, $CDCl_3$) δ 8.34-8.42 (m, 2H), 7.38-7.42 (m, 4H), 7.28-7.30 (m, 2H), 7.15 (dd, $J_1$ = 8.0 *Hz*, $J_2$ = 20.8 Hz, 2H), 6.23 (d, *J* = 14.0 Hz, 1H), 6.12 (d, *J* = 14.0 Hz, 1H), 4.08-4.13 (m, 4H), 2.70 (q, *J* = 6.0 Hz, 4H), 2.47 (t, *J* = 7.2 Hz, 2H), 1.97-2.00 (m, 2H), 1.85-1.89 (m, 2H), 1.75-1.79 (m, 2H), 1.74 (d, *J* = 0.8 Hz, 12H), 1.53-1.57 (m, 2H), 1.45 (t, *J* = 7.2 Hz, 3H).<br>LCMS: m/z = 643.3 $C_{38}H_{46}BrN_2O_2^+$.<br>Green solid. |
| 97 | 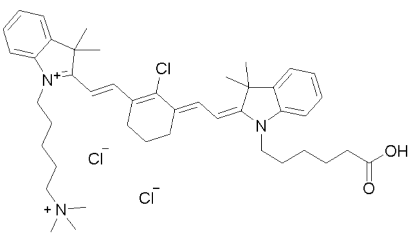<br>97 | $^1$H NMR (400 MHz, DMSO)δ 8.23-8.31 (m, 2H), 7.64 (t, *J* = 8.0 Hz, 2H), 7.43-7.50 (m, 4H), 7.28-7.34 (m, 2H), 6.33 (dd, $J_1$ = 14.4 Hz, $J_2$ = 27.2 Hz, 2H), 4.19-4.27 (m, 4H), 3.22-3.27 (m, 4H), 3.01 (s, 9H), 2.67-2.74 (m, 4H), 2.21 (t, *J* = 7.2 Hz, 2H), 1.84-1.88 (m, 2H), 1.74-1.80 (m, 6H), 1.68 (s, 12H), 1.54-1.58 (m, 2H), 1.36-1.42 (m, 2H).<br>LCMS: m/z = 349.2 $C_{44}H_{60}ClN_3O_2^{2+}/2$.<br>Green solid. |

(continued)

| Example No. | Compound structure and reaction formula | NMR or MS, and appearance |
|---|---|---|
| | 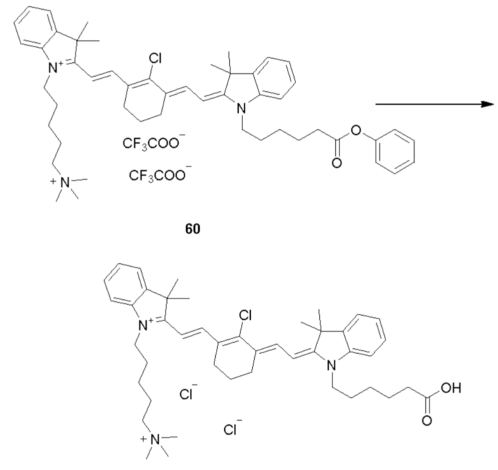 60 97 | |
| 98 | 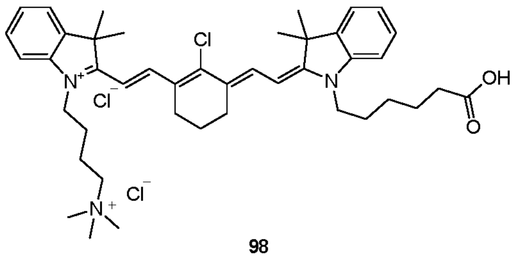 98 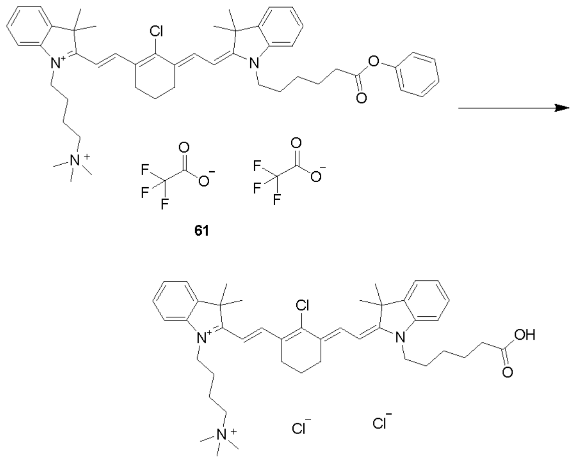 61 98 | $^1$H NMR (400MHz, DMSO-$d_6$)δ 12.01 (s, 1H), 8.22-8.33 (m, 2H), 7.61-7.68 (m, 2H), 7.46-7.51 (m, 4H), 7.26-7.35 (m, 2H), 6.23-6.42 (m, 2H), 4.20-4.28 (m, 4H), 3.36-3.38 (m, 2H), 3.05 (s, 9H), 2.71 (d, $J$ = 4.8 Hz, 4H), 2.20 (t, $J$ = 7.2 Hz, 2H), 1.86-1.87 (m, 4H), 1.68-1.77 (m, 16H), 1.55-1.58 (m, 2H), 1.37-1.40 (m, 2H). LCMS: m/z = 342.2 $C_{43}H_{58}ClN_3O_2^{2+}/2$. Green solid. |
| 99 | 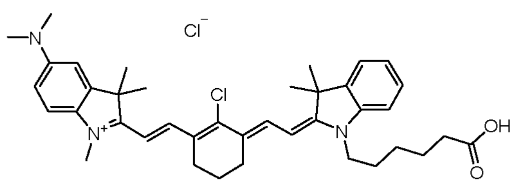 99 | $^1$H NMR (400MHz, MeOD)δ 8.61 (t, $J$ = 10.0 Hz, 1H), 8.22 (t, $J$ = 8.80 Hz, 1H), 7.91 (d, $J$ = 2.00 Hz, 1H), 7.72 - 7.64 (m, 2H), 7.58 - 7.51 (m, 2H), 7.44 (t, $J$ = 7.60 Hz, 1H), 7.30 (d, $J$ = 14.8 Hz, 1H), 6.61 (d, $J$ = 14.8 Hz, 1H), 6.08 (d, $J$ = 13.6 Hz, 1H), 4.39 (t, $J$ = 7.20 Hz, 2H), 3.54 (s, 3H), 3.33 (s, 6H), 2.76 (dd, $J_1$ = 6.00 Hz, $J_2$ = 12.4 Hz, 4H), 2.33 (t, $J$ = 7.20 Hz, 2H), 1.98 - 1.93 (m, 4H), 1.77 (d, $J$ = 8.00 Hz, 12H), 1.73 - 1.69 (m, 2H), 1.54 - 1.51(m, 2H). |

(continued)

| Example No. | Compound structure and reaction formula | NMR or MS, and appearance |
|---|---|---|
| | 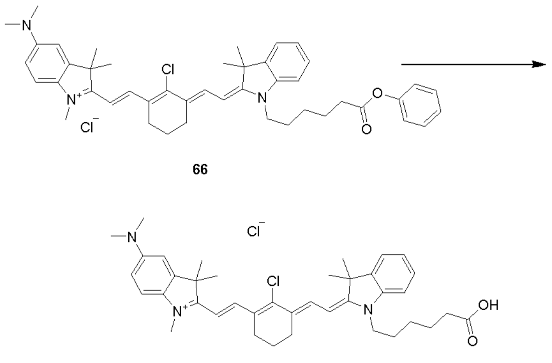 66 99 | LCMS: m/z = 626.4 $C_{39}H_{49}ClN_3O_2^+$. Green paste. |
| 100 | 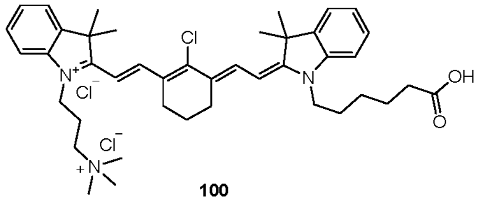 100 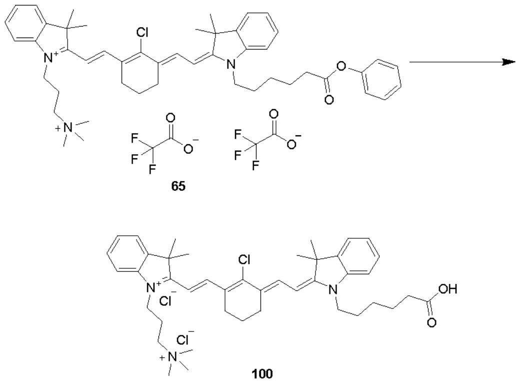 65 100 | $^1H$ NMR (400MHz, $CDCl_3$)$\delta$ 8.30 - 8.42 (m, 2H), 7.30 - 7.44 (m, 5H), 7.09-7.13 (m, 3H), 6.41-6.44 (m, 1H), 6.04 - 6.12 (m, 1H), 3.85-4.37 (m, 6H), 3.25 (d, *J* = 34.0 Hz, 9H), 2.64-2.74 (m, 4H), 2.27-2.38 (m, 12H), 1.81-1.86 (m, 4H), 1.72 (t, *J* = 4.8 Hz, 4H), 1.41-1.63 (m, 4H). LCMS: m/z = 335.0 $C_{42}H_{56}ClN_3O_{22}^+/2$. Green solid. |

Example 101: Preparation of 6-[(2E)-2-[(2E)-2-[3-[(E)-2-(1-ethyl-3,3-dimethyll-indol-1-ium-2-yl)ethenyl]-2-thiophe nol-cyclopent-2-en-1-methylene]ethylidene]-3,3-dimethyl-indol-1-yl]hexanoate;2,2,2-tr ifluoroacetate (101)

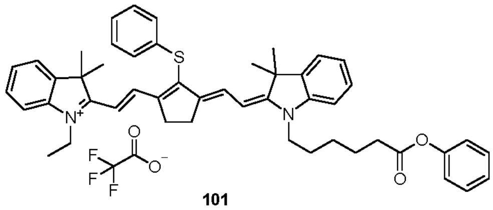

101

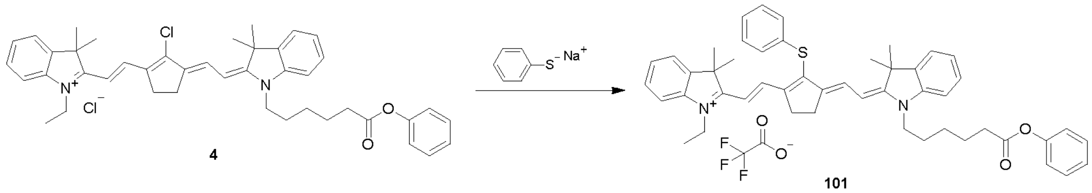

4

101

Triethylamine (78.5 mg, 775 μmol, 107 μL, 3.00 eq) and sodium thiophenolate (41.0 mg, 310 μmol, 1.20 eq) were added to a solution of Compound 4 (200 mg, 258 μmol, 1.00 eq) in THF (1 mL). The mixture was stirred at 25°C for 3 hours. The resulting mixture was concentrated under reduced pressure, and the residues were purified by preparative HPLC (chromatographic column: Phenomenex luna C18 150mm×40mm×15μm; mobile phase: [water(TFA)-ACN]; gradient: 60%-90% B, 10 min) to obtain Compound 101(86.0 mg, 96.0 μmol, 37.1% yield, 94.6% purity) as a blue solid.

$^1H$ NMR (400MHz, MeOD) δ 8.06 - 8.12 (m, 2H), 7.36 - 7.45 (m, 10H), 7.29 - 7.32 (m, 6H), 6.99 - 7.01 (m, 2H), 6.07 - 6.13 (m, 2H), 4.41 - 4.19 (m, 4H), 2.96 - 3.02 (m, 4H), 2.61 (t, J = 14.4 Hz, 2H), 1.81 - 1.92 (m, 4H), 1.56 - 1.57 (m, 2H), 1.48 (d, J = 2.8 Hz, 12H), 1.39 (t, J = 14.4 Hz, 3H).

LCMS: m/z = 733.3, $C_{49}H_{53}N_2O_2S^+$.

According to the preparation method of Example 101 above, the following compounds were prepared using corresponding raw materials:

| Example No. | Compound structure and reaction formula | NMR or MS, and appearance |
|---|---|---|
| 102 | 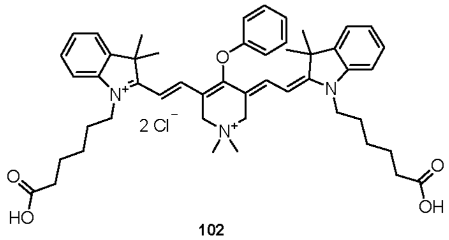 102 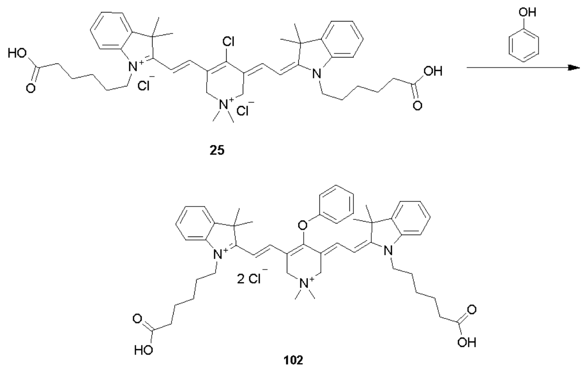 25 102 | $^1H$ NMR (400 MHz, MeOD) δ 8.04 (d, *J* = 15.2 Hz, 2H), 7.42-7.50 (m, 6H), 7.38-7.40 (m, 2H), 7.29-7.33 (m, 2H), 7.25-7.27 (m, 2H), 7.13-7.17 (m, 1H), 6.21 (d, *J* = 14.8 Hz, 2H), 4.79 (s, 4H), 4.24 (t, *J* = 14.8 Hz, 4H), 3.50 (s, 6H), 2.33 (t, *J* = 14.4 Hz, 4H), 1.83-1.87 (m, 4H), 1.68-1.72 (m, 4H), 1.48-1.52 (m, 4H), 1.37 (s, 12H). LCMS: m/z = 385.9, $C_{49}H_{61}N_3O_5^{2+}$. Green solid. |

(continued)

| Example No. | Compound structure and reaction formula | NMR or MS, and appearance |
|---|---|---|
| 103 | 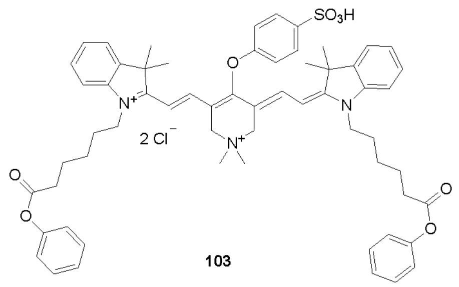 103 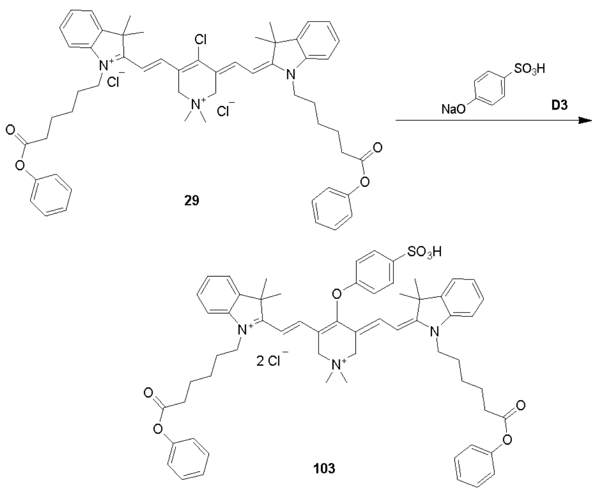 29 103 | $^1$H NMR (400 MHz, MeOD) δ 7.89-7.96 (m, 4H), 7.46-7.47 (m, 2H), 7.37-7.42 (m, 8H), 7.25-7.31 (m, 6H), 7.02-7.04 (m, 4H), 6.14 (d, *J* = 14.8 Hz, 2H), 4.62 (s, 4H), 4.21-4.25 (m, 4H), 3.19 (s, 6H), 2.64 (t, *J* = 7.4 Hz, 4H), 1.81-1.89 (m, 8H), 1.55-1.60 (m, 4H), 1.34 (s, 12H). LCMS: m/z = 502.0, $C_{61}H_{69}N_3O_8S^{2+}$.<br><br>Green solid. |
| 104 | 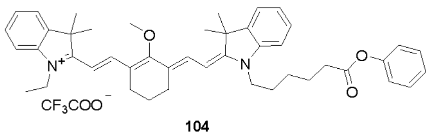 104 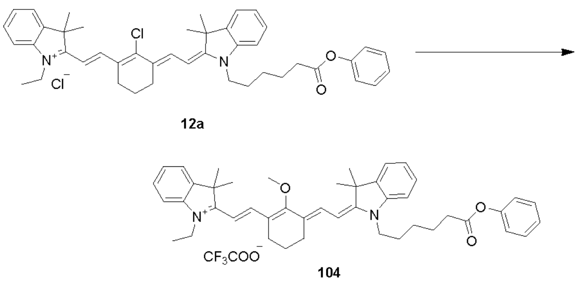 12a 104 | $^1$H NMR (400MHz, $CDCl_3$) δ 7.94 - 7.86 (m, 2H), 7.39 - 7.35 (m, 4H), 7.24 - 7.06 (m, 9H), 6.10 (dd, *J* =22.0, 14.0 Hz, 2H), 4.22-4.09 (m, 4H), 3.66 (s, 3H), 2.79-2.76 (m, 4H), 2.35 (t, J=7.2 Hz,2H), 2.09 - 2.06 (m, 2H), 1.84 - 1.78 (m, 2H), 1.73 - 1.67 (m,2H), 1.55 - 1.48 (m, 2H), 1.42 (t, J=7.2 Hz, 3H), 1.33 - 1.32 (m,12H). LCMS: m/z = 669.41, $C_{45}H_{53}N_2O_3^+$.<br>Green solid. |

(continued)

| Example No. | Compound structure and reaction formula | NMR or MS, and appearance |
|---|---|---|
| 106 | 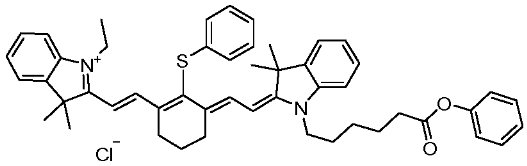 **106** 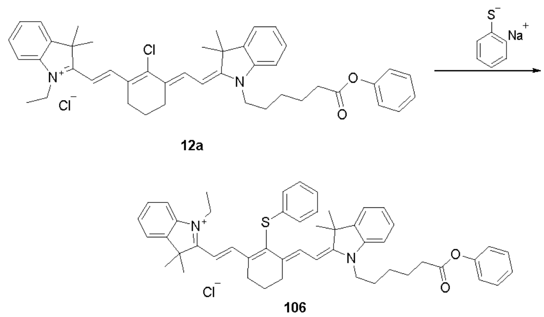 12a 106 | $^1$H NMR (400MHz, $CDCl_3$) $\delta$ 8.66 - 8.72 (m, 2H), 7.35 - 7.38 (m, 4H), 7.28 - 7.32 (m, 2H), 7.22 - 7.25 (m, 7H), 7.07 - 7.16 (m, 3H), 7.03 (d, $J$ = 7.6 Hz, 2H), 6.27 - 6.32 (m, 2H), 4.19 - 4.26 (m, 4H), 2.81 (s, 4H), 2.62 (t, $J$ = 7.2 Hz, 2H), 2.05 (m, 2H), 1.83 - 1.90 (m, 4H), 1.62 - 1.66 (m, 2H), 1.43 - 1.47 (m, 15H). LCMS: m/z = 747.6, $C_{50}H_{55}N_2O_2S^+$.<br>Blue solid. |
| 107 | 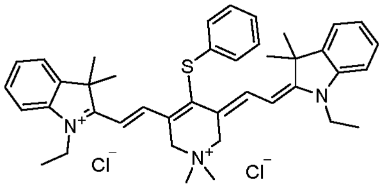 **107** 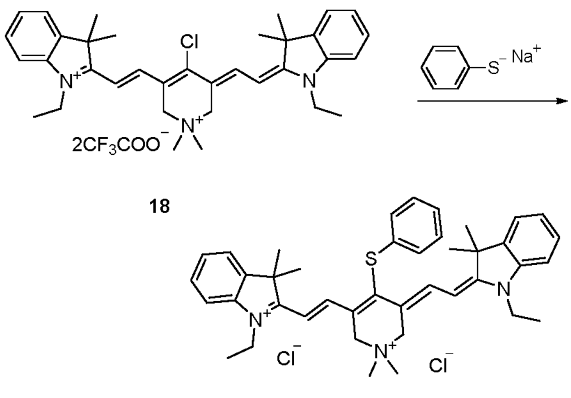 **18** **107** | $^1$H NMR (400MHz, MeOD) $\delta$ 8.79 (d, $J$ = 14.8 Hz, 2H), 7.53 - 7.36 (m, 12H), 7.34 - 7.21 (m, 1H), 6.37 (d, J = 14.8 Hz, 2H), 4.81 (s, 4H), 4.34 (dd, $J_1$ = 7.2 Hz, $J_2$ = 14.4 Hz, 4H), 3.43 (s, 6H), 1.52 (s, 12H), 1.44 (t, $J$ = 7.2 Hz, 6H).<br>LCMS: m/z = 614.4, $C_{41}H_{49}N_3S^{2+}$.<br>Blue solid. |

(continued)

| Example No. | Compound structure and reaction formula | NMR or MS, and appearance |
|---|---|---|
| 108 | 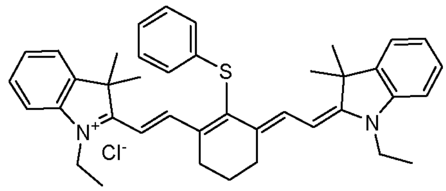 108 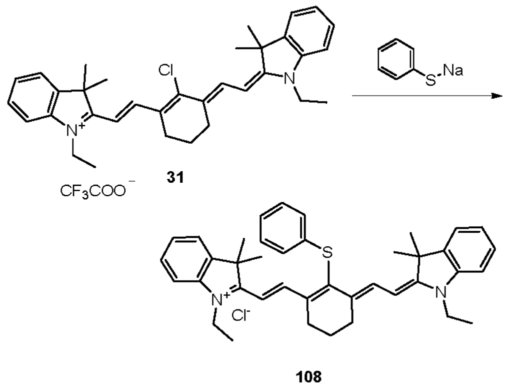 31 108 | $^{1}H$ NMR: (400 MHz, MeOD) δ 8.80-8.83 (m, 2H), 7.40-7.45 (m, 4H), 7.26-7.30 (m, 9H), 7.11-7.24 (m, 1H), 6.28-6.32 (m, 1H), 4.15-4.21 (m, 4H), 2.78-2.81 (m, 4H), 2.03-2.06 (m, 2H), 1.49 (s, 12H), 1.38 (t, *J* = 7.2 Hz, 6H). LCMS: m/z = 585.4, $C_{40}H_{45}N_2S^+$. Blue solid. |
| 112 | 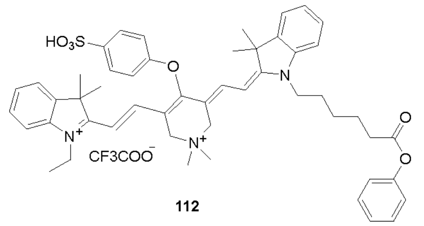 112 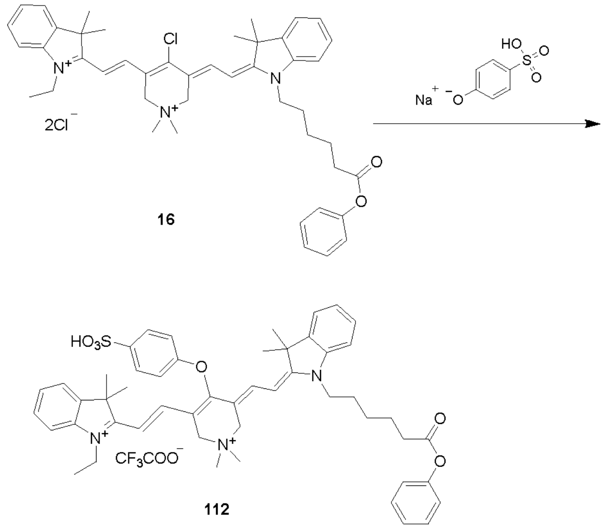 16 112 | $^{1}H$ NMR (400MHz, $CDCl_3$) δ 7.91 (d, *J* = 8.0 Hz, 2H), 7.80 (d, *J* = 14.0 Hz, 2H), 7.39 - 7.35 (m, 2H), 7.29 - 7.28 (m, 1H), 7.22 - 7.15 (m, 5H), 7.09 - 6.96 (m, 7H), 6.38 - 6.21(m, 2H), 4.94 - 4.76 (m, 2H), 4.27 - 4.10 (m, 2H), 3.31 (s, 4H), 2.73 - 2.61 (m, 6H), 2.57 - 2.54 (m, 2H), 1.85 - 1.75 (m, 2H), 1.58 - 1.53 (m, 2H), 1.24 - 1.22 (m, 12H), 0.88 - 0.86 (m, 5H). LCMS: m/z = 841.0 , $C_{51}H_{59}N_3O_6S^{2+}$. Blue solid. |

(continued)

| Example No. | Compound structure and reaction formula | NMR or MS, and appearance |
|---|---|---|
| 113 | 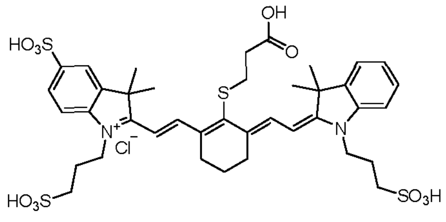 113 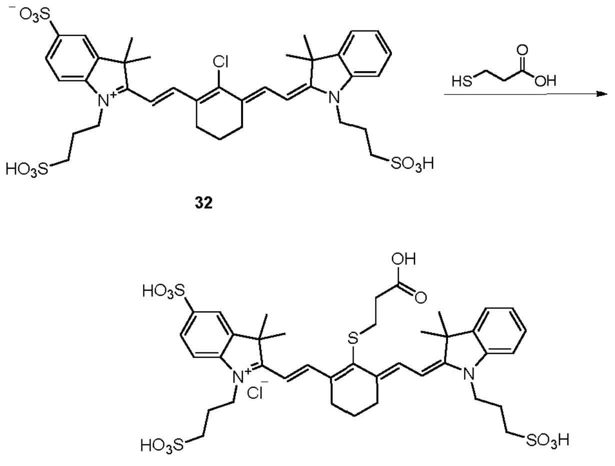 32 113 | $^1$H NMR (400MHz, $CDCl_3$) δ 9.04 (d, *J* = 14.4 Hz, 1H), 8.73 (*d, J* = 13.6 Hz, 1H), 7.86 - 7.84 (m, 2H), 7.57 - 7.52 (m, 2H), 7.36 - 7.30 (m, 2H), 6.62 (d, J= 14.4 Hz, 1H), 6.37 (d, *J* = 13.6 Hz, 1H), 4.49 - 4.45 (m, 2H), 4.31 - 4.27 (m, 2H), 3.08 (t, *J* = 6.4 Hz, 2H), 3.00 - 2.96 (m, 4H), 2.75 - 2.72 (m, 4H), 2.60 (t, *J* = 6.8 Hz, 2H), 2.32 - 2.20 (m, 4H), 1.95 - 1.92 (m, 2H), 1.79 (s, 6H), 1.77 (s, 6H).<br>LCMS: m/z = 849.2, $C_{39}H_{49}N_2O_{11}S_4$.<br>Green solid. |
| 114 | 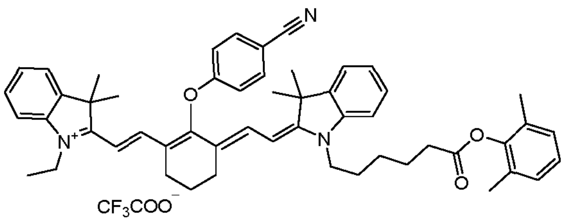 114 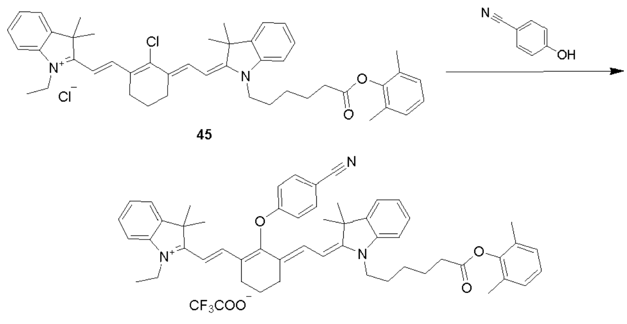 45 114 | $^1$H NMR (400MHz, $CDCl_3$) δ 7.70 - 7.75 (m, 4H), 7.35 - 7.37 (m, 2H), 7.28 - 7.30 (m, 1H), 7.20 - 7.26 (m, 5H), 7.11 -7.15 (m, 2H), 7.05 (s, 3H), 6.02 - 6.08 (m, 2H), 4.05 - 4.13 (m, 4H),2.66 (t, *J* = 7.2 Hz, 4H), 2.11 (s, 6H), 2.02 - 2.04 (m, 2H), 1.85 - 1.89 (s, 5H), 1.56 - 1.59 (m, 1H), 1.52 - 1.53 (m, 2H), 1.40 - 1.43 (m, 3H), 1.34 (d, *J* = 2.0 Hz, 12H).<br>LCMS: m/z = 784.6, $C_{53}H_{58}N_3O_3^+$.<br>Blue solid. |

(continued)

| Example No. | Compound structure and reaction formula | NMR or MS, and appearance |
|---|---|---|
| 115 | 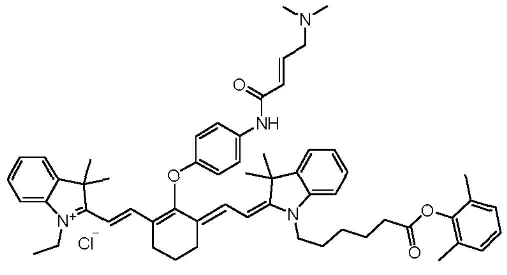 115 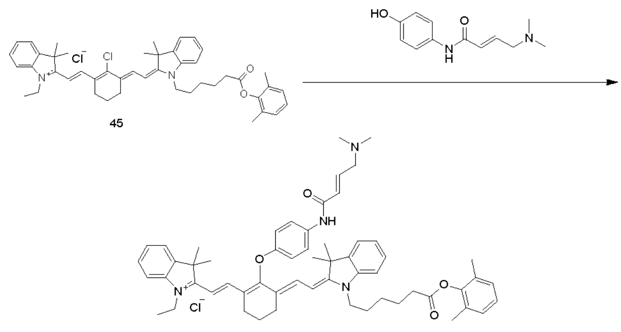 | $^1$H NMR (400MHz, MeOD) δ 7.98 - 8.09 (m, 2H), 7.70 (d, J = 9.2 Hz, 2H), 7.39 (s, 5H), 7.14 - 7.24 (m, 2H), 7.03 - 7.12 (m, 6H), 6.80 - 6.84 (m, 1H), 6.54 (d, J = 15.2 Hz, 1H), 4.84 (s, 8H), 4.04 - 4.16 (m, 2H), 3.98 (d, J = 7.6 Hz, 2H), 2.91 (s, 6H), 2.67 (t, J = 7.2 Hz, 2H ), 2.06 (s, 6H), 1.81 - 1.90 (m, 6H), 1.57 - 1.58 (m, 2H), 1.34 - 1.37 (m, 15H). LCMS: m/z = 886.7, $C_{58}H_{69}N_4O_4^+$. Green solid. |
| 116 | 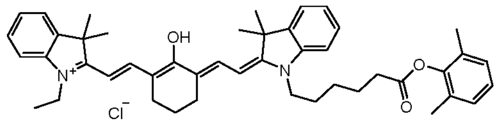 116 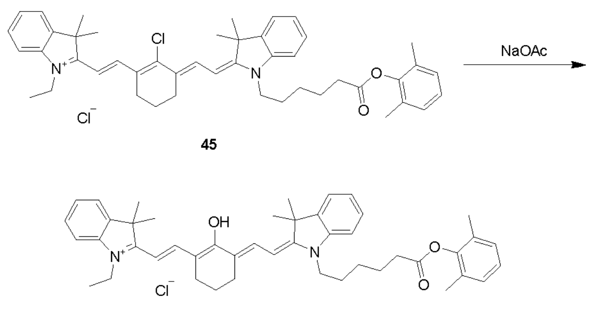 | LCMS: m/z = 683.3, $C_{46}H_{55}N_2O_3^+$. Black oil. |

Example 117: Preparation of 5-carboxy-2-((E)-2-((E)-2-chloro-3-(2-((E)-3,3-dimethyl-1-(2-(3-oxo-3-phenoxypropox y) ethyl)indolin-2-ylidene)ethenyl)cyclohex-1-en-1-yl)ethenyl)-1-ethyl-3,3-dimethyl-3H -indol-1-ium 2,2,2-trifluoroacetate (117)

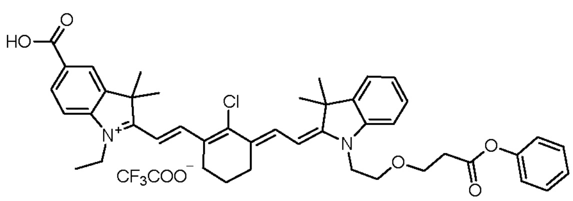

117

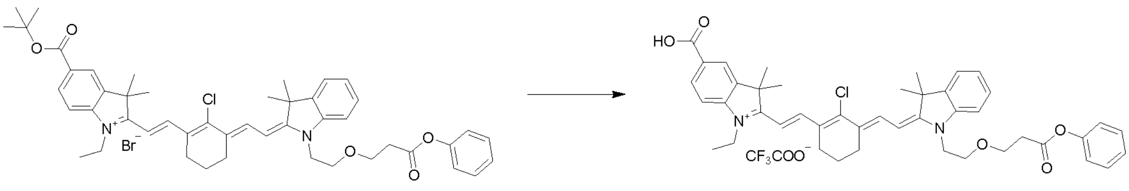

74 117

TFA (8.81 g, 77.3 mmol, 5.74 mL, 20.0 eq) was added to a solution of Compound 74 (3.00 g, 3.86 mmol, 1.00 eq) in DCM (10.0 mL), and stirred at 20°C for 4 hours. The resulting mixture was concentrated under reduced pressure, and the residues were purified by reversed-phase HPLC (Phenomenex Synergi Polar-RP 100mm×25 mm×4 μm;mobile phase: [water(TFA)-ACN]; B%: 25%-85%;9 min) to obtain Compound 117 as a blue solid after lyophilization, with a yield of 9.63%.

$^1$H NMR (400 MHz, MeOD) δ 8.50-8.65 (m, 1H), 8.16-8.27 (m, 1H), 8.03-8.07 (m, 2H), 7.62 (d, J = 7.2 Hz, 1H), 7.51-7.57 (m, 2H), 7.22-7.25 (m, 1H), 7.42-7.46 (m, 2H), 7.26-7.30 (m, 2H), 7.14-7.19 (m, 2H), 6.83-6.85 (m, 1H), 6.09 (br, 1H), 4.60 (s, 2H), 4.08 (s, 2H), 3.99 (t, J = 4.4 Hz, 2H), 3.79 (t, J = 5.6 Hz, 2H), 2.64-2.77 (m, 6H), 1.85-1.88 (m, 2H), 1.72 (d, J = 6.4 Hz, 12H), 1.36 (t, J = 7.2 Hz, 3H).

LCMS: m/z = 719.4, $C_{44}H_{48}ClN_2O_5$.

Example 118: Preparation of 5-carboxy-2-((E)-2-((E)-2-chloro-3-(2-((E)-3,3-dimethyl-1-(6-oxo-6-phenoxyhexyl)ind olin-2-ylidene)ethenyl)cyclohex-1-en-1-yl)ethenyl)-1-ethyl-3,3-dimethyl-3H-indol-1-iu m 2,2,2-trifluoroacetate (118)

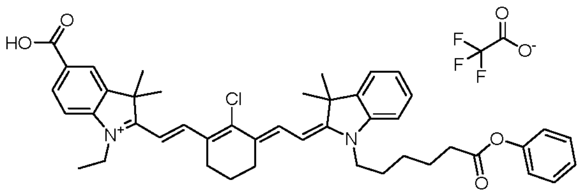

118

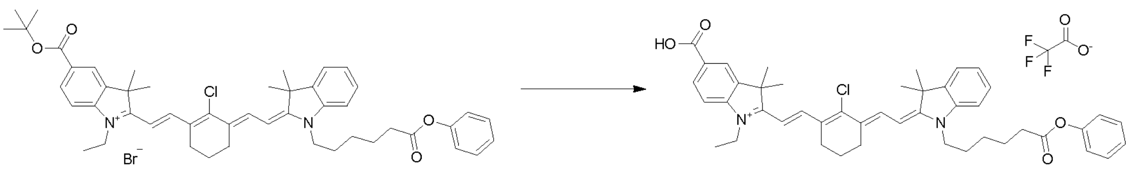

41 118

Compound 118 was prepared as a blue solid according to the preparation method similar to Example 117, except that Compound 41 was used instead of Compound 74.

$^1$H NMR (400 MHz, MeOD) δ 8.61 (d, *J* = 14.4 Hz, 1H), 8.28 (d, *J* = 13.6 Hz, 1H), 8.05-8.10 (m, 2H), 7.63-7.65 (m, 1H), 7.50-7.57 (m, 2H), 7.42-7.46 (m, 1H), 7.36 (t, *J* = 8.0 Hz, 2H), 7.20-7.24 (m, 2H), 6.99 (d, *J* = 7.6 Hz, 2H), 6.59 (d, *J* = 14.8 Hz, 1H), 6.14 (d, *J* = 13.6 Hz, 1H), 4.36-4.41 (m, 2H), 4.08-4.17 (m, 2H), 2.70-2.75 (m, 4H), 2.62 (t, *J* = 7.2 Hz, 2H), 1.90-2.00 (m, 4H), 1.80-1.87 (m, 2H), 1.75 (d, *J* = 13.2 Hz, 12H), 1.58-1.64 (m, 2H), 1.38 (t, *J* = 7.2 Hz, 3H).

LCMS: m/z = 717.4, $C_{45}H_{50}ClN_2O_4^+$.

Example 119: Preparation of 5-carboxy-2-((E)-2-((E)-3-(2-((E)-1-(5-carboxypentyl)-3,3-dimethylindolin-2-ylidene)et henyl)-2-chlorocyclohex-1-en-1-yl)ethenyl)-1,3,3-trimethyl-3H-indol-1-ium 2,2,2-trifluoroacetate (119)

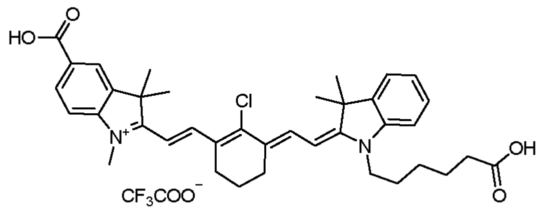

119

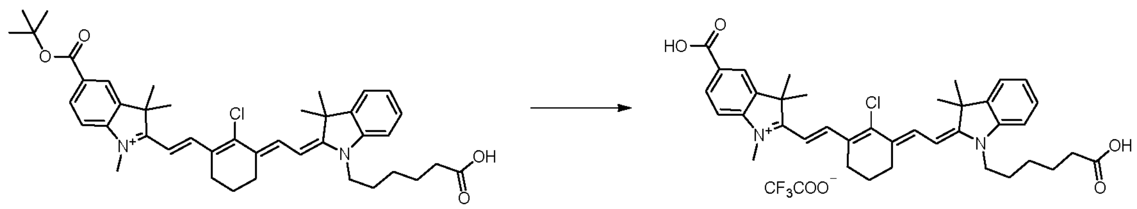

43 119

Compound 119 was prepared according to the preparation method similar to Example 117, except that Compound 43 was used instead of Compound 74.

$^1H$ NMR (400 MHz, MeOD) $\delta$ 8.63 (d, J = 14.8 Hz, 1H), 8.27 (d, J = 13.6 Hz, 1H), 8.03-8.08 (m, 2H), 7.64 (d, J = 7.6 Hz, 1H), 7.53-7.54 (m, 2H), 7.44-7.46 (m, 1H), 7.21 (d, J = 8.4 Hz, 1H), 6.59 (d, J = 14.8 Hz, 1H), 6.11 (d, J = 13.6 Hz, 1H), 4.36 (t, J = 7.2 Hz, 2H), 3.55 (s, 3H), 2.72-2.80 (m, 4H), 2.33 (t, J = 7.2 Hz, 2H), 1.91-1.99 (m, 4H), 1.78 (s, 6H), 1.74 (s, 6H), 1.67-1.71 (m, 2H), 1.50-1.54 (m, 2H).

LCMS: m/z = 627.3, $C_{38}H_{44}ClN_2O_4^+$.

Example 120: Preparation of 5-carboxy-2-((E)-2-((E)-2-chloro-3-(2-((E)-3,3-dimethyl-1-(6-oxo-6-phenoxyhexyl)indolin-2-ylidene)ethenyl)cyclohex-1-en-1-yl)ethenyl)-1,3,3-trimethyl-3H-indol-1-ium 2,2,2-trifluoroacetate (120)

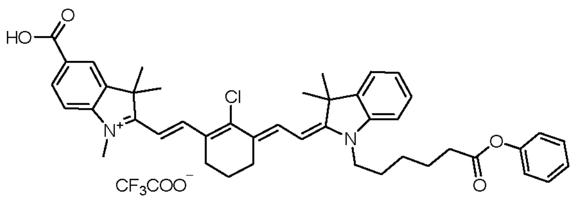

120

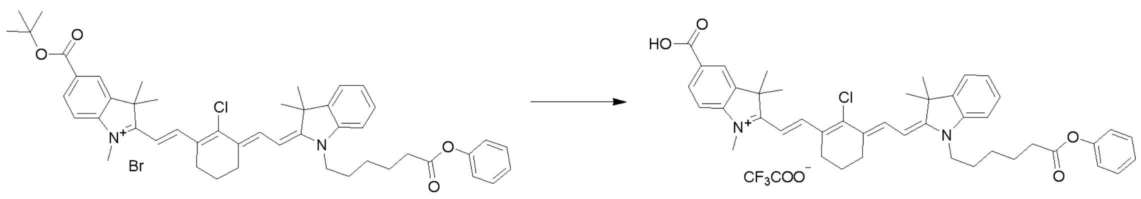

44 120

Compound 120 was prepared according to the preparation method similar to Example 117, except that Compound 44 was used instead of Compound 74.

$^1H$ NMR (400 MHz, MeOD) $\delta$ 8.61 (d, J = 14.8 Hz, 1H), 8.27 (d, J = 14.0 Hz, 1H), 8.03-8.08 (m, 2H), 7.64 (d, J = 7.2 Hz, 1H), 7.50-7.57 (m, 2H), 7.43-7.46 (m, 1H), 7.34-7.37 (m, 2H), 7.20-7.23 (m, 2H), 6.98-7.00 (m, 2H), 6.59 (d, J = 14.8 Hz, 1H), 6.10 (d, J = 13.6 Hz, 1H), 4.39 (t, J = 6.8 Hz, 2H), 3.55 (s, 3H), 2.67-2.76 (m, 4H), 2.62 (t, J = 7.2 Hz, 2H), 1.96-2.00 (m, 2H), 1.87-1.94 (m, 2H), 1.80-1.85 (m, 2H), 1.76 (s, 6H), 1.73 (s, 6H), 1.58-1.64 (m, 2H).

LCMS: m/z = 703.3, $C_{44}H_{48}ClN_2O_4^+$.

Example 121: Preparation of 2-((E)-2-((E)-2-(((E)-4-(dimethylamino)but-2-enoyl)oxy)-3-(2-((E)-1-(6-(2,6-dimethylp henoxy)-6-oxohexyl)-3,3-dimethylindolin-2-ylidene)ethenyl)cyclohex-1-en-1-yl)etheny l)-1-ethyl-3,3-dimethyl-3H-indol-1-ium 2,2,2-trifluoroacetate (121)

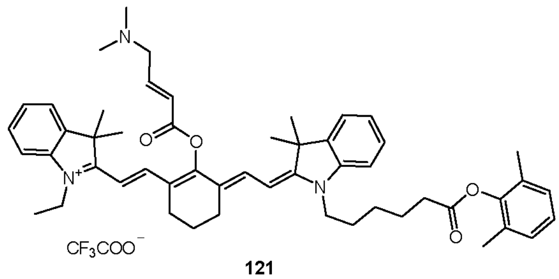

121

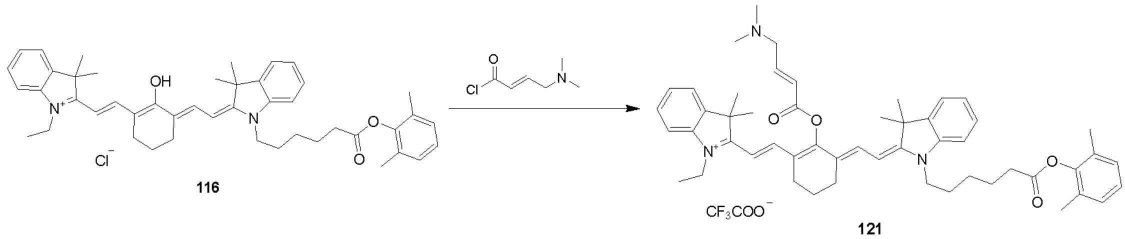

116

121

Pyridine (27.5 mg, 347 μmol, 28.1 μL, 0.500 eq) was added to a solution of Compound 116 (500 mg, 695 μmol, 1.00 eq), (E)-4-(dimethylamino)but-2-enyl hydrochloride (820 mg, 5.56 mmol, 8.00 eq) in DCM (10 mL). The reaction mixture was stirred at 45°C for 12 hours. The mixture was concentrated under reduced pressure, and the residues were purified by reversed-phase HPLC (column: Phenomenex luna C18 150mm×40mm×15μm; mobile phase: [water(HCl)-ACN]; gradient: 35%-65% B, 10 min) to obtain Compound 121 (40.0 mg, 30.6 μmol, 4.40% yield, 69.5% purity) as a green solid.

$^1$H NMR (400MHz, $CDCl_3$) δ 7.70 - 7.77 (m, 2H), 7.55 - 7.61 (m, 1H), 7.42 - 7.45 (m, 4H), 7.36 - 7.38 (m, 1H), 7.23 -7.25 (m, 1H), 7.03 - 7.07 (m, 5H), 6.83 - 6.87 (m, 1H), 5.94 - 6.00 (m, 2H), 4.33 (d, J = 3.6 Hz, 2H), 3.97 - 4.06 (m, 4H), 3.00 (s, 6H), 2.61 - 2.68 (m, 6H), 2.12 (s, 6H), 1.86 - 2.07 (m, 8H), 1.59 (s, 12H), 1.43 (t, J = 6.8 Hz, 3H).

LCMS: m/z = 794.5, $C_{52}H_{64}N_3O_4^+$.

Example 122: Preparation of 2-((E)-2-((E)-2-chloro-3-(2-((E)-3,3-dimethyl-1-(5-phosphonylpentyl)indolin-2-ylidene ) ethenyl)cyclohex-1-en-1-yl)ethenyl)-1,3,3-trimethyl-3H-indol-1-ium chloride (122)

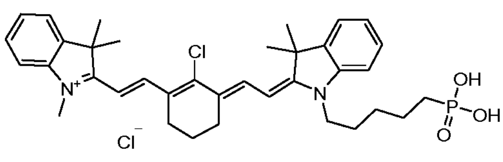

122

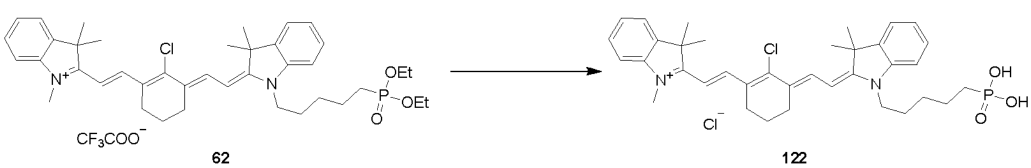

62

122

HCl/dioxane (6.00 M, 1.27 mL, 10.0 eq) was added to a solution of Compound 62 (0.600 g, 760 μmol, 1.00 eq) in MeCN (3.00 mL) at 0°C. The mixture was heated to 100°C and stirred for 24 hours. The resulting mixture was filtered, and the filtrate was concentrated. The residues were purified by reversed-phase HPLC (column: Phenomenex luna C18 150 mm×40mm×15μm; mobile phase: [water(HCl)-ACN]; gradient: 45%-75% B, 10 min) to obtain Compound 122 (27.0 mg,

39.5 μmol, 5.20% yield, 95.9% purity) as a green solid.

$^1$H NMR (400MHz, DMSO-d6)δ 8.24-8.28 (m, 2H), 7.63 (t, J = 7.6 Hz, 2H), 7.42-7.46 (m, 4H), 7.27-7.30 (m, 2H), 6.31 (t, J = 14.4 Hz, 2H), 4.17-4.20 (m, 2H), 3.70 (s, 3H), 2.70-2.72 (m, 4H), 1.86-1.87 (m, 2H), 1.71-1.73 (m, 2H), 1.67 (s, 12H), 1.47-1.55 (m, 8H).

LCMS: m/z = 619.3, $C_{36}H_{45}ClN_2O_3P^+$.

Example 123: Preparation of 5-carboxy-2-((E)-2-((E)-2-chloro-3-(2-((E)-3,3-dimethyl-1-(2-(3-oxo-3-phenoxypropox y) ethyl)indolin-2-ylidene)ethenyl)cyclohex-1-en-1-yl)ethenyl)-1-ethyl-3,3-dimethyl-3H -indol-1-ium 2,2,2-trifluoroacetate (123)

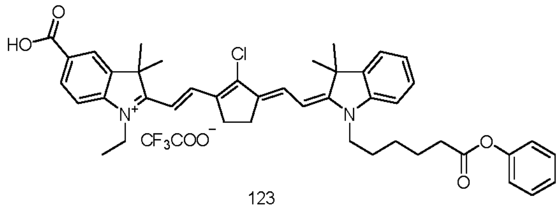

123

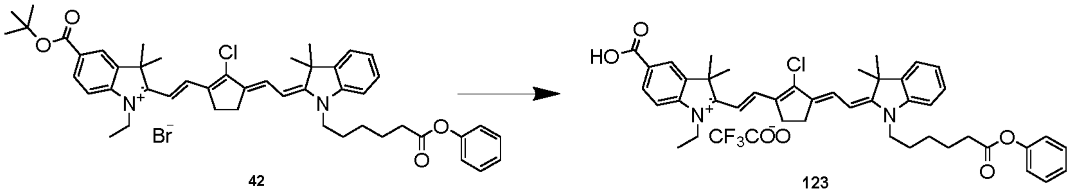

42 123

Compound 123 was prepared as a blue solid according to the preparation method similar to Example 118, except that Compound 42 was used instead of Compound 41.

$^1$H NMR (400 MHz, $CDCl_3$) δ 8.04 (d, J = 8.4 Hz, 1H), 7.98-8.01 (m, 2H), 7.62 (d, J = 13.2 Hz, 1H), 7.41-7.53 (m, 2H), 7.30-7.37 (m, 4H), 7.21 (t, J = 16.0 Hz, 1H), 7.01 (d, J = 7.6 Hz, 2H), 6.95 (d, J = 8.0 Hz, 1H), 6.71 (d, J = 14.0 Hz, 1H), 5.87 (d, J = 13.6 Hz, 1H), 4.66 (s, 2H), 4.07 (br, 2H), 3.18 (br, 2H), 2.96 (br, 2H), 2.60-2.67 (m, 2H), 1.98 (br, 2H), 1.76-1.87 (m, 4H), 1.71 (d, J = 19.2 Hz, 12H), 1.35-1.45 (m, 3H).

LCMS: m/z = 703.3, $C_{44}H_{48}ClN_2O_4^+$.

In the present invention, the following compounds are also synthesized:

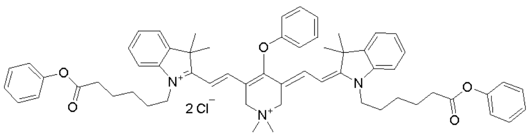
,
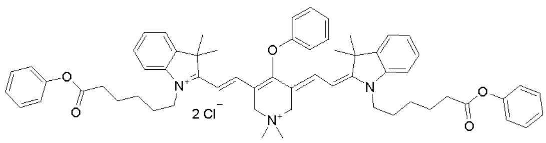
,

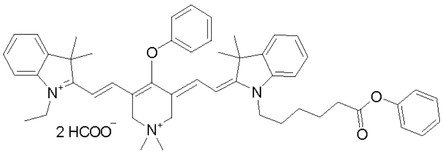
,
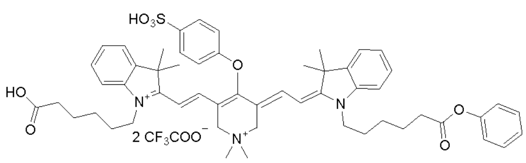
,

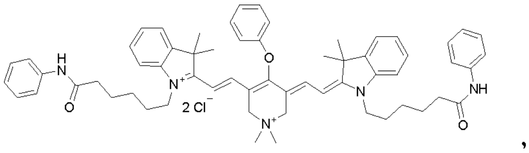
,
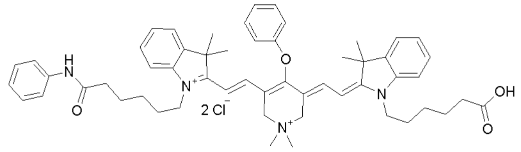
,

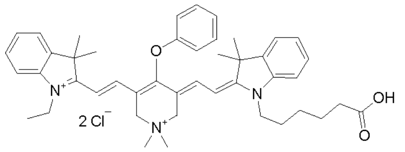 , 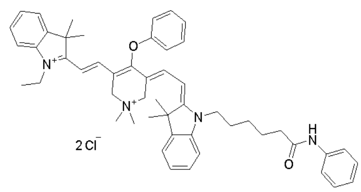 ,

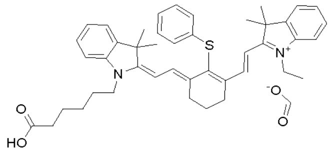 .

Comparative Example 1: Preparation of 2-((E)-2-((E)-2-chloro-3-(2-((E)-3,3-dimethyl-1-(6-oxo-6-phenoxyhexyl)indolin-2-ylide ne)ethenyl)cyclohex-1-en-1-yl)ethenyl)-1-ethyl-3,3-dimethyl-3H-indol-1-ium chloride (12a)

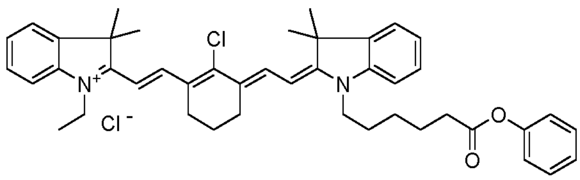

**12a**

Compound **12a** was prepared according to the same preparation method as Example 34, except that Compound C1 was used instead of Compound C10.

$^1$H NMR (400MHz, $CDCl_3$)δ 8.35 (t, *J* = 13.2 Hz, 2H), 7.43 - 7.35 (m, 6H), 7.25 - 7.13 (m, 5H), 7.03 (d, *J* = 8.4 Hz, 2H), 6.25 - 6.19 (m, 2H), 4.22 - 4.16 (m, 4H), 2.71 - 2.66 (m, 4H), 2.62 (t, *J* = 6.8 Hz, 2H), 2.01 - 1.88 (m, 4H), 1.87 - 1.83 (m, 2H), 1.73 - 1.72 (m, 12H), 1.67 - 1.62 (m, 2H), 1.46 (t, *J* = 6.8 Hz, 3H).

LCMS: m/z = 673.4 , $C_{44}H_{50}N_2O_2Cl_2$.

Test Examples

Test Example 1. Solubility, hERG, optical property, and MRC5 tests

(1) Method for solubility test

The compounds were weighed in an appropriate amount and dissolved in DMSO to prepare a 10 mM stock solution. 30 uL of the stock solution was taken and 970 μL of phosphate buffered saline solution (PBS) was added thereto. The solution was shaken at 25°C on a thermostatic mixer for 2 hours, and centrifuged at 12000 rpm for 10 minutes. The supernatant was taken, and the kinetic solubility of each compound in phosphate buffered saline solution (PBS) was determined by Waters Acquity Arc HPLC. The test results are shown in Table 1 below.

(2) Method for HERG test

1) Materials and instruments

| Material | Manufacturer (batch number) |
|---|---|
| 6 cm cell-culture dish | Nunc (150288) |
| 3.5 cm cell-culture dish | Nunc (153066) |
| Dialyzed fetal bovine serum | BIOSUN (BS-0005-500) |

(continued)

| Material | Manufacturer (batch number) |
|---|---|
| DMSO | Merk (102952) |
| DMEM medium | Gibco (10569) |
| HEPES | Gibco (15630080) |
| TrypLE™ trypsin | Gibco (12604) |
| 1×PBS, free of $Ca^{2+}$/$Mg^{2+}$ | Gibco (14190) |
| Penicillin - streptomycin | Gibco (15140-122) |
| MEM+NEAA | Gibco (11140) |
| Geneticin, G418 | Gibco (11811031) |
| Blasticidin | Gibco (R21001) |
| Polylysine | Sigma (P4832) |
| Dofetilide | TRC (D525700) |
| Doxycycline | Sigma (D9891) |
| **Instrument** | **Manufacturer** |
| Steri-Cycle $CO_2$ incubator | Thermo(371) |
| Micropipette puller | Sutter (P-97 Model) |
| Micromanipulator | Siskiyou Controller (MC1000e) |
| | Sutter (ROE-200; MP285) |
| Multilamp700B amplifier | HEKA (EPC 10) |
| EPC10 amplifier | AXON (Multiclamp 700B) |
| Microscope | Olympus (IX51/71/73) |

2) Cell line and cell culture

The HEK 293 cell line (K1236) stably expressing hERG was purchased from Invitrogen. Cells were cultured in 85% DMEM, 10% dialyzed FBS, 0.1 mM NEAA, 25 mM HEPES, 100 U/mL penicillin-streptomycin, 5 μg/mL blasticidin and 400 μg/mL geneticin. Cells were split using TrypLE™ about three times a week, and maintained at a confluence of about 40% to about 80%. Before measurement, cells were cultured at a density of $5 \times 10^5$ in a 6 cm culture dish and induced with 1 μg/mL doxycycline for 48 hours.

3) Solution preparation

Extracellular solution (mM): 132 NaCl, 4 KCl, 13 $CaCl_2$, 0.5 $MgCl_2$, 11.1 glucose, 10 HEPES (pH 7.35 adjusted with NaOH);
Intercellular solution (mM): 140 KCl, 2 $MgCl_2$, 10 EGTA, 10 HEPES, 5 MgATP (pH 7.35 adjusted with KOH).

4) Preparation of working solution of test compounds

The test compounds were formulated in DMSO to prepare a stock solution with a final concentration of 10 mM.
Then, the stock solution of each compound was diluted with DMSO in a 1:3 serial dilution to prepare five intermediate solutions with concentrations of 3, 1, 0.33, 0.12, 0.04 mM or 3.33, 1.11, 0.37, 0.12 mM, respectively.
Before testing, the final working solutions were prepared by diluting the intermediate solutions 1000 times with the extracellular solution. The concentration of DMSO in the working solutions was within a range around 0.1%.
The working solutions at five different concentrations of 3, 1, 0.33, 0.12 and 0.04 μM were used to determine the potential inhibition effect of the compounds on hERG channels and to fit concentration-response curves and calculate $IC_{50}$.

5) Experimental procedures

The coverslip was removed from the cell culture dish and placed on a microscope stage.

A 10x objective was used to locate cells to be observed. An electrode tip was located under the microscope using the 10x objective by focusing above the plane of the cells. Once the tip was in focus, the electrode was advanced downward towards the cells under rough control of the manipulator, while the objective was moved so as to keep the tip in focus.

When the electrode was directly over a cell, the objective was switched to a 40x objective and fine control of the manipulator was used to enable to gradually approach the cell surface.

With gentle suction through a side port of an electrode holder, a giga-ohm seal was formed.

$C_{fast}$ was used to eliminate the capacitive current consistent with the voltage step. Brief and strong suction was applied repeatedly until the membrane patch ruptured and the whole-cell configuration was obtained.

At this point, membrane potential was set to -60 mV in order to ensure that hERG channels were not open. Then, $C_{slow}$ on the amplifier was used to remove the spikes of capacitive current.

Holding potential was set to -90 mV for 500 ms. Current was recorded at 20 kHz and filtering was carried out at 10 kHz. Leak current was tested at -80 mV for 500 ms.

The hERG current was elicited by depolarizing at +30 mV for 4.8 seconds, and then the voltage was restored to -50 mV for 5.2 seconds to remove the inactivation and observe the deactivating tail current. The maximum of tail current size was used to determine hERG current amplitude.

Current was recorded for 120 seconds to evaluate the current stability. Only stable cells with recording parameters above thresholds were used for drug administrations.

Firstly, vehicle control was applied to the cells to establish the baseline. Once the hERC current was found to be stabilized for 5 minutes, the compound was applied. The hERG current in the presence of the test compound was recorded for about 5 minutes to reach a steady state, and then 5 sweeps were captured. For dose-response testing, multiple compounds were applied to the cells cumulatively from low to high concentrations (multiple compounds were detected simultaneously). The positive control, Dofetilide at a concentration of 150 nM, was also applied to each cell post hERG current measurement at the highest concentration of the test compound, which was used as an internal low control for normalization of percentage inhibition. In order to ensure the good performance of cultured cells and operations, 5 doses of Dofetilide as a positive control was also used to test the same batch of cells for the compound.

6) Data analysis

Data acceptance criteria:

The following criteria were used to determine data acceptability:

Initial seal resistance >1 GΩ;
Leak current < 50% of the control peak tail current at any time;
Peak tail amplitude >250 pA;
Membrane resistance Rm >200 MΩ;
Access resistance Ra >15 MΩ;
Apparent drop of peak current < 2.5% per minute.

After perfusing the blank solvent or compound gradient solution, the average of 5 consecutive current values obtained stably was taken and used as "Tail current size $_{blank}$" or "Tail current size $_{compound}$" respectively.

The data that met the above criteria for hERG current quality were further analyzed according to the following procedure:

The percentage of current inhibition was calculated using the following formula (wherein PatchMaster or Clampfit software was used to extract the peak currents from raw data), where the blank control was 0.1% DMSO as the blank solvent perfused;

$$\text{Tail current inhibition} = \left(1 - \frac{\text{Tail current size}_{compound} - \text{Tail current size}_{positive\ drug}}{\text{Tail current size}_{compound} - \text{Tail current size}_{positive\ drug}}\right) \times 100$$

Dose-effect curves of the test compounds were obtained by fitting using Graphpad Prism 8.0, and the concentrations corresponding to 50% inhibition, i.e., the concentrations in Table 1 were derived from the curves.
The hERG test results are shown in Table 1.

7) References

[1] 1: Roche et al. A Virtual Screening Method for Prediction of the hERG Potassium Channel Liability of Compound Libraries. (2002) ChemBioChem. 3, 455-459.
[2] Glenn E. Kirsch et al. Variability in the measurement of hERG potassium channel inhibition: effects of temperature and stimulus patter. (2004) Journal of Pharmacological and Toxicological Methods 50, 93-101.
[3] Roger Marrannes et al. Computer programs to facilitate the estimation of time-dependent drug effects on ion channels. (2004) Computer Methods and Programs in Biomedicine 74, 167-181.

Table 1. Solubility and hERG values of the example compounds of the present invention and the compound of comparative example

| Example number | Kinetic solubility (mg/mL) | hERG $IC_{50}$ (μM) |
|---|---|---|
| Comparative Example 1 | 0.005 | <0.12 |
| 10 | 0.026 | 1.33 |
| 87 | 0.038 | 1.417 |
| 95 | 0.099 | 0.98 |
| 68 | >0.25 | 0.89 |
| 70 | >0.25 | 0.568 |
| 73 | >0.25 | 1.662 |

(3) Method for optical property test

The UV-VIS absorption spectra of the compounds (10 μM, DMSO solution) were detected using Shimadzu UV2600 spectrophotometer, and the maximum absorption peak and absorbance of each compound were obtained. The results are shown in Table 2.

Table 2

| Example number | Absorption peak nm | Absorbance $\times 10^4$ |
|---|---|---|
| Comparative Example 1 | 794.5 | 2.16 |
| 1A | 860 | 2.82 |
| 1B | 859.5 | 2.03 |
| 2 | 833.5 | 2.09 |
| 3 | 835 | 1.88 |
| 4 | 821 | 2.36 |
| 5 | 796.5 | 1.17 |
| 6 | 795 | 1.8 |
| 7 | 794.5 | 1.92 |
| 8 | 794.5 | 1.96 |
| 10 | 821 | 2.08 |
| 11 | 796.5 | 2.27 |
| 12 | 781.5 | 2.07 |
| 13 | 632 | 0.68 |

(continued)

| Example number | Absorption peak nm | Absorbance $\times 10^4$ |
|---|---|---|
| 14 | 767.5 | 1.71 |
| 16 | 766 | 1.71 |
| 17 | 766 | 0.88 |
| 18 | 764.5 | 2.12 |
| 20 | 782 | 2.60 |
| 21 | 821 | 2.466 |
| 22 | 827 | 1.97 |
| 25 | 767.5 | 2.265 |
| 26 | 797.5 | 2.44 |
| 27A | 796.5 | 2.29 |
| 27B | 797 | 2.117 |
| 28 | 766 | 1.856 |
| 35 | 794.5 | 2.21 |
| 36 | 795.5 | 3.368 |
| 37A | 794.5 | 2.118 |
| 37B | 801 | 1.563 |
| 38 | 584 | 0.364 |
| 40 | | 0.93 |
| 47 | | 2 |
| 48 | | 2.02 |
| 49 | | 2.3 |
| 50 | | 1.97 |
| 51 | | 1.9 |
| 52 | | 2.14 |
| 53 | | 0.6 |
| 54 | | 2.57 |
| 55 | | 3.48 |
| 56 | | 1.85 |
| 58 | | 2.21 |
| 63 | | 0.31 |
| 64 | | 0.5 |
| 68 | 796 | 2.74 |
| 70 | 796.5 | 2.09 |
| 73 | 796.5 | 2.15 |
| 77 | 794.5 | 4.344 |
| 78 | 794.5 | 4.082 |
| 79 | 787.5 | 5.223 |
| 80 | 789 | 4.457 |
| 81 | 794 | 3.867 |

(continued)

| Example number | Absorption peak nm | Absorbance $\times 10^4$ |
|---|---|---|
| 82 | 793.5 | 3.686 |
| 83 | 820 | 3.079 |
| 84 | | 2.41 |
| 85A | 796.5 | 2.239 |
| 85B | 796.5 | 1.397 |
| 87 | 785 | 1.91 |
| 88 | | 1.75 |
| 89 | | 1.86 |
| 91 | | 0.46 |
| 92 | | 1.49 |
| 93 | | 1.3 |
| 94 | | 1.86 |
| 95 | 793.5 | 2.41 |
| 96 | | 2.45 |
| 101 | 836.5 | 1.971 |
| 103 | 757.5 | 2.483 |
| 104 | 781.5 | 2.054 |
| 106 | 807.5 | 1.997 |
| 107 | 778.5 | 2.028 |
| 108 | 805.5 | 1.752 |
| 112 | 756 | 0.911 |
| 114 | | 2.46 |
| 115 | | 2.24 |
| 117 | | 1.36 |
| 118 | | 1.73 |
| 119 | | 1.52 |
| 120 | | 1.73 |
| 123 | | 1.3 |

(4) MRC5 test

1. Cell line

MRC5 (ATCC; Cat. No.: CCL-171)

2. Reagents

Culture medium EMEM (ATCC, 30-2003)
Fetal bovine serum (Gibco, 10091148)
Penicillin-streptomycin double antibody (Gibco, S110JV)
DPBS (Gibco, 14190-144)
CellTiter-Glo® assay kit (Promega, G7573)

Trypsin (0.25%) (Gibco, 25200-056)
DMSO (Sigma, D2650)

3. Instruments

Biosafety cabinet (AIRTECH, BSC-1300A II)
Automated cell counter (Life Technologies, countess II)
Multifunctional microplate reader (Biotek, H1FM)
Shaker (Hangzhou Allsheng Instruments Co., Ltd., OS-100)
XLFIT 5.3 (Shanghai Inforstack Co., Ltd.)

4. Test method

For this test method, the following experiment was performed in a biosafety cabinet with the specific steps as follows.

(1) Cells were seeded in a 96-well plate (in which the culture medium was EMEM). One day later, the previous culture medium EMEM was poured off, and 5 mL of DPBS was added to wash the cells. Then DPBS was pipetted and discarded. An appropriate amount of trypsin was then added. The plate was placed in a 5% $CO_2$ cell incubator at 37° C to digestion for about 2 to 5 minutes, and then taken out. New culture medium EMEM was added. The cells were pipetted up and down to resuspend evenly. The cells were then counted by an automated cell counter.
(2) Cells were seeded in columns 1 to 11 of a 96-well plate (Corning, Cat. No. 3610) at a density of 3000 cells per well in 100 μL of culture medium EMEM, and 100 μL of culture medium EMEM free of cells was added into column 12 of the wells. Then, the plate was placed in the 5% $CO_2$ incubator at 37° C for 24 hours.
(3) Next, the compound of the present invention was 2-fold serially diluted into 8 dose points starting at 10 μM. 10 μL of the serially diluted compound was pipetted and added to 100 μL of cells in columns 1 to 10, and 10 μL of the culture medium EMEM containing 2% DMSO was added to both columns 11 and 12. The plate was placed in the 5% $CO_2$ incubator at 37° C and incubated for 3 days (Note: column 11 was used as a MAX well with cells but without the compound; column 12 was used as a MIN well without both cell and compound).
(4) The cell viability detection reagent in the above CellTiter-Glo® assay kit was then added to the 96-well plate at 50 μL per well according to the instruction, and shaken on a shaker in dark for 5 to 10 minutes. The cell viability was then measured using a multifunctional microplate reader. Finally, the inhibition of the compound of the present invention on cell proliferation was plotted by XLFIT software, and the half inhibitory concentration, $IC_{50}$ value of the compound of the present invention was calculated therefrom.

The test results of the inhibition of the compounds of the present invention on MRC5 are shown in Table 3 below.

Table 3. Inhibitory effect of the compounds of the present invention on proliferation of normal cells ($IC_{50}$, nM)

| Example number | MRC5 ($IC_{50}$, nM) |
|---|---|
| Comparative Example 1 | 2698.2 |
| 87 | > 10000 |
| 95 | > 10000 |
| 68 | > 10000 |
| 70 | > 10000 |
| 73 | > 10000 |

The results indicated that the compounds of the present invention significantly improved solubility compared to the compound of Comparative Example 1. This offers great convenience for application of drug injection. In addition, the compounds of the present invention significantly reduced hERG values, i.e. cardiac toxicity, making them safer for blood medication or other systemic medications. Furthermore, the present inventors evaluated the killing effect of some compounds on normal cells and found that they were all greater than 10 μM, indicating that they would not have adverse effects on normal tissues or cells.

Test Example 2. *In vitro* cellular uptake test of contrast agent molecules

2.1. Fluorescence localization imaging of Comparative Example 1 and Examples 2, 6, 4, 7, 8 and 10 in human bladder cancer cell line RT112 (Nanjing Cobioer; Cat. No.: CBP60316).

RT112 cells ($1.5 \times 10^4$/well) were cultured in DMEM medium (containing 10% fetal bovine serum (FBS), 1% antibiotics (100 U/mL penicillin and 100 U/mL streptomycin)) and seeded in a 96-well plate in advance. Then, the plate was cultured in a 5% $CO_2$ cell culture incubator at 37°C overnight. The following day, the culture medium was sucked away, and the culture medium containing an appropriate concentration (1-20 μM) of the compound and surfactin (the molar ratio of the test compound to the surfactin (OKA: Cat. No.: A55976-1g) being 1:5) was added to the test wells, and incubated for 30 minutes. The cells were rinsed three times with PBS solution, and the near-infrared fluorescence intensity in the bladder cancer cells was observed using an integrated fluorescence microscopic imaging system from Keyence. The conditions for near-infrared photography were Ex = 775/50 nm and Em = 845/55 nm. The results are shown in Figure 1.

2.2. Fluorescence localization imaging of Compounds 12a, 2, 6, 4, 7, 8 and 10 in human bladder cancer cell line UM-UC-3 (ATCC; Cat. No.: CRL-1749).

UM-UC -3 cells ($1.5 \times 10^4$/well) were cultured in EMEM medium (containing 10% fetal bovine serum (FBS) and 1% antibiotics (100 U/mL penicillin and 100 U/mL streptomycin)) and seeded in a 96-well plate in advance. The plate was cultured in a 5% $CO_2$ cell culture incubator at 37°C overnight. The following day, the culture medium was sucked away, and the culture medium containing an appropriate concentration (1-20 μM) of the compound as well as surfactin (the molar ratio of the test compound to the surfactin being 1:5) was added to the test wells, and incubated for 30 minutes. The cells were rinsed three times with PBS solution, and the near-infrared fluorescence intensity in the bladder cancer cells was observed using an integrated fluorescence microscopic imaging system from Keyence. The conditions for near-infrared photography were Ex = 775/50 nm and Em = 845/55 nm. The results are shown in Figure 2.

Test Example 3. *In vitro* imaging effect test with addition of BSP

RT112 cells ($1.5 \times 10^4$/well) were cultured in DMEM medium (containing 10% fetal bovine serum (FBS), 1% antibiotics (100 U/mL penicillin and 100 U/mL streptomycin)) and seeded in a 96-well plate in advance. The plate was cultured in a 5% $CO_2$ cell culture incubator at 37°C overnight. The following day, the culture medium was sucked away, and 250 μM of BSP (bromosulfophthalein, purchased from MACKLIN; Cat. No.: S914901) was added to the test wells and preconditioned for 10 minutes, followed by addition of the culture medium containing an appropriate concentration (1-20 μM) of the compound and surfactin (the molar ratio of the test compound to the surfactin being 1:5) with or without 250 μM of BSP, and then incubated for 30 minutes. The cells were rinsed three times with PBS solution, and the near-infrared fluorescence intensity in the bladder cancer cells was observed using an integrated fluorescence microscopic imaging system from Keyence. The conditions for near-infrared photography were Ex = 775/50 nm and Em = 845/55 nm. The results are shown in Figure 3.

It can be seen from Figure 3 that the uptake of Compounds 12a, 2, 6, 4, 7, 8 and 10 by human bladder cancer cell line RT112 may be weakened or enhanced by BSP.

UM-UC -3 ($1.5 \times 10^4$/well) were cultured in EMEM medium (containing 10% fetal bovine serum (FBS) and 1% antibiotics (100 U/mL penicillin and 100 U/mL streptomycin)) and seeded in a 96-well plate in advance. The plate was cultured in a 5% $CO_2$ cell culture incubator at 37°C overnight. The following day, the culture medium was sucked away, and 250 μM of BSP was added to the test wells and preconditioned for 10 minutes, followed by addition of the culture medium containing an appropriate concentration (1-20 μM) of the compound and surfactin (the molar ratio of the test compound to the surfactin being 1:5) with or without 250 μM of BSP, and then incubated for 30 minutes. The cells were rinsed three times with PBS solution, and the near-infrared fluorescence intensity in the bladder cancer cells was observed using an integrated fluorescence microscopic imaging system from Keyence. The conditions for near-infrared photography were Ex = 775/50 nm and Em = 845/55 nm. The results are shown in Figure 4.

It can be seen from Figure 4 that the uptake of Compounds 12a, 2, 6, 4, 7, 8 and 10 by human bladder cancer cell line UM-UC-3 may be weakened or enhanced by BSP.

Test Example 4. Imaging of the compounds of the invention in a bladder cancer tumor model

The imaging of Compounds 7, 8 and 10 in a bladder cancer tumor model was detected.

All animal experiments were conducted exactly in accordance with the guidelines for the care and use of animals issued by the regional animal committee. A mouse model of xenograft RT112 tumor was established by subcutaneous injection of RT112 cell suspension ($2 \times 10^6$ cells/mouse, 0.1 ml PBS) into the upper right limb of 8- to 10-week-old BALB/C female nude mice (20 to 25 g). BABL/c nude mice (15 to 20 g) were purchased from Beijing Vital River Laboratory Animal Technology Co., Ltd. For *in vivo* near-infrared fluorescence (NIRF) imaging, the RT112 tumor-bearing mice were intraperitoneally injected with different doses of free compounds (2 mg/kg or 0.4 mg/kg), respectively. At 48 hours after

injection, near-infrared imaging was performed using an IVIS Lumina spectroscopic imaging system (excitation/emission, 780/845 nm). The results are shown in Figure 5.

As shown in Figure 5, Compounds 7, 8 and 10 were all specifically enriched at the tumor sites under the administration dose condition of 2 mg/kg or 0.4 mg/kg (NIR standing for near-infrared fluorescence signal). The ratio of fluorescence intensity at the tumor site to background fluorescence intensity was greater than 1.5 times.

Test Example 5. Irradiance of the compounds of the invention

The compounds of the present invention, such as Compound 10, were superior to ICG (a dye used for angiography).

Test Example 6. Tumor/normal cell signal ratio of the compounds of the invention

The compounds of the present invention exhibited good contrast at different concentrations (1-20 μM), i.e., the fluorescence intensity of tumor cells was significantly higher than that of normal cells.

Test Example 7. Tumor/background ratio of the compounds of the invention

The subcutaneous tumor model mice inoculated with RT112 tumor were selected. Each compound was administrated at a dose of 2 mpk, and intraperitoneally injected. At 48 hours after injection, the ratio of fluorescence intensity at the tumor site to background fluorescence intensity was observed using a small animal imaging device. The results are shown in Table 4.

Table 4

| Example number | Tumor/background ratio |
|---|---|
| Comparative Example 1 | 2.4 |
| 10 | 3.3 |
| 87 | 3.3 |
| 95 | 2.8 |
| 68 | 2.5 |
| 70 | 2.7 |
| 73 | 2.7 |

In addition, the experimental results in an in situ tumor model showed that the compounds of the present invention exhibited a good tumor/background ratio, and the fluorescence intensity in the bladder with tumor was significantly higher than that in the normal bladder.

**Claims**

1. A compound with a structure of general formula (I) or a pharmaceutically acceptable salt thereof,

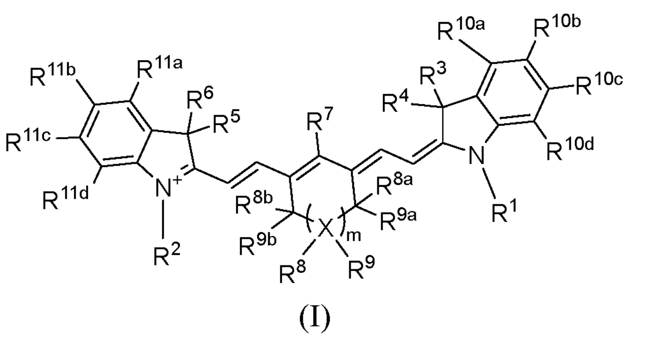

(I)

wherein:

X is C, O, S or $N^+$;
$R^1$ and $R^2$ are each independently selected from the group consisting of $L_1$-$L_2$-$L_3$-Q;
$L_1$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
$L_2$ is selected from the group consisting of bond, aryl, heteroaryl, cycloalkyl and heterocyclyl;
$L_3$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
Q is selected from the group consisting of $R^{12}$, $-OR^{13}$, $-C(O)R^{12}$, $-C(O)OR^{13}$, $-C(O)NHR^{13}$, $-SO_3H$ and

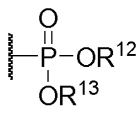 ;

$R^{12}$ is selected from the group consisting of hydrogen, halogen, amino, hydroxy, thiol, alkyl, alkylamino, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{13}$ is selected from the group consisting of hydrogen, alkyl, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^3$ and $R^4$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^5$ and $R^6$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^7$ is selected from the group consisting of hydrogen, halogen, nitro, cyano, hydroxy, thiol, oxo, alkyl, $OR^a$, $SR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^8$ and $R^9$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
$R^{8a}$ and $R^{9a}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{8b}$ and $R^{9b}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{10a}$, $R^{10b}$, $R^{10c}$ and $R^{10d}$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl,

wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{10a}$, $R^{10b}$, $R^{10c}$ and $R^{10d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^{11a}$, $R^{11b}$, $R^{11c}$ and $R^{11d}$ are each independently selected from the group consisting of hydrogen, halogen, nitro, cyano, hydroxy, thiol, oxo, alkyl, $OR^a$, $NR^bR^c$, $-SO_3H$, $-S(O)_2R^a$, $-C(O)OR^a$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

or any two adjacent ones of $R^{11a}$, $R^{11b}$, $R^{11c}$ and $R^{11d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

$R^a$ is selected from the group consisting of hydrogen, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;

$R^b$ and $R^c$ are each independently selected from the group consisting of hydrogen, alkyl, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;

m is 0 or 1;

p is an integer from 1 to 10;

q is an integer from 1 to 10;

v is an integer from 1 to 10.

**2.** A compound represented by general formula (IA) or a pharmaceutically acceptable salt thereof,

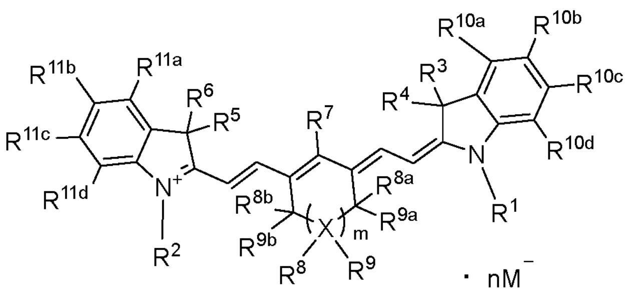

(IA)

wherein:

X is C, O, S or $N^+$;

$M^-$ is an acid ion, and preferably chloride ion, bromide ion, iodide ion, formate ion, acetate ion or trifluoroacetate ion;

N is 1 or 2, when X is one $N^+$, n is 2, and when X is C, O or S, n is 1;

$R^1$ and $R^2$ are each independently selected from the group consisting of $L_1-L_2-L_3-Q$;

$L_1$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;

$L_2$ is selected from the group consisting of bond, aryl, heteroaryl, cycloalkyl and heterocyclyl;

$L_3$ is selected from the group consisting of bond, $-(CH_2)_p-$, $-(CH_2O)_q-$, $-(CH_2CH_2O)_v-$, $-(CH_2O)_q-(CH_2)_p-$, $-(CH_2)_p-(CH_2O)_q-$, $-(CH_2)_p-(CH_2O)_q-(CH_2)_p-$, $-(CH_2CH_2O)_v-(CH_2)_p-$ and $-(CH_2CH_2O)_v-(CH_2O)_q-$;
Q is selected from the group consisting of $R^{12}$, $-OR^{13}$, $-C(O)R^{12}$, $-C(O)OR^{13}$, $-C(O)NHR^{13}$, $-SO_3H$ and

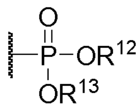
;

$R^{12}$ is selected from the group consisting of hydrogen, halogen, amino, hydroxy, thiol, alkyl, alkylamino, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{13}$ is selected from the group consisting of hydrogen, alkyl, alkenyl, alkynyl, cycloalkyl, heterocyclyl, aryl and heteroaryl; the alkyl, alkenyl, alkynyl, cycloalkyl, heterocyclyl, aryl or heteroaryl is optionally further substituted by one or more groups selected from the group consisting of halogen, amino, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^3$ and $R^4$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^5$ and $R^6$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^7$ is selected from the group consisting of hydrogen, halogen, nitro, cyano, hydroxy, thiol, oxo, alkyl, $OR^a$, $SR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^8$ and $R^9$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
$R^{8a}$ and $R^{9a}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{8b}$ and $R^{9b}$ are each independently selected from the group consisting of hydrogen, halogen, amino, nitro, cyano, hydroxy, thiol, oxo, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, alkyl, haloalkyl, alkoxy, haloalkoxy, hydroxyalkyl, alkenyl, alkynyl, cycloalkyl, heterocyclyl, aryl and heteroaryl;
$R^{10a}$, $R^{10b}$, $R^{10c}$ and $R^{10d}$ are each independently selected from the group consisting of hydrogen, halogen, nitro, cyano, hydroxy, thiol, oxo, alkyl, $OR^a$, $NR^bR^c$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
or any two adjacent ones of $R^{10a}$, $R^{10b}$, $R^{10c}$ and $R^{10d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl,

haloalkyl, alkoxy, haloalkoxy, hydroxyalkyl, alkenyl, alkynyl, cycloalkyl, heterocyclyl, aryl and heteroaryl;
$R^{11a}$, $R^{11b}$, $R^{11c}$ and $R^{11d}$ are each independently selected from the group consisting of hydrogen, halogen, nitro, cyano, hydroxy, thiol, oxo, alkyl, $OR^a$, $NR^bR^c$, $-SO_3H$, $-S(O)_2R^a$, $-C(O)OR^a$, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
or any two adjacent ones of $R^{11a}$, $R^{11b}$, $R^{11c}$ and $R^{11d}$, together with the atoms to which they are attached, form an aryl or heteroaryl, and the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
$R^a$ is selected from the group consisting of hydrogen, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;
$R^b$ and $R^c$ are each independently selected from the group consisting of hydrogen, alkyl, alkenyl, alkynyl, cycloalkyl, heterocyclyl, aryl and heteroaryl, wherein the alkyl, alkoxy, alkenyl, alkynyl, cycloalkyl, heterocyclyl, aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl;
m is 0 or 1;
p is an integer from 1 to 10;
q is an integer from 1 to 10;
v is an integer from 1 to 10.

**3.** The compound or a pharmaceutically acceptable salt thereof according to claim 1, being a compound with a structure of general formula (II) or a pharmaceutically acceptable salt thereof,

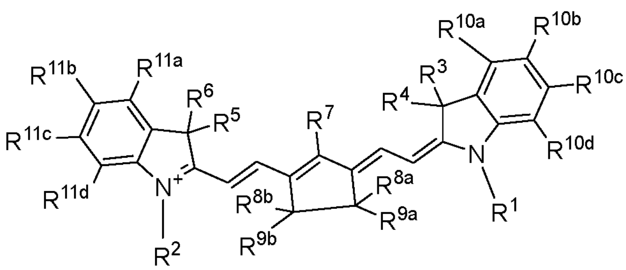

(II)

wherein , $R^1$ to $R^7$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 1.

**4.** The compound or a pharmaceutically acceptable salt thereof according to claim 1, being a compound with a structure of general formula (III) or a pharmaceutically acceptable salt thereof,

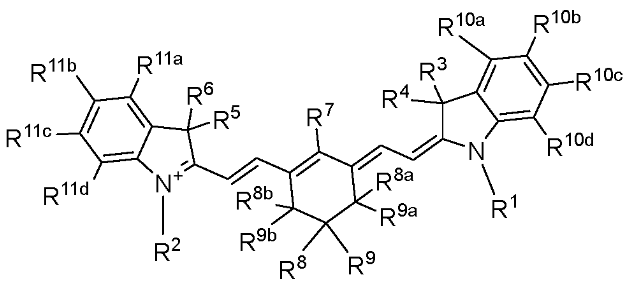

(III)

wherein , $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 1.

**5.** The compound represented by general formula (IA) or a pharmaceutically acceptable salt thereof according to claim 2, being a compound represented by general formula (IIA) or (IIIA) or a pharmaceutically acceptable salt thereof,

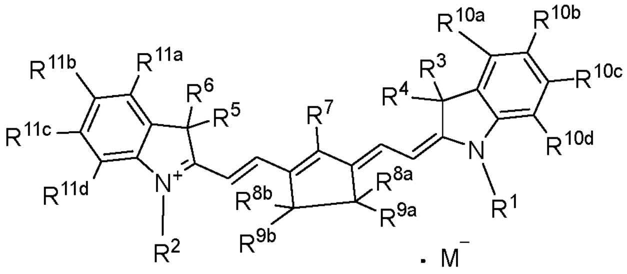

(IIA)

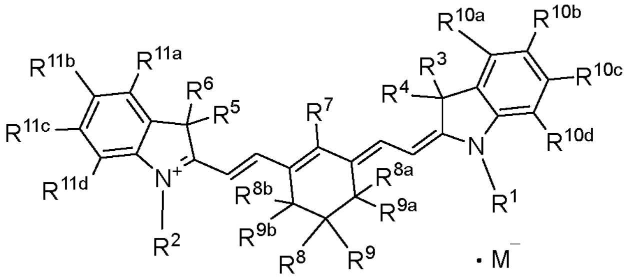

(IIIA)

wherein, $M^-$, $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 2.

**6.** The compound or a pharmaceutically acceptable salt thereof according to claim 1, being a compound with a structure of general formula (IV) or a pharmaceutically acceptable salt thereof,

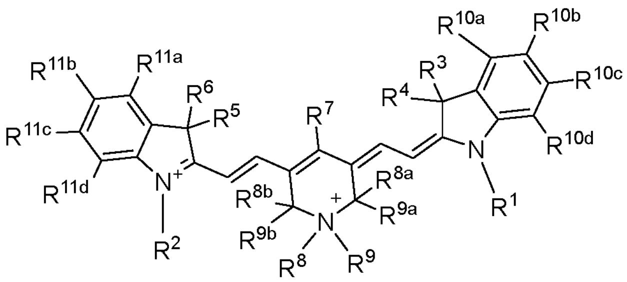

(IV)

wherein , $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 1.

**7.** The compound represented by general formula (IA) or a pharmaceutically acceptable salt thereof according to claim 2, being a compound represented by general formula (IVA) or a pharmaceutically acceptable salt thereof,

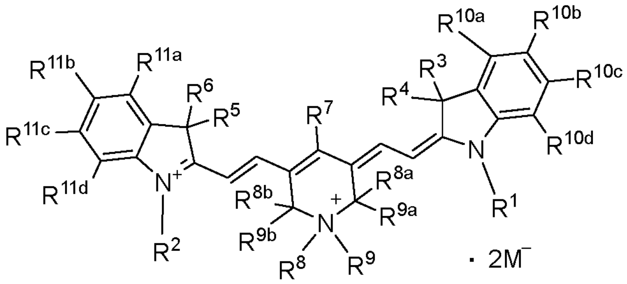

wherein, $M^-$, $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 2.

**8.** The compound or a pharmaceutically acceptable salt thereof according to any one of claims 1 to 7, wherein, $R^1$ is $L_1$-$L_2$- Q;

$L_1$ is selected from the group consisting of $-(CH_2)_p-$ and $-(CH_2O)_q-(CH_2)_p-$;
$L_2$ is selected from the group consisting of bond, $C_{6-10}$ aryl and 5 to 10 membered heteroaryl, and preferably selected from the group consisting of bond and phenyl;
Q is selected from the group consisting of $R^{12}$, -C(O)O$R^{13}$, -C(O)NH$R^{13}$, $-SO_3H$ and

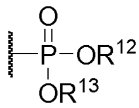
;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably selected from the group consisting of hydrogen and phenyl;
p is an integer from 1 to 10, and preferably an integer from 1 to 6;
q is an integer from 1 to 10, and preferably an integer from 1 to 6.

**9.** The compound or a pharmaceutically acceptable salt thereof according to any one of claims 1 to 8, wherein, $R^2$ is $L_1$-Q;

$L_1$ is $-(CH_2)_p-$;
Q is selected from the group consisting of $R^{12}$, -C(O)O$R^{13}$, -C(O)NH$R^{13}$, $-SO_3H$ and

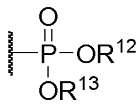
;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably selected from the group consisting of hydrogen and phenyl;
p is an integer from 1 to 10, and preferably an integer from 1 to 6.

**10.** The compound or a pharmaceutically acceptable salt thereof according to any one of claims 1 to 9, wherein $R^{10a}$, $R^{10b}$, $R^{10c}$ and $R^{10d}$ are each independently selected from the group consisting of hydrogen and O$R^a$, and $R^a$ is $C_{1-6}$ alkyl.

**11.** The compound or a pharmaceutically acceptable salt thereof according to any one of claims 1 to 10, wherein $R^{11a}$, $R^{11b}$, $R^{11c}$ and $R^{11d}$ are each independently selected from the group consisting of hydrogen, carboxy, O$R^a$, N$R^b$$R^c$, $-SO_3H$, $-S(O)_2R^a$ and -C(O)O$R^a$,

$R^a$ is $C_{1-6}$ alkyl;
$R^b$ and $R^c$ are each independently $C_{1-6}$ alkyl.

**12.** The compound or a pharmaceutically acceptable salt thereof according to claim 1, wherein any two adjacent ones of

$R^{10a}$, $R^{10b}$, $R^{10c}$ and $R^{10d}$, together with the atoms to which they are attached, form a $C_{6-10}$ aryl or 5 to 10 membered heteroaryl, and preferably phenyl; the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl.

**13.** The compound or a pharmaceutically acceptable salt thereof according to claim 1, wherein any two adjacent ones of $R^{11a}$, $R^{11b}$, $R^{11c}$ and $R^{11d}$, together with the atoms to which they are attached, form a $C_{6-10}$ aryl or 5 to 10 membered heteroaryl, and preferably phenyl; the aryl or heteroaryl is optionally further substituted by one or more substituents selected from the group consisting of halogen, amino, oxo, thioxo, nitro, cyano, hydroxy, thiol, carboxy, ester, alkyl, haloalkyl, alkoxy, haloalkoxy, hydroxyalkyl, alkenyl, alkynyl, cycloalkyl, heterocyclyl, aryl and heteroaryl.

**14.** The compound or a pharmaceutically acceptable salt thereof according to claim 1, being a compound with a structure of general formula (V) or a pharmaceutically acceptable salt thereof,

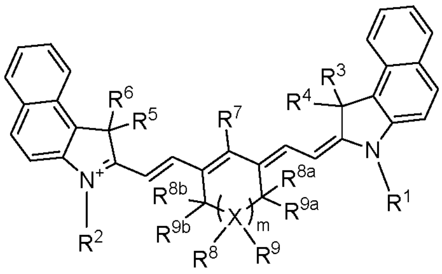

(V)

wherein, $R^1$ to $R^9$, $R^{8a}$, $R^{8b}$, $R^{9a}$, $R^{9b}$, X and m are as defined in claim 1.

**15.** The compound or a pharmaceutically acceptable salt thereof according to any one of claims 1 to 14, wherein,

$R^1$ is $L_1$- Q;
$L_1$ is $-(CH_2)_p-$;
Q is selected from the group consisting of $-C(O)OR^{13}$, $-C(O)NHR^{13}$, $-SO_3H$ and

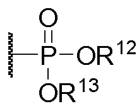
;

$R^{13}$ is selected from the group consisting of hydrogen, $C_{6-10}$ aryl and 5 to 10 membered heteroaryl; and preferably selected from the group consisting of hydrogen and phenyl;
p is an integer from 1 to 10, and preferably an integer from 1 to 6;
$R^2$ is $C_{1-6}$ alkyl.

**16.** The compound or a pharmaceutically acceptable salt thereof according to any one of claims 1 to 14, wherein,

$R^1$ is $L_1$- Q;
$L_1$ is $-(CH_2O)_q-(CH_2)_p-$;
Q is selected from the group consisting of $-C(O)OR^{13}$ and $-C(O)NHR^{13}$,
$R^{13}$ is selected from the group consisting of hydrogen, $C_{6-10}$ aryl and 5 to 10 membered heteroaryl; and preferably phenyl;
p is an integer from 1 to 6, preferably an integer from 1 to 4, and more preferably 1 or 2;
q is an integer from 1 to 6, preferably an integer from 1 to 4, and more preferably 1 or 2;
$R^2$ is $C_{1-6}$ alkyl.

**17.** The compound or a pharmaceutically acceptable salt thereof according to any one of claims 1 to 14, wherein,

$R^1$ is $L_1$-$L_2$- Q;
$L_1$ is $-(CH_2)_p-$;

$L_2$ is selected from the group consisting of $C_{6-10}$ aryl and 5 to 10 membered heteroaryl, and preferably phenyl;
Q is $-C(O)OR^{13}$,
$R^{13}$ is selected from the group consisting of hydrogen, $C_{6-10}$ aryl and 5 to 10 membered heteroaryl; and preferably phenyl;
p is an integer from 1 to 4, and preferably an integer from 1 to 2;
$R^2$ is $C_{1-6}$ alkyl.

**18.** The compound or a pharmaceutically acceptable salt thereof according to any one of claims 1 to 14, wherein,

$R^1$ is $C_{1-6}$ alkyl;
$R^2$ is $C_{1-6}$ alkyl.

**19.** The compound or a pharmaceutically acceptable salt thereof according to any one of claims 1 to 14, wherein,

$R^1$ and $R^2$ are each independently selected from $L_1$- Q;
$L_1$ is $-(CH_2)_p-$;
Q is selected from the group consisting of $R^{12}$, $-C(O)OR^{13}$, $-C(O)NHR^{13}$, $-SO_3H$ and

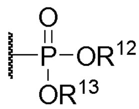
;

$R^{12}$ is selected from the group consisting of hydrogen, hydroxy and $C_{1-6}$ alkyl;
$R^{13}$ is selected from the group consisting of hydrogen, $C_{6-10}$ aryl and 5 to 10 membered heteroaryl; and preferably selected from the group consisting of hydrogen and phenyl;
p is an integer from 1 to 10, and preferably an integer from 1 to 6.

**20.** The compound or a pharmaceutically acceptable salt thereof according to any one of claims 1 to 19, wherein, $R^7$ is selected from the group consisting of hydrogen, halogen, hydroxy, $OR^a$, $SR^a$ and $NR^bR^c$;

$R^a$ is selected from the group consisting of $C_{6-10}$ aryl and 5 to 10 membered heteroaryl, and preferably phenyl; wherein, the aryl or heteroaryl is optionally further substituted by one or more substituents selected from the group consisting of halogen, amino, oxo, thioxo, nitro, cyano, hydroxy, thiol, carboxy, ester, sulfonic group, $-NHC(O)-L_4-NR^bR^c$, $-OC(O)-L_4-NR^bR^c$, alkyl, haloalkyl, alkoxy, haloalkoxy, hydroxyalkyl, alkenyl, alkynyl, cycloalkyl, heterocyclyl, aryl and heteroaryl; wherein $-L_4-$ is alkenylene;
$R^b$ and $R^c$ are each independently selected from the group consisting of hydrogen and $C_{1-10}$ alkyl, wherein the $C_{1-10}$ alkyl is optionally further substituted by carboxy.

**21.** The compound or a pharmaceutically acceptable salt thereof according to any one of claims 1 to 19, wherein $R^7$ is $SR^a$; $R^a$ is $C_{1-6}$ alkyl, and the $C_{1-6}$ alkyl is optionally substituted by carboxy.

**22.** The compound or a pharmaceutically acceptable salt thereof according to any one of claims 1 to 21, wherein $R^8$ and $R^9$ are each independently selected from the group consisting of hydrogen, $C_{1-6}$ alkyl and $NR^bR^c$; $R^b$ and $R^c$ are each independently selected from the group consisting of hydrogen and $C_{1-6}$ alkyl.

**23.** The compound or a pharmaceutically acceptable salt thereof according to any one of claims 1 to 22, wherein $R^{8a}$ and $R^{9a}$ are each independently hydrogen.

**24.** The compound or a pharmaceutically acceptable salt thereof according to any one of claims 1 to 23, wherein $R^{8b}$ and $R^{9b}$ are each independently hydrogen.

**25.** The compound or a pharmaceutically acceptable salt thereof according to any one of claims 1 to 24, wherein $R^3$ and $R^4$ are each independently selected from the group consisting of hydrogen and $C_{1-6}$ alkyl.

**26.** The compound or a pharmaceutically acceptable salt thereof according to any one of claims 1 to 25, wherein $R^5$ and $R^6$ are each independently selected from the group consisting of hydrogen and $C_{1-6}$ alkyl.

**27.** The compound or a pharmaceutically acceptable salt thereof according to claim 1, wherein the pharmaceutically acceptable salt is halide salt or carboxylate salt, and preferably hydrochloride salt, formate salt, acetate salt or trifluoroacetate salt.

**28.** The compound or a pharmaceutically acceptable salt thereof according to any one of claims 1 to 27, being selected from the group consisting of

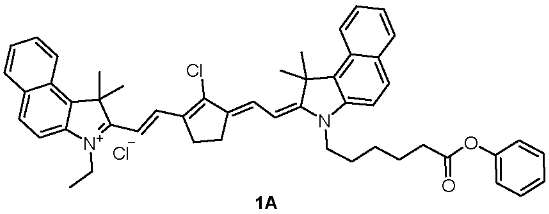
1A ,

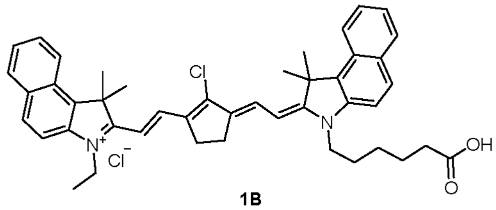
1B ,

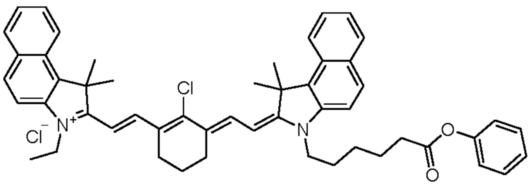
2 ,

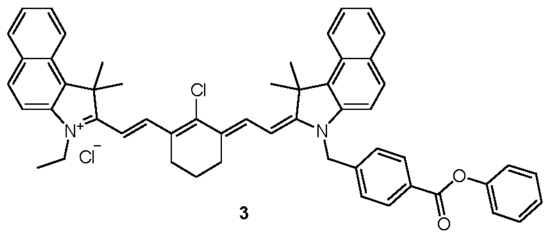
3 ,

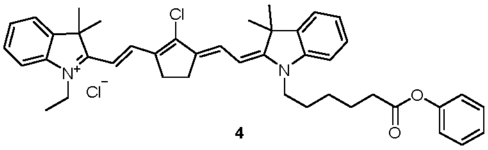
4 ,

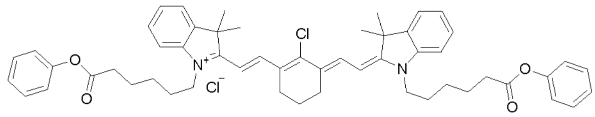
5 ,

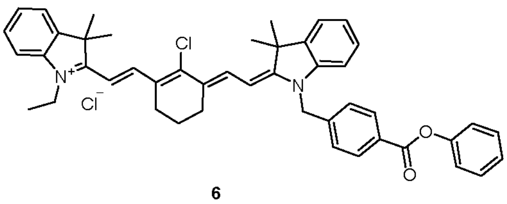
6 ,

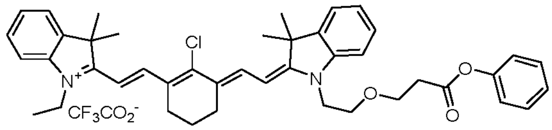
7 ,

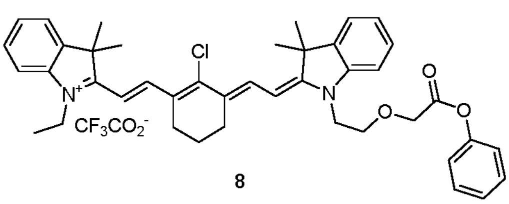
8 ,

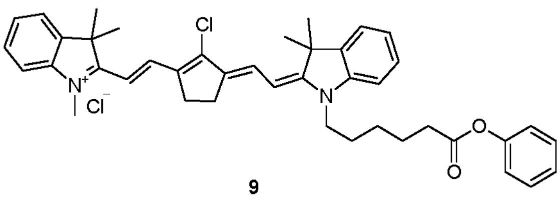
9 ,

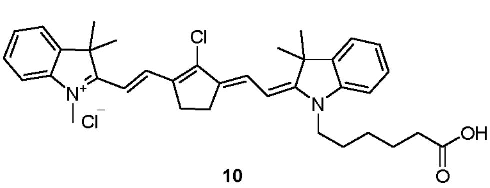
10 ,

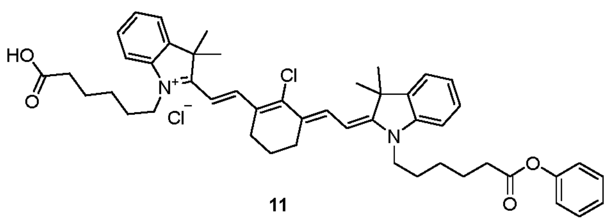
11 ,

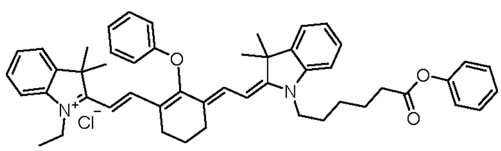

12

,

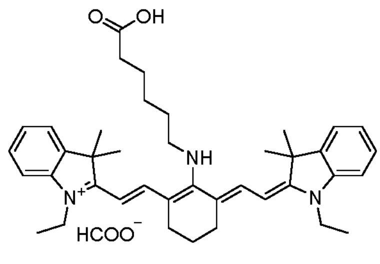

13

,

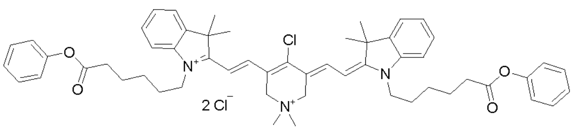

14

,

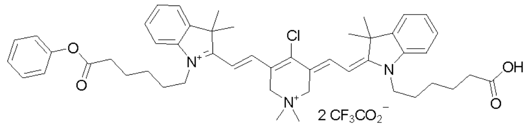

15

,

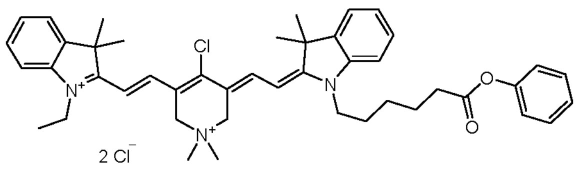

16

,

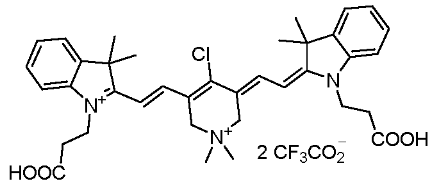

17

,

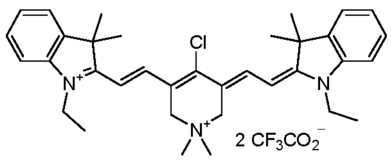

18

,

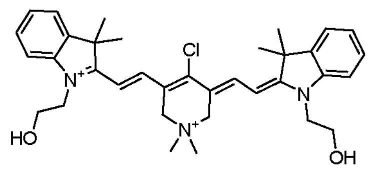

19

,

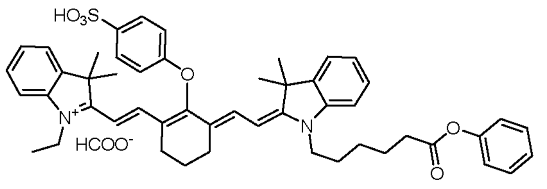

20

,

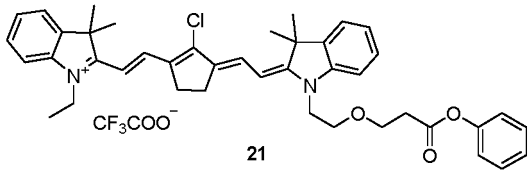

21

,

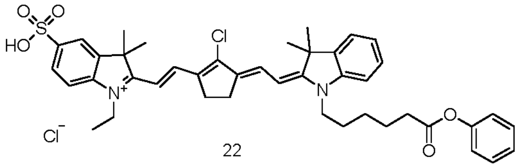

22

,

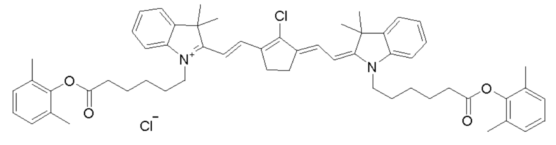

23

,

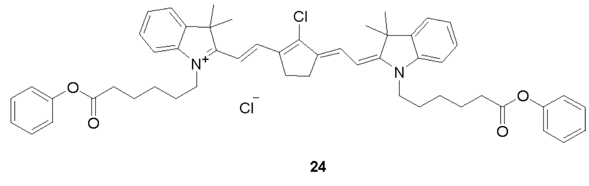

24

,

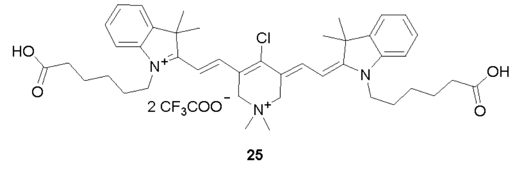

25

,

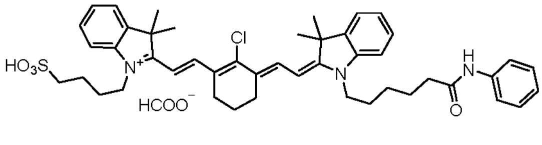

26

,

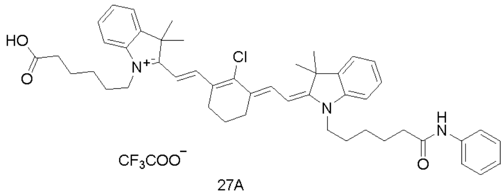

27A

,

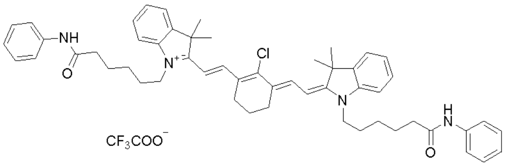

27B

,

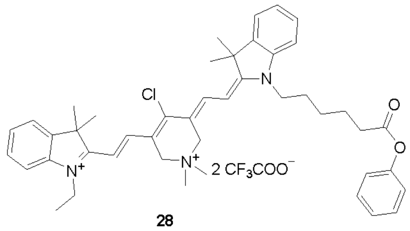

28

,

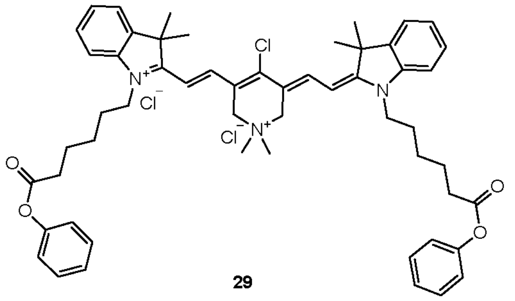

29

,

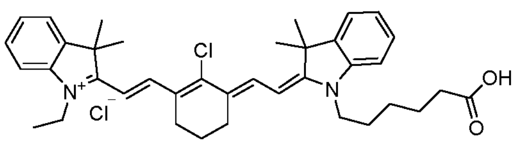

30

,

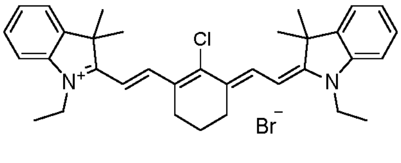

31

,

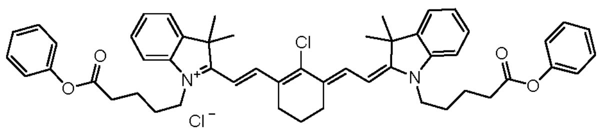

33

,

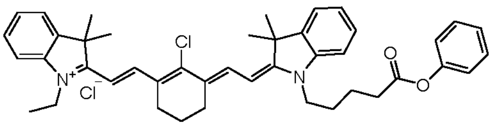

34

,

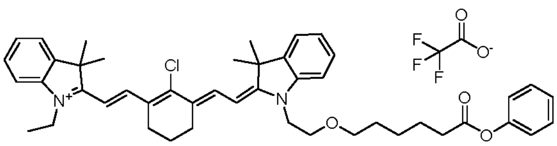

35

,

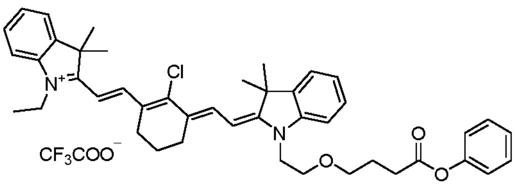

36

,

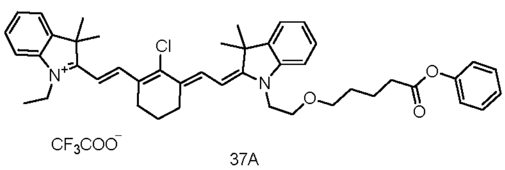

37A

,

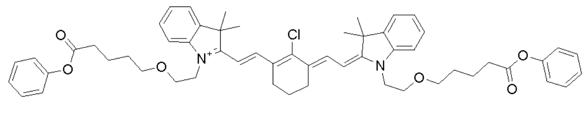

37B,

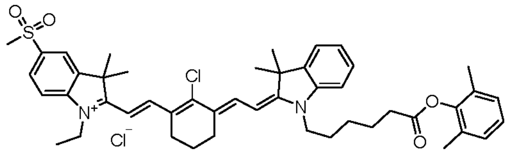

39,

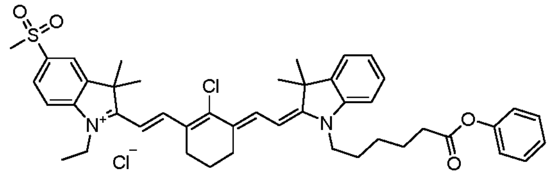

40,

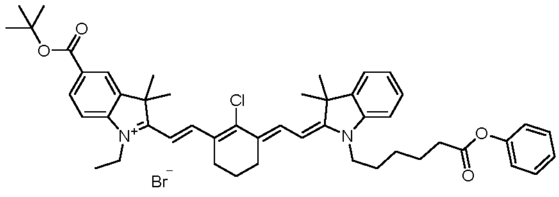

41,

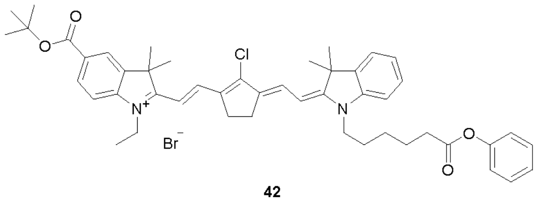

42,

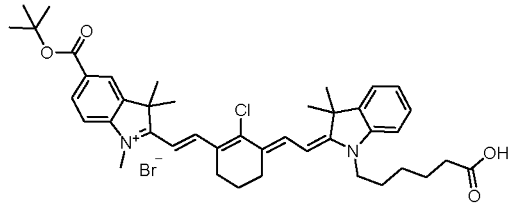

43,

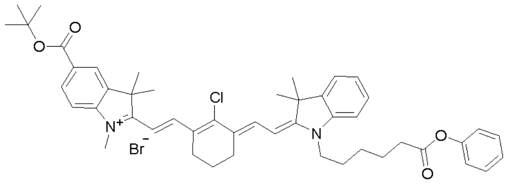

44,

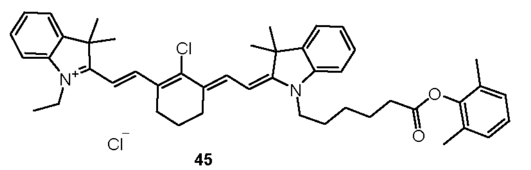

45,

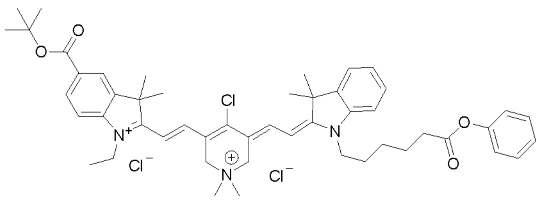

46,

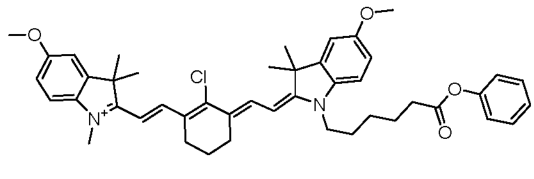

47,

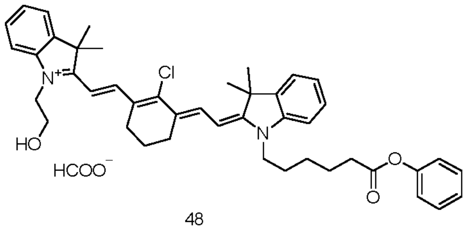

48,

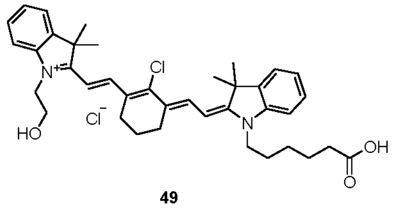

49,

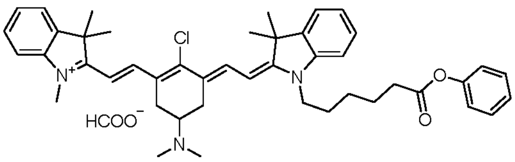

50

,

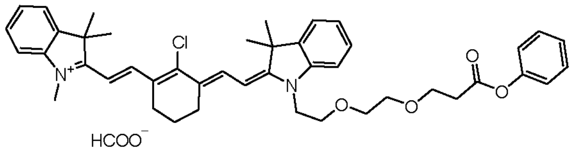

51

,

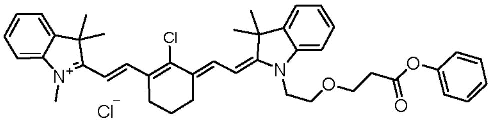

52

,

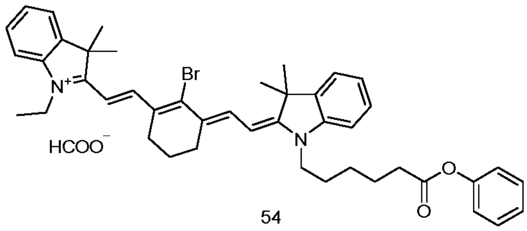

54

,

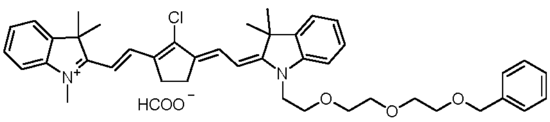

55

,

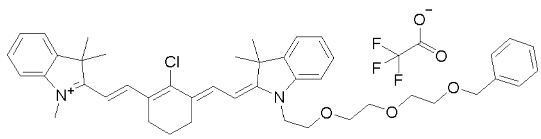

56

,

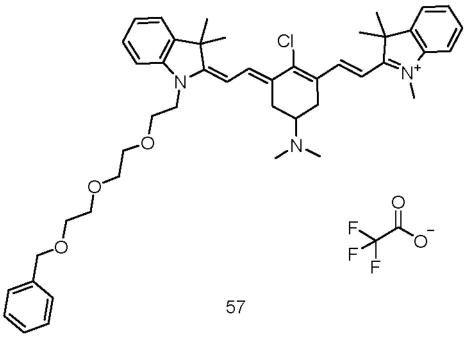

57

,

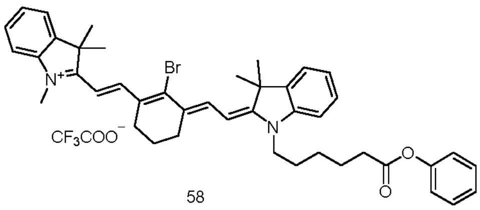

58

,

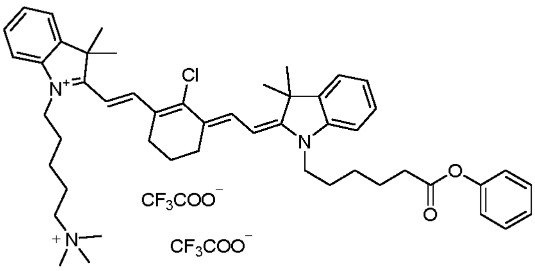

60

,

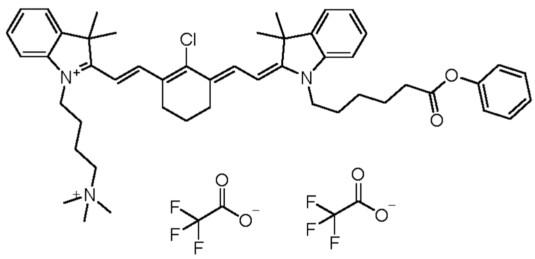

61

,

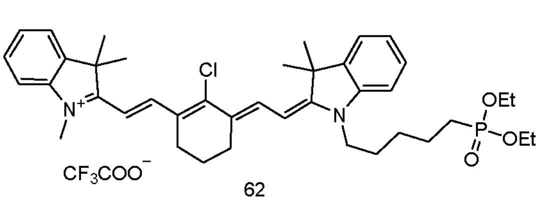

62

,

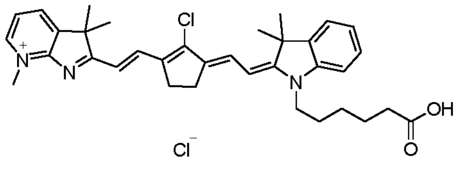

63

,

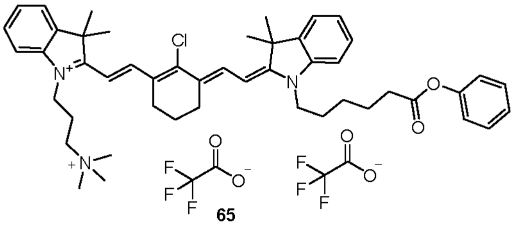

65

,

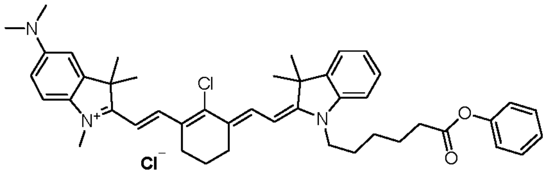

66

,

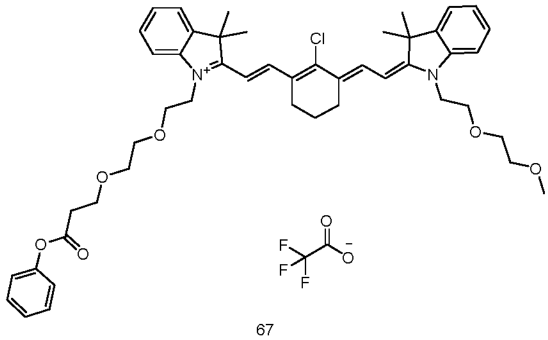

67

,

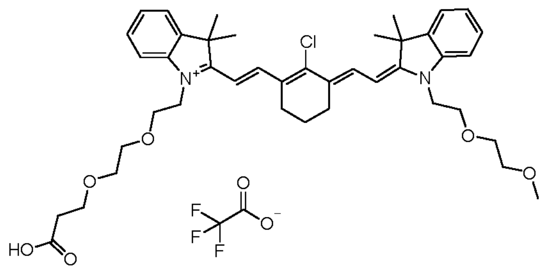

68

,

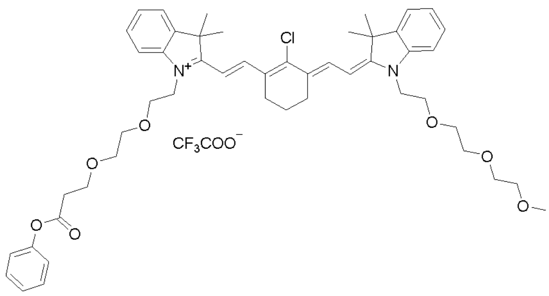

69

,

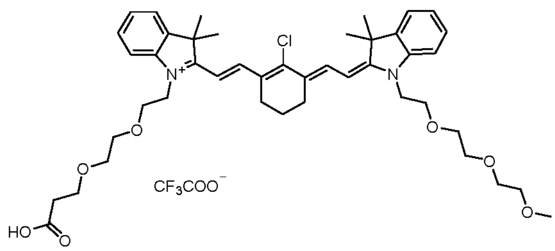

70

,

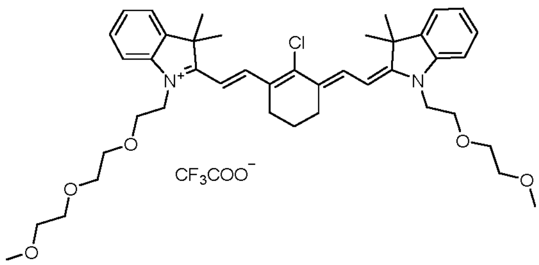

71

,

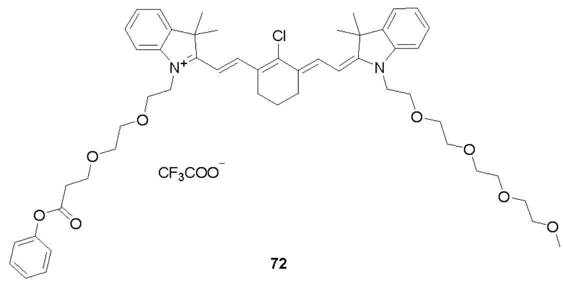

72

,

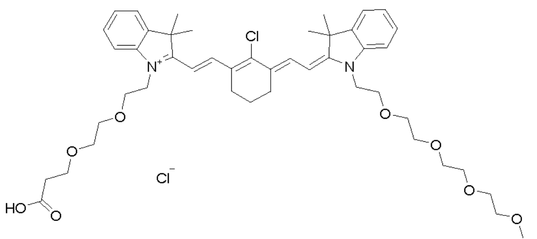

73

,

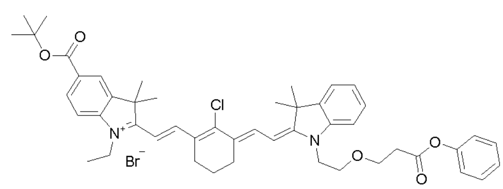

74

,

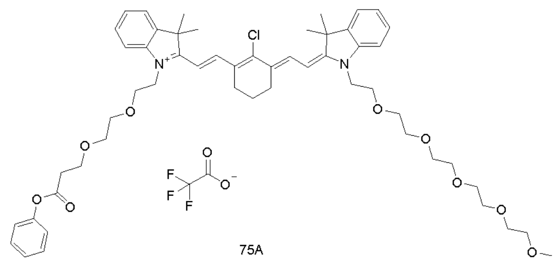

75A ,

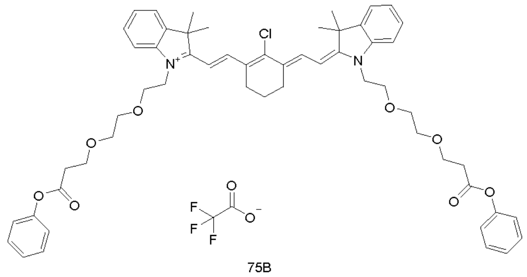

75B ,

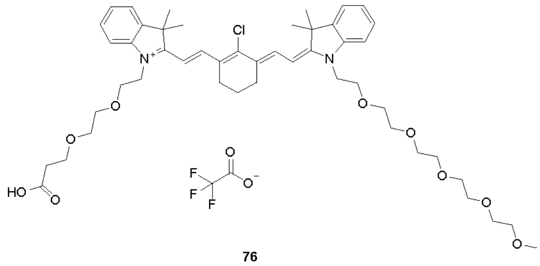

76 ,

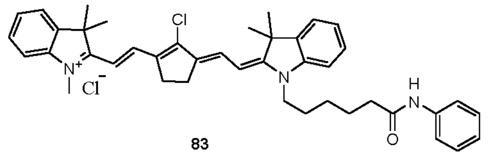

83 ,

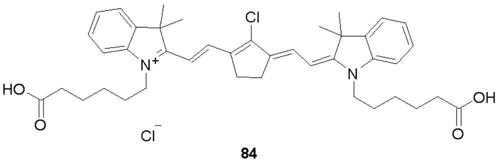

84 .

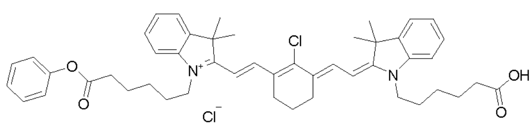

85A .

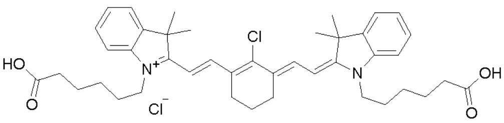

85B ,

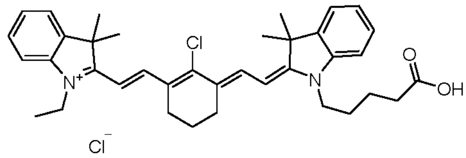

86 ,

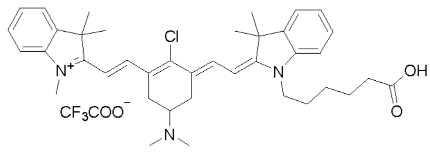

87 .

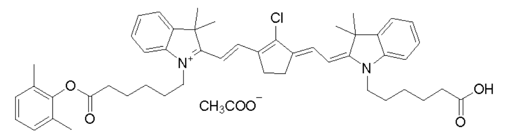

88 .

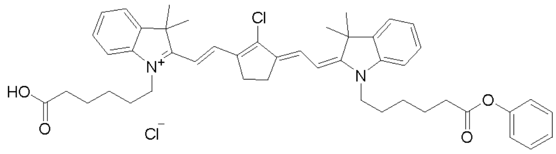

89 ,

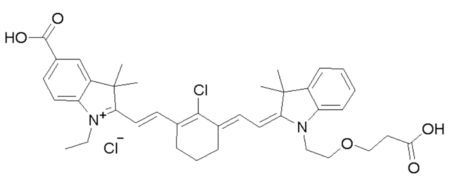

91 ,

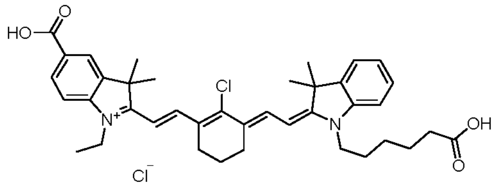

92,

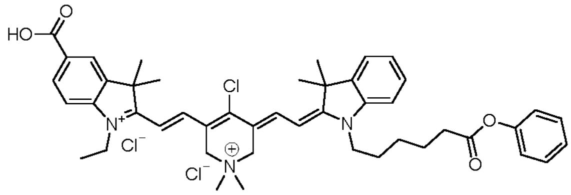

93,

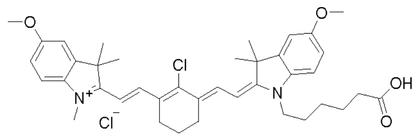

94,

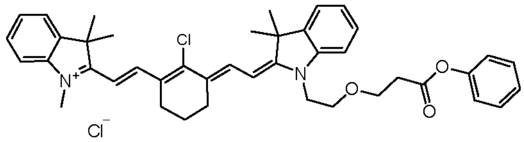

95,

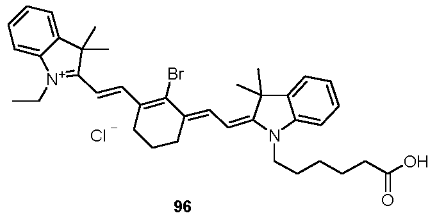

96,

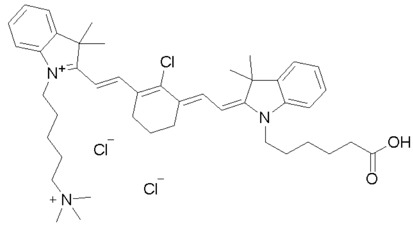

97,

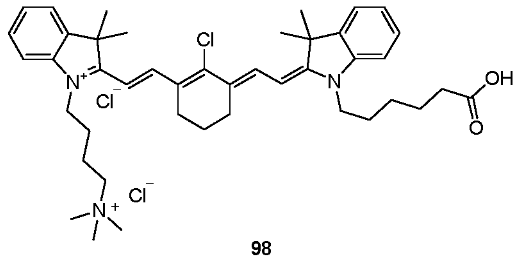

98,

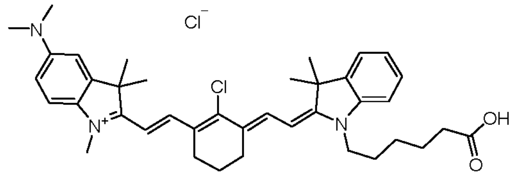

99,

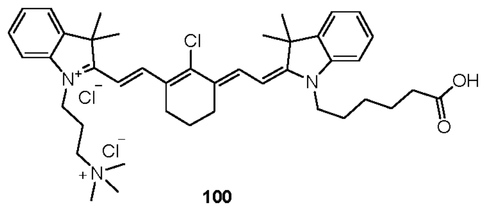

100,

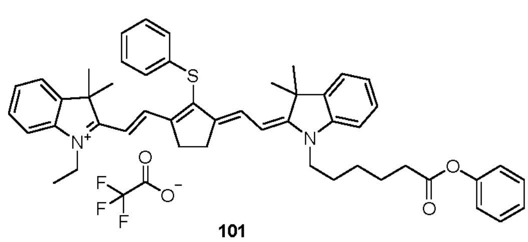

101,

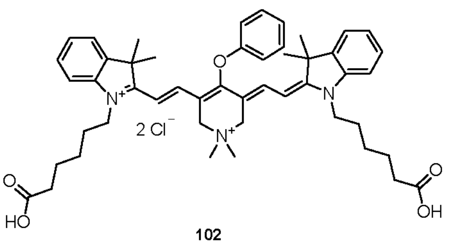

102,

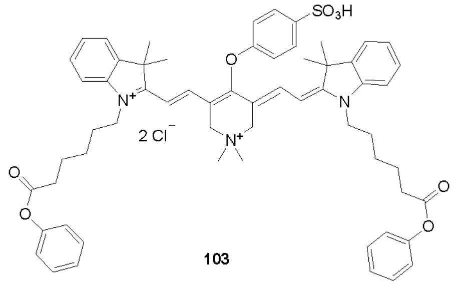

103,

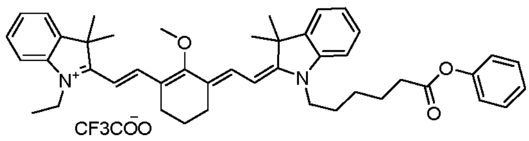

104

,

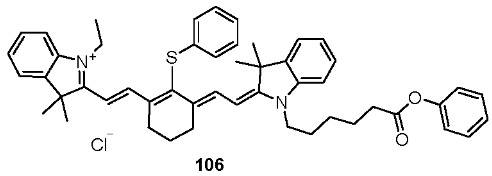

106

,

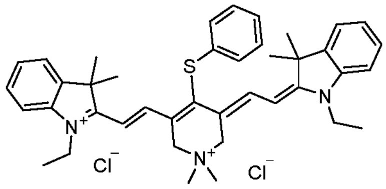

107

,

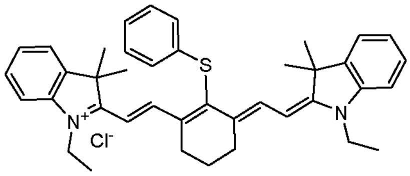

108

,

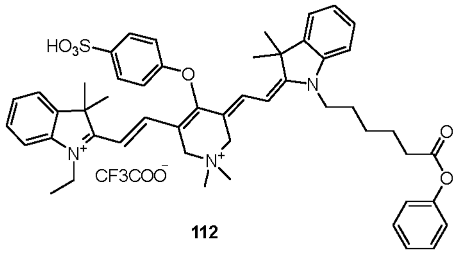

112

,

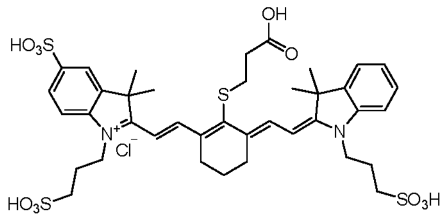

113

,

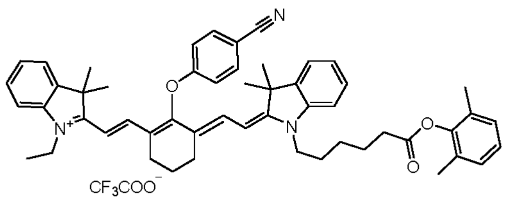

114

,

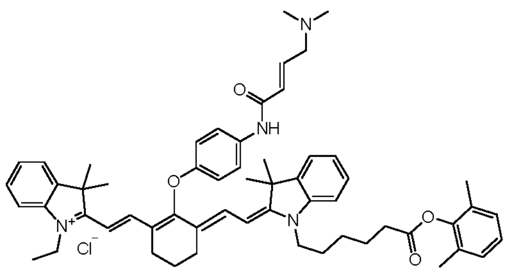

115

,

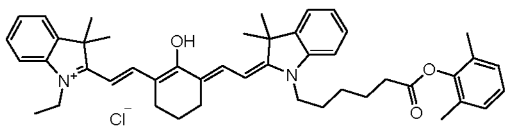

116

,

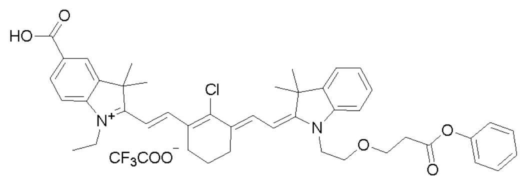

117

,

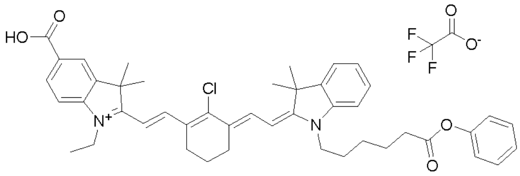

118

,

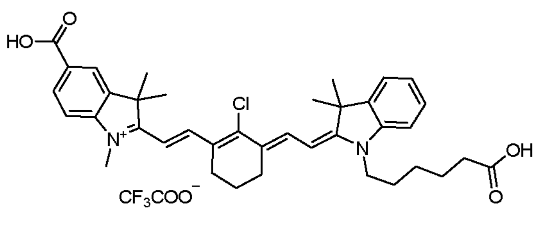

119

,

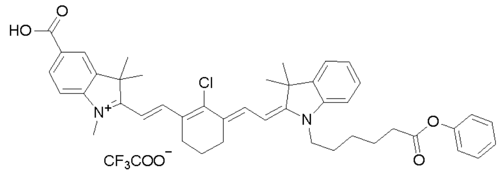

120

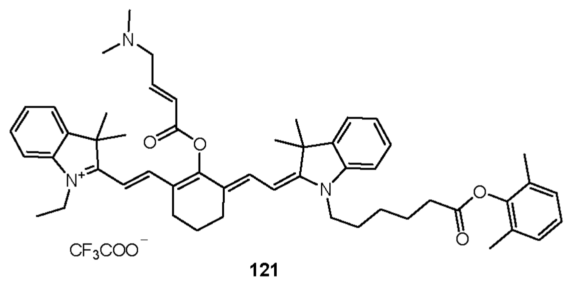

121

,

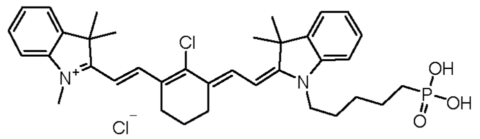

122

,

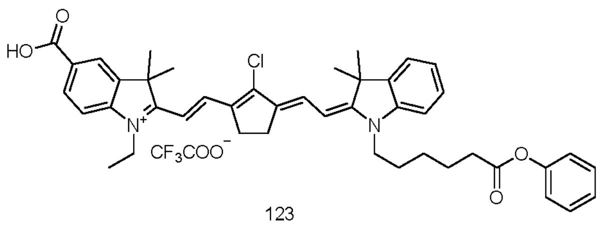

123

.

**29.** A compound with a structure of general formula (VI) or a pharmaceutically acceptable salt thereof,

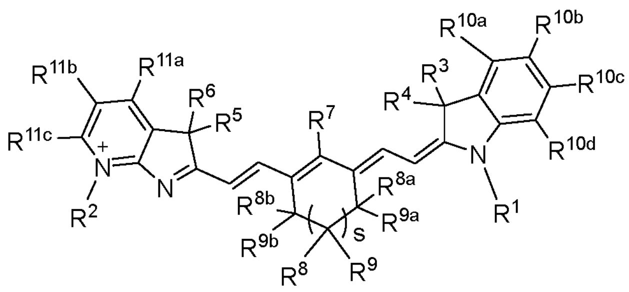

(VI)

wherein, $R^1$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$ and $R^{11c}$ are as defined in claim 1; and s is 0 or 1;

preferably,

$R^1$ is $L_1$-$L_2$- Q;

$L_1$ is $-(CH_2)_p-$;

$L_2$ is a bond;

Q is $-C(O)OR^{13}$;

$R^{13}$ is selected from the group consisting of hydrogen and phenyl;

$R^2$ is $C_{1-6}$ alkyl;

$R^3$ and $R^4$ are each independently selected from the group consisting of hydrogen and $C_{1-6}$ alkyl, and preferably $C_{1-6}$ alkyl;

$R^5$ and $R^6$ are each independently selected from the group consisting of hydrogen and $C_{1-6}$ alkyl, and preferably $C_{1-6}$ alkyl;

$R^7$ is a halogen;

$R^8$ and $R^9$ are each independently hydrogen;

$R^{8a}$ and $R^{9a}$ are each independently hydrogen;

$R^{8b}$ and $R^{9b}$ are each independently hydrogen;

$R^{10a}$, $R^{10b}$, $R^{10c}$ and $R^{10d}$ are each independently hydrogen;

$R^{11a}$, $R^{11b}$ and $R^{11c}$ are each independently hydrogen.

**30.** The compound or a pharmaceutically acceptable salt thereof according to claim 29, being selected from the group consisting of:

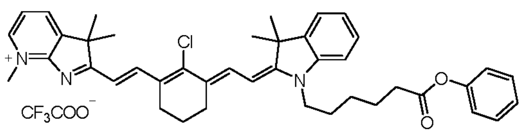

53

and

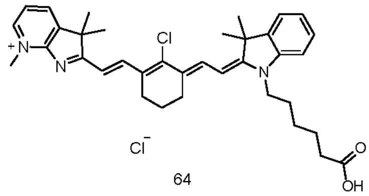

64 .

**31.** A compound with a structure of general formula (VII) or a pharmaceutically acceptable salt thereof,

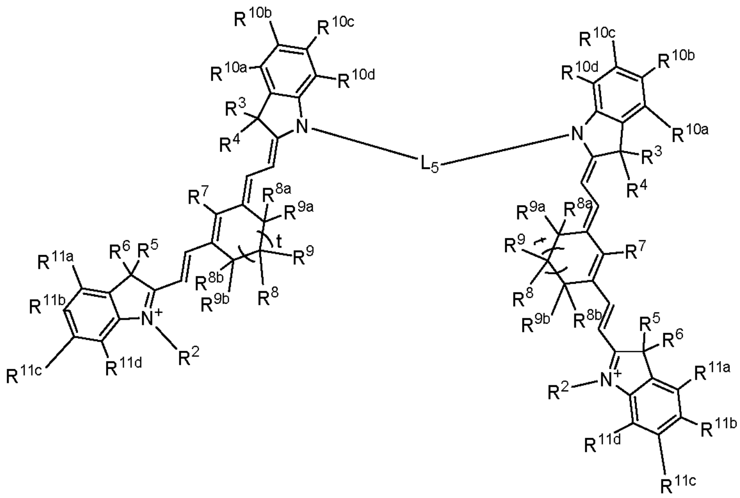

(VII)

wherein, $R^2$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 1;
t is 0 or 1;
$L_5$ is $-(CH_2)_{t1}-C(O)NH-(CH_2)_{t2}-NHC(O)-(CH_2)_{t3}-$;
$t_1$, $t_2$ and $t_3$ are each independently selected from the group consisting of integers from 1 to 10; and preferably selected from the group consisting of integers from 2 to 6;
preferably,
$R^2$ is $C_{1-6}$ alkyl;
$R^3$ and $R^4$ are each independently selected from the group consisting of hydrogen and $C_{1-6}$ alkyl, and preferably $C_{1-6}$ alkyl;
$R^5$ and $R^6$ are each independently selected from the group consisting of hydrogen and $C_{1-6}$ alkyl, and preferably $C_{1-6}$ alkyl;
$R^7$ is a halogen;
$R^8$ and $R^9$ are each independently hydrogen;
$R^{8a}$ and $R^{9a}$ are each independently hydrogen;
$R^{8b}$ and $R^{9b}$ are each independently hydrogen;
$R^{10a}$, $R^{10b}$, $R^{10c}$ and $R^{10d}$ are each independently hydrogen;
$R^{11a}$, $R^{11b}$ and $R^{11c}$ are each independently hydrogen.

**32.** The compound or a pharmaceutically acceptable salt thereof according to claim 31, being selected from the group consisting of

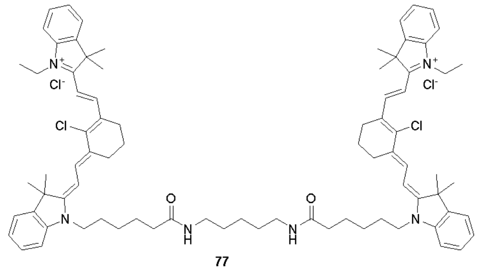

77

,

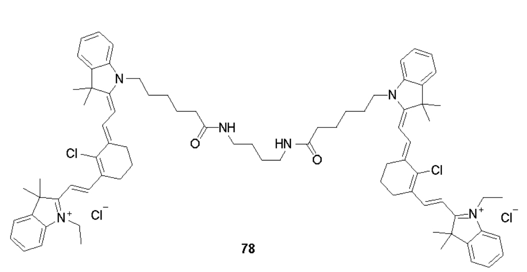

78

,

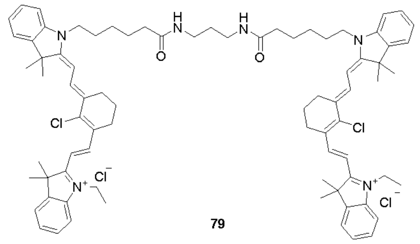

79

,

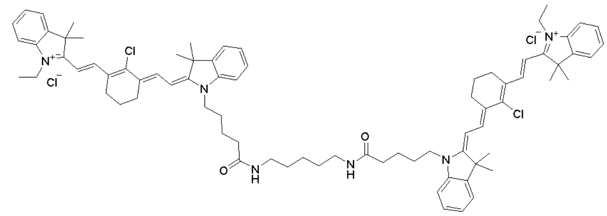

80

,

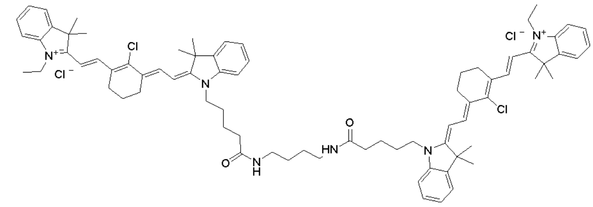

81

and

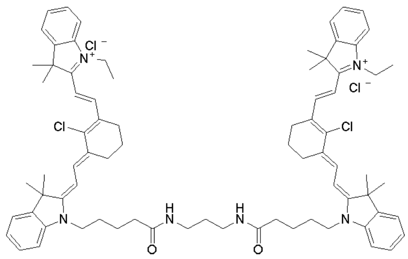

82

.

**33.** A compound with a structure of general formula (VIII) or a pharmaceutically acceptable salt thereof,

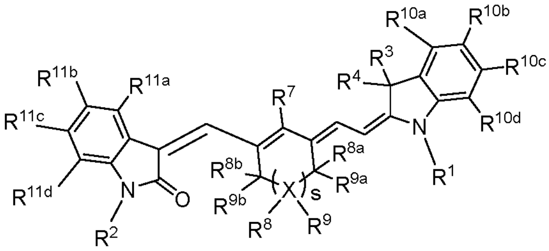

(VI)

wherein, X, $R^1$ to $R^4$, $R^7$ to $R^9$, $R^{8a}$, $R^{8b}$, $R^{9a}$, $R^{9b}$, $R^{10a}$, $R^{10b}$, $R^{10c}$, $R^{10d}$, $R^{11a}$, $R^{11b}$, $R^{11c}$ and $R^{11d}$ are as defined in claim 1; and s is 0 or 1;

preferably,

X is $N^+$;

s is 1;

$R^1$ is $L_1$-$L_2$- Q;

$L_1$ is $-(CH_2)_p-$;

$L_2$ is a bond;

Q is $-C(O)OR^{13}$;

$R^{13}$ is selected from the group consisting of hydrogen and phenyl;

$R^2$ is $C_{1-6}$ alkyl;

$R^3$ and $R^4$ are each independently selected from the group consisting of hydrogen and $C_{1-6}$ alkyl, and preferably $C_{1-6}$ alkyl;

$R^7$ is a halogen;

$R^8$ and $R^9$ are each independently $C_{1-6}$ alkyl;

$R^{8a}$ and $R^{9a}$ are each independently hydrogen;
$R^{8b}$ and $R^{9b}$ are each independently hydrogen;
$R^{10a}$, $R^{10b}$, $R^{10c}$ and $R^{10d}$ are each independently hydrogen;
$R^{11a}$, $R^{11b}$ and $R^{11c}$ are each independently hydrogen.

**34.** The compound or a pharmaceutically acceptable salt thereof according to claim 33, being selected from the group consisting of

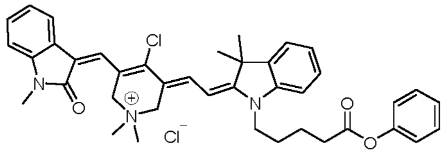

38

.

**35.** A contrast agent comprising the compound or a pharmaceutically acceptable salt thereof according to any one of claims 1 to 34 and an anionic surfactant.

**36.** The contrast agent according to claim 35, wherein, the anionic surfactant is selected from the group consisting of carboxylate salt, sulfonate salt, sulfate salt, phosphate salt and lipopeptide anionic surfactants, preferably is sulfonate salt or lipopeptide anionic surfactant, and more preferably is surfactin, sodium dodecyl sulfonate, and/or sodium dodecyl benzene sulfonate; and/or

the molar ratio of the compound or a pharmaceutically acceptable salt thereof according to any one of claims 1 to 34 to the anionic surfactant ranges from 1:20 to 10:1, preferably from 1:10 to 10:1, and further preferably from 1:5 to 5:1; and/or
the compound or the pharmaceutically acceptable salt thereof according to any one of claims 1 to 34 in the contrast agent has a final concentration ranging from 1 to 100 μM, and preferably from 5 to 50 μM or from 10 to 30 μM; and/or
a solution of the contrast agent has a particle size ranging from 10 to 200 nm, preferably from 30 to 150 nm, and more preferably from 50 to 100 nm; and/or
the contrast agent is in a form of solution or lyophilization powder.

**37.** An assay kit comprising the compound or a pharmaceutically acceptable salt thereof according to any one of claims 1 to 34 and an anionic surfactant;

preferably, the anionic surfactant is selected from the group consisting of carboxylate salt, sulfonate salt, sulfate salt, phosphate salt and lipopeptide anionic surfactants, preferably is sulfonate salt or lipopeptide anionic surfactant, and more preferably is surfactin, sodium dodecyl sulfonate, and/or sodium dodecyl benzene sulfonate; and/or
the molar ratio of the compound or a pharmaceutically acceptable salt thereof according to any one of claims 1 to 34 to the anionic surfactant ranges from 1:20 to 10:1, preferably from 1:10 to 10:1, and further preferably from 1:5 to 5:1; and/or
the compound or a pharmaceutically acceptable salt thereof according to any one of claims 1 to 34 in the contrast agent has a final concentration ranging from 1 to 100 μM, and preferably from 5 to 50 μM or from 10 to 30 μM; and/or
a solution of the contrast agent has a particle size ranging from 10 to 200 nm, preferably from 30 to 150 nm, and more preferably from 50 to 100 nm; and/or
the contrast agent is in a form of solution form or lyophilization powder.

**38.** A pharmaceutical composition comprising the compound or a pharmaceutically acceptable salt thereof according to any one of claims 1 to 34, and one or more pharmaceutically acceptable carriers.

**39.** Use of the compound or a pharmaceutically acceptable salt thereof according to any one of claims 1 to 34 or the pharmaceutical composition according to claim 38 in the preparation of a contrast agent for tumor, wherein the tumor is preferably bladder cancer.

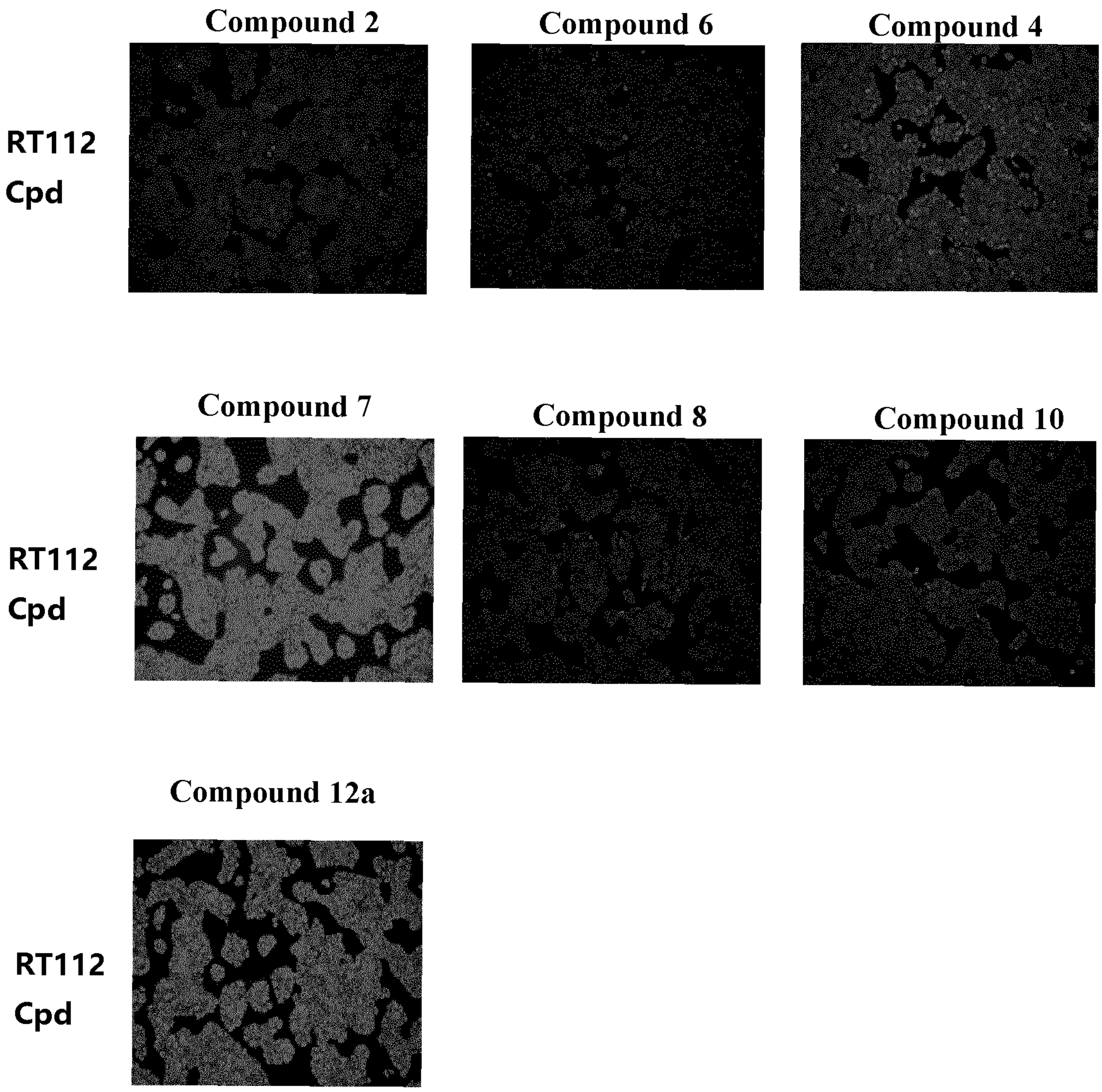
Compound 2
Compound 6
Compound 4
RT112
Cpd
Compound 7
Compound 8
Compound 10
RT112
Cpd
Compound 12a
RT112
Cpd

Figure 1

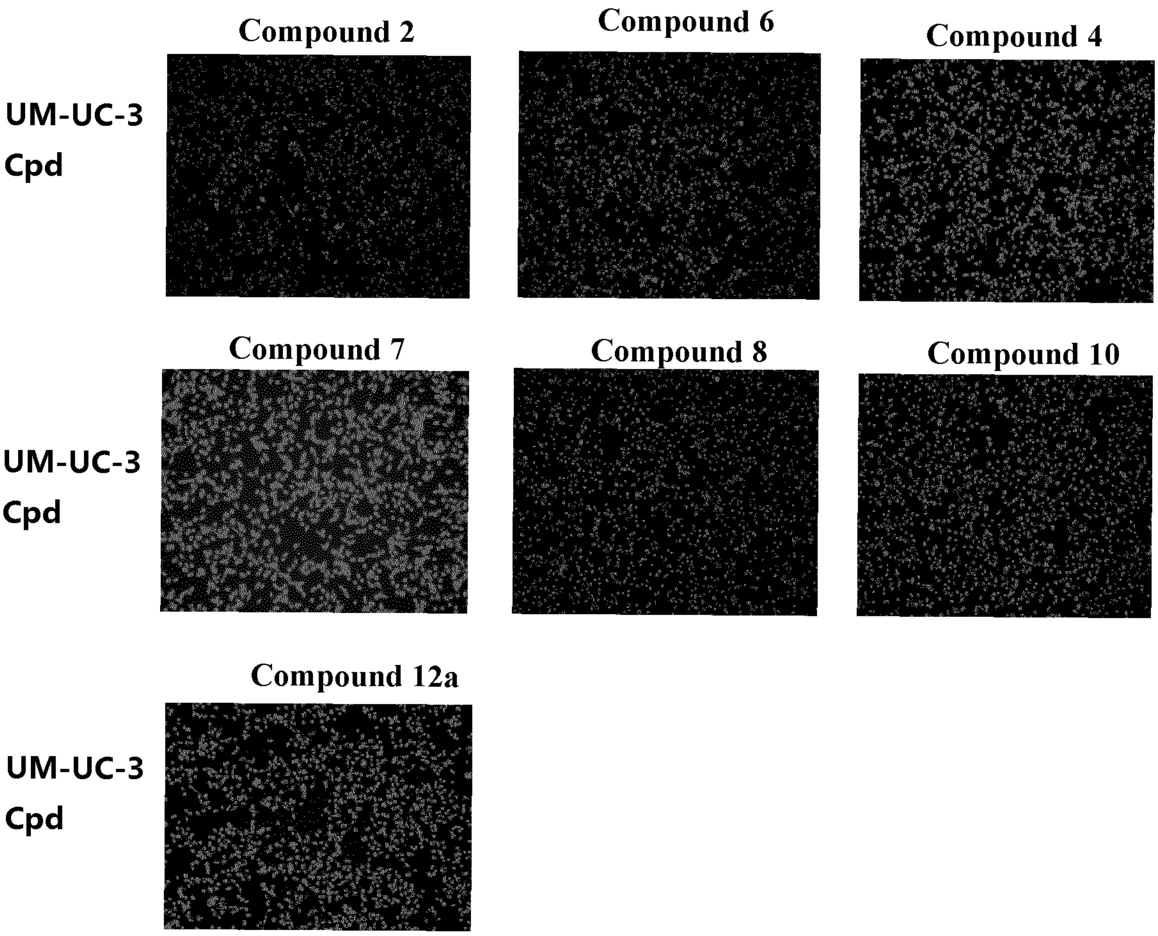
Compound 2
Compound 6
Compound 4
UM-UC-3
Cpd
Compound 7
Compound 8
Compound 10
UM-UC-3
Cpd
Compound 12a
UM-UC-3
Cpd

Figure 2

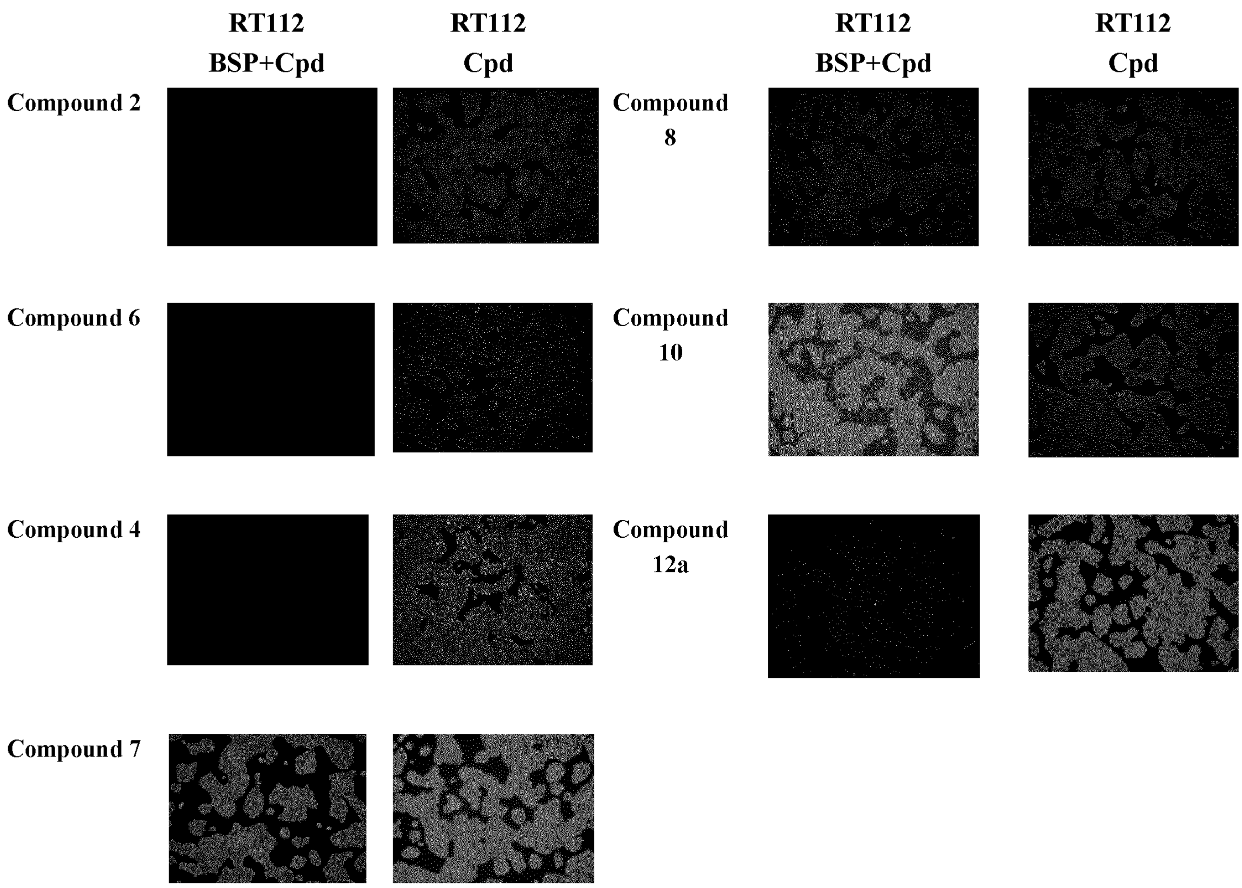
RT112
BSP+Cpd
RT112
Cpd
RT112
BSP+Cpd
RT112
Cpd
Compound 2
Compound 8
Compound 6
Compound 10
Compound 4
Compound 12a
Compound 7

Figure 3

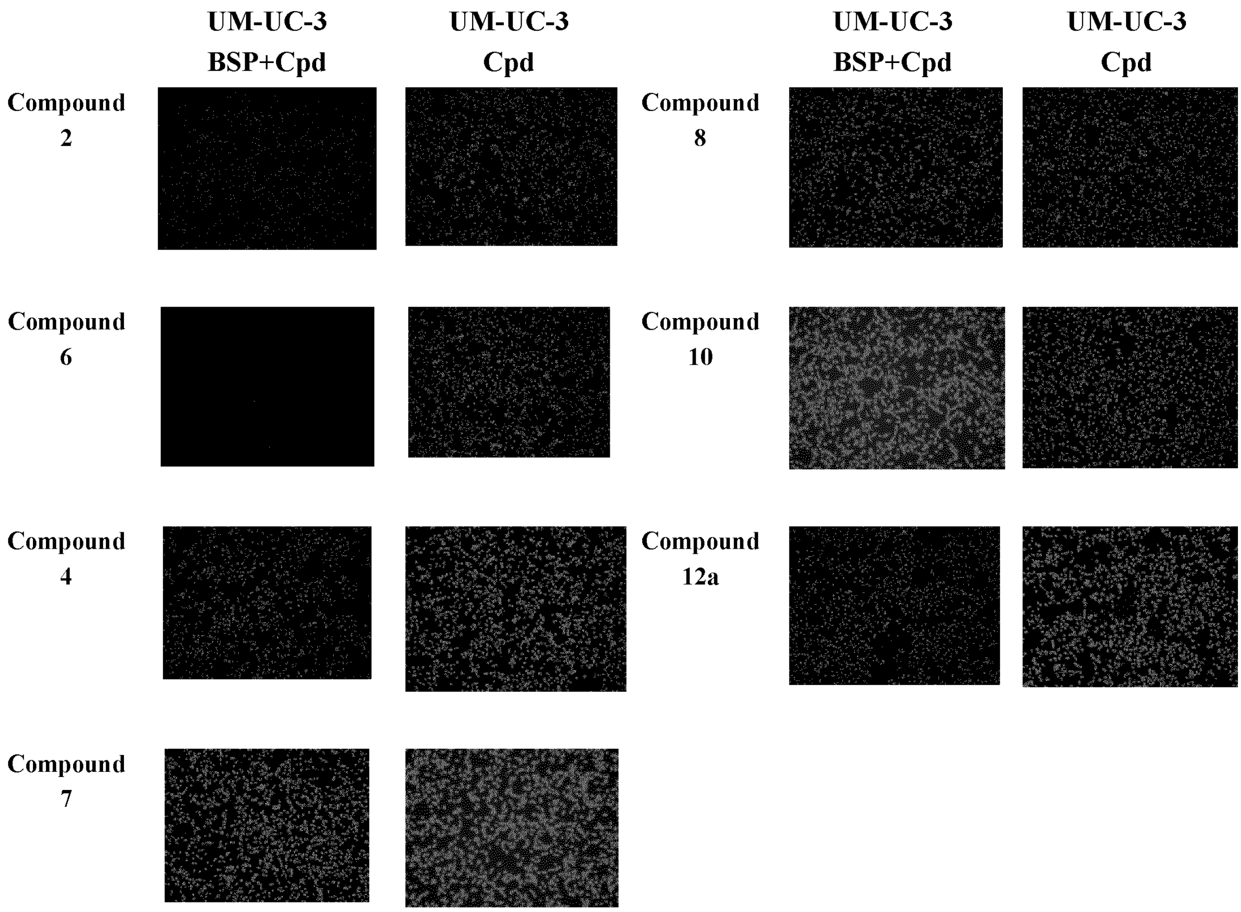
UM-UC-3
BSP+Cpd
UM-UC-3
Cpd
UM-UC-3
BSP+Cpd
UM-UC-3
Cpd
Compound 2
Compound 8
Compound 6
Compound 10
Compound 4
Compound 12a
Compound 7

Figure 4

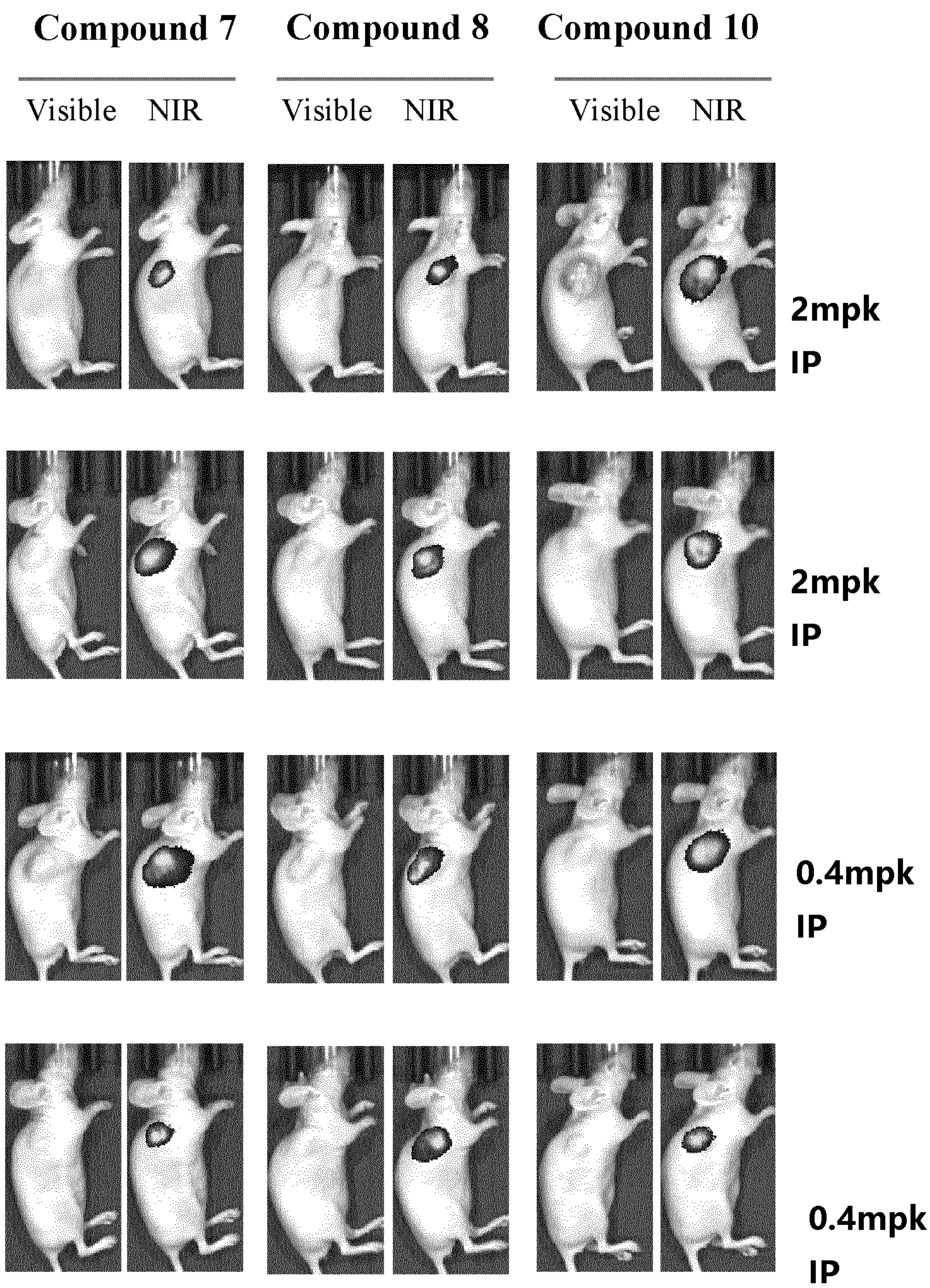
Compound 7
Compound 8
Compound 10
Visible
NIR
Visible
NIR
Visible
NIR
2mpk
IP
2mpk
IP
0.4mpk
IP
0.4mpk
IP

Figure 5

**INTERNATIONAL SEARCH REPORT**

International application No.
**PCT/CN2023/124246**

**A. CLASSIFICATION OF SUBJECT MATTER**

C07D209/60(2006.01)i; C07D209/08(2006.01)i; C07D401/06(2006.01)i; C07D401/14(2006.01)i; C07D471/04(2006.01)i; C09K11/06(2006.01)i; A61K49/06(2006.01)i

According to International Patent Classification (IPC) or to both national classification and IPC

**B. FIELDS SEARCHED**

Minimum documentation searched (classification system followed by classification symbols)

IPC: C07D C09K A61K

Documentation searched other than minimum documentation to the extent that such documents are included in the fields searched

Electronic data base consulted during the international search (name of data base and, where practicable, search terms used)

CNTXT, VEN, CNKI, 万方数据, WANFANG DATA, STNext(CAplus, Registry, Marpat): 造影剂, 显影, 成像, 影像, 七甲川花青, 七甲川花菁, 肿瘤, 癌, contrast, imaging, diagnostic, tumor, cancer, structure search

**C. DOCUMENTS CONSIDERED TO BE RELEVANT**

| Category* | Citation of document, with indication, where appropriate, of the relevant passages | Relevant to claim No. |
|---|---|---|
| X | CN 111925311 A (SHANGHAI KUBO BIOTECHNOLOGY CO., LTD.) 13 November 2020 (2020-11-13)<br>entire document, and in particular, claims 1-10 and 19 | 1-5, 8-28, 35-39 |
| Y | CN 111925311 A (SHANGHAI KUBO BIOTECHNOLOGY CO., LTD.) 13 November 2020 (2020-11-13)<br>entire document, and in particular, claims 1-10 and 19 | 1-30, 35-39 |
| A | CN 111925311 A (SHANGHAI KUBO BIOTECHNOLOGY CO., LTD.) 13 November 2020 (2020-11-13)<br>entire document | 31-34 |
| Y | WANG, Xu et al. "Screening and investigation of a cyanine fluorescent probe for simultaneous sensing of glutathione and cysteine under single excitation." *Chemical Communications,* Vol. 50, 23 August 2014 (2014-08-23), pages 15439-15422, and Supporting Information<br>see entire document | 1-5, 8-20, 22-28, 35-39 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 January 2024** | **01 February 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/124246**

**C. DOCUMENTS CONSIDERED TO BE RELEVANT**

| Category* | Citation of document, with indication, where appropriate, of the relevant passages | Relevant to claim No. |
|---|---|---|
| X | THAVORNPRADIT, Sopida et al. "QuatCy: a heptamethine cyanine modification with improved characteristics" *Theranostics,* Vol. 9, No. 10, 18 July 2019 (2019-07-18), pages 2856-2867 ISSN: 1838-7640, entire document, in particular, abstract, and pages 2857-2862 | 1-2, 6-13, 15, 20, 22-28, 38 |
| Y | THAVORNPRADIT, Sopida et al. "QuatCy: a heptamethine cyanine modification with improved characteristics" *Theranostics,* Vol. 9, No. 10, 18 July 2019 (2019-07-18), pages 2856-2867 ISSN: 1838-7640, entire document, in particular, abstract, and pages 2857-2862 | 1-2, 6-28, 35-39 |
| X | WU, Jason Boyang et al. "Monoamine Oxidase A Inhibitor–Near-Infrared Dye Conjugate Reduces Prostate Tumor Growth" *Journal of the American Chemical Society,* Vol. 137, No. 6, 13 January 2015 (2015-01-13), pages 2366-2374 ISSN: 1520-5126, page 2367, scheme 1 | 1-2, 4-5, 8-11, 19, 20, 22-28 |
| X | WO 2009012109 A2 (EMORY UNIVERSITY et al.) 22 January 2009 (2009-01-22) claims 1-30, and description, paragraphs 0049-0060 | 1-5, 8-28, 38 |
| Y | WO 2009012109 A2 (EMORY UNIVERSITY et al.) 22 January 2009 (2009-01-22) claims 1-30, and description, paragraphs 0049-0060 | 1-30, 35-39 |
| Y | WO 2012054749 A1 (LI-COR, INC. et al.) 26 April 2012 (2012-04-26) claims 1 and 170, and description, paragraph 0310 | 29-30, 35-39 |
| A | CN 105813648 A (BLAZE BIOSCIENCE, INC. et al.) 27 July 2016 (2016-07-27) entire document | 1-39 |
| A | JP 2004219649 A (FUJI PHOTO FILM CO., LTD.) 05 August 2004 (2004-08-05) entire document | 1-39 |

Form PCT/ISA/210 (second sheet) (July 2022)

**INTERNATIONAL SEARCH REPORT**
**Information on patent family members**

International application No.
**PCT/CN2023/124246**

| Patent document cited in search report | | | Publication date (day/month/year) | Patent family member(s) | | | Publication date (day/month/year) |
|---|---|---|---|---|---|---|---|
| CN | 111925311 | A | 13 November 2020 | CN | 111925311 | B | 02 November 2021 |
| WO | 2009012109 | A2 | 22 January 2009 | US | 2015183736 | A1 | 02 July 2015 |
| | | | | US | 2011262354 | A1 | 27 October 2011 |
| | | | | US | 2015335765 | A1 | 26 November 2015 |
| | | | | US | 2017354747 | A1 | 14 December 2017 |
| | | | | US | 11738095 | B2 | 29 August 2023 |
| | | | | WO | 2009012109 | A3 | 30 December 2009 |
| WO | 2012054749 | A1 | 26 April 2012 | US | 2013039860 | A1 | 14 February 2013 |
| | | | | US | 2013274452 | A1 | 17 October 2013 |
| | | | | US | 2013280172 | A1 | 24 October 2013 |
| | | | | US | 2015119553 | A1 | 30 April 2015 |
| | | | | US | 8927719 | B2 | 06 January 2015 |
| | | | | US | 9089603 | B2 | 28 July 2015 |
| | | | | US | 9248203 | B2 | 02 February 2016 |
| | | | | US | 9408924 | B2 | 09 August 2016 |
| | | | | EP | 2630196 | A1 | 28 August 2013 |
| | | | | EP | 2630196 | B1 | 06 September 2017 |
| CN | 105813648 | A | 27 July 2016 | EP | 3046572 | A1 | 27 July 2016 |
| | | | | EP | 3046572 | A4 | 28 June 2017 |
| | | | | JP | 2020019779 | A | 06 February 2020 |
| | | | | JP | 6994011 | B2 | 03 February 2022 |
| | | | | IL | 244665 | A0 | 21 April 2016 |
| | | | | IL | 244665 | B | 31 March 2019 |
| | | | | AU | 2021202814 | A1 | 27 May 2021 |
| | | | | AU | 2021202814 | B2 | 02 February 2023 |
| | | | | AU | 2020200116 | A1 | 30 January 2020 |
| | | | | AU | 2020200116 | B2 | 25 February 2021 |
| | | | | CA | 2924521 | A1 | 26 March 2015 |
| | | | | JP | 2016534147 | A | 04 November 2016 |
| | | | | JP | 6652923 | B2 | 26 February 2020 |
| | | | | US | 2016096869 | A1 | 07 April 2016 |
| | | | | WO | 2015042202 | A1 | 26 March 2015 |
| | | | | AU | 2023202389 | A1 | 06 July 2023 |
| | | | | US | 2020188536 | A1 | 18 June 2020 |
| | | | | BR | 112016005802 | A2 | 12 September 2017 |
| | | | | AU | 2014323563 | A1 | 14 April 2016 |
| | | | | AU | 2014323563 | B2 | 10 October 2019 |
| | | | | AU | 2014323563 | B9 | 24 October 2019 |
| JP | 2004219649 | A | 05 August 2004 | JP | 4085005 | B2 | 30 April 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T. W. GREENE** ; **G. M. WUTS**. Protective Groups in Organic Synthesis. Wiley, 1999
- **ROCHE et al.** A Virtual Screening Method for Prediction of the hERG Potassium Channel Liability of Compound Libraries. *ChemBioChem*, 2002, vol. 3, 455-459
- **GLENN E. KIRSCH et al.** Variability in the measurement of hERG potassium channel inhibition: effects of temperature and stimulus patter. *Journal of Pharmacological and Toxicological Methods*, 2004, vol. 50, 93-101
- **ROGER MARRANNES et al.** Computer programs to facilitate the estimation of time-dependent drug effects on ion channels. *Computer Methods and Programs in Biomedicine*, 2004, vol. 74, 167-181